(19) Europäisches Patentamt
European Patent Office
Office européen des brevets

(11) EP 1 529 110 B1

(12) **EUROPEAN PATENT SPECIFICATION**

(45) Date of publication and mention of the grant of the patent:
**05.10.2011 Bulletin 2011/40**

(21) Application number: **03787738.8**

(22) Date of filing: **01.08.2003**

(51) Int Cl.:
***C12N 15/63*** *(2006.01)* ***C12N 15/02*** *(2006.01)*
***C12Q 1/68*** *(2006.01)*

(86) International application number:
**PCT/DK2003/000521**

(87) International publication number:
**WO 2004/016791 (26.02.2004 Gazette 2004/09)**

# (54) METHODS OF MIXING LARGE NUMBERS OF HETEROLOGOUS GENES

VERFAHREN ZUR MISCHUNG VON VIELEN HETEROLOGEN GENEN

PROCEDES POUR MELANGER DE GRANDS NOMBRES DE GENES HETEROLOGUES

(84) Designated Contracting States:
**AT BE BG CH CY CZ DE DK EE ES FI FR GB GR HU IE IT LI LU MC NL PT RO SE SI SK TR**

(30) Priority: **01.08.2002 DK 200201173**
**23.05.2003 DK 200300787**

(43) Date of publication of application:
**11.05.2005 Bulletin 2005/19**

(73) Proprietor: **Evolva Ltd.**
**4153 Reinach (CH)**

(72) Inventors:
- **SORENSEN, Alexandra, M.P., Santana**
  **DK-2840 Holte (DK)**
- **NIELSEN, Soren, V.S.**
  **DK-3450 Allerod (DK)**
- **GOLDSMITH, Neil**
  **Oxford, Oxfordshire OX4 1NX (GB)**
- **NIELSEN, Curt, Aimé, Friis**
  **DK-5250 Odense SV (DK)**

(74) Representative: **Høiberg A/S**
**St. Kongensgade 59 A**
**1264 Copenhagen K (DK)**

(56) References cited:

| | |
|---|---|
| **WO-A-00/04190** | **WO-A-00/52180** |
| **WO-A-01/73000** | **WO-A-97/35966** |
| **WO-A-98/17811** | **WO-A-98/31837** |
| **WO-A-02/059290** | **WO-A-02/059296** |
| **WO-A1-96/34112** | **WO-A1-03/062419** |
| **WO-A2-02/059297** | **WO-A2-03/106639** |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

SEQUENCE LISTING

<110> Evolva Biotech AS
Goldsmith, Neil
Sorensen, Alexandra M. P.
Nielsen, Søren V.S.
Curt Aimé Friis Nielsen

<120> Methods of mixing large numbers of heterologous genes

<130> P 669 PC00

<160> 5

<170> PatentIn version 3.1

<210> 1
<211> 3417
<212> DNA
<213> Artificial sequence

<220>
<223> Synthetic
<220>
<221> misc_feature
<222> (1902)..(2759)
<223> Ampicillin resistance gene

<220>
<221> rep_origin
<222> (959) .. (1899)
<223> ColE1

<220>
<221> misc_feature
<222> (2891)..(3347)
<223> f1-phage origin of replication

<220>

<221> terminator
<222> (495)..(823)
<223> ADH1

<220>
<221> promoter
<222> (49).. (437)
<223> Met25 promoter

<400> 1

```
ctgatttgcc cgggcagttc aggctcatca ggcgcgccat gcagggattc ttcggatgca     60
agggttcgaa tcccttagct ctcattattt tttgcttttt ctcttgaggt cacatgatcg    120
caaaatggca aatggcacgt gaagctgtcg atattgggga actgtggtgg ttggcaaatg    180
actaattaag ttagtcaagg cgccatcctc atgaaaactg tgtaacataa taaccgaagt    240
gtcgaaaagg tggcaccttg tccaattgaa cacgctcgat gaaaaaaata agatatatat    300
aaggttaagt aaagcgtctg ttagaaagga agtttttcct ttttcttgct ctcttgtctt    360
ttcatctact atttccttcg tgtaatacag ggtcgtcaga tacatagata caattctatt    420
acccccatcc atacaagctt ggcgccgaat tcgtcgaccc ggggatccgc ggccgcaggc    480
ctaaattgat ctagagcttt ggacttcttc gccagaggtt tggtcaagtc tccaatcaag    540
gttgtcggct tgtctacctt gccagaaatt tacgaaaaga tggaaaaggg tcaaatcgtt    600
```

```
ggtagatacg ttgttgacac ttctaaataa gcgaatttct tatgatttat gatttttatt    660
attaaataag ttataaaaaa aataagtgta tacaaatttt aaagtgactc ttaggtttta    720
aaacgaaaat tcttgttctt gagtaactct ttcctgtagg tcaggttgct ttctcaggta    780
tagcatgagg tcgctcttat tgaccacacc tctaccggca tgcccatggg ttaactgatc    840
aatgcatcct gcatggcgcg cctgatgagc ctgaactgcc cgggcaaatc agctggacgt    900
ctgcctgcat taatgaatcg gccaacgcgc ggggagaggc ggtttgcgta ttgggcgctc    960
ttccgcttcc tcgctcactg actcgctgcg ctcggtcgtt cggctgcggc gagcggtatc   1020
agctcactca aaggcggtaa tacggttatc cacagaatca ggggataacg caggaaagaa   1080
catgtgagca aaaggccagc aaaaggccag gaaccgtaaa aaggccgcgt tgctggcgtt   1140
tttccatagg ctccgccccc ctgacgagca tcacaaaaat cgacgctcaa gtcagaggtg   1200
gcgaaacccg acaggactat aaagatacca ggcgtttccc cctggaagct ccctcgtgcg   1260
ctctcctgtt ccgaccctgc cgcttaccgg atacctgtcc gcctttctcc cttcgggaag   1320
cgtggcgctt tctcatagct cacgctgtag gtatctcagt tcggtgtagg tcgttcgctc   1380
caagctgggc tgtgtgcacg aaccccccgt tcagcccgac cgctgcgcct tatccggtaa   1440
ctatcgtctt gagtccaacc cggtaagaca cgacttatcg ccactggcag cagccactgg   1500
taacaggatt agcagagcga ggtatgtagg cggtgctaca gagttcttga agtggtggcc   1560
taactacggc tacactagaa ggacagtatt tggtatctgc gctctgctga agccagttac   1620
cttcggaaaa agagttggta gctcttgatc cggcaaacaa accaccgctg gtagcggtgg   1680
tttttttgtt tgcaagcagc agattacgcg cagaaaaaaa ggatctcaag aagatccttt   1740
gatcttttct acggggtctg acgctcagtg gaacgaaaac tcacgttaag ggattttggt   1800
catgagatta tcaaaaagga tcttcaccta gatcctttta aattaaaaat gaagttttaa   1860
atcaatctaa agtatatatg agtaaacttg gtctgacagt taccaatgct taatcagtga   1920
ggcacctatc tcagcgatct gtctatttcg ttcatccata gttgcctgac tccccgtcgt   1980
gtagataact acgatacggg agggcttacc atctggcccc agtgctgcaa tgataccgcg   2040
agacccacgc tcaccggctc cagatttatc agcaataaac cagccagccg gaagggccga   2100
gcgcagaagt ggtcctgcaa ctttatccgc ctccatccag tctattaatt gttgccggga   2160
agctagagta agtagttcgc cagttaatag tttgcgcaac gttgttgcca ttgctacagg   2220
catcgtggtg tcacgctcgt cgtttggtat ggcttcattc agctccggtt cccaacgatc   2280
aaggcgagtt acatgatccc ccatgttgtg caaaaaagcg gttagctcct tcggtcctcc   2340
gatcgttgtc agaagtaagt tggccgcagt gttatcactc atggttatgg cagcactgca   2400
```

```
taattctctt actgtcatgc catccgtaag atgcttttct gtgactggtg agtactcaac   2460
caagtcattc tgagaatagt gtatgcggcg accgagttgc tcttgcccgg cgtcaatacg   2520
ggataatacc gcgccacata gcagaacttt aaaagtgctc atcattggaa aacgttcttc   2580
ggggcgaaaa ctctcaagga tcttaccgct gttgagatcc agttcgatgt aacccactcg   2640
tgcacccaac tgatcttcag catcttttac tttcaccagc gtttctgggt gagcaaaaac   2700
aggaaggcaa aatgccgcaa aaaagggaat aagggcgaca cggaaatgtt gaatactcat   2760
actcttcctt tttcaatatt attgaagcat ttatcagggt tattgtctca tgagcggata   2820
catatttgaa tgtatttaga aaaataaaca aataggggtt ccgcgcacat ttccccgaaa   2880
agtgccacct gacgcgccct gtagcggcgc attaagcgcg gcgggtgtgg tggttacgcg   2940
cagcgtgacc gctacacttg ccagcgccct agcgcccgct cctttcgctt tcttcccttc   3000
ctttctcgcc acgttcgccg gctttccccg tcaagctcta aatcgggggc tccctttagg   3060
gttccgattt agtgctttac ggcacctcga ccccaaaaaa cttgattagg gtgatggttc   3120
acgtagtggg ccatcgccct gatagacggt ttttcgccct ttgacgttgg agtccacgtt   3180
ctttaatagt ggactcttgt tccaaactgg aacaacactc aaccctatct cggtctattc   3240
ttttgattta taagggattt tgccgatttc ggcctattgg ttaaaaaatg agctgattta   3300
acaaaaattt aacgcgaatt ttaacaaaat attaacgctt acaatttcca ttcgccattc   3360
aggctgcgca actgttggga agggcgatcg gtgcgggcct cttcgctatt acgccag      3417
```

<210> 2
<211> 3501
<212> DNA
<213> Artificial sequence

<220>
<223> Synthetic
<220>
<221> misc_feature
<222> (1986) .. (2843)
<223> Ampicillin resistance gene

<220>
<221> rep_origin
<222> (1043)..(1983)
<223> ColE1

<220>
<221> misc_feature
<222> (2975)..(3431)
<223> f1-phage origin of replication

<220>

<221> terminator
<222> (579)..(907)
<223> ADH1

<220>
<221> promoter
<222> (49)..(519)
<223> Cup1 promoter

<400> 2

```
ctgatttgcc cgggcagttc aggctcatca ggcgcgccat gcagggataa gccgatccca      60
ttaccgacat ttgggcgcta tacgtgcata tgttcatgta tgtatctgta tttaaaacac     120
ttttgtatta tttttcctca tatatgtgta taggtttata cggatgattt aattattact     180
tcaccaccct ttatttcagg ctgatatctt agccttgtta ctagttagaa aaagacattt     240
ttgctgtcag tcactgtcaa gagattcttt tgctggcatt tcttctagaa gcaaaaagag     300
cgatgcgtct ttccgctga accgttccag caaaaaagac taccaacgca atatggattg     360
tcagaatcat ataaaagaga agcaaataac tccttgtctt gtatcaattg cattataata     420
tcttcttgtt agtgcaatat catatagaag tcatcgaaat agatattaag aaaaacaaac     480
```

tgtacaatca atcaatcaat catcacataa aatgttcaaa gcttggcgcc gaattcgtcg 540
acccggggat ccgcggccgc aggcctaaat tgatctagag ctttggactt cttcgccaga 600
ggtttggtca agtctccaat caaggttgtc ggcttgtcta ccttgccaga aatttacgaa 660
aagatggaaa agggtcaaat cgttggtaga tacgttgttg acacttctaa ataagcgaat 720
ttcttatgat ttatgatttt tattattaaa taagttataa aaaaaataag tgtatacaaa 780
ttttaaagtg actcttaggt tttaaaacga aaattcttgt tcttgagtaa ctctttcctg 840
taggtcaggt tgctttctca ggtatagcat gaggtcgctc ttattgacca cacctctacc 900
ggcatgccca tgggttaact gatcaatgca tcctgcatgg cgcgcctgat gagcctgaac 960
tgcccgggca aatcagctgg acgtctgcct gcattaatga atcggccaac gcgcggggag 1020
aggcggtttg cgtattgggc gctcttccgc ttcctcgctc actgactcgc tgcgctcggt 1080
cgttcggctg cggcgagcgg tatcagctca ctcaaaggcg gtaatacggt tatccacaga 1140
atcaggggat aacgcaggaa agaacatgtg agcaaaaggc cagcaaaagg ccaggaaccg 1200
taaaaaggcc gcgttgctgg cgtttttcca taggctccgc ccccctgacg agcatcacaa 1260
aaatcgacgc tcaagtcaga ggtggcgaaa cccgacagga ctataaagat accaggcgtt 1320
tccccctgga agctccctcg tgcgctctcc tgttccgacc ctgccgctta ccggatacct 1380
gtccgccttt ctcccttcgg gaagcgtggc gctttctcat agctcacgct gtaggtatct 1440
cagttcggtg taggtcgttc gctccaagct gggctgtgtg cacgaacccc ccgttcagcc 1500
cgaccgctgc gccttatccg gtaactatcg tcttgagtcc aacccggtaa gacacgactt 1560
atcgccactg gcagcagcca ctggtaacag gattagcaga gcgaggtatg taggcggtgc 1620
tacagagttc ttgaagtggt ggcctaacta cggctacact agaaggacag tatttggtat 1680
ctgcgctctg ctgaagccag ttaccttcgg aaaaagagtt ggtagctctt gatccggcaa 1740
acaaaccacc gctggtagcg gtggtttttt tgtttgcaag cagcagatta cgcgcagaaa 1800
aaaaggatct caagaagatc ctttgatctt ttctacgggg tctgacgctc agtggaacga 1860
aaactcacgt taagggattt tggtcatgag attatcaaaa aggatcttca cctagatcct 1920
tttaaattaa aaatgaagtt ttaaatcaat ctaaagtata tatgagtaaa cttggtctga 1980
cagttaccaa tgcttaatca gtgaggcacc tatctcagcg atctgtctat ttcgttcatc 2040
catagttgcc tgactccccg tcgtgtagat aactacgata cgggagggct taccatctgg 2100
ccccagtgct gcaatgatac cgcgagaccc acgctcaccg gctccagatt tatcagcaat 2160
aaaccagcca gccggaaggg ccgagcgcag aagtggtcct gcaactttat ccgcctccat 2220
ccagtctatt aattgttgcc gggaagctag agtaagtagt tcgccagtta atagtttgcg 2280

caacgttgtt gccattgcta caggcatcgt ggtgtcacgc tcgtcgtttg gtatggcttc 2340
attcagctcc ggttcccaac gatcaaggcg agttacatga tcccccatgt tgtgcaaaaa 2400
agcggttagc tccttcggtc ctccgatcgt tgtcagaagt aagttggccg cagtgttatc 2460
actcatggtt atggcagcac tgcataattc tcttactgtc atgccatccg taagatgctt 2520
ttctgtgact ggtgagtact caaccaagtc attctgagaa tagtgtatgc ggcgaccgag 2580
ttgctcttgc ccggcgtcaa tacgggataa taccgcgcca catagcagaa ctttaaaagt 2640
gctcatcatt ggaaaacgtt cttcggggcg aaaactctca aggatcttac cgctgttgag 2700
atccagttcg atgtaaccca ctcgtgcacc caactgatct tcagcatctt ttactttcac 2760
cagcgtttct gggtgagcaa aaacaggaag gcaaaatgcc gcaaaaaagg gaataagggc 2820
gacacggaaa tgttgaatac tcatactctt cctttttcaa tattattgaa gcatttatca 2880
gggttattgt ctcatgagcg gatacatatt tgaatgtatt tagaaaaata aacaaatagg 2940
ggttccgcgc acatttcccc gaaaagtgcc acctgacgcg ccctgtagcg gcgcattaag 3000
cgcggcgggt gtggtggtta cgcgcagcgt gaccgctaca cttgccagcg ccctagcgcc 3060
cgctcctttc gctttcttcc cttcctttct cgccacgttc gccggctttc cccgtcaagc 3120
tctaaatcgg gggctccctt tagggttccg atttagtgct ttacggcacc tcgaccccaa 3180
aaaacttgat tagggtgatg gttcacgtag tgggccatcg ccctgataga cggtttttcg 3240
ccctttgacg ttggagtcca cgttctttaa tagtggactc ttgttccaaa ctggaacaac 3300
actcaaccct atctcggtct attcttttga tttataaggg attttgccga tttcggccta 3360
ttggttaaaa aatgagctga tttaacaaaa atttaacgcg aattttaaca aaatattaac 3420
gcttacaatt tccattcgcc attcaggctg cgcaactgtt gggaagggcg atcggtgcgg 3480
gcctcttcgc tattacgcca g 3501

<210> 3
<211> 4188
<212> DNA
<213> Artificial sequence

<220>
<223> Synthetic
<220>
<221> misc_feature
<222> (2673) .. (3530)
<223> Ampicillin resistance gene

<220>
<221> rep_origin
<222> (1730)..(2670)
<223> ColE1

<220>
<221> misc_feature
<222> (3662).. (4118)
<223> f1-phage origin of replication

<220>
<221> terminator
<222> (1027)..(1355)
<223> ADH1

<220>
<221> promoter
<222> (582)..(969)
<223> Met25 promoter

<220>
<221> misc_feature
<222> (1365)..(1603)
<223> ARS1 (autonomous replicating sequence) for Yeast replication

<220>
<221> misc_feature
<222> (49) .. (574)
<223> lambda spacer DNA (22428-22923)

<400> 3

```
ctgatttgcc cgggcagttc aggctcatca ggcgcgccat gcagggattc tggaaattgc     60
aacgaaggaa gaaacctcgt tgctggaagc ctggaagaag tatcgggtgt tgctgaaccg    120
tgttgataca tcaactgcac ctgatattga gtggcctgct gtccctgtta tggagtaatc    180
gttttgtgat atgccgcaga aacgttgtat gaaataacgt tctgcggtta gttagtatat    240
tgtaaagctg agtattggtt tatttggcga ttattatctt caggagaata atggaagttc    300
tatgactcaa ttgttcatag tgtttacatc accgccaatt gcttttaaga ctgaacgcat    360
gaaatatggt ttttcgtcat gttttgagtc tgctgttgat atttctaaag tcggtttttt    420
ttcttcgttt tctctaacta ttttccatga aatacatttt tgattattat ttgaatcaat    480
tccaattacc tgaagtcttt catctataat tggcattgta tgtattggtt tattggagta    540
gatgcttgct tttctgagcc atagctctga tatcagatct tcttcggatg caagggttcg    600
aatcccttag ctctcattat tttttgcttt ttctcttgag gtcacatgat cgcaaaatgg    660
caaatggcac gtgaagctgt cgatattggg gaactgtggt ggttggcaaa tgactaatta    720
agttagtcaa ggcgccatcc tcatgaaaac tgtgtaacat aataaccgaa gtgtcgaaaa    780
ggtggcacct tgtccaattg aacacgctcg atgaaaaaaa taagatatat ataaggttaa    840
gtaaagcgtc tgttagaaag gaagtttttc ctttttcttg ctctcttgtc ttttcatcta    900
ctatttcctt cgtgtaatac agggtcgtca gatacataga tacaattcta ttacccccat    960
ccatacaagc ttggcgccga attcgtcgac ccggggatcc gcggccgcag gcctaaattg   1020
atctagagct ttggacttct tcgccagagg tttggtcaag tctccaatca aggttgtcgg   1080
cttgtctacc ttgccagaaa tttacgaaaa gatggaaaag ggtcaaatcg ttggtagata   1140
cgttgttgac acttctaaat aagcgaattt cttatgattt atgattttta ttattaaata   1200
agttataaaa aaaataagtg tatacaaatt ttaaagtgac tcttaggttt taaaacgaaa   1260
attcttgttc ttgagtaact ctttcctgta ggtcaggttg ctttctcagg tatagcatga   1320
ggtcgctctt attgaccaca cctctaccgg catgcccatg ggttcttttg aaaagcaagc   1380
```

```
ataaaagatc taaacataaa atctgtaaaa taacaagatg taaagataat gctaaatcat   1440
ttggcttttt gattgattgt acaggaaaat atacatcgca ggggggttgac ttttaccatt   1500
tcaccgcaat ggaatcaaac ttgttgaaga gaatgttcac aggcgcatac gctacaatga   1560
cccgattctt gctagccttt tctcggtctt gcaaacaacc gccaactgat caatgcatcc   1620
tgcatggcgc gcctgatgag cctgaactgc ccgggcaaat cagctggacg tctgcctgca   1680
ttaatgaatc ggccaacgcg cggggagagg cggtttgcgt attgggcgct cttccgcttc   1740
ctcgctcact gactcgctgc gctcggtcgt tcggctgcgg cgagcggtat cagctcactc   1800
aaaggcggta atacggttat ccacagaatc aggggataac gcaggaaaga acatgtgagc   1860
aaaaggccag caaaaggcca ggaaccgtaa aaaggccgcg ttgctggcgt ttttccatag   1920
gctccgcccc cctgacgagc atcacaaaaa tcgacgctca agtcagaggt ggcgaaaccc   1980
gacaggacta taaagatacc aggcgtttcc ccctggaagc tccctcgtgc gctctcctgt   2040
tccgaccctg ccgcttaccg gatacctgtc cgcctttctc ccttcgggaa gcgtggcgct   2100
ttctcatagc tcacgctgta ggtatctcag ttcggtgtag gtcgttcgct ccaagctggg   2160
ctgtgtgcac gaacccccg ttcagcccga ccgctgcgcc ttatccggta actatcgtct   2220
tgagtccaac ccggtaagac acgacttatc gccactggca gcagccactg gtaacaggat   2280
tagcagagcg aggtatgtag gcggtgctac agagttcttg aagtggtggc ctaactacgg   2340
ctacactaga aggacagtat ttggtatctg cgctctgctg aagccagtta ccttcggaaa   2400
aagagttggt agctcttgat ccggcaaaca aaccaccgct ggtagcggtg gtttttttgt   2460
ttgcaagcag cagattacgc gcagaaaaaa aggatctcaa gaagatcctt tgatcttttc   2520
tacggggtct gacgctcagt ggaacgaaaa ctcacgttaa gggattttgg tcatgagatt   2580
atcaaaaagg atcttcacct agatcctttt aaattaaaaa tgaagtttta aatcaatcta   2640
aagtatatat gagtaaactt ggtctgacag ttaccaatgc ttaatcagtg aggcacctat   2700
ctcagcgatc tgtctatttc gttcatccat agttgcctga ctccccgtcg tgtagataac   2760
tacgatacgg gagggcttac catctggccc cagtgctgca atgataccgc gagacccacg   2820
ctcaccggct ccagatttat cagcaataaa ccagccagcc ggaagggccg agcgcagaag   2880
tggtcctgca actttatccg cctccatcca gtctattaat tgttgccggg aagctagagt   2940
aagtagttcg ccagttaata gtttgcgcaa cgttgttgcc attgctacag gcatcgtggt   3000
gtcacgctcg tcgtttggta tggcttcatt cagctccggt tcccaacgat caaggcgagt   3060
tacatgatcc cccatgttgt gcaaaaaagc ggttagctcc ttcggtcctc cgatcgttgt   3120
cagaagtaag ttggccgcag tgttatcact catggttatg gcagcactgc ataattctct   3180
```

```
tactgtcatg ccatccgtaa gatgcttttc tgtgactggt gagtactcaa ccaagtcatt   3240
ctgagaatag tgtatgcggc gaccgagttg ctcttgcccg gcgtcaatac gggataatac   3300
cgcgccacat agcagaactt taaaagtgct catcattgga aaacgttctt cggggcgaaa   3360
actctcaagg atcttaccgc tgttgagatc cagttcgatg taacccactc gtgcacccaa   3420
ctgatcttca gcatctttta ctttcaccag cgtttctggg tgagcaaaaa caggaaggca   3480
aaatgccgca aaaaagggaa taagggcgac acggaaatgt tgaatactca tactcttcct   3540
ttttcaatat tattgaagca tttatcaggg ttattgtctc atgagcggat acatatttga   3600
atgtatttag aaaaataaac aaataggggt tccgcgcaca tttccccgaa aagtgccacc   3660
tgacgcgccc tgtagcggcg cattaagcgc ggcgggtgtg gtggttacgc gcagcgtgac   3720
cgctacactt gccagcgccc tagcgcccgc tcctttcgct ttcttccctt cctttctcgc   3780
cacgttcgcc ggctttcccc gtcaagctct aaatcggggg ctccctttag ggttccgatt   3840
tagtgcttta cggcacctcg accccaaaaa acttgattag ggtgatggtt cacgtagtgg   3900
gccatcgccc tgatagacgg tttttcgccc tttgacgttg gagtccacgt tctttaatag   3960
tggactcttg ttccaaactg gaacaacact caaccctatc tcggtctatt cttttgattt   4020
ataagggatt ttgccgattt cggcctattg gttaaaaaat gagctgattt aacaaaaatt   4080
taacgcgaat tttaacaaaa tattaacgct tacaatttcc attcgccatt caggctgcgc   4140
aactgttggg aagggcgatc ggtgcgggcc tcttcgctat tacgccag                4188
```

<210> 4
<211> 11466
<212> DNA
<213> Artificial sequence

<220>
<223> Synthetic
<220>
<221> misc_feature
<222> (3560)..(4247)
<223> Tetrahymena thermophila macronuclear telomere

<220>
<221> misc_feature
<222> (6024)..(6711)
<223> Tetrahymena thermophila macronuclear telomere

<220>
<221> misc_feature
<222> (9644)..(10388)
<223> Autonomous replicating sequence

<220>

<221> misc_feature
<222> (10488)..(11465)
<223> Centromere IV

<220>
<221> rep_origin
<222> (7198)..(7198)
<223> Origin of replication, PMB1

<220>
<221> misc_feature
<222> (1962) .. (2765)
<223> Ultra3, orotidine-5'-phosphate decarboxylase coding sequence

<220>
<221> misc_feature
<222> (4893)..(5552)
<223> HIS3, imidazoleglycerolphosphate dehydratase, coding sequence

<220>
<221> misc_feature
<222> (7956)..(8816)
<223> AP(R), beta-lactamase, ampR ampicillin resistance, coding sequenc e

<220>
<221> misc_feature
<222> (9129)..(9803)
<223> TRP1, phosphoribosylanthranilate isomerase, coding sequence

<400> 4

```
ttctcatgtt tgacagctta tcatcgataa gctttaatgc ggtagtttat cacagttaaa     60
ttgctaacgc agtcaggcac cgtgtatgaa atctaacaat gcgctcatcg tcatcctcgg    120
caccgtcacc ctggatgctg taggcatagg cttggttatg ccggtactgc cgggcctctt    180
gcgggatatc gtccattccg acagcatcgc cagtcactat ggcgtgctgc tagcgctata    240
tgcgttgatg caatttctat gcgcacccgt tctcggagca ctgtccgacc gctttggccg    300
ccgcccagtc ctgctcgctt cgctacttgg agccactatc gactacgcga tcatggcgac    360
cacacccgtc ctgtggatca attcccttta gtataaattt cactctgaac catcttggaa    420
ggaccggtaa ttatttcaaa tctctttttc aattgtatat gtgttatgtt atgtagtata    480
ctctttcttc aacaattaaa tactctcggt agccaagttg gtttaaggcg caagacttta    540
atttatcact acggaattgg cgcgccaatt ccgtaatctt gagatcgggc gttcgatcgc    600
cccgggagat ttttttgttt tttatgtctt ccattcactt cccagacttg caagttgaaa    660
tatttctttc aagggaattg atcctctacg ccggacgcat cgtggccggc atcaccggcg    720
ccacaggtgc ggttgctggc gcctatatcg ccgacatcac cgatggggaa gatcgggctc    780
gccacttcgg gctcatgagc gcttgtttcg gcgtgggtat ggtggcaggc cccgtggccg    840
ggggactgtt gggcgccatc tccttgcatg caccattcct tgcggcggcg gtgctcaacg    900
gcctcaacct actactgggc tgcttcctaa tgcaggagtc gcataaggga gagcgtcgac    960
cgatgccctt gagagccttc aacccagtca gctccttccg gtgggcgcgg ggcatgacta   1020
```

tcgtcgccgc acttatgact gtcttcttta tcatgcaact cgtaggacag gtgccggcag 1080
cgctctgggt cattttcggc gaggaccgct ttcgctggag cgcgacgatg atcggcctgt 1140
cgcttgcggt attcggaatc ttgcacgccc tcgctcaagc cttcgtcact ggtcccgcca 1200
ccaaacgttt cggcgagaag caggccatta tcgccggcat ggcggccgac gcgctgggct 1260
acgtcttgct ggcgttcgcg acgcgaggct ggatggcctt ccccattatg attcttctcg 1320
cttccggcgg catcgggatg cccgcgttgc aggccatgct gtccaggcag gtagatgacg 1380
accatcaggg acagcttcaa ggatcgctcg cggctcttac cagcctaact tcgatcactg 1440
gaccgctgat cgtcacggcg atttatgccg cctcggcgag cacatggaac gggttggcat 1500
ggattgtagg cgccgcccta taccttgtct gcctccccgc gttgcgtcgc ggtgcatgga 1560
gccgggccac ctcgacctga atggaagccg gcggcacctc gctaacggat tcaccactcc 1620
aagaattgga gccaatcaat tcttgcggag aactgtgaat gcgcaaacca acccttggca 1680
gaacatatcc atcgcgtccg ccatctccag cagccgcacg cggcgcatcc ccccccccct 1740
ttcaattcaa ttcatcattt tttttttatt cttttttttg atttcggttt ctttgaaatt 1800
tttttgattc ggtaatctcc gaacagaagg aagaacgaag gaaggagcac agacttagat 1860
tggtatatat acgcatatgt agtgttgaag aaacatgaaa ttgcccagta ttcttaaccc 1920
aactgcacag aacaaaaacc tgcaggaaac gaagataaat catgtcgaaa gctacatata 1980
aggaacgtgc tgctactcat cctagtcctg ttgctgccaa gctatttaat atcatgcacg 2040
aaaagcaaac aaacttgtgt gcttcattgg atgttcgtac caccaaggaa ttactggagt 2100
tagttgaagc attaggtccc aaaatttgtt tactaaaaac acatgtggat atcttgactg 2160
atttttccat ggagggcaca gttaagccgc taaaggcatt atccgccaag tacaattttt 2220
tactcttcga agacagaaaa tttgctgaca ttggtaatac agtcaaattg cagtactctg 2280
cgggtgtata cagaatagca gaatgggcag acattacgaa tgcacacggt gtggtgggcc 2340
caggtattgt tagcggtttg aagcaggcgg cagaagaagt aacaaaggaa cctagaggcc 2400
ttttgatgtt agcagaattg tcatgcaagg gctccctatc tactggagaa tatactaagg 2460
gtactgttga cattgcgaag agcgacaaag attttgttat cggctttatt gctcaaagag 2520
acatgggtgg aagagatgaa ggttacgatt ggttgattat gacacccggt gtgggtttag 2580
atgacaaggg agacgcattg ggtcaacagt atagaaccgt ggatgatgtg gtctctacag 2640
gatctgacat tattattgtt ggaagaggac tatttgcaaa gggaagggat gctaaggtag 2700
agggtgaacg ttacagaaaa gcaggctggg aagcatattt gagaagatgc ggccagcaaa 2760
actaaaaaac tgtattataa gtaaatgcat gtatactaaa ctcacaaatt agagcttcaa 2820

```
tttaattata tcagttatta ctcgggcgta atgattttta taatgacgaa aaaaaaaaaa    2880
ttggaaagaa aagggggggg gggcagcgtt gggtcctggc cacgggtgcg catgatcgtg    2940
ctcctgtcgt tgaggacccg gctaggctgg cggggttgcc ttactggtta gcagaatgaa    3000
tcaccgatac gcgagcgaac gtgaagcgac tgctgctgca aaacgtctgc gacctgagca    3060
acaacatgaa tggtcttcgg tttccgtgtt tcgtaaagtc tggaaacgcg gaagtcagcg    3120
ccctgcacca ttatgttccg gatctgcatc gcaggatgct gctggctacc ctgtggaaca    3180
cctacatctg tattaacgaa gcgctggcat tgaccctgag tgattttct ctggtcccgc     3240
cgcatccata ccgccagttg tttaccctca caacgttcca gtaaccgggc atgttcatca    3300
tcagtaaccc gtatcgtgag catcctctct cgtttcatcg gtatcattac ccccatgaac    3360
agaaattccc ccttacacgg aggcatcaag tgaccaaaca ggaaaaaacc gcccttaaca    3420
tggcccgctt tatcagaagc cagacattaa cgcttctgga gaaactcaac gagctggacg    3480
cggatgaaca ggcagacatc tgtgaatcgc ttcacgacca cgctgatgag ctttaccgca    3540
gccctcgagg gataagcttc atttttagat aaaatttatt aatcatcatt aatttcttga    3600
aaaacatttt atttattgat cttttataac aaaaaaccct tctaaaagtt tatttttgaa    3660
tgaaaaactt ataaaaattt atgaaaacta caaaaaataa aatttttaat taaaataatt    3720
ttgataagaa cttcaatctt tgactagcta gcttagtcat tttgagatt taattaatat     3780
tttatgttta ttcatatata aactattcaa aatattatag aatttaaaca ttttaacatc    3840
ttaatcattc ataaataact aaaaatcaaa gtattacatc aataaataac ttttactcaa    3900
tgtcaaagaa ttattggggt tggggttggg gttggggttg gggttggggt tggggttggg    3960
gttggggttg gggttggggt tggggttggg gttggggttg gggttggggt tggggttggg    4020
gttggggttg gggttggggt tggggttggg gttggggttg gggttggggt tggggttggg    4080
gttggggttg gggttggggt tggggttggg gttggggttg gggttggggt tggggttggg    4140
gttggggttg gggttggggt tggggttggg gttggggttg gggtgggaaa acagcattca    4200
ggtattagaa gaatatcctg attcaggtga aaatattgtt gatgcgcggg atcctcgggg    4260
acaccaaata tggcgatctc ggccttttcg tttcttggag ctgggacatg tttgccatcg    4320
atccatctac caccagaacg gccgttagat ctgctgccac cgttgtttcc accgaagaaa    4380
ccaccgttgc cgtaaccacc acgacggttg ttgctaaaga agctgccacc gccacggcca    4440
ccgttgtagc cgccgttgtt gttattgtag ttgctcatgt tatttctggc acttcttggt    4500
tttcctctta agtgaggagg aacataacca ttctcgttgt tgtcgttgat gcttaaattt    4560
```

tgcacttgtt cgctcagttc agccataata tgaaatgctt ttcttgttgt tcttacggaa 4620
taccacttgc cacctatcac cacaactaac tttttcccgt tcctccatct cttttatatt 4680
ttttttctcg atcgagttca agagaaaaaa aaagaaaaag caaaaagaaa aaaggaaagc 4740
gcgcctcgtt cagaatgaca cgtatagaat gatgcattac cttgtcatct tcagtatcat 4800
actgttcgta tacatactta ctgacattca taggtataca tatatacaca tgtatatata 4860
tcgtatgctg cagctttaaa taatcggtgt cactacataa gaacaccttt ggtggaggga 4920
acatcgttgg taccattggg cgaggtggct tctcttatgg caaccgcaag agccttgaac 4980
gcactctcac tacggtgatg atcattcttg cctcgcagac aatcaacgtg gagggtaatt 5040
ctgctagcct ctgcaaagct ttcaagaaaa tgcgggatca tctcgcaaga gagatctcct 5100
actttctccc tttgcaaacc aagttcgaca actgcgtacg gcctgttcga aagatctacc 5160
accgctctgg aaagtgcctc atccaaaggc gcaaatcctg atccaaacct tttactccca 5220
cgcgccagta gggcctcttt aaaagcttga ccgagagcaa tcccgcagtc ttcagtggtg 5280
tgatggtcgt ctatgtgtaa gtcaccaatg cactcaacga ttagcgacca gccggaatgc 5340
ttggccagag catgtatcat atggtccaga aaccctatac ctgtgtggac gttaatcact 5400
tgcgattgtg tggcctgttc tgctactgct tctgcctctt tttctgggaa gatcgagtgc 5460
tctatcgcta ggggaccacc ctttaaagag atcgcaatct gaatcttggt ttcatttgta 5520
atacgcttta ctagggcttt ctgctctgtc atctttgcct tcgtttatct tgcctgctca 5580
ttttttagta tattcttcga agaaatcaca ttactttata taatgtataa ttcattatgt 5640
gataatgcca atcgctaaga aaaaaaaaga gtcatccgct aggtggaaaa aaaaaaatga 5700
aaatcattac cgaggcataa aaaaatatag agtgtactag aggaggccaa gagtaataga 5760
aaaagaaaat tgcgggaaag gactgtgtta tgacttccct gactaatgcc gtgttcaaac 5820
gatacctggc agtgactcct agcgctcacc aagctcttaa aacgagaatt aagaaaaagt 5880
cgtcatcttt cgataagttt ttcccacagc aaagcaatag tagaaaaaaa caatgggaaa 5940
cgttgaatga agacaaagcg tcgtggttta aaaggaaata cgctcacgta catgctaggg 6000
aacaggaccg tgcagcggat cccgcgcatc aacaatattt tcacctgaat caggatattc 6060
ttctaatacc tgaatgctgt tttcccaccc caaccccaac cccaacccca accccaaccc 6120
caaccccaac cccaacccca accccaaccc caaccccaac cccaacccca accccaaccc 6180
caaccccaac cccaacccca accccaaccc caaccccaac cccaacccca accccaaccc 6240
caaccccaac cccaacccca accccaaccc caaccccaac cccaacccca accccaaccc 6300
caaccccaac cccaacccca accccaaccc caaccccaac cccaacccca accccaataa 6360

ttctttgaca ttgagtaaaa gttatttatt gatgtaatac tttgattttt agttatttat 6420
gaatgattaa gatgttaaaa tgtttaaatt ctataatatt ttgaatagtt tatatatgaa 6480
taaacataaa atattaatta aatctcaaaa atgactaagc tagctagtca aagattgaag 6540
ttcttatcaa aattatttta attaaaaatt ttattttttg tagttttcat aaatttttat 6600
aagtttttca ttcaaaaata aacttttaga agggtttttt gttataaaag atcaataaat 6660
aaaatgtttt tcaagaaatt aatgatgatt aataaatttt atctaaaaat gaagcttatc 6720
cctcgagggc tgcctcgcgc gtttcggtga tgacggtgaa aacctctgac acatgcagct 6780
cccggagacg gtcacagctt gtctgtaagc ggatgccggg agcagacaag cccgtcaggg 6840
cgcgtcagcg ggtgttggcg ggtgtcgggg cgcagccatg acccagtcac gtagcgatag 6900
cggagtgtat actggcttaa ctatgcggca tcagagcaga ttgtactgag agtgcaccat 6960
atgcggtgtg aaataccgca cagatgcgta aggagaaaat accgcatcag gcgctcttcc 7020
gcttcctcgc tcactgactc gctgcgctcg gtcgttcggc tgcggcgagc ggtatcagct 7080
cactcaaagg cggtaatacg gttatccaca gaatcagggg ataacgcagg aaagaacatg 7140
tgagcaaaag gccagcaaaa ggccaggaac cgtaaaaagg ccgcgttgct ggcgtttttc 7200
cataggctcc gcccccctga cgagcatcac aaaaatcgac gctcaagtca gaggtggcga 7260
aacccgacag gactataaag ataccaggcg tttccccctg gaagctccct cgtgcgctct 7320
cctgttccga ccctgccgct taccggatac ctgtccgcct ttctcccttc gggaagcgtg 7380
gcgctttctc atagctcacg ctgtaggtat ctcagttcgg tgtaggtcgt tcgctccaag 7440
ctgggctgtg tgcacgaacc ccccgttcag cccgaccgct gcgccttatc cggtaactat 7500
cgtcttgagt ccaacccggt aagacacgac ttatcgccac tggcagcagc cactggtaac 7560
aggattagca gagcgaggta tgtaggcggt gctacagagt tcttgaagtg gtggcctaac 7620
tacggctaca ctagaaggac agtatttggt atctgcgctc tgctgaagcc agttaccttc 7680
ggaaaaagag ttggtagctc ttgatccggc aaacaaacca ccgctggtag cggtggtttt 7740
tttgtttgca agcagcagat tacgcgcaga aaaaaaggat ctcaagaaga tcctttgatc 7800
ttttctacgg ggtctgacgc tcagtggaac gaaaactcac gttaagggat tttggtcatg 7860
agattatcaa aaaggatctt cacctagatc cttttaaatt aaaaatgaag ttttaaatca 7920
atctaaagta tatatgagta aacttggtct gacagttacc aatgcttaat cagtgaggca 7980
cctatctcag cgatctgtct atttcgttca tccatagttg cctgactccc cgtcgtgtag 8040
ataactacga tacgggaggg cttaccatct ggccccagtg ctgcaatgat accgcgagac 8100

ccacgctcac cggctccaga tttatcagca ataaaccagc cagccggaag ggccgagcgc 8160
agaagtggtc ctgcaacttt atccgcctcc atccagtcta ttaattgttg ccgggaagct 8220
agagtaagta gttcgccagt taatagtttg cgcaacgttg ttgccattgc tgcaggcatc 8280
gtggtgtcac gctcgtcgtt tggtatggct tcattcagct ccggttccca acgatcaagg 8340
cgagttacat gatcccccat gttgtgcaaa aaagcggtta gctccttcgg tcctccgatc 8400
gttgtcagaa gtaagttggc cgcagtgtta tcactcatgg ttatggcagc actgcataat 8460
tctcttactg tcatgccatc cgtaagatgc ttttctgtga ctggtgagta ctcaaccaag 8520
tcattctgag aatagtgtat gcggcgaccg agttgctctt gcccggcgtc aacacgggat 8580
aataccgcgc cacatagcag aactttaaaa gtgctcatca ttggaaaacg ttcttcgggg 8640
cgaaaactct caaggatctt accgctgttg agatccagtt cgatgtaacc cactcgtgca 8700
cccaactgat cttcagcatc ttttactttc accagcgttt ctgggtgagc aaaaacagga 8760
aggcaaaatg ccgcaaaaaa gggaataagg gcgacacgga aatgttgaat actcatactc 8820
ttcctttttc aatattattg aagcatttat cagggttatt gtctcatgag cggatacata 8880
tttgaatgta tttagaaaaa taaacaaata ggggttccgc gcacatttcc ccgaaaagtg 8940
ccacctgacg tctaagaaac cattattatc atgacattaa cctataaaaa taggcgtatc 9000
acgaggccct ttcgtcttca agaattaatt cggtcgaaaa aagaaaagga gagggccaag 9060
agggagggca ttggtgacta ttgagcacgt gagtatacgt gattaagcac acaaaggcag 9120
cttggagtat gtctgttatt aatttcacag gtagttctgg tccattggtg aaagtttgcg 9180
gcttgcagag cacagaggcc gcagaatgtg ctctagattc cgatgctgac ttgctgggta 9240
ttatatgtgt gcccaataga aagagaacaa ttgacccggt tattgcaagg aaaatttcaa 9300
gtcttgtaaa agcatataaa aatagttcag gcactccgaa atacttggtt ggcgtgtttc 9360
gtaatcaacc taaggaggat gttttggctc tggtcaatga ttacggcatt gatatcgtcc 9420
aactgcatgg agatgagtcg tggcaagaat accaagagtt cctcggtttg ccagttatta 9480
aaagactcgt atttccaaaa gactgcaaca tactactcag tgcagcttca cagaaacctc 9540
attcgtttat tcccttgttt gattcagaag caggtgggac aggtgaactt ttggattgga 9600
actcgatttc tgactgggtt ggaaggcaag agagccccga aagcttacat tttatgttag 9660
ctggtggact gacgccagaa aatgttggtg atgcgcttag atttaaatggc gttattggtg 9720
ttgatgtaag cggaggtgtg gagacaaatg gtgtaaaaga ctctaacaaa atagcaaatt 9780
tcgtcaaaaa tgctaagaaa taggttatta ctgagtagta tttatttaag tattgtttgt 9840
gcacttgcct gcaggccttt tgaaaagcaa gcataaaaga tctaaacata aaatctgtaa 9900

aataacaaga tgtaaagata atgctaaatc atttggcttt ttgattgatt gtacaggaaa 9960
atatacatcg caggggggttg acttttacca tttcaccgca atggaatcaa acttgttgaa 10020
gagaatgttc acaggcgcat acgctacaat gacccgattc ttgctagcct tttctcggtc 10080
ttgcaaacaa ccgccggcag cttagtatat aaatacacat gtacatacct ctctccgtat 10140
cctcgtaatc atttcttgt atttatcgtc ttttcgctgt aaaaacttta tcacacttat 10200
ctcaaataca cttattaacc gcttttacta ttatcttcta cgctgacagt aatatcaaac 10260
agtgacacat attaaacaca gtggtttctt tgcataaaca ccatcagcct caagtcgtca 10320
agtaaagatt tcgtgttcat gcagatagat aacaatctat atgttgataa ttagcgttgc 10380
ctcatcaatg cgagatccgt ttaaccggac cctagtgcac ttaccccacg ttcggtccac 10440
tgtgtgccga acatgctcct tcactatttt aacatgtgga attaattcta aatcctcttt 10500
atatgatctg ccgatagata gttctaagtc attgaggttc atcaacaatt ggattttctg 10560
tttactcgac ttcaggtaaa tgaaatgaga tgatacttgc ttatctcata gttaactcta 10620
agaggtgata cttatttact gtaaaactgt gacgataaaa ccggaaggaa gaataagaaa 10680
actcgaactg atctataatg cctattttct gtaaagagtt taagctatga aagcctcggc 10740
atttggccg ctcctaggta gtgcttttt tccaaggaca aaacagtttc tttttcttga 10800
gcaggtttta tgtttcggta atcataaaca ataaataaat tatttcattt atgtttaaaa 10860
ataaaaaata aaaaagtatt ttaaattttt aaaaaagttg attataagca tgtgaccttt 10920
tgcaagcaat taaattttgc aatttgtgat tttaggcaaa agttacaatt tctggctcgt 10980
gtaatatatg tatgctaaag tgaactttta caaagtcgat atggacttag tcaaaagaaa 11040
ttttcttaaa aatatatagc actagccaat ttagcacttc tttatgagat atattataga 11100
ctttattaag ccagatttgt gtattatatg tatttacccg gcgaatcatg gacatacatt 11160
ctgaaatagg taatattctc tatggtgaga cagcatagat aacctaggat acaagttaaa 11220
agctagtact gttttgcagt aattttttc ttttttataa gaatgttacc acctaaataa 11280
gttataaagt caatagttaa gtttgatatt tgattgtaaa ataccgtaat atatttgcat 11340
gatcaaaagg ctcaatgttg actagccagc atgtcaacca ctatattgat caccgatata 11400
tggacttcca caccaactag taatatgaca ataaattcaa gatattcttc atgagaatgg 11460
cccaga 11466

<210> 5
<211> 4313
<212> DNA
<213> Artificial sequence

<220>
<223> Synthetic
<220>
<221> misc_feature
<222> (3787) .. (4243)
<223> f1-phage origin of replication

<220>
<221> misc_feature
<222> (2798)..(3655)
<223> Ampicillin resistance gene

<220>
<221> terminator
<222> (1100)..(1428)
<223>

<220>
<221> promoter
<222> (655)..(1042)
<223> Met25 promotor

<220>
<221> rep_origin
<222> (1855)..(2795)
<223> ColE1

<400> 5

ctgatttgcc cgggcagttc aggctcatca ggcgcgccat gcagggatcg gcgttttccg 60
gaactggaaa accgacatgt tgatttcctg aaacgggata tcatcaaagc catgaacaaa 120
gcagccgcgc tggatgaact gataccgggg ttgctgagtg aatatatcga acagtcaggt 180
taacaggctg cggcattttg tccgcgccgg gcttcgctca ctgttcaggc cggagccaca 240
gaccgccgtt gaatgggcgg atgctaatta ctatctcccg aaagaatccg cataccagga 300
agggcgctgg gaaacactgc cctttcagcg ggccatcatg aatgcgatgg gcagcgacta 360
catccgtgag gtgaatgtgg tgaagtctgc ccgtgtcggt tattccaaaa tgctgctggg 420
tgtttatgcc tactttatag agcataagca gcgcaacacc cttatctggt tgccgacgga 480
tggtgatgcc gagaacttta tgaaaaccca cgttgagccg actattcgtg atattccgtc 540
gctgctggcg ctggccccgt ggtatggcaa aaagcaccgg gataacacgc tcaccatgaa 600
gcgtttcact aatgggcgtg gcttctggtg cctgggcggt aaagcggaga tcttcttcgg 660
atgcaagggt tcgaatccct tagctctcat tattttttgc tttttctctt gaggtcacat 720
gatcgcaaaa tggcaaatgg cacgtgaagc tgtcgatatt ggggaactgt ggtggttggc 780
aaatgactaa ttaagttagt caaggcgcca tcctcatgaa aactgtgtaa cataataacc 840
gaagtgtcga aaaggtggca ccttgtccaa ttgaacacgc tcgatgaaaa aaataagata 900
tatataaggt taagtaaagc gtctgttaga aaggaagttt ttcctttttc ttgctctctt 960
gtcttttcat ctactatttc cttcgtgtaa tacagggtcg tcagatacat agatacaatt 1020
ctattacccc catccataca agcttggcgc cgaattcgtc gacccgggga tccgcggccg 1080
caggcctaaa ttgatctaga gctttggact tcttcgccag aggtttggtc aagtctccaa 1140
tcaaggttgt cggcttgtct accttgccag aaatttacga aaagatggaa aagggtcaaa 1200
tcgttggtag atacgttgtt gacacttcta aataagcgaa tttcttatga tttatgattt 1260
ttattattaa ataagttata aaaaaaataa gtgtatacaa attttaaagt gactcttagg 1320
ttttaaaacg aaaattcttg ttcttgagta actctttcct gtaggtcagg ttgctttctc 1380
aggtatagca tgaggtcgct cttattgacc acacctctac cggcatgccc atggatgacc 1440
cctccagcgt gttttatctc tgcgagcata atgcctgcgt catccgccag caggagctgg 1500
actttactga tgcccgttat atctgcgaaa agaccgggat ctggacccgt gatggcattc 1560
tctggttttc gtcatccggt gaagagattg agccacctga cagtgtgacc tttcacatct 1620
ggacagcgta cagcccgttc accacctggg tgcagattgt caaagactgg atgaaaacga 1680

aaggggatac gggaaaacgt aaaaccttcg taaacaccac gctcggtgag atgatcaatg 1740
catcctgcat ggcgcgcctg atgagcctga actgcccggg caaatcagct ggacgtctgc 1800
ctgcattaat gaatcggcca acgcgcgggg agaggcggtt tgcgtattgg gcgctcttcc 1860
gcttcctcgc tcactgactc gctgcgctcg gtcgttcggc tgcggcgagc ggtatcagct 1920
cactcaaagg cggtaatacg gttatccaca gaatcagggg ataacgcagg aaagaacatg 1980
tgagcaaaag gccagcaaaa ggccaggaac cgtaaaaagg ccgcgttgct ggcgtttttc 2040
cataggctcc gcccccctga cgagcatcac aaaaatcgac gctcaagtca gaggtggcga 2100
aacccgacag gactataaag ataccaggcg tttccccctg gaagctccct cgtgcgctct 2160
cctgttccga ccctgccgct taccggatac ctgtccgcct ttctcccttc gggaagcgtg 2220
gcgctttctc atagctcacg ctgtaggtat ctcagttcgg tgtaggtcgt tcgctccaag 2280
ctgggctgtg tgcacgaacc ccccgttcag cccgaccgct gcgccttatc cggtaactat 2340
cgtcttgagt ccaacccggt aagacacgac ttatcgccac tggcagcagc cactggtaac 2400
aggattagca gagcgaggta tgtaggcggt gctacagagt tcttgaagtg gtggcctaac 2460
tacggctaca ctagaaggac agtatttggt atctgcgctc tgctgaagcc agttaccttc 2520
ggaaaaagag ttggtagctc ttgatccggc aaacaaacca ccgctggtag cggtggtttt 2580
tttgtttgca agcagcagat tacgcgcaga aaaaaaggat ctcaagaaga tcctttgatc 2640
ttttctacgg ggtctgacgc tcagtggaac gaaaactcac gttaagggat tttggtcatg 2700
agattatcaa aaaggatctt cacctagatc cttttaaatt aaaaatgaag ttttaaatca 2760
atctaaagta tatatgagta aacttggtct gacagttacc aatgcttaat cagtgaggca 2820
cctatctcag cgatctgtct atttcgttca tccatagttg cctgactccc cgtcgtgtag 2880
ataactacga tacgggaggg cttaccatct ggccccagtg ctgcaatgat accgcgagac 2940
ccacgctcac cggctccaga tttatcagca ataaaccagc cagccggaag ggccgagcgc 3000
agaagtggtc ctgcaacttt atccgcctcc atccagtcta ttaattgttg ccgggaagct 3060
agagtaagta gttcgccagt taatagtttg cgcaacgttg ttgccattgc tacaggcatc 3120
gtggtgtcac gctcgtcgtt tggtatggct tcattcagct ccggttccca acgatcaagg 3180
cgagttacat gatcccccat gttgtgcaaa aaagcggtta gctccttcgg tcctccgatc 3240
gttgtcagaa gtaagttggc cgcagtgtta tcactcatgg ttatggcagc actgcataat 3300
tctcttactg tcatgccatc cgtaagatgc ttttctgtga ctggtgagta ctcaaccaag 3360
tcattctgag aatagtgtat gcggcgaccg agttgctctt gcccggcgtc aatacgggat 3420

```
aataccgcgc cacatagcag aactttaaaa gtgctcatca ttggaaaacg ttcttcgggg   3480
cgaaaactct caaggatctt accgctgttg agatccagtt cgatgtaacc cactcgtgca   3540
cccaactgat cttcagcatc ttttactttc accagcgttt ctgggtgagc aaaaacagga   3600
aggcaaaatg ccgcaaaaaa gggaataagg gcgacacgga aatgttgaat actcatactc   3660
ttcctttttc aatattattg aagcatttat cagggttatt gtctcatgag cggatacata   3720
tttgaatgta tttagaaaaa taaacaaata ggggttccgc gcacatttcc ccgaaaagtg   3780
ccacctgacg cgccctgtag cggcgcatta agcgcggcgg gtgtggtggt tacgcgcagc   3840
gtgaccgcta cacttgccag cgccctagcg cccgctcctt tcgctttctt cccttccttt   3900
ctcgccacgt tcgccggctt tccccgtcaa gctctaaatc gggggctccc tttagggttc   3960
cgatttagtg ctttacggca cctcgacccc aaaaaacttg attagggtga tggttcacgt   4020
agtgggccat cgccctgata gacggttttt cgccctttga cgttggagtc cacgttcttt   4080
aatagtggac tcttgttcca aactggaaca acactcaacc ctatctcggt ctattctttt   4140
gatttataag ggattttgcc gatttcggcc tattggttaa aaaatgagct gatttaacaa   4200
aaatttaacg cgaattttaa caaaatatta acgcttacaa tttccattcg ccattcaggc   4260
tgcgcaactg ttgggaaggg cgatcggtgc gggcctcttc gctattacgc cag          4313
```

## Description

### Field of invention

The present invention relates to methods of mixing large numbers of heterologous genes, which are located on artificial chromsomes. The methods of the present invention are useful for evolution of cells and whole genomes to acquire new functionality(ies), such as the ability to synthesise novel secondary metabolites 1and/or the evolution of novel metabolic pathways.

### Background of invention

Recombination of cells in order to optimise or produce heterologous proteins is a well-established practice in molecular biology.

The traditional approach to engineered molecular evolution relates to optimisation of an individual gene having a specific phenotype. The strategy is to clone a gene, identify a function for the gene and an assay for selecting the gene, mutate selected positions in the gene and select variants of the gene for improvement in the known function of the gene. A variant having a desired function may then be expressed in a suitable host cell.

However, the traditional approach has several drawbacks when it comes to evolution of cells having new properties, since the approach only relates to discrete genes. Multiple genes that cooperatively confer a single phenotype cannot be optimised in this manner. Furthermore, the traditional approach only leads to a very limited number of combinations or permutations in a cell or even for a single gene.

Evolution of cells having new properties have been described in for example WO 98/31837 wherein a method of evolving cells towards acquisition of new properties employing iterative cycles of recombination and selection/screening for evolution is discussed.

In WO 97/35966 a process of recursive sequence recombination in order to evolve new metabolic pathways are discussed, and in WO 00/04190 a process of recursive sequence recombination in order to evolve whole cells and organisms having desired properties.

Whether using the traditional approach of optimising individual genes or conducting iterative cycles of recombination, the individual genes in the cells in question are recombined, i.e. changed with foreign genetic material evolving new genes.

A major drawback when evolving new genes in this manner is, that each cycle of recombination may as well result in a failure leading to a nonsense gene as a success leading to an optimised gene. Furthermore, these methods are not very useful for evolution of novel metabolic pathways, since very few gene combination are produced.

Methods are known to provide recombined combinatorial gene expression libraries by crossing and recombination between cells comprising expression constructs (WO 00/52180 Terragen Discovery Ltd). Through the recombination, which may be carried out in vitro using the recA recombination enzyme, novel genes are obtained, which may or may not be functional in the host cell.

WO 98/17811 discloses a combinatorial gene expression library, comprising a pool of expression constructs in which the cDNA or genomic DNA fragment is operably-associated with one or more regulatory regions that drives expression of genes encoded by the cDNA or genomic DNA fragment in an appropriate host organism.

In WO 01/7300 a method for controlling a complex phenotype is disclosed. The method comprises providing a library of nucleic acids, which nucleic acids comprise one or more polynucleotide segments operably linked to at least one transcription regulatory sequence; introducing the library of nucleic acids into a population of recipient cells or intracellular organelles, whereby subsets of two or more members of the library, which subsets alter expression or activity of one or more components of a multigenic phenotype, are introduced into a plurality of the recipient cells or organelles; and identifying at least one recipient cell, intracellular organelle or organism comprising a recipient cell, with a desired phenotype.

WO 96/34112 discloses a combinatorial gene expression library with a pool of expression constructs each construct containing a cDNA or genomic DNA fragment from a plurality of donor organisms with the purpose of generating new metabolic pathways. The publication also discloses a combinatorial gene expression library in which each cell comprises a concatemer of cDNA fragments being operably associated with regulatory regions to drive expression of the genes encoded by the concatenated cDNA in a host organism. Once the gene cassettes have been cloned into the vector, it is not possible to excise the cassettes or the complete concatemer from the vector using a restriction enzyme. The reference is silent on the possibility of changing and optimising the combination of genes in each cell.

Consequently there is a need for developing methods for generation and optimisation of novel metabolic pathways

## Summary of invention

A first aspect of the invention relates to a method of mixing heterologous genes in expression cassettes located on artificial chromosomes, said method comprising the steps of:

providing two initial populations of cells that can mate with each other,
said initial populations comprising at least 2 cells in each population, and at least two cells in each population having different combinations of heterologous genes and/or different combinations of expression cassettes, wherein the expression cassettes are located on a nucleotide concatemer comprising in the 5'→3' direction a cassette of nucleotide sequence of the general formula

$$[rs_2\text{-SP-PR-X- TR-SP-}rs_1]_0$$

wherein

$rs_{1_}$and $rs_{2_}$ together denote a functional restriction site,
SP individually denotes a spacer of at least two nucleotide bases,
PR denotes a promoter, capable of functioning in a cell.
X denotes an expressible nucleotide sequence,
TR denotes a terminator, and
SP individually denotes a spacer of at least two nucleotide bases, and
$n \geq 2$, and
wherein at least a first cassette is different from a second cassette.

each cell comprising at least a first type of artificial chromosome, the at least first type of artificial chromosome comprising both at least two expression cassettes comprising heterologous genes and at least one selectable marker, the selectable markers being allocated to artificial chromosomes so that each type of artificial chromosome from each population can be individually selected for, mating the cells with each other, and
selecting mated cells that carry at least a subset of the selectable markers present on the artificial chromosomes in the two initial populations.

By "a type of artificial chromosome" is meant a group of artificial chromosomes sharing the same selectable markers. According to the present invention such a group of artificial chromosomes comprise artificial chromosomes with different expression cassettes or with different combinations of expression cassettes. Methods for generation of such artificial chromosomes and the differences between artificial chromosomes within the same type are disclosed in the detailed description part of the invention.

The method provides a possibility for changing the combination of genes located on artificial chromosomes in a cell by using simple means as mating and subsequent meiosis. Thus the methods of the present invention provides solutions to the problem of obtaining further mixing of genes that have already been mixed as they were selected for insertion into the two initial populations.

Hence, the method adapts simple mating techniques and makes them suitable for complex crossings. Because the content of individual cells of a library is not known, crossing cells of a library is more complex than crossing cells of a specific clone. For example, selection of cells comprising artificial chromosomes must be performed in order to preserve many different combinations of heterologous genes/gene cassettes.

The presence of the artificial chromosomes in the mated cells is ensured by the use of selectable markers located on all the artificial chromosomes, that select for mated cells After mating any sub-set of types of artificial chromosomes that select for mated cells can be used. One example of this is selecting for marker combinations present on at least one type of artificial chromosome present in each of the initial populations.

However in order to conserve the majority of genes while keeping the number of selective media/conditions reasonably low, the subset of the selectable markers selected for should include selecton for at least 70 % of all diploid types present in the mated population. More preferably the subset of the selectable markers selected for should include selection for at least 80 % of all diploid types present in the mated population, such as at least 90%, for example at least 95%, such as at least 99%, for example 100%.

The process may be continued by allowing the mated cells to subsequently undergo meiosis. Preferably meiosis is performed under conditions where cells without artificial chromosomes and cells that have not undergone meiosis do not survive. Standard protocols are available to do this e.g. for yeast cells.

Once cells have undergone meiosis and until the next mating round, the cells are kept under conditions where cells without artificial chromosomes do not survive. As the mixing method results in the generation of novel gene combinations it may advantageously be combined with screening of mated cells for a parameter related to a desired functionality(ies) and selecting cells having a predefined selection criterion(a) to undergo meiosis and mating. By screening cells at the diploid level, more genes are present in each cell, and consequently the chance of generating novel metabolic pathways is higher than at the haploid level.

Screening may alternatively comprise screening cells that have undergone meiosis for a parameter related to a desired functionality(ies) and selecting cells having a predefined selection criterion(a) to undergo mating and meiosis.

The mixing, mating, selection and preferably also further meiosis steps may be repeated to stepwise optimise the gene combinations or evolve novel gene combinations by repeating the process at least twice, such as 3 times, for example 4 times, such as 5 times, for example 6 times, such as 7 times, for example 8 times, such as 9 times, for example 10 times, such as 11 times, for example 12 times, such as 13 times, for example 14 times, such as 15 times, for example 16 times, such as 17 times, for example 18 times, such as 19 times, for example 20 times, such as 25 times, for example at least 30 times, such as at least 40 times, for example at least 50 times, such as at least 75 times, for example at least 100 times, such as at least 200 times, for example.at least 300 times, such as at least 500 times, for example at least 1000 times.

In order to reduce the accumulation of mutations in the host cells the method may further comprise subjecting the populations of cells to physical isolation of artificial chromosomes from the populations for every 4-5 rounds of meiosis and selection, and transferring the isolated artificial chromosomes into new host cells. The physical isolation may e.g. comprise amplification of artificial chromosomes in the host cells so that the artificial chromosomes constitute up to 20% of total DNA in the cells. Alternatively the physical isolation may comprise cutting expression cassettes from concatamers of expression cassettes on artificial chromosomes, cloning and amplification of these and re-assembling expression cassettes into an artificial chromosome vector backbone and transforming these into new host cells.

After meiosis, cells of the mating types may be separated from each other.

When working with a spore forming host species, the method may comprise mixing spores from different populations prior to mating. The two populations can then be mixed very efficiently before mating and thus there will be less mating within each population.

As there is no guarantee that the mixing method according to the invention results in improved genotypes compared to the initial populations or to early rounds of mixing and selection, the method preferably comprises storing a sub-population of mated and selected cells, while another sub-population undergoes' further meiosis and mating. According to this embodiment, the method preferably additionally comprises screening of at least a stored sub-population together with a population that has undergone at least one further round of meiosis and mating at a higher selection threshold than the previous screening, selecting cells above the higher selection threshold, and mating the selected cells with each other. The method may also comprise screening together with the stored sub-population and/or a population that has undergone at least one further round of meiosis, mating and screening, cells that contain expression cassettes or combinations of cassettes that have not been screened before.

It is also possible to add a further population of cells with artificial chromosomes comprising at least two expression cassettes with heterologous genes, the cells being capable of mating with the cells that have undergone mating and meiosis, the further population comprising at least 2 cells with combinations of expression cassettes different from the combinations in the cells of the initial population, the artificial chromosomes of said further population carrying at least one selectable marker. Preferably the artificial chromosomes of said further population have the same markers as the initial populations. The further population may comprise a 50/50 mixture of cells of the two mating types of the initial populations or it may comprise cells of one of the mating types of the initial populations.

At least one of the two initial populations of cells that can mate with each other may further carry at least a second type of artificial chromosome with expression cassettes comprising heterologous genes, the first and second types of artificial chromosome carrying at least one selectable marker so that said first and second types of artificial chromosome can be individually selected for. More preferably, at least one of the two initial populations of cells that can mate with each other further carries at least a third type of artificial chromosome with expression cassettes comprising heterologous genes, the first, second, and third types of artificial chromosome carrying at least one selectable marker so that said first, second, and third type of artificial chromosome can be individually selected for. More preferably at least one of the two initial populations of cells that can mate with each other further carries at least a fourth type of artificial chromosome with expression cassettes comprising heterologous genes, the first, second, third, and fourth type of artificial chromosome carrying at least one selectable marker so that said first, second, third, and fourth type of artificial chromosome can be individually selected for.

More generally speaking the two initial populations of cells that can mate with each other may carry from 1 to 10 types of artificial chromosomes, each artificial chromosome of each population carrying at least one selectable marker so that each of the types of artificial chromosomes from each of the two populations can be individually selected for.

Similarly, the further population of cells with artificial chromosomes capable of mating with the cells that have undergone mating and meiosis may carry from 1 to 10 types of artificial chromosomes, each type of artificial chromosome of said further population carrying at least one selectable marker so that each of the types of artificial chromosomes can be individually selected for.

According to one embodiment, each cell may carry 2 artificial chromosomes per cell that can mate. According to another embodiment each cell may carry 3 artificial chromosomes per cell that can mate. The number of artificial chromosomes per cell is today considered a practical number, at least in the case where yeast is the host species. This is because this number of artificial chromosomes can be efficiently transferred into and be stably maintained at least in yeast and also because too high a number of artificial chromosomes may cause centromere toxicity, at least when all the centromeres of the artificial chromosomes are identical. It is expected that within the term of the present patent, methods will be developed for stable maintenance of a higher number of artificial chromosomes in yeast and possibly also in other species.

Preferably each artificial chromosome carries at least two selectable markers, the selectable markers being allocated to types of artificial chromosomes so that each type of artificial chromosome from each population can be individually selected for. In this case, one marker is normally' located on each arm of the artificial chromosomes to ensure that the artificial chromosomes do not lose any of the arms. Preferably, all artificial chromosomes carry a common marker so that it is possible at any point to select for cells that contain at least one artificial chromosome. Suitable selectable markers are selected from drug resistance, colour, morphology, resistance against electromagnetic radiation, salt tolerance, oxidative stress resistance, markers based on fluorescence probes, and auxotrophy markers, markers that can be used to produce high copy numbers (e.g. a poorly expressed LEU2 gene (leu-2d), heterologous thymidine kinase (TK), heterologous dihydrofolate reductase gene), heterologous genes that give a growth advantage. More preferably the markers are auxotrophy markers. "Oxidative stress resistance" is meant to encompompass resistance or tolerance to reative oxygen species, i.e. free oxygen radikals.

Specific examples of these markers include but are not limited to: NPT", LEU 2, TRP 1, HIS 3, LYS 2, URA 3, ADE 2, Amyloglucosidase, β-lactamase, CUP 1, $G418^R$, $TUN^R$, KILk1, C230, SMR1, SFA, $Hygromycin^R$, $methotrexate^R$, $chloramphenicol^R$, $Diuron^R$, $Zeocin^R$, $Canavanine^R$, ARG 4, THR4, Luciferase, GUS, GFP, LUX.

It is preferred that said at least 2 cells of each population comprise at least 2, more preferably at least 5, even more preferably at least 10, yet more preferably at least 20, for example at least 50, such as at least 100, for example at least 200, such as in the range of 10 to 1000, for example in the range of 40 to 700, such as in the range of 60 to 300 expression cassettes comprising different heterologous genes.

When possible, it is preferred that the two initial populations are of different mating types. The size of the initial populations may either be selected to that the two initial populations have approximately the same number of cells. Alternatively, the number of cells in one population is higher than the number of cells in the other population.

In a further aspect the invention relates to a method of mixing heterologous genes in expression cassettes located on artificial chromosomes, said method comprising the steps of

providing two initial populations of protoplasts or cells that can be fused,

said initial populations comprising at least 2 cells in each population, and at least two cells in each population having different combinations of heterologous genes and/or different combinations of expression cassettes, wherein the expression cassettes are located on a nucleotide concatemer comprising in the 5'→3' direction a cassette of nucleotide sequence of the general formula

$$[rs_2\text{-}SP\text{-}PR\text{-}X\text{-}TR\text{-}SP\text{-}rs_1]_0$$

wherein

$rs_1$ and $rs_2$ together denote a functional restriction site,
SP individually denotes a spacer of at least two nucleotide bases,
PR denotes a promoter, capable of functioning in a cell,
X denotes an expressible nucleotide sequence,
TR denotes a terminator, and
SP individually denotes a spacer of at least two nucleotide bases, and
$n \geq 2$, and
wherein at least a first cassette is different from a second cassette.

each cell comprising at least a first type of artificial chromosome, said at least first type of artificial chromosome comprising both at least two expression cassettes comprising heterologous genes and at least one selectable marker,

the selectable markers being allocated to artificial chromosomes so that each type of artificial chromosome from each population can be individually selected for, performing protoplast fusion and regeneration of cell walls or performing fusion of cells, and

selecting fused cells that carry at least a subset of the selectable markers present on the artificial chromosomes in the two initial populations.

As for the first aspect of this invention, the second aspect provides a method for mixing and optimising gene combinations for expressible genes located on artificial chromosomes. The method may be used even in cases where it is not possible to mate cells in vitro.

Preferably, the cells or protoplasts caused to fuse are haploid, so that the chromosome number of the fused cells is diploid. It is also possible in some species to fuse cells with higher ploidy level and thus obtain polyploid fused cells.

The process may be repeated one or more times.

Preferably the species of cells are selected from fungi, algae, and plants, for which protocols for isolation and fusion of protoplasts and subsequent regeneration of cell walls are known. More preferably, the species of cells is a fungus species, in which it is possible to induce spore formation in vitro. Other relevant species include prokaryots, which can be fused.

However, it is also contemplated, that it becomes possible to fuse animal cells, which do not have a cell wall, so that the species of cells may include animal cells, including human cells.

Preferably, the species of cells is one for which extensive in vitro protocols are known, and for which standard molecular biology methods have been developed. These include industrial microorganisms, preferably fungi, more preferably yeast. Suitable examples of yeast species are disclosed in the detailed description of the present invention. Other species include carrot, Arabidopsis thaliana, Nicotiana spp., Nicotiana tabacum, maize, wheat, rice, soybean, tomato, peanut, potato; sugar beets, sunflower, yam, rape seed, conifers, and petunia. The list is expected to grow in future as the field expands.

As for the mating based method, the mixing may advantageously be combined with screening cells that result from protoplast fusion for a desired functionalty(ies) and selecting cells having the desired functionalty(ies) above a defined threshold, isolating protoplasts from these cells and performing protoplast fusion and cell regeneration on the selected cells.

According to a further aspect the invention relates to a method for mixing heterologous genes in expression cassettes located on artificial chromosomes, said method comprising the steps of
providing two initial populations of cells,
said initial populations comprising at least 2 cells in each population, and at least two cells in each population having different combinations of heterologous genes and/or different combinations of expression cassettes, wherein the expression cassettes are located on a nucleotide concatemer comprising in the 5'→3' direction a cassette of nucleotide sequence of the general formula

$$[rs_2\text{-SP-PR-X-TR-SP-}rs_1]_0$$

wherein

$rs_1$ and $rs_2$ together denote a functional restriction site,
SP individually denotes a spacer of at least two nucleotide bases,
PR denotes a promoter, capable of functioning in a cell,
X denotes an expressible nucleotide sequence,
TR denotes a terminator, and
SP individually denotes a spacer of at least two nucleotide bases, and
$n \geq 2$, and
wherein at least a first cassettes is different from a second cassette.

each cell comprising at least a first type of artificial chromosome, the at least first type of artificial chromosome comprising both at least two expression cassettes comprising heterologous genes and at least one selectable marker,
the selectable markers being allocated to artificial chromosomes so that each type of artificial chromosome from each population can be individually selected for, mating the cells with each other,
amplifying the artificial chromosomes in the host cells,
isolating the artificial chromosomes,
mixing the isolated artificial chromosomes,
transferring subsets of said isolated and mixed artificial chromosomes into host cells, and
selecting cells that carry at least a subset of the selectable markers present on the artificial chromosomes in the two initial populations.

According to this aspect, use is made of the fact that artificial chromosomes can be amplified in the host cells, for example in yeast it is possible to use vectors that permitte copy number amplification. The vectors include a conditional centromere that can be turned on or off. The disruption of the centromere activity by high levels of transcription towards conserved centromeric elements leads to a segregation bias during cell division wherein the mother cell receives both copies of the artificial chromsome, in this case a YAC. At the same time, a strong selective pressure for extra copies of the artificial chromosome can be applied by selecting for the expression of a heterologous gene such as thymidine kinase. Selection for the TK gene can be accomplished by adding exogenous thymidine in the presence of methotrexate and sulfanilamide. The later two compounds inhibit enzymes involved in the recycling or de novo synthesis of folate cofactos required for the synthesis of deoxythymidilic acid. When this system is used, artificial chromosomes were readily amplified 10- to 20- fold. Reactivation of the centromere in amplified artificial chromosome clones resulted in stable maintenance of an elevated copy number (Smith et al, PNAS, 1990, vol 87:8242-48).

The method may advantageously be combined with the other gene mixing methods according to the invention, in particular with the mating based method, which in a preferred embodiment includes physical isolation of artificial chromosomes.

The host cells into which the subsets of mixed artificial chromosomes are transferred may already contain artificial chromosomes with expression cassettes with heterologous genes.

There is provided a method of mixing heterologous genes in expression cassettes located on artificial chromosomes, said method comprising the steps of:

a) obtaining at least one population of cells, the cells of said at least one population comprising a concatemer of expression cassettes of the following formula:

$$[rs_2\text{-SP-PR-X-TR-SP-}rs_1]_0$$

wherein

$rs_1$ and $rs_2$ together denote a restriction site,
SP individually denotes a spacer,
PR denotes a promoter, capable of functioning in the cells,
X denotes an expressible nucleotide sequence,
TR denotes a terminator, and
$n \geq 2$,
the cells differing from each other with respect to combinations of expressible nucleotide sequences and/or promoters and/or terminators and/or spacers,

b) isolating at least some of the cassettes of the selected cells by cutting the concatemers with a restriction enzyme cleaving $rs_1rs_2$,
c) amplifying at least some of the isolated cassettes,
d) assembling the expression cassettes of step c) into artificial chromosomes, and
e) optionally transferring the artificial chromosomes into host cells.

In one embodiment of the invention, n is at least 10, such as at least 15, for example at least 20, such as at least 25, for example at least 30, such as from 30 to 60 or more than 60, such as at least 75, for example at least 100, such as at least 200, for example at least 500, such as at least 750, for example at least 1000, such as at least 1500, for example at least 2000.

The concatemers can be used to make novel and non-native combinations of genes for co-ordinated expression in a host cell. Thereby new metabolic pathways can be generated, which may lead to the production of new metabolites, and/or to the metabolisation of compounds, which are otherwise not metabolisable by the host cells. The new gene combinations may also lead to metabolic pathways which produce metabolites in new quantities or in new compartments of the cell or outside the cell. Depending on the purpose, the selection of genes can be made completely random based on sourcing of expressible nucleotide sequences across the different kingdoms. However, it may also be advantageous to source genes from sources known to have certain metabolic pathways in order to make targeted new gene combinations. It may also be advantageous to source genes from organisms/tissues known to have relevant properties, e.g. a specific pharmaceutical activity.

One of several advantages of the concatemers of the present invention is that the expression cassettes can be cut out from the concatemers at any point to make new combinations of expression cassettes. During re-assembly, further genes comprised in similar expression cassettes may be added if desired to modify the expression pattern. In this way, the concatemers according to the present invention present a powerful tool in generating novel gene combinations.

One advantage of the structure of the concatemer is that cassettes can be recovered from the host cell through nucleotide isolation and subsequent digestion with a restriction enzyme specific for the $rs_1$-$rs_2$ restriction site. The building blocks of the concatemers may thus be disassembled and reassembled at any point.

The amplification step ensures that the copy number of the cassettes is high enough to be able to perform the re-assembly conveniently. The amplification step may include PCR with primers that tag $rs_1$ and $rs_2$ and/or inserting isolated cassettes into a vector having a cloning site compatible with $rs_1rs_2$ and multiplying this vector in a suitable host.

Further expression cassettes may be added for the assembly step if desired. Conveniently, the method may be combined with screening cells with assembled artificial chromosomes for a desired functionalty(ies) and selecting cells having the desired functionalty(ies). The process may be repeated by subjecting the selected cells to further isolation and amplification of cassettes and assembly of artificial chromosomes. The method may also be combined with any of the other gene mixing methods according to the invention.

In a still further aspect the invention relates to a method for mixing expressible nucleotide sequences, said method comprising the steps of

a) obtaining at least one population of cells, the cells of said at least one population comprising at least two expression cassettes of the following formula:

[$rs_2$-SP-PR-rs1'-X-rs2'-TR-SP-$rs_1$]

wherein

$rs_1$ and $rs_2$ together denote a restriction site,
rs1' and rs2' together denote a different restriction site,
SP individually denotes an optional spacer,
PR denotes a promoter, capable of functioning in the cells,
X denotes an expressible nucleotide sequence,
TR denotes a terminator,

b) isolating at least some of the expressible nucleotide sequences of the selected cells by cutting the cassettes with a restriction enzyme cleaving rs1'rs2', or by amplifying the sequences with primer pairs templating sequences in rs1' and rs2',
c) re-inserting the expressible nucleotide sequences into other similar backbone,
d) re-mixing the expression cassettes, and
e) transferring the re-expression cassettes into host cells.

The method provides a way of isolating the coding sequence for re-insertion into new expression constructs in order to increase the number of expression contexts.

Preferably, the isolated expressible nucleotide sequences are inserted into primary vectors comprising a nucleotide sequence cassette of the general formula in 5'→3' direction:

[RS1-RS2-SP-PR-CS-TR-SP-RS2'-RS1']

wherein

RS1 and RS1' denote restriction sites,
RS2 and RS2' denotes restriction sites different from RS1 and RS1',
SP individually denotes a spacer sequence of at least two nucleotides,
PR denotes a promoter,
CS denotes a cloning site,
TR denotes a terminator.

Expression cassettes can be isolated from these primary vectors and assembled into concatamers of expression cassettes with new gene combinations.

In a further aspect the invention relates to a method of mixing heterologous genes in expression cassettes located on plasmids said method comprising the steps of

providing two initial populations of cells that can mate with each other,
said initial populations comprising at least 2 cells in each population, and at least two cells in each population having different combinations of heterologous genes and/or different combinations of expression cassettes, wherein the expression cassettes are located on a nucleotide concatemer comprising in the 5'→3' direction a cassette of nucleotide sequence of the general formula

$[rs_2\text{-SP-PR-X-TR-SP-}rs_1]_0$

wherein

$rs_1$ and $rs_2$ together denote a functional restriction site,
SP individually denotes a spacer of at least two nucleotide bases,
PR denotes a promoter, capable of functioning in a cell,
X denotes an expressible nucleotide sequence,
TR denotes a terminator, and
SP individual denotes a spacer of at least two nucleotide bases, and
$n \geq 2$, and
wherein at least a first cassette is different from a second cassette.

each cell comprising at least a first plasmid, the at least first plasmid comprising both at least two expression cassettes comprising heterologous genes and at least one selectable marker, the selectable markers being allocated to plasmids so that each type of plasmid from each population can be individually selected for,
mating the cells with each other, and
selecting mated cells that carry at least a subset of the selectable markers present on the plasmids in the two initial populations.

There are several advantages associated with having the expression cassettes located on a plasmid. Among these is the possibility of using a shuttle vector which makes it possible to amplify the plasmids in bacteria and later transform them into another cellular host, such as yeast. Plasmids may also be purified in bacteria by simply isolating total DNA from the host cells and transform this into bacteria. As only the plasmids have an origin of replication, which is functional in a bacterium, only the plasmids are replicated, and the plasmids are therefore selectably amplified. The selectable markers located on the plasmids are used to select for bacteria harbouring the plasmids. The plasmids can then be re-isolated from the bacteria and re-inserted into the other host cells.

Preferably, in the plasmid based method, the expression cassettes are located on a nucleotide concatemer comprising in the 5'→3' direction a cassette of nucleotide sequence of the general formula

$[rs_2\text{-SP-PR-X-TR-SP-}rs_1]_n$

wherein

$rs_1$ and $rs_2$ together denote a functional restriction site,
SP individually denotes a spacer of at least two nucleotide bases,
PR denotes a promoter, capable of functioning in a cell,
X denotes an expressible nucleotide sequence,
TR denotes a terminator, and
SP individually denotes a spacer of at least two nucleotide bases, and
$n \geq 2$, and
wherein at least a first cassette is different from a second cassette.

In a separate aspect of the invention there is provided a method for mixing of heterologous genes in expression cassettes located on artificial chromosomes or plasmids, wherein the expression cassettes are located on a nucleotide concatemer comprising in the 5'→3' direction a cassette of nucleotide sequence of the general formula

$rs_2\text{-SP-PR-X-TR-SP-}rs_1]_{0-}$

wherein

$rs_1$ and $rS_2$ together denote a functional restriction site,
SP individually denotes a spacer of at least two nucleotide bases,
PR denotes a promoter, capable of functioning in a cell,
X denotes an expressible nucleotide sequence.
TR denotes a terminator, and,
SP individually denotes a spacer of at least two nucleotide bases, and
$n \geq 2$, and

wherein at least a first cassette is different from a second cassette,
wherein a biological selection pressure is used to ensure that only cells with the desired functionality(ies) survive this selection pressure. The biological selection pressure may e.g. be based on a reporter system which is transformed into the host cells prior to transformation of the artificial chromosomes or plasmids with the expression cassettes or it may be a medium based selection pressure or any of the other selection methods described in the present application. The biological selection pressure is applied as soon as possible after insertion of the artificial chromosomes or plasmids, just allowing the cells time to recover after the transformation. This method more closely resembles natural evolution of cells and will eventually cause cells to evolve the properties selected for. As with the other methods this method can be combined with any other method (mating, protoplast fusion, physical isolation) according to the present invention.

## Definitions

Diploid type: A mated cell that contains at least one artificial chromosome from each parent population of a mating round. Depending on the number of artificial chromosomes in the parent populations, mating will result in many diploid types. The presence of artificial chromosome of type A and B in one parent population and type C and D in another results in the following diploid types: AC, AD, BC, BD, ABC, ABD, ACD, BCD, ABCD.

Fused cell type: a cell resulting from protoplast or cell fusion, which carries at least one artificial chromosome from each of the two populations that formed the fused cells. Depending on the number of artificial chromosomes in the two initial populations, fusion will result in many fused cell types. The presence of artificial chromosome of type A and B in one initial population and type C and D in another results in the following fused cell types: AC, AD, BC, BD, ABC, ABD, ACD, BCD. ABCD.

Protoplast: a cell from which the cell wall has been removed.

Artificial chromosomes: As used herein, an artificial chromosome (AC) is a piece of DNA that can stably replicate and segregate alongside endogenous chromosomes. For eukaryotes the artificial chromosome may also be described as a nucleotide sequence of substantial length comprising a functional centromer, functional telomeres, and at least one autonomous replicating sequence. It has the capacity to accommodate and express heterologous genes inserted therein. It is referred to as a mammalian artificial chromosome (MAC) when it contains an active mammalian centromere. Plant artificial chromosome and insect artificial chromosome (BUGAC) refer to chromosomes that include plant and insect centromers, respectively. A human artificial chromosome (HAC) refers to a chromosome that includes human centromeres, AVACs refer to avian artificial chromosomes. A yeast artificial chromosome (YAC) refers to chromosomes are functional in yeast, such as chromosomes that include a yeast centromere.

As used herein, stable maintenance of chromosomes occurs when at least about 85%, preferably 90%, more preferably 95%, more preferably 99% of the cells retain the chromosome. Stability is measured in the presence of a selective agent. Preferably these chromosomes are also maintained in the absence of a selective agent. Stable chromosomes also retain their structure during cell culturing, buffering neither intrachromosomal nor interchromosomal rearrangements.

Expression cassettes comprising heterologous genes: By the term "expression cassettes" is meant controllably expressed nucleotide sequences, of the formula:

PR-X-TR

wherein

- PR denotes a promoter, capable of functioning in a cell,
- X denotes an expressible nucleotide sequence,
- TR denotes a terminator, capable of functioning in a cell.

The promoter and expressible nucleotide sequence may be natively associated but preferably the promoter is heterologous to the expressible nucleotide sequence.

Selectable marker: any gene, which provides the cell with a function, for which selection can be made to ensure the presence of the selectable marker in the cell. Typical examples of selectable markers include auxotrophic markers and drug resistance markers. Further examples of selectable markers include markers giving a colour or a particular morphology, which may be selected for in a flow cytometer or by hand. Still further examples of selectable markers include resistance against physical or chemical conditions, e.g. radiation resistance, salt resistance. These may be selected for on the basis of survival. Other types of markers includes nucleotide probes labelled with a stain/dye molecule or preferably a fluorescent signal molecule.

## Description of the drawings

Fig. 1 shows one schematic example of mixing of genes using mating and selection. Different shades of the artificial chromsomes (native chromosomes left out) illustrate different types of artificial chromosomes.

Fig. 2 shows an example of multiple parameter screening for compounds synthesised by cells, where the compounds inhibit Cox-2 and NF-κB and do not ihibit Cox1. It is shown that in early rounds, cells that meet one, two or all three of the criteria are selected. In later rounds, only cells that fit all the selection criteria are selected.

Fig. 3 shows an example of a multiple parameter screen including S. Aureus growth inhibition, DNA Polymerase III inhibition and P450 compound inactivation. The screen is assembled by, for example, transforming a library of producer strains with GFP reporter systems for a few selected human P450s and for recombinant Bacillus subtilis DNA Pol III. The library is then plated and overlayed with an MRSA strain. The compounds have to cross the producer's cell wall and reach the MRSA strain thus the screen will also select for compounds that have a reasonable solubility profile. Producer cells in zones cleared of MRSA cells and which produce the desired combination of fluorescent colours are selected.

Fig. 4a illustrates a multiple parameter screen set-up for cancer chemoprotectant. In the assay, a producer species library is encapsulated so that on average each capsule has 1 cell and allow to grow for a few generations. These clonal lines are then double encapsulated with a permeabilised yeast that contains a human DNA Topo II a reporter system. The gel droplet environment contains etoposide (a poison) and a flourescent DNA double strand break stain. Gel droplets where the yeast cells in the outer layer have survived and that do not fluoresce are selected.

Fig. 4b illustrates a method of screening single yeast cells. Cells were encapsulated in alginate beads (diameter 20 μ). Beads were inoculated into selective medium and cells allowed to grow for c. 6 generations resulting in each bead containing c. 100 clonal cells. Beads were then placed in inducive medium for 24 h before co-encapsulation w. reporter yeast. The reporter yeast cells were S. cerevisiae strains JN394 and JN 394top2-5 (Nitiss and Nitiss(2001), Methods Mol Biol.;95:315-27). JN394 -deGFP(ise2 and RAD52 mutant) DNA repair deficient and hypersensitive to Topo2 poisons, expresses deGFP constitutively. JN394top2-5-deCFP (contains a TOPO 2 that is resistant to topo-poisons), expresses deCFP constitutively. Double encapsulation beads were then placed in selective medium containing a lignan precursor and incubated for 8 hours. Geldroplets showing CFP flourescense but no GFP fluorescence were selected and producer cells retained for further evolution. Gel droplets shoving no flourescense, or double flourescense were discarded.

Fig. 5 shows a multiple parameter screen set up where a producer species library reporting RXR-RXR activation is co-encapsulated with a mammalian cell line reporting PPARγ-RXR activation as well as P450 compound inactivation. Gel droplets that indicate PPARγ-RXR activation but not RXR-RXR or P450 inhibition are selected.

Fig. 6 shows an example of a multiple parameter screen for absorption and a pharmacological activity: By using a dual culture system and timing the time of cell selection, it is possible to select producer cells that have the desired pharmacological activity and a good absorption profile.

Fig. 7 shows an example of a screening system which minimises the number of false positives generated by compounds that are rapidly metabolised by the human DMEs and also leads to the discovery of compounds that are active after being metabolised and which would otherwise remain undiscovered.

Fig. 8 shows a schematic representation of a screening system of the present invention to evaluate target activity, metabolism by DMEs and cytotoxicity: Using a double gel encapsulation system where in the first droplet are clonal lines of the producer species transformed with the pharmacological target and DMEs, and in the second droplet are hepatocytes, it is possible to screen for target activity, DME metabolism and hapatotoxicity.

Fig. 9 shows a flow chart of the steps leading from an expression state to incorporation of the expressible nucleotide sequences in an entry library (a nucleotide library according to the invention).

Fig. 10 shows a flow chart of the steps leading from an entry library comprising expressible nucleotide sequences to evolvable artificial chromosomes (EVAC) transformed into an appropriate host cell. Fig. 10a shows one way of producing the EVACs which includes concatenation, size selection and insertion into an artificial chromosome vector. Fig. 10b shows a one step procedure for concatenation and ligation of vector arms to obtain EVACs.

Fig. 11 shows a model entry vector. MCS is a multi cloning site for inserting expressible nucleotide sequences. Amp R is the gene for ampicillin resistance. Col E is the origin of replication in E. coli. R1 and R2 are restriction enzyme recognition sites.

Fig. 12 shows an example of an entry vector according to the invention, EVE4.

MET25 is a promoter, ADH1 is a terminator, f1 is an origin of replication for filamentous phages, e.g. M13. Spacer 1 and spacer 2 are constituted by a few nucleotides deriving from the multiple cloning site, MCS, Scfl and Ascl are restriction enzyme recognition sites. Other abbreviations, see Fig. 11. The sequence of the vector is set forth in SEQ ID NO 1.

Fig 13 shows an example of an entry vector according to the invention, EVE5. CUP1 is a promoter, ADH1 is a terminator, f1 is an origin of replication for filamentous phages, e.g. M13. Spacer 1 and spacer 2 are constituted by a few nucleotides deriving from the multiple cloning site, MCS, Scfl and Ascl are restriction enzyme recognition sites. Other abbreviations, see Fig. 11. The sequence of the vector is set forth in SEQ ID NO 2.

Fig 14 shows an example of an entry vector according to the invention, EVE8. CUP1 is a promoter, ADH1 is a terminator, f1 is an origin of replication for filamentous phages, e.g. M13. Spacer3 is a 550 bp fragment of lambda phage DNA fragment. Spacer4 is a ARS1 sequence from yeast. Scfl and Ascl are restriction enzyme recognition sites. Other abbreviations, see Fig. 11. The sequence of the vector is set forth in SEQ ID NO 3.

Fig. 15 shows an example of an entry vector according to the invention, EVE9. Met25 is a promoter, ADH1 is a terminator. Spacer 5 and 6 are lambda phage DNA. SEQ ID NO 5.

Fig. 16 shows a vector (pYAC4-Ascl) for providing arms for an evolvable artificial chromosome (EVAC) into which a concatemer according to the invention can be cloned. TRP1, URA3, and HIS3 are yeast auxotrophic marker genes, and AmpR is an E. coli antibiotic marker gene. CEN4 is a centromere and TEL are telomeres.

ARS1 and PMB1 allow replication in yeast and E. coli respectively. BamHI and Asc I are restriction enzyme recognition sites. The nucleotide sequence of the vector is set forth in SEQ ID NO 4.

Fig. 17. shows the general concatenation strategy. On the left is shown a circular entry vector with restriction sites, spacers, promoter, expressible nucleotide sequence and terminator. These are excised and ligated randomly.

| Lane | F/Y |
|---|---|
| 1 | 100/1 |
| 2 | 50/1 |
| 3 | 20/1 |
| 4 | 10/1 |
| 5 | 5/1 |
| 6 | 2/1 |
| 7 | 1/1 |
| 8 | 1/2 |
| 9 | 1/5 |
| Legend: Lane M: molecular weight marker, λ-phage DNA digested w. Pst1. Lanes 1-9, concatenation reactions. Ratio of fragments to yac-arms(F/Y) as in table. | |

Fig 18a and 18b. illustrates the integration of concatenation with synthesis of evolvable artificial chromosomes and how concatemer size can be controlled by controlling the ratio of vector arms to expression cassettes, as described in example 14.

Fig 19. EVAC gel Legend: PFGE of EVAC containing clones:

Lanes. a: Yeast DNA PFGE markers(strain YNN295), b: lambda ladder, c: non-transformed host yeast, 1 - 9 : EVAC containing clones. EVACs in size range 1400-1600 kb. Lane 2 shows a clone containing 2 EVACs sized ~1500 kb and -550 kb respectively. The 550kb EVAC is comigrating with the 564kb yeast chromosome and is resulting in an increased intensity of the band at 564 kb relative to the other bands in the lane. Arrows point up to EVAC bands.

Fig. 20 shows an example of generation of an EVAC containing cell population. EVACs (Evolvable Artificial Chromosome) are artificial chromosomes composed of concatemers of expression cassettes containing heterologous DNA, so that each gene is under the control of an externally controllable promoter. Large numbers of heterologous genes from multiple sources can thus be combined in a single host cell.

Fig. 21 shows the general principle for screening EVAC containing cell populations. The cell population is amplified and subjected to a panel of screens that are relevant to a desired functionality. Positive subpopulations are selected.

Fig. 22 shows how cell populations evolve through a tiered set of selection conditions, always taking the best performing cell populations further in the process until an optimal functionality/property is evolved.

Fig. 23 shows a general screening strategy. Independent populations are subjected to the same set of screens, and genetic material from the different selected sub-populations is combined together with novel genetic diversity introduced between selection rounds.

Fig. 24 shows physical remixing of EVACs. EVACs are isolated from the host and used for transformation of either empty host cells or for transformation of host cells already containing EVACS to obtain new combinations of EVACs in each host cell.

Fig. 25 shows one example of evolution. Cells that are resistant to a poison may be selected in liquid media. The surviving cells are cells containing EVACs that result in the production of compounds that prevent the poison from interacting with its target.

Fig. 26 shows how an evolution programme based on a screen for compounds that activate (or prevents) activation of a reporter system may be designed. Using the appropriate marker (e.g. GFP) positive clones can be selected using e.g. flow cytometry.

Fig. 27 shows an example of controllable gene expression in a cell population containing EVACs enriched in genes that code for carotenoid synthetic enzymes. The expression cassettes contain either a Met 25 or a CUP I promoter. Orange, yelllow and red colonies are obtained as a function of the promoter activation. Intensity of colour and number of coloured colonies increases in the following order: CUP + Met > CUP > Met. Uninduced colonies are white.

Figure 28 shows oxidative stress resistance of cells after 1, 2 and 3 rounds of screening. CEY (compound enhanced yeast) indicate selected yeast cells.

Figure 29 shows yeasts producing pink, yellow, orange and green colours obtained after two and three rounds of screening. CEY (compound enhanced yeast) indicate selected yeast cells.

Figure 30 shows the carotenoid content of 10 selected yeast clones. CEY (compound enhanced yeast) indicate selected yeast cells.

**Detailed Description of the Invention**

The following provides a background description on how to apply the gene mixing methods according to the invention and how to use these in the directed to evolution of cells to acquire new functionalities.

The present invention relates to methods of mixing of genes for the purpose of evolving cells having at least one desired functionality, preferably evolving cells that produce compounds, novel substances and/or comprise novel metabolic pathways. The mixing and/or evolution may lead to the production of novel molecules of commercial value, such as pharmaceuticals, cosmetics, flavours, other food and animal feed ingredients, agricultural chemicals, colouring agents, diagnostic markers, industrial chemials and intermediates for industrial purposes.

By "Evolution of a cell" is meant change of a cell's phenotype towards a novel phenotype due to expression of a novel combination of genes. By "evolution of a composition" is meant change of the properties of a composition due to a novel combination of cells expressing a novel combination of genes.

In the following the term mixing is mainly used for describing the assembly of expression casettes and the term remixing is mainly used for the process referring to further mixing of expression casettes as described in the claims. However, as both terms encompass mixing, the term mixing is also used for the remixing steps.

In seeking to evolve molecules with defined pharmaceutical, industrial, nutritional properties one must have a method of selecting for those genetic patterns that encode for phenotypes that are consistent with these properties.

Each cell in a cell population, given that it is genetically different from other cells, has an intrinsic variability that can potentially express itself in one or more ways. For the purposes of the current invention the term Output shall be taken to mean a property of the cell that is consequent to the expression of one or more expression cassettes. Optionally the property may be consequent to both the expression of one or more expression cassettes and the expression of a certain set of host genes.

Outputs can be measured according to various different criteria. These criteria may be directly or indirectly linked to the functional or structural properties that are being optimised. Alternatively they may be inversely linked to functional or structural properties that are not desired.

Outputs can be measured either directly or by means of a reporter construct. For the purposes of this document the term Reporter Construct shall be taken to mean a genetic or molecular device for measuring whether a given cell or subset of cells in a cell population vary in respect of a given output from other cells or subsets of cells in the cell population. Example reporter constructs include a genetic construct that produces a fluorescent protein in response to the activation of a transcription factor by an output. Another example of a reporter construct is a coloured enzyme substrate, to which an enzyme is added that converts the substrate to another molecule with a different colour. Should the cell produce an output that inhibits the enzyme, the colour change will not occur.

Outputs that can be measured without a reporter construct include without limitation the survival of cells subjected to the screening criteria, cells able to metabolise a predetermined substance, cells able to produce a substance that preferentially absorbs electromagnetic radiation at one or more frequencies, cells having enzymatic efficacy in the media etc.

Reporter constructs can be placed proximal either before or after the expression construct is engineered into the cell. Methods of incorporating the reporter construct into a proximal location include but are not limited to standard transformation techniques, the mating of two different yeast mating types, or systems providing .physical proximity between cell and reporter construct, for example gel microdroplet co-encapsulation of cell and reporter construct.

The term Proximal shall be taken to mean a location that is either in the same cell as the expression construct or sufficently close to said cell such that the concentration of a molecule or molecules diffusing from an intact or lysed cell, or being actively pumped from the cell, is at least one picomole in the vicinity of the location

Outputs of cells that may be measured either by proximal reporter contructs or by other means include, but are not limited to:

- Novel spectral properties
- Induced cytochrome oxidase activity
- Changed size, morphology, stickiness or adhesive properties or lack thereof
- Ability to grow on substrates they cannot normally grow on
- Ability to grow on sublethal substrates
- Ability to grow in the absence of normal essential requirements
- Ability to grow on media comprising one or more inhibitors
- Ability to grow under changed physical conditions, such as temperature, osmolarity, electromagnetic radiation including light of certain wavelengths.
- Ability to grow under magnetic field of certain force.
- Secretion or the lack of it from the cell
- The inhibition or prevention of inhibition of an enzyme
- The activation of a receptor.
- The prevention of an activating molecule binding to a receptor.
- The inhibition or promotion of binding of small molecules or proteins to nucleic acid or peptide sequences.
- The inhibition or promotion of transcription or translation of post translational processing.
- Changes in the transport or localisation of molecules within the cell or within organelles.
- Changes in the DNA content or morphology of the cell.
- The production of small molecules with certain properties that allow their selective isolation (e.g. all the chromoatography principles available to the skilled practitioner).
- The production of small molecules with certain spectroscopic properties (defined broadly to include visible light, microwaves, IR, UV, X-ray, etc.).
- Changes in the morphology of the cell, including the prevention or promotion of cell differentiation.
- The induction of apoptotic pathways.
- Chemical indicator.

### Diverse Genetic Patterns

Given that evolution is a statistical process it is necessary to provide sufficient genetic variation on which selection processes can act. In the present invention, this comprises two elements

- Providing a sufficiency large and diverse population
- Controlling the genetic basis of the diversity and how it expresses

Selection requires genetic diversity on which to operate. Thus the first requirement of the current invention is to provide a population of cells that embodies a genetic diversity. The term *"genetic diversity"* means that substantially all cells are different, in that they comprise different genes, and/or identical genes under control of different control system, such as different promoters, such that almost each cell initially represents a genotype not represented in any of the other cells. Of course due to cell division a few cells may be substantially identical.

The term "Cell Population" shall be taken to mean a population of cells where at least $10^4$ cells, such as at least $10^5$ cells, such as at least $10^6$ cells, such as at least $10^7$ cells, such as at least $10^8$ cells, such as at least $10^9$ cells, such as at least $10^{10}$ cells, such as at least $10^{11}$ cells, such as at least $10^{12}$ cells in the population represent a genotype not represented in any of the other cells.

Thus, the principle of the evolution method according to the invention is to obtain a population of cells having a very high genetic diversity.

One particular embodiment of this principle is to produce cells with combinations of concatemers comprising cassettes with expressible nucleotide sequences from a number of different expression states, which may be from any number of unrelated or distantly or closely related species, or from species from different kingdoms or phylae, novel and random combinations of gene products are produced in one single cell.

By inserting novel genes into the host cell, and especially by inserting a high number of novel genes from different expression states, such as from a wide variety of species into a host cell, the gene products from this array of novel genes will interact with the pool of metabolites of the host cell and with each other and modify known metabolites and/or intermediates in novel ways to create novel compounds. Due to the high number of substantially different cells that can be generated using the methods according to the present invention, for example at least $10^4$ cells, such as at least $10^5$ cells, such as at least $10^6$ cells, such as at least $10^7$ cells, such as at least $10^8$, such as at least $10^9$, for example at least $10^{10}$, such as at least $10^{12}$, it is more or less inevitable or at least likely that such large populations will lead to a sub-population having such an interaction. The sub-population having such interaction may comprise at most $10^{10}$ cells, such as at most $10^9$ cells, such as at most $10^8$ , such as at most $10^7$ cells, such as at most $10^6$ cells, such as at most $10^5$ cells, such as at most $10^4$ cells, such as at most $10^3$ cells, such as at most $10^2$ cells or just 10 cells.

**Generation of Novel Genetic Compositions**

It is a requirement of evolutionary processes that new patterns are generated either in parallel to or sequential to selection steps. In systems where the patterns are based on genetic elements this requires that either new genetic elements are introduced or new combinations of existing genetic elements are created or both.

In the present invention new patterns can be achieved through one or more of the following processes. The term combining or remixing shall be taken to mean a process of generating new combinations of expression constructs using one or more of these approaches. The combination or remixing may be conducted at any step of the selection process and a preferred timing is when cells having elements of the predetermined functionality have been found in at least one of the compositions, and preferably in at least 0.1%, such as at least 1%, such as at least 2%, such as at least 5%, such as at least 10% or at least 50% of compositions. The term Daughter Population shall be taken to mean a cell population that is predominately genetically descendant from those cells in one or more cell populations that had a fitness score above a certain threshold and that is further characterised by most of the cells in the daughter population having been generated through a remixing step.

In principle the combination or remixing may be conducted by at least the following approaches: physical isolation and remixing of expression cassettes, physical isolation and remixing of artificial chromosomes containing expression cassettes, sexual crosses, cell- or protoplast fusion (vide Hugerat Y, Spencer F, Zenwirth D, Simchen G (1994). Genomics 22(1), p. 108-117), and YAC-duction (vide Curran BP, Bugeja VC (1996), Methods Mol. Biol. 53, p 45-49.

Physical isolation of the expression cassettes and subsequently mixing the cassettes may be used together with the mating and protoplast/cell fusion methods of the present invention. One advantage of this approach is that any accumulating host mutations are removed by the remixing of genes into new host lines. Reporter genes can also be introduced as part of this process, allowing for the introduction of intracellular reporter assays. The remixing is preferably carried out in vitro by removing the expressible sequences from at least two different cells, combining the individual expressible sequences in vitro, and introducing at least two combined expressible sequences into at least two cells.

Due to the common structure of the expression cassettes according to a preferred embodiment of the invention, these may easily be excised from the host cells again using a restriction enzyme specific for the $rs_1$-$rs_2$ restriction site According to the present invention the enzyme specific for the $rs_1$-$rs_2$ restriction site is preferably a rare cutter therefore the likelihood of cutting host genomic DNA fragments with a size similar to the size of the expression cassettes is very limited. After excision the expression cassettes may be mixed with other expression cassettes of similar structure and be re-concatenated and re-inserted into another host cell in another combination creating a greater diversity during the evolution steps.

The combination of expressible sequences may of course also be a combination of full length chromosomes in the cells, such as combination of artificial chromosomes. Combination of the artificial chromosomes may be achieved in at least 4 ways depending on the host cells. These are physical isolation, crosses, protoplast fusion and YAC-duction as described herein.

An alternative way of physically remixing expression cassettes is to isolate the artificial chromosomes from one or more cell populations and re-transform new host cells. The host cells may or may not already contain artificial chromosomes containing expression cassettes.

**Addition of new genetic material.**

The remixing is preferably conducted with addition of new genetic material from another cell composition. The other composition may be chosen from compositions capable of expressing at least one predetermined phenotype, such as a protein or a metabolite, or it may be chosen at random.

In one embodiment it is desirable to conduct selection in a series of isolated populations that are then brought together once they have independently evolved useful traits. In this manner the use of independent selections for same phenotype provides different genetic backgrounds (a form of parallel evolution) that can then ideally act synergistically with each other.

In another embodiment the result of selection on two or more compositions is mixed at a certain step of evolution to create further modified compositions when aiming for at least one cell having the desired functionality.

Recombination of the expressible sequences, i.e. changes of the genetic material by for example cross-over, may be optionally avoided, due the construction of the genetic inserts, in particular spacer sequences, as well as due to a general attempt to suppress recombination in the cells. Thereby combination of the genetic material is favoured, leading to combination of intact genes or cDNA material, without the risk of destroying the function of the genetic material due to recombination.

After having obtained daughter populations exhibiting the desired functionality, the daughter population may then be subjected to further steps of screening and selection in order to optimise the cells.

**Novel Molecules and Pathways**

The aim of the evolution method according to the present invention is to evolve cells capable of producing new substances, such as new metabolites, new proteins, and/or having new pathways.

Thus, in a further aspect the present invention relates to a substance produced by the' cells evolved according to the present invention, said substance being metabolites, proteins, carbohydrates, poly- and oligosaccharides, and ribonucleic acids. Since some of the interactions that produce the novel phenotypes are mediated by enzymes it is likely that the result will include novel compounds with chiral centres, which are especially difficult to produce via chemical synthesis.

Creation of novel pathways, may lead to the capability of creating cells capable of metabolising, i.e. converting, a compound, which is not metabolisable by the native, un-evolved cell. Thus, in particular the substance is a metabolite.

**MULTIPLE PARAMETER SCREENING**

For a compound to be useable as a drug it must fulfil multiple functional requirements. It must interact with the target(s) and affect the function of the target in the desired manner. At the same time it should not interact with many other (often similar) targets, have major non-specific effects. And then it must further have the right physical-chemical parameters and be metabolised by the body in an acceptable manner.

Because of this intrinsic difficulty and complexity, the process of discovering and developing drugs has a very poor, success rate and is thus extremely expensive ($600mn per successful compound) and very time consuming (c. 8-12 years from discovery to clinic). Only c. 1 in 15 primary screens produce a compound that makes it into pre-clinical development and only 1 in 10 of these compounds then make it to market. The average pharmaceutical company spends 250 man-years of research and development effort for every compound that enters the clinic. Most pharmaceutical companies are, in consequence, failing to launch new drugs at the rate they require to satisfy their investors.

An alternative to the current process is the evolution of small molecules compounds towards multiple properties simultaneously, with these properties being related, either directly or indirectly to the therapeutic target(s) the small molecule has to interact with, the targets it should not interfere with, the ADMET properties it should fulfil, etc.

***Multiple Pharmacological Activities***

Due to the vast number of known targets and relationships between those targets that are currently known, it is not in the scope of the present invention to describe all know targets and their correlations. Table 1 discloses a list of relevant pharmacological targets.

Table 1: Drug targets

| |
|---|
| 3β hydroxysteroid dehydrogenase |
| 3-hydroxy-3-methylglutaryl coenzyme A |
| 5-adenosyl homocysteine hydrolase |
| 5-$HT_3$ receptor |
| 5-$HT_4$ receptor |
| 23S rRNA of the 50S ribosomal unit |
| 30S rRNA from 50S ribosomal unit |
| 50S ribosomal unit binding site |

(continued)

α2 antiplasmin
α-adrenergic receptor
α-subunit of $Na^+/K^+$ ATPase (3 isoforms)
α-amylase
α-glucosidase
ACTH receptor
Adenosine deaminase
Adrenocortical steroid synthesis
Adrenocorticosteroid receptor
Adrenergic receptor $\beta_1$, $\beta_2$
Adrenocorticotropic hormone
Androgen receptor
Angiotensin-converting enzyme (ACE)
Angiotensin II formation
Angiotensin II receptor
Antiplatelet/antithrombotic agent
Arginine vasopressin receptor
Angiotensin receptors, AT1, AT2
ATP-sensitive $K^+$channel
Antigcoagulant protein C
Antigcoagulant protein S
Androgen receptor
Apoptosis
Aminoacyl tRNA site on 30S ribosomal unit (tetracycline)
Acetylcholinesterase
Adrenergic receptors α1, α2, β1, β2, β3
Aromatase
ATP sensitive $K^+$ channels
Ascorbic acid

β-amyloid
β-adrenergic receptor
β-lactamase
β-subunit of DNA-dependent RNA polymerase
β-adrenergic receptors, b1
β-tubulin subunit of microtubules
Benzodiazepine receptor
Butyrylcholinesterase
Bradykinin receptors, $B_1$, and $B_2$

Carbonic anhydrase, type IV, II
$Ca^{2+}$ channel
$Ca^{2+}$ channel, Voltage-activated T-type
Catechol-O-methyltranferase
Calcitonin
Cell surface receptors for sulfonylureas on pancreatic β cells
*Cell surface receptors for glitinides on pancreatic* β *cells*

Cholecystokinin ($CCK_A$, $CCK_B$)
Choline acetyltransferase
Cholinesterase

(continued)

Carnitine
Calcinerin
Corticosteroid nuclear receptor
Cyclophilin, cyclosporin binding protein
$CD_3$ glycoprotein on T lymphocytes
*CD33 receptor*
*CD20 receptor*
CG-rich DNA (actinomycin)
Coagulation factor II, VII, IX, X
Corticosteroid adrenocorticotropin receptors
Cyclooxygenase 1, 2 (COX-1, COX-2)
Cyclic nucleotide phosphodiesterase
Cyclooxygenase
Cytochrome P450 reductase
Cytochrome P450 11β (11β hydroxylase)
Cytochrome P450 17α C17-20 lyase
Cytochrome P450 aldo, aldosterone synthase
Cytochrome P450 side chain cleavage (scc) enzyme
Cytochrome P450-dependent sterol 14 α-demethylase

$_D$-alanyl $_D$-alanine synthetase
Dihydropteroate synthetase
Deoxycytidine kinase
Dihydroorotate dehydrogenase
Dihydrofolate reductase
Dopamine D1-D5 receptors
DNA chain elongation factor
DNA cross-linking
DNA-dependent RNA polymerase
DNA gyrase, subunit a
DNA methylation
DNA polymerases I+III
DNA primase
DNA topoisomerase
DNA alkylation
DNA topoisomerase I, II and/or IV
DNA alkylation (oxamniquine)

Erythropoietin
Endo-β-d-glucuronidase
Estrogen receptor

Factors VII; VIII
Fusion protein (respiratory syncytial virus)
FKBP, tacrolimus binding protein, FK506 binding protein
Folic acid
Follicle-stimulating hormone (FSH)
FSH receptor

Glycerol phosphate oxidase
$GABA_A$ receptor (6α variants, 3β, 2δ, 3γ variants

(continued)

GABA transaminase
$GABA_A$-associated ion channel
Glutamic acid decarboxylase
Glutamate/aspartate receptors, AMPA, GLU 1-4, KA, GLU 5-7, NMDA $1,2_{A-D}$, mGLU 1-7
Glycinamide ribonucleotide transformylase
Granulocyte colony-stimulating factor receptor
GHRH receptor
*Glucagon receptor*
Glucoamylase
Glucocorticoid receptor (GR)
GnRH receptor
Gonadotropin releasing hormone (GnHR)
Guanylyl kinase
G-protein coupled adenosine receptor
Ganglionic adrenergic neurons/norepinephrine transporter
Guanylate cyclase (nitroprusside)
Guanylyl cyclase (NO)
Granulocyte colony-stimulating factor
Granulocyte-macrophage colony-stimulating factor
Growth hormone receptor
Growth hormone-releasing hormone (GHRH)
Glycine receptor $\alpha$, $\beta$

$H^+$, $K^+$ ATPase, proton pump
$H_1$ histamine receptor
$H_2$ histamine receptor
HCl secretion by gastric cells
Helicase
HIV Protease
HSV thymidine kinase
Hemoglobulin protease
Heparin antagonist
Hypoxanthine-guanine phosphoribosyl transferase
*Her-2* receptor
Histamine receptors $H_{1,}$ $H_2$, $H_3$
Hepatic sulfotransferase as a catalyst

Intercellular adhesion molecule 1
Interleukin 1 receptor
Interleukin (IL-1, -2, -3, -4, -5, -6, -7, -8, -9, -10, -11, -12
Interleukin-2 receptor
IGF-1 receptor, IGF-2 receptor
lodothyrinine-59-deiodinase, type 1, type 2
Influenza A virus $M_2$ protein
Inosine 5' phosphatedehydrogenase
Insulin-like growth factor 1
Interleukin-2 receptor

Inosinate dehydrogenase
Interferon a

(continued)

Interferon a receptor
Inosine monophosphate dehydrogenase
Integrase
Interferon $\alpha$
Interferon $\alpha$ receptor
Interferon $\gamma$
Insulin
Insulin-like growth factor (IGF-1, IGF-2)
Insulin receptor, $\alpha$ and $\beta$ subunits
Insulin transporter

Kallikrein, aprotinin, C-esterase, $\alpha$2 macroglobulin
Kinin

L-alanyl racemase
L-aromatic amino acid decarboxylase
L-type voltage-sensitive $Ca^{2+}$ channel
Leukocyte integrins
Leukotriene A hydrolase
Leukotriene $B_4$ receptor
Leukotriene $C_4$ receptor
Leukotriene C synthase
Leukotriene $D_4/E_4$ receptor
Lipocortin (protein), inhibits phospholiphase $A_2$
Lipoxygenases (12-lipoxygenase (platelets), 5-lipoxygenase (leukocytes)
LH/choriogonadotropin (CG) receptor
Luteinizing hormone (LH)
Lactamase
Lipoprotein lipase

$M_1$ receptor, muscarinic cholinergic
$\mu$ and $\delta$ receptor in gastrointestinal tract
Macrophage colony-stimulating factor
Microbial dihydrofolate reductase
Microtubular protein
Mineralocorticoid receptor
Mineralocorticoid receptor (MR)
Monoamine oxidase (MAO)-A
Monoamine oxidase (MAO)-B
Muscarinic receptor, $M_1$, 3 subunits
Muscarinic receptor, $M_2$, 3 subunits
Muscarinic receptor, $M_3$, 3 subunits
Muscarinic receptor, $M_4$, 3 subunits
Mycobacterial RNA polymerase

N-acyl hydrolase
$Na^+$ channel, $\alpha$1, $\beta$1, $\beta$3
$Na^+$ channel $\alpha$, $\beta$, $\gamma$
$Na^+$/Cl-symporter
$Na^+/K^+$/2Cl-symporter

(continued)

Niacin receptor
Nicotinic acid
Nicotinic receptor
Nicotinic cholinergic receptors, muscle $N_M$ α, β, δ, γ, ε
Nicotinic cholinergic receptors, neuronal, $N_N$ α2, α3, α4, α5, α6, α7, α8, α9, β2, β3, β4
Neuramidase
Neuropeptide Y, Y1, Y2 receptors
Noradrenaline transporter

Opioid receptors $\mu_{1-2}$, $\delta_{1-2}$, $\kappa_{1-3}$
Oxytocin & receptor

Platelet-derived growth factor
Parathyroid hormone (PTH)
Peroxidase
Progesterone receptor
Prolactin
Prolactin receptor
Parasite β-tubulin
Parasite dihydrofolate reductase
Parasite glutamate gated $Cl^-$ channel
Penicillin-binding protein 1a (PBP 1a, 1b), transpeptidase
PBP 2a, 2b
PBP 3, 4, 5, 6, 7
Platelet glycoprotein IIb/IIIa (fibrinogen receptor)
Plasma protein transferrin (β1 glycprotein)
Pyridoxine receptor
Penicilloyl enzyme
Peptidyl site of the 50S ribosomal unit
Primase
Phosphodiesterase (type IV, cyclic nucleotide phosphodiesterase)
Phospholiphase $A_2$, C
Platelet-activating factor
Prostacyclin synthase
Plasmodial heme polymerase
Progesterone receptor
Pyridoxine
Phospholipase Cβ
Purine receptors, P1 ($A_{1,2a,2b,3}$), $P_{2x}$, $P_{2Y}$
Peroxisome proliferator-activated receptor
Pancrelipase
Potassium channel
Prostaglandin 15-OH dehydrogenase
Prostaglandin D-DP receptor
Prostaglandin E1, E2, E3-EP receptor
Prostaglandin F-FP receptor
Prostaglandin I2-IP receptor
Prostaglandin $I_2$ ($PGI_2$) receptor
Prostaglandin $F_2$ receptor
Prostaglandin synthetase

(continued)

Prostaglandin $I_2$ receptor
Reverse transcriptase
Ribosomal protein from 50S ribosomal unit (streptomycin)
Rh 0
Riboflavin receptor
Retinoic acid α, X receptors
Ribonucleoside diphosphate reductase
Ribonucelotide reductase

Somatostatin
Somatostatin receptors, several
Steroid 5 α reductase 1, 2
Sucrase
Squalene epoxidase
Stem cell factor, c-kit ligand
Serotonin receptors (5-HT) 5-$HT_{1A-F,}$ 5-$HT_{2A-C}$, 5-$HT_3$, 5-$HT_{4-7}$
Succinic semialdehyde dehydrogenase
Spindle formation
Scission of DNA
Secretion of vasopressin K receptor

Topoisomerase I, II, III, IV
Tubulin
Thrombopoietin
Thrombin
Tissue plasminogen activtor
Thymidylate synthetase
Tachykinins, NK1, NK2, NK3
Tryptaminergic receptor
Thromboxane $A_2$ TP receptor, platelet and non-platelet
Thromboxane synthase
Thyroid-stimulating hormone (TSH) receptor, TRα 1,2, TRβ 1,2
Tumor necrosis factor receptor
Trypanothione reductase
Type I cyclic nucleotide phosphodiesterase
Type III cyclic AMP phosphodiesterase
Type V cyclic nucleotide phosphodiesterase
Transpeptidase
Thymic lymphocyte antibodies
Tumour necrosis factor alpha
Thiamine

Uridine monophosphate pyrophosphorylase

Vascular cellular adhesion molecule 1 receptor
Vasopressin receptors $V_{1a}$, $V_{1b}$, $V_2$,
Viral DNA polymerase
Vitamin A nuclear receptor
Vitamin E
Vitamin K & receptor
Vitamin $B_{12}$ receptor

(continued)

| |
|---|
| Vitamin D nuclear receptor |
| Voltage-activated $Ca^{2+}$ channel, L-type |

Below are examples of diseases and the different targets involved in these diseases. It is also presented in outline examples of how new potential drugs for these targets would be screened using the present invention.

**1. Disease Target: Bacterial infections (inhibition of DNA Polymerase III, P450 inhibition and Multi-drug resistance *S. aureus* growth inhibition)**

The widespread emergence of resistance has significantly limited the efficacy of classical antibiotic therapy for bacterial disease. Fuelled largely by the excessive and often unnecessary use of antibiotics in humans and animals, antibiotic resistance has resulted in increased patient morbidity, mortality and overall cost of health care. Methicillin-resistant *Staphylococcus aureus* (MRSA) is now the most prevelant nosocomial pathogen in the United States, and the enterococci, as opportunistic pathogens, are among the top four causes of nosocomial infection. Indeed, the percentage of enterococcal isolates resistant to essentially every antibiotic, including vancomycin, continues to increase. Thus a premium is placed upon the discovery of inhibitors that function by a novel or at least different mechanism than currently approved antibiotics, as these would be expected to circumvent current bacterial resistance mechanisms.

*S. aureus* is a very important human pathogen and has favorable growth characteristics for use in high-throughput screening. Use of an antibiotic-resistant strain will *a priori* select for hits that have activity against a multi-drug resistant strain.

DNA Polymerase III is a DNA polymerase-exonuclease (Pol-Exo) that is essential for the replicative DNA synthesis of Gram positive organisms. Since DNA Pol III is essential for the replication of Gram positive bacteria, the inhibition of DNA Pol III offers a specific and alternative way to treat antibiotic resistant gram positive bacteria.

Many patients with severe disease may be administered multiple anti-infectives as well as other drugs to treat (non-infectious) underlying disease. In this case, drug classes that are not metabolized via the major P450 liver enzymes are preferable.

Desired therapeutic profile:

- *Gram positive-specific:* Systemic administration of agents with a very broad spectrum has the undesired effect of creating resistance in the normal host Gastrointestinal flora. Therefore, more disease-specific antibiotics might have an advantage in gaining hospital formulary approval and overall wider acceptance.
- *Orally-active:* The ideal drug candidate would be orally-active with additional formulations for intravenous use. Multiple dosing is acceptable however anything approaching continuous infusion requires very careful consideration. Improvement or equivalence with dosing regimens of competitive therapies is important.
- *Safety*: The ideal drug candidate would be microorganism-specific and devoid of significant side-effects and drug interactions within at least 10-fold of $C_{max}$ in the therapeutic dosing range.

Multiple parameter screens:

A multiple parameter screen would thus include S. Aureus growth inhibition, DNA Polymerase III inhibition and P450 inhibition. A screen could be assembled by, for example, transforming a library of producer strains with GFP reporter systems for a few selected human P450s and for recombinant Bacillus subtilis DNA Pol III. The library would then be plated and overlayed with an MRSA strain. An assay where the compounds have to cross the producer's cell wall and reach the reporter strain will also select for compounds that have a reasonable solubility profile.

Figure 3 exemplifies such a multiple parameter screen where producer cells in zones cleared of MRSA cells and which produce the desired combination of fluorescent colours would be selected.

**2. Disease target: Cancer - Inhibition of solid tumour growth and prevention of metastasis (inhibition of NF-**

**κB, inhibition Cox-2, no inhibition Cox-1)**

Cancer is the second leading cause of death in the US, causing one in every four deaths. Existing treatments for surgically inoperable cancers include chemotherapy and radiation treatments. These are highly toxic because they are non selective or at best only partially selective. There exists a critical need for new therapeutics to inhibit tumor growth and prevent metastasis. A premium is placed upon molecules that prevent metastasis and which work through a selective mechanism so as to avoid or minimize side effects.

Nuclear Factor κB (NF-κB) is a transcription factor that, by regulating the expression of multiple inflammatory and immune genes, plays a critical role in host defense and several pathogenic processes. Its most common inducible form is composed of the proteins p65 and p50, and usually exists as a molecular complex with one of several inhibitory molecules, the IκBs, in the cytoplasm. Proteins that are regulated by NF-κB include TNFα, IL-1β, IL-2, IL-6, IL-8, iNOS, COX-2, intercellular adhesion molecule-1 (ICAM-1), vascular-cell adhesion molecule-1 (VCAM-1) and E-selectin (Cancer J., 1998, 4, S92; Int. J. Biochem. Cell. Biol., 1997, 29, (6), 867).

Activation of NF-κB can lead to the synthesis of the inducible form of cyclooxygenase (COX-2). This enzyme has a critical role in the response of tissues to injury or infectious agents and are essential components of the inflammatory response, the ultimate repair of injury, and carcinogenesis. Several population-based studies have detected a 40-50% decrease in relative risk for colorectal cancer in persons who regularly use Aspirin and other NSAIDs. Attempts to determine the molecular basis for these observations found that both human and animal colorectal tumors express high levels of COX-2, whereas the normal intestinal mucosa has low to undetectable COX-2 expression. These findings led to the hypothesis that COX-2 plays a role in colon cancer growth and progression (Faseb, 1998, 12, 1063). Since Aspirin also inhibits NF-κB these findings also suggest that inhibiting NF-κB may prevent tumour growth and progression. Another way in which COX-2 seems to be involved in cancerinogenesis is by protecting cells from apoptosis (J. Nat. Cancer Inst., 1998, 90, (11), 802). Therefore, inhibition of NF-κB can help control tumor growth by one further process since it leads to less COX-2 induced protection from apoptosis. Inhibition of NF-κB also leads to an increase in Tumor Necrosis Factor (TNF) which in turn leads to an increase in apoptosis.

Immense effort is being devoted to developing new molecules that are direct inhibitors of the enzymatic activity of COX-2. However, an alternative approach is to find new agents that can prevent expression of the respective genes coding for the activities since there are already examples that inhibition of a single mediator does not eliminate all symptoms of a disease (Inflamm. Res., 1997, 46, 282).

Desired therapeutic profile:

- *Selective NF-κB inhibitor:* There are several drugs that act by partial inhibition of NF-κB but they all produce side effects due to interactions with other targets. Any new NF-κB inhibitor would have to be selective.
- *Selective COX-2 inhibitor with a Cox-2/Cox-1 differential inhibitory activity as low* as *possible*: Prostanoids that are derived from the COX-1 pathway regulate platelet aggregation via thromboxane A2, the function and integrity of gut mucosa, and kidney function via prostaglandin E2 and prostacyclin. Cox-2 is expressed in various cell types, including monocytes, fibroblasts and synovial cells, in response to inflammatory stimuli. Consequently, COX-1 inhibition by NSAIDs is associated with gastrointestinal and renal toxicity, whereas, COX-2 inhibition limits the formation of pro-inflammatory at the site of the inflammatory response and has anticancer effects.
- *Orally-active:* Given the severity of the medical problem, an orally-active drug would be desired but not essential.
- *Safety:* The ideal candidate would be selective and devoid of significant side-effects and drug interactions. However again, given the severity of many cancers and lack of therapeutic options, there is significant history of compounds that are less-than-ideal in these parameters.

Multiple Parameter Screen:

A multiple parameter screen set up could for example be the double gel encapsulation of a producer species library with 2 different mammalian cell lines. The first gel capsule would contain the producer cell and a mammalian cell reporting NF-κB and Cox-1 inhibition while the second capsule would contain a second mammalian cell line reporting Cox-2 inhibition. Gel droplets producing the desired fluorescence output would be selected.

**3. Disease target: Cancer (survival in the presence of DNA Topoisomerase II α poisons, no production of DNA**

**double strand breaks and inhibition of human DNA topoisomerase II activity)**

Chemotherapy is one of the most common approaches to the treatment of cancer. All chemotherapy drugs interfere with cell growth, and they all have some form of side effects. These vary from the highly undesirable to side effects so severe as to prevent further chemotherapy.

An underlying problem of chemotherapy is that cancer cells are not that different from normal undifferentiated or fast growing tissues and therefore, killing a cancer cell tends to kill such cells as well. This side effect effectively limits the dose at which the chemotherapeutic can be applied, and hence limits the efficacy that can be achieved. Consequently, there is a need for the development of novel chemotherapeutic agents to overcome these central problems of cancer chemotherapy.

The most common way to address the above problems of cancer chemotherapy is to look for compounds or delivery systems that increase the specificity for cancer cells. However an alternative approach is to use compounds that protect vulnerable normal tissue against the proposed chemotherapeutic agent. Such protectants should of course not be harmful to the normal cells and either not reach, or not be functional in the cancer cells. A number of protectant approaches are in clinical use to day.

Many chemotherapeutic agents, e.g., Doxorubicin and Etoposide have a large part of their toxicity (and hence clinical utility) due to the specific way in which they "poison" the enzyme Topoisomerase II, an enzyme with a crucial role in the elongation and termination stages of DNA replication. Rather than blocking the enzyme specifically, these drugs stabilise an intermediate DNA/enzyme/drug complex, creating double-stranded breaks in the DNA of treated cells. A second class of structures act by blocking the Topoisomerase II catalytic cycle at other points in the cycle and do not create double-stranded DNA breaks. These two types of compounds are antagonists to each other since they stabilise different points in the cycle. If one binds, the other cannot. Therefore, inhibitors of Topo II can be used to offset the effects of Topo II poisons.

Two highly homologous isoforms of mammalian topoisomerase II have been identified in tumor cells, topoisomerase II $\alpha$ (170 kDa) and topoisomerase II $\beta$ (180 kDa) (Malonne, H. and Atassi, G., Anti-Cancer Drugs, 1997, 8, 811-822). The two isoforms differ in several biochemical and pharmacological properties, such as optimal salt concentration for *in vitro* catalytic activity, thermal stability and sensitivity to teniposide (a non-intercalative DNA topoisomerase II poison). Topoisomerase II $\alpha$ is the major drug target isoform in mammalian cells (Sehested et al, Cancer Research, 1998, 58, 1460-1468.

The discovery of new inhibitors of DNA topoisomerase II would enable the protection of certain vulnerable tissues against Topo II poisons and hence expand the efficacy of existing chemotherapy drugs and reduce side effects.

<u>Desired therapeutic profile:</u>

- *DNA topoisomerase II a inhibitor:* Any new compound would have to be an inhibitor and not a poison of the enzyme.
- *Reversible inhibition of normal cell growth:* The effects of the drug should only last long enough to off set the effects of the chemotherapeutic agent
- *Orally-active:* Given the severity of the medical problem, an orally-active drug would be desired but probably not essential.
- *Safety:* The ideal candidate would have modest toxicity such that it does not by itself place an additional toxicity burden on the patient.

<u>Multiple Parameter Screen:</u>

A preferred example of a' multiple parameter screen set-up for cancer chemoprotectant is illustrated in Fig. 4b. In the assay, a producer species library is encapsulated so that on average each capsule has 1 cell and allow to grow for a few generations. These clonal lines are then double encapsulated with a permeabilised yeast that contains a human DNA Topo II $\alpha$ reporter system. The gel droplet environment contains etoposide (a poison) and a DNA double strand break stain. Gel droplets where the yeast cells in the outer layer have survived and that do not fluoresce or are stained are selected.

**4. Disease target: Diabetes (ligand activation of RXR$\alpha$, ligand specific activation of RXR-PPAR$\gamma$, adipocyte**

**differentiation).**

Type 2 diabetes is one of the most common chronic diseases and is associated with co-morbidities, such as obesity, hypertension, hyperlipidemia and cardiovascular disease.

Peroxisome proliferator-activated receptors (PPARs) and retinoid X receptors (RXR) are transcription factors belonging to the family of ligand-inducible nuclear receptors. There are three related but distinct PPARs called PPAR-alpha, PPAR-beta/delta and PPAR-gamma that form heterodimmers with RXR. These receptors regulate expression of genes involved in fat and carbohydrate metabolism. RXR is unique among retinoid receptors as it can form homo- or heterodimers with multiple nuclear receptors including PPARs, retinoic acid receptors (RARs), vitamin D receptor, and thyroid hormone receptor.

PPARγ/RXR regulates adipogenesis and insulin sensitivity both when activated by PPARγ ligands and/or RXR ligands. For example, insulin sensitizers, such as the drugs from the thiazolidinedione class (TZDs), exert their antidiabetic effects through a mechanism that involves activation of the gamma isoform of the nuclear receptor of the peroxisome proliferator-activated receptor (PPARγ).

Activation of RXRα increases activation of PPARγ and insulin sensitivity. Clinical studies show that co-administration of retinoids (LG100268) +TZDs increases insulin sensitivity and glucose uptake by 60%

The retinoid receptors mediate the biological effects of natural and synthetic vitamin A derivatives, such as retinoic acid. RXR ligands interact with many different proteins, including members of the following protein families: RXR, RAR, retinoic acid receptor-related orphan receptor (RZR), cytoplasmic retinoic acid-binding proteins, retinal-binding protein, P-glycoprotein and cytochrome P450. The expression level of each of these proteins is likely to affect the potency and efficacy of retinoids in various cell types.

Rexinoids may have undesirable effects mediated by RXR homodimers or heterodimers partners other than PPARγ. Rexinoids for treating type 2 diabetes should thus be selective for the PPARγ/RXR heterodimer.

<u>Desired therapeutic profile:</u>

- *Selectivity:* RXR agonists should be selective for the PPARγ/RXR heterodimer.
- *Orally-active:* The ideal drug candidate would be orally-active.
- *Safety*: The ideal drug candidate would be devoid of significant side-effects and drug interactions.

<u>*Multiple Parameter Screen:*</u>

A multiple parameter screen set up could be the gel encapsulation of a producer species library reporting RXR-RXR activation with a mammalian cell line reporting PPARγ-RXR activation as well as P450 inhibition. Gel droplets that indicate PPARγ-RXR activation but not RXR-RXR or P450 inhibition are selected. Figure 5 exemplifies such a system.

***Absorption, Distribution, Metabolism, Excretion & Toxicity (ADMET)***

Major reasons for the failure of lead compounds in development often involve inappropriate kinetics or toxicity, thus there is a strong need to obtain the relevant information as early as possible in the discovery process in order to spend as little as possible on inadequate compounds. The pharmaceutical and biotech industries are thus currently focusing on transforming the traditionally very low throughput processes of physicochemical, pharmacokinetic and toxicity optimization studies into high throughput selection methods in order to obtain the relevant information as early in the discovery process as possible.

Though the use of evolutionary strategies and cell based systems, the present invention enables the inclusion of ADMET requirements in the lead generation process and thus reduces significantly the production and screening of thousands of compounds that are not drug like.

*Solubility*

For drugs to be effective they must be able to reach their targets in effective amounts. In cell free assays the only limitation that exists in this regard is the compound's solubility in the assay buffer. In cell-based assays with intracellular targets, the ability of compounds to diffuse across cell membranes is dependent on their ability to partition into and out of lipid-rich membranes. This process is more efficient when compounds have a certain degree of lipophilicity in addition to being sufficiently water-soluble. If the cell culture medium contains proteins (such as from the presence of fetal calf serum) the degree of binding of the compound to serum proteins influences the freely diffusible fraction of compound and hence the amount available for interaction with the target.. The extent of drug binding to serum proteins has a number of important implications in the living organism including transport and distribution.

The present invention uses a host species to produce the compounds. In a preferred embodiment it uses assays external to the producer species. Thus it is an inherent part of the process to evaluate the ability of compounds to diffuse across cell membranes. The presence of medium proteins is also an inherent part of the system.

Another aspect of the invention is the control of the expression of the host's drug resistance pumps. This control allows significant pumping of the compounds produced in the first rounds of screening, when the solubility of compounds produced is not a key selection criteria. In later rounds of screening the expression of the pumps will be progressively turned off in order for the compounds that reach the disease targets to have to cross the host's cell membrane and thus have a reasonable solubility profile.

*Absorption*

The preferred route of drug delivery is oral administration. The intestinal membrane permeability is a critical characteristic that determines the extent and rate of drug absorption and ultimately the bioavailability. Other cells which are relevant for drug uptake indluce epithelial, epidermis, nasal, blood-brain and blood-testis barriers, as well as the kidney, liver, intestinal epithelial and lung cells; which are also routes for uptake of drugs.

Most models of absorption involve the use of cultured, immortalised cells, which are generally intestinal in nature and which give a good correlation with absorption in vivo. Most notable among them are CaCO-2 cells that derive from a human colon carcinoma cell line or a subclone of the Caco-2 cell line, TC7. Other usefull cell lines for absorption studies are dog kidney cell line, the Madin-Derby Canine kidney cell line (MDCK) and everted intestinal rings and brush-border membrane vesicles (BBMV). These cell lines are grown in a confluent monolayer and used for permeability measurements which are based on the rate of appearance of test compound in the receiver compartment. The apical (donor) surface of the monolayer contains microvilli and thus retains many characteristics of the intestinal brush border. Furthermore, the apically located efflux pump, P-glycoprotein, the monocarboxylic acid transported, the dipeptide transporter, the transporter for large neutral amino acids (LNAA) [Inui K-I, Yamamoto M, Saito H. T, J Pharmacol Exp Ther, 1992; 261: 195-201; Lu S, Guttendorf RJ, Stewart BH, Pharm Res, 1994; 11: S-258.] and metabolic enzymes [Bjorge S, Halelehle KL, Homan R, Rose SE, Turluck DA, Wright DS., Pharm Res, 1991; 8: 1441-1443] are all functionally expressed.

Figure 6 shows an example of a multiple parameter screen for absorption and a pharmacological activity. Using a dual culture system and timing the time of cell selection, it is possible to select producer cells that have the desired pharmacological activity and a good absorption profile.

Drugs that inhibit P-glycoprotein can alter the absorption, disposition and elimination of co-administered drugs and can enhance bioavailability or cause unwanted drug-drug interactions. Thus another important aspect of absorption studies is to determine if a compound is a PGP inhibitor by a direct measure of inhibition of PGP-mediated digoxin transport across polarized human PGP cDNA -expressing LLC-PK1 cell monolayers.

In mammals, the ABC transporters, like MDR1 and MRP1, have a key role in the functioning of the blood-brain and blood-testis barriers, as well as the kidney, liver, lung, and intestinal epithelial cells. MDR1 is expressed normally on apical membranes of cells derived from excretory tissues, as well as on the luminal surface of cerebral capillary cells (*Gottesman et al., 1993; Cordon-Cardo et al., 1989*). MDR1 and MRP1 are present in the epithelia of the choroid plexus (CP) and both transporters participate in the blood-CSF permeation barrier *(Rao et al., 1999).* MDR1-Pgp contributes to the drug-permeation barrier in cerebral capillary endothelial cells and takes part in elimination of organic cations and xenobiotics from the central nervous system (CNS) (*Rao et al., 1999; Schinkel et al., 1997).* MRP1 contributes to the basolateral broad-specifity drug-permeation barrier in CP, protects this epithelium from xenobiotics and extrudes organic anions and probably also some hydrophobic compounds from the CSF (*Wijnholds et al., 2000).* Some ABC transporters form and regulate specific membrane channels, while others are involved in the elimination of detoxified drug- conjugates, transport of phospholipids or bile acids, and even the initiation of antiviral immune-reaction or specific self-destruction in various cell types. Moreover, members of the ABC transporter family were shown to provide multidrug resistance in pathogenic bacteria and parasites (e.g. Plasmodium and Leishmania species), while also allowing multixenobiotic resistance (MXR) in a large variety of organisms living in a chemically polluted environment *(Kurelec et al., 1989; 1992).*

In order to predict the penetration of a compound through different pharmacological barriers, a wide range of ABC transporters-compound interactions are also being tested, e.g., Pgp/MDR1, MRP1, MRP2, MDR3, MRP3, MRP5, MRP6, MXR (BCRP, ABCG2).

*Metabolism*

A drug, once it enters an organism, can experience a variety of biological fates. Drug metabolizing enzymes, including cytochromes P450, (present at high levels in liver, kidney, gut and other organs), can catalyze the chemical conversion of a particular drug to entities (metabolites) which are more aqueous-soluble and more readily excreted than the parent drug from which they were derived. If a parent drug is inherently metabolically unstable, undesirable pharmacokinetic behavior, such as an inappropriately short duration of action or poor oral bioavailability, can be observed. It is therefore, common practice in the industry to gain knowledge about the metabolic stability of lead candidates in order to identify compounds that may turn out to have poor pharmacokinetic profiles.

In addition, studies in drug metabolism can address the issue of possible drug-drug interactions, which are closely linked to the safe use of drugs in polytherapies. Most undesirable drug-drug interactions occur when two or more compounds compete for the same drug-metabolizing enzyme. The result is usually altered pharmacokinetics for one or more of the compounds involved, sometimes accounting for compound blood levels which are outside of the therapeutic window. These types of interactions can be foreseen with the assistance of studies of the inhibitory effects of test compounds with specific drug metabolizing enzymes.

Various *in vitro* methods are available which are being increasingly incorporated into drug discovery strategies. Among the most popular and widely utilized systems in use today are hepatic microsomes. These preparations retain activity of those enzymes that reside in the smooth endoplasmic reticulum, such as cytochromes P450 (CYP), flavin monooxygenases (FMOs), sulfotransferases, UDP-glycosyl transferases, glutathione transferases and N-acetyl transferases. Isolated hepatocytes appear to retain a broader spectrum of enzymatic activities, including not only reticular systems, but cytosolic and mitochondrial enzymes as well. Liver slices, which like hepatocytes retain a wide array of enzyme activities, are also increasingly used. Furthermore, both hepatocytes and liver slices are capable of assessing of enzyme induction *in vitro.* Isolated heterologous human CYP enzymes have been available for several years, being expressed from cDNA in yeast (Saccharomyces cerevisiae), bacterial *(Escherichia coli),* and mammalian (B-lymphoblastoid) cell lines [Ohgiya S, Komori M, Fujitani T, Miura T, Shinriki N, Kamataki T., Biochem Int, 1989; 18: 429-438; Winters DK, Cederbaum Al, Biochim Biophys Acta 1992; 1156: 43-49; Crespi CL, Gonzalez FJ, Steimel DT, Turner TR, Gelboin HV, Penman BW, Langenbach R, Chem Res Toxicol, 1991; 4: 566-572]. These systems have been used to ascertain whether a compound is a substrate for a particular CYP isozyme and, if so, what metabolite is generated by that enzyme.

Assays using recombinant human cytochromes P450, (including CYP2D6 &CYP2C19 that are polymorphically-encoded) as well as assays using isozyme-specific substrate and metabolite combinations in liver microsomal preparations can provide valuable information regarding a test compound's drug-drug interaction potential.

In the present invention the generation of small molecules is carried out by host cells that can themselves be transformed with a range of enzymes involved in human metabolism. These minimises the number of false positives generated by compounds that are rapidly metabolised by the human DMEs and also leads to the discovery of compounds that are active after being metabolised and which would otherwise remain undiscovered. (see figure 7).

In another aspect of the invention the drug metabolising enzymes are included extracellular to the small molecule producer cell in either cell free or cell based assays. When using cell based assays, one preferred approach is the use of hepatocytes.

In yet another aspect of the invention, the drug metabolising enzyme(s) are associated with reporter systems in order to gain information on enzyme inhibition. Alternatively competition assays for drug-drug interactions can be carried out.

In specific cases, some of the dug metabolising enzymes are themselves the disease targets since several of these enzymes are known to be associated with several diseases.

Conceptually, the drug metabolizing enzymes are divided into two groups. Oxidative drug metabolizing enzymes, which include CYP450s and FMOs, catalyze the introduction of an oxygen atom into substrate molecules, generally resulting in hydroxylation or demethylation. The conjugative enzyme families include the UDP-glycosyltransferases (UGTs), glutathione transferases (GSTs), sulfotransferases (SULTs), and N-acetyltransferases (NATs). The conjugative drug metabolizing enzymes catalyze the coupling of endogenous small molecules to xenobiotics that usually results in the formation of soluble compounds that are more readily excreted.

<u>Cytochrome P450s</u>

Cytochrome P450 proteins in humans are drug metabolizing enzymes and enzymes that are used to make cholesterol, steroids and other important lipids such as prostacyclins and thromboxane A2. These last two are metabolites of arachidonic acid. Mutations in cytochrome P450 genes or deficiencies of the enzymes are responsible for several human diseases. Induction of some P450s is a risk factor in several cancers since these enzymes can convert procarcinogens to carcinogens.

CYP450 enzyme in the liver catalyze the initial step in the biotransformation of xenobiotic compounds, including most drugs. These enzymes are members of a large family of mixed-function oxidases that catalyze the introduction of an oxygen atom into substrate molecules, often resulting in hydroxylated or dealkylated metabolites. The metabolism takes place in two phases. Phase I is chemical modification to add a functional group that can be used to attach a conjugate. The conjugate makes the modified compound more water soluble so it can be excreted in the urine. Many P450s add a hydroxyl group in a Phase I step of drug metabolism. The hydroxyl then serves as the site for further modifications in Phase 2 drug metabolism.

More than fifty CYP450 isozymes are known to exist in humans and they have been classified into 18 families and 43 subfamilies based on amino acid sequence similarities. Proteins from the same family are greater than 40% identical at the amino acid level, while those in the same subfamily are greater than 55% identical (Nelson, D.R. (1999) Arch. Biochem. Biophys. 369:1-10). In the standard nomenclature, the family is designated by a number followed by a letter designation for the subfamily, and a second number that identifies the individual member of that subfamily.

CYP1 drug metabolism (3 subfamilies, 3 genes, 1 pseudogene)
CYP2 drug and steroid metabolism (13 subfamilies, 16 genes, 16 pseudogenes)
CYP3 drug metabolism (1 subfamily, 4 genes, 2 pseudogenes)
CYP4 arachidonic acid or fatty acid metabolism (5 subfamilies, 11 genes, 10 pseudogenes)
CYP5 Thromboxane A2 synthase (1 subfamily, 1 gene)
CYP7A bile acid biosynthesis 7-alpha hydroxylase of steroid nucleus (1 subfamily member)
CYP7B brain specific form of 7-alpha hydroxylase (1 subfamily member)
CYP8A prostacyclin synthase (1 subfamily member)
CYP8B bile acid biosynthesis (1 subfamily member)
CYP11 steroid biosynthesis (2 subfamilies, 3 genes)
CYP17 steroid biosynthesis (1 subfamily, 1 gene) 17-alpha hydroxylase
CYP19 steroid biosynthesis (1 subfamily, 1 gene) aromatase forms estrogen
CYP20 Unknown function (1 subfamily, 1 gene)
CYP21 steroid biosynthesis (1 subfamily, 1 gene, 1 pseudogene)
CYP24 vitamin D degradation (1 subfamily, 1 gene)
CYP26A retinoic acid hydroxylase important in development (1 subfamily member)
CYP26B probable retinoic acid hydroxylase (1 subfamily member)
CYP26C probabvle retinoic acid hydroxylase (1 subfamily member)
CYP27A bile acid biosynthesis (1 subfamily member)
CYP27B Vitamin D3 1-alpha hydroxylase activates vitamin D3 (1 subfamily member)
CYP27C Unknown function (1 subfamily member)
CYP39 unknown function (1 subfamily member)
CYP46 cholesterol 24-hydroxylase (1 subfamily member)
CYP51 cholesterol biosynthesis (1 subfamily, 1 gene, 3 pseudogenes) lanosterol 14-alpha demethylase

The bulk of drugs are metabolised by a few members of the CYP1, 2, and 3 families arid the metabolism occurs primarily in the liver, which contains the highest concentration of CYP450 in the body. However, the importance of extrahepatic metabolism in tissues such as the intestine and lung is also recognized.

The xenobiotic metabolizing P450s are approximately 50 kDa proteins anchored in the endoplasmic reticulum (ER) by a single transmembrane helix in the N-terminus. Cell fractionation using differential centrifugation results in particulate preparations enriched in endoplasmic reticulum, commonly referred to as microsomes. Detailed examination of microsomal fractions from many different individuals has demonstrated significant variability in expression patterns of individual isozymes, however some generalizations are possible (Guengerich, F.P. (1995) Cytochrome P450: Structure, Mechanism, and Biochemistry (Second Edition), Chapter 14, edited by Paul R. Ortiz de Montellano, Plenum Press, New York, Shimada, T., et al. (1994) J. Pharmacol. Exp. Ther. 270:414-23). On average, 70% of the P450s expressed in adult human liver consist of the following isozymes: 1 A2, 2A6, 2B6, the 2C subfamily (2C8, 2C9, 2C18, and 2C19), 2D6, 2E1, and the 3A subfamily (3A4 and 3A5).

Another very important aspect of the P450s is that polymorphisms cause significant differences in drug metabolism from population to population and individuo to individuo. A polymorphism is a difference in DNA sequence found at 1% or higher in a population. These differences in DNA sequence can lead to differences in drug metabolism, so they are important features of P450 genes in humans. CYP2C19 has a polymorphism that changes the enzyme's ability to metabolize mephenytoin (a marker drug). In Caucasians, the polymorphism for the poor metabolizer phenotype is only seen in 3% of the population. However, it is seen in 20% of the asian population. Because of this difference, it is important to be aware of a person's race when drugs are given that are metabolized differently by different populations. Some drugs that have a narrow range of effective dose before they become toxic might be overdosed in a poor metabolizer.

A cytochrome P450 allele website is available from Sweden at http:/www.imm.ki.se/CYPalleles/

Another aspect of the current invention is the ability to evolve drugs designed for specific populations or even individuos since the drug metabolic aspects are addresses during the drug generation process.

Oxidation of organic molecules by P450s is quite complex (Ortiz de Montellano, P.R. (1995) Cytochrome P450: Structure, Mechanism, and Biochemistry (Second Edition), Chapter 8, edited by Paul R. Ortiz de Montellano, Plenum Press, New York), but the overall reaction can be represented simply by **Equation 1:**

**Equation 1: RH + O2 + NADPH + H+ →ROH + H2O + NADP+**

An electron from NADPH is transferred via the flavin domain of NADPH-P450 reductase to the heme domain of the CYP450 where the activation of molecular oxygen occurs. Substrates react with one of the oxygen atoms and the other is reduced to water. In some cases, the second electron can come from NADPH via cytochrome *b*5 reductase and cytochrome b5. During *in vitro* reconstitution experiments, cytochrome *b*5 can stimulate metabolism of various substrates by some CYP450 isozymes, notably 3A4, 2E1, and 2C9. However, the mechanism of this stimulation is not clearly understood. Apocytochrome b5 was shown to be as effective as the holoenzyme in stimulating reconstituted CYP3A4 reactions, so at least in this instance, it does not appear to be playing a direct role in electron transfer (Yamazaki, H., et al. (1996) J. Biol. Chem. 271:27438-44). The most widely held hypothesis is that cytochrome b5 acts allosterically to enhance the interaction between CYP450 and NADPH-P450 reductase, or it improves substrate binding.

*Flavin Monooxygenases (FMOs)*

Flavin monooxygenases, like the CYP450 enzymes, are associated with the endoplasmic reticulum and catalyze the oxidation of organic compounds using molecular oxygen and NADPH as the source of electrons for the reduction of one of the oxygen atoms **(Equation 1)**. However, they are mechanistically distinct from the CYP450s in that they react with oxygen and NADPH in the absence of substrate to form a 4$\alpha$-hydroperoxy flavin enzyme intermediate. Thus, the FMOs exist in an activated form in the cell, and their interaction with a nucleophilic group such as an amine, thiol, or phosphate, is all that is required for completion of the catalytic cycle (Rettie, A.E. and Fisher, M.B. (1999) in Handbook of Drug Metabolism, pp131-147, edited by Thomas F. Woolf, Marcel Dekker, Inc, New York). The capacity to remain stable while poised in an activated state is a possible explanation for the extremely broad substrate specificity of the FMO isozymes. It has been proposed that essentially all of the energy required for catalysis is captured in the oxygen-activated intermediate, and that alignment or distortion of the substrate molecules is not required (Ziegler, D.M. (1993) Annu. Rev. Pharmacol. Toxicol. 33:179). It follows that the active site of FMOs is much less sterically defined than for other enzymes. FMO3 is the most abundant form in human liver and is believed to be the dominant member of this enzyme family in terms of overall drug metabolism (Rettie, A.E. and Fisher, M.B. (1999) in Handbook of Drug Metabolism, pp131-147, edited by Thomas F. Woolf, Marcel Dekker, Inc, New York).

*UDP glycosyltransferases (UGTs)*

UDP glycosyltransferases catalyze the glucuronidation of xenobiotics at hydroxyl, carboxyl, amino, imino, and sulfyhydryl groups using UDP-glucuronic acid as a donor molecule **(Equation 2)**. In general, this generates products that are more hydrophilic and thus more readily excreted in bile or urine.

**Equation 2: UDP-glucuronic acid + R →UDP + R-glucuronide**

Although glucuronidation generally is classified as Phase II metabolism - the phase occurring after CYP450 dependent oxidative metabolism - many compounds do not require prior oxidation because they already possess functional groups that can be glucuronidated. Examples of first-pass metabolism catalyzed by UGTs include the UGT2B7-dependent glucuronidation of morphine (Coffman, B., et al. (1996) Drug Metab. Dispos. 25:1-4) and the glucuronidation of 5-lipoxygenase inhibitors (antiinflammatories) (Coffman, B., et al. (1997) Drug Metab. Dispos. 25:1032-8); in the latter case, glucuronidation was demonstrated to be the rate-limiting step for *in vivo* plasma clearance. UGTs are 50-60 kDa integral membrane proteins with the major portion of the protein, including the catalytic domain, located in the lumen of the endoplasmic reticulum and a C-terminal anchoring region of 15-20 amino acids spanning the ER membrane (Radominska-Pandya, A., et al. (1999) Drug Metab. Rev. 31:817-99.11. Radominska- Pandya, A., et al. (1999) Drug Metab. Rev. 31:817-99). The aglycone-binding site is believed to be in the N-terminal portion the UGT polypeptide, which is the region of the protein that shows the greatest variability in sequence among UGT isozymes. The UDPGA binding domain is in the highly conserved C-terminal half of the protein. Although not a certainty, it has been hypothesized that association with lipid is required for UGT activity and may influence the access of aglycones to the active site. Two UGT families - UGT1 and UGT2 - have been identified in humans: Although members of these families are less than 50% identical in primary amino acid sequence, they exhibit significant overlap in substrate specificity (Radominska- Pandya, A., et al. (1999) Drug Metab. Rev. 31:817-99). The members of the UGT1 family that are expressed in human liver, where the majority of xenobiotic metabolism takes place, includes UGT1A1, 1A3, 1A4, 1A6, and 1A9. Although the UGT2 family has not been as extensively studied, it is known that UGT2B4, 2B7, 2B10, 2B11 and 2B15 are expressed in the liver (Radominska-Pandya, A., et al. (1999) Drug Metab. Rev. 31:817-99.11. Radominska- Pandya, A., et al. (1999) Drug Metab. Rev. 31:817-99). As is the case for other drug metabolizing enzymes such as CYP450s, inter-individual differences in UGT expression levels have been observed and linked to differences in drug responses (Weber, W. (1997) Pharmacogenetics, Oxford University Press, New York).

The human UGT1 family includes the major bilirubin metabolizing isoform (UGT1A1) and the isoform that preferentially conjugates planar phenols (UGT1A6). Isoforms in the UGT2 family metabolize a variety of endogenous steroid compounds, as well as xenobiotics. As with the CYP450s, classification of the UGTs based on substrate specificity is somewhat limited since there is a great deal of overlap in the biotransformation capacity for most of the human UGTs.

*Glutathione transferases (GSTs)*

Glutathione transferases catalyze the formation of thioether conjugates between glutathione (GSH) and reactive xenobiotics by direct addition **(Equation 3)** or displacement of an electron-withdrawing group **(Equation 4).**

**Equation 3: GSH + R →GS-R**

**Equation 4: GSH + R-X →GS-R + HX**

The major biological function of GSTs is believed to provide defense against electrophilic chemical species. The majority of GSTs are cytosolic homodimers composed of approximately 25 kDa subunits from one of four structural classes: Alpha ($\alpha$), Mu ($\mu$), Pi ($\pi$), and Theta ($\theta$). The $\alpha$ isoform (GST A1-1) is restricted to a few tissues in mammals, including kidney, intestine, lung and liver. The $\mu$ isoform (GST M1-1) is found in the liver, but relatively few other tissues. In contrast, the $\pi$ isoform (GST P1-1) is widely distributed throughout the body, although it is notably absent in the liver. Additionally, GST P1-1 is abundant in most types of tumor cells.

*Sulfotransferases (SULTs)*

Sulfotransferase enzymes catalyze the conjugation of sulfate groups onto a variety of xenobiotic and endogenous substrates that possess acceptor moieties such as hydroxyl and amine groups **(Equation 5).**

**Equation 5: R-XH + PAPS →R-SO4 + phosphoadenosine + H+**

The cofactor 3'-phosphoadenosine 5'-phosphosulfate (PAPS) is required for sulfonation by these enzymes. Although sulfonation generally causes molecules to lose their biological activity, several documented examples indicate that the addition of sulfate can lead to formation of highly reactive metabolic intermediates, such as minoxidil, and reactive electrophilic cations, such as sulfated N-hydroxy 2-acetylaminofluorene (McCall, J., et al. (1983) J. Med. Chem. 26: 1791-3; Miller, J.A. (1994) Chem. Bio. Interact. 92:329-41). Several sulfotransferase enzymes with different biochemical properties have been characterized in animal and human tissue. Two general classes exist in tissue fractions: the cytosolic enzymes, which are considered important in drug metabolism; and the membrane bound enzymes, which are involved in the sulfonation of glycosaminoglycans and glycoproteins (Weinshilboum, R.M., et al. (1997) FASEB J. 11:

3-14). The human cytosolic sulfotransferase isozymes function as homodimers of 32-35 kDa subunits. There are currently 10 known sulfotransferases in humans, five of which are known to be expressed in adult liver (SULT1A1, SULT1A2, SULT1A3, SULT1E and SULT2A1). It is expected that other new genes encoding sulfotransferases will be identified. The nomenclature of the different genes, their mRNA and protein products has recently been revised so that "SULT" is the accepted superfamily abbreviation (Raftogianis, R.B., et al. (1997) BBRC 239:298- 304). Allelic variants of sulfotransferase enzymes do exist and studying their frequency and functional role in drug disposition is a very active area of research.

N-acetyl Transferases

N-acetyltransferases (NATs) catalyze the biotransformation of aromatic amines or hydrazines to the respective amides and hydrazides **(Equation 6)** using acetyl coenzyme A as a donor. They also will catalyze the O-acetylation of N-hydroxyaromatic amines to acetoxy esters **(Equation 7)**.

**Equation 6: R-NH2 + CoA—S—COCH3 →R-NCOCH3 + CoA—SH**

**Equation 7: R—NHOH + CoA—S—COCH3 →R—NHOCOCH3 + CoA—SH**

There are two known NAT isoforms in humans called NAT1 and NAT2; both are 33 kDa cytosolic proteins found in the liver. NAT1 is also expressed in many other tissues, whereas NAT2 is expressed only in the liver and gut. The two isoforms have different, but overlapping substrate specificities, with no single substrate appearing to be exclusively acetylated by one isoform or the other. Genetic polymorphisms for N-acetylation are well documented, and may play a role in the susceptibility of certain individuals to bladder and colon cancer, as the NATs are involved in both the activation and detoxification of heterocyclic aromatic amine carcinogens (Weber, W. (1997) Pharmacogenetics, Oxford University Press, New York).

**Toxicity**

One of the main forms of toxicity is hepatotoxycity. Freshly isolated human hepatocytes represent the best in vitro biological system in which to evaluate toxicity. Some human liver cell lines have been developed that reflect normal human liver metabolism (e.g., ACTIVTox from Amphioxus Inc, and Hep G2 from Cerep). These cell lines can be used in cell proliferation assays that give very good correlation with in vivo results.

In the present invention toxicity assessment is an inherent parameter of the screens since the compounds are produced in a host organism. Any compound that is very toxic will not be selected or detected since it will kill the host organism. A more accurate human toxicity assay can be incorporated in the multiple parameter screening procedure by for example encapsulating hepatocytes with the producer species and disease target(s) and select screening units that activated the disease target(s) in the desired way and have not inhibit hepatocyte growth.

Figure 8 shows a schematic representation of a screening system of the present invention to evaluate target activity, metabolism by DMEs and cytotoxicity: Using a double gel encapsulation system where in the first droplet are clonal lines of the producer species transformed with the pharmacological target and DMEs, and in the second droplet are hepatocytes, it is possible to screen for target activity, DME metabolism and hapatotoxicity.

**Mutagenesis**

The mutagenic ability of a compound is another aspect that has to be addressed in a drug discovery programme. The mutagenicity of a compound can be evaluated by measuring the reverse-mutation rate in microorganisms. For example, there are several different strains with differing and complementary sensitivities to potential mutagens of the bacterium *Salmonella typhimurium.*

**MULTIPLE PARAMETER SCREENING FOR OTHER PURPOSES**

**Screening for herbicides.**

The effect of a compound as a herbicide can be screened with in vitro assays. Primary screens that test the effect as a herbicide-include: toxicity, inhibition of photosynthesis, inhibition of central metabolic enzymes.

Examples of further screens that can be assayed simultaneously with the first group include: uptake (using hairy root cultures, organ cultures (including shoot cultures), metabolism, lack of toxicity towards other plants (in particular crops) or towards other organisms (animals, humans, insects, fungi).

**Screening for fungicides (agricultural)**

Primary screens are much like the ones used in screening for or evolving herbicides except that fungal cells are used as reporter cells and for uptake.

Secondary screens are also more or less of the same type. One particular screen to perform is lack of toxicity towards plants, in particular crop plants.

**Screening for insecticides**

Primary screens include the assays four the function of the compounds as insecticides, i.e. cell based assay for toxicity towards a specific species or group of species of insects, and/or assays for inhibition of specific enzymes in key metabolic functions of insects, or inhibition of reproduction.

Secondary screens may include uptake in specific insect organs using e.g. a confluent monolayer of insect cells from the organ in which the insecticide is to be taken up. A further screen includes metabolism by insect metabolic enzymes to test whether the compounds are metabolised or activated by these. Furthermore, it is relevant to screen for lack of toxicity or mutagenicity or teratogenicity towards animals and/or human beings. Another example of a secondary screen is lack of toxicity towards other species of insects.

**Screening for cosmetics**

Primary screens are directed to the function of the compounds as cosmetics.

Secondary screens include the same as for screening for or evolution of pharamaceuticals, i.e. absorption (if relevant), distribution (if relevant), metabolism, excretion (if relevant) and toxicity, mutagenicity and teratogenicity.

**Screening for flavours**

Primary screens may include automatic assaying for the desired flavour. "Artificial noses" have been developed that can assay for particular flavours or tastes. Artificial noses, or olfactory or vapor-selective detectors can detect low levels of odorants. Examples of such noses are disclosed in e.g. US 6,368,558 and references cited therein. The technique is also know as artificial olfactometry.

Secondary screens typically include toxicity, mutagenicity, teratogenicity, metabolism (by e.g. saliva enzymes).

Fine chemicals: other examples of multiple parameter screening and evolution include the evolution and screening for fine chemicals, food and feed additives, and catalysts.

**SCREENING TECHNIQUES**

The selection of the positive cells can be achieved by establishing screens where only positive cells survive or by phyisically selecting positive cells. Survival of positive clones can e.g. be achieved by using assays based on

a. Survival in the presence of toxic substances
b. Survival in the presence of other organisms
c. Use of nutritional reporter genes, e.g., His, or of reporter genes that when giving desired response produce a vital protein, e.g., CDC25

Physical selection of positive cells can be done by the use of:

a. FACS & intracellular reporter assays (native or engineered)
b. FACS & Gel encapsulation (single, double or more) & extracellular reporter systems [cell based (native or engineered) or cell free]
c. Overlay assay & extracellular reporter systems-[cell based (native or engineered) or cell free] & picking (manual or automatic)
d. Single clonal cell line confinement to microtiter plate, & extracellular reporter systems [cell based (native or engineered) or cell free] & picking (manual or automatic)

e. Plating & picking (manual or automatic)

Flow cytometry

In traditional flow cytometry, it is common to analyze very large numbers of cells in a short period of time. Newly developed flow cytometers can analyze and sort up to 100,000 cells per second. In a typical flow cytometer, individual particles pass through an illumination zone and appropriate detectors, gated electronically, measure the magnitude of a pulse representing the extent of light scattered. The magnitude of these pulses are sorted electronically into "bins" or "channels", permitting the display of histograms of the number of cells possessing a certain quantitative property versus the channel number. It was recognized early on that the data accruing from flow cytometric measurements could be analyzed (electronically) rapidly enough that electronic cell-sorting procedures could be used to sort cells with desired properties into separate "buckets", a procedure usually known as fluorescence-activated cell sorting.

Fluorescence-activated cell sorting has been primarily used in studies of human and animal cell lines and the control of cell culture processes. Fluorophore labeling of cells and measurement of the fluorescence can give quantitative data about specific target molecules or subcellular components and their distribution in the cell population. Flow cytometry can quantitate virtually any cell-associated property or cell organelle for which there is a fluorescent probe (or natural fluorescence).

Cell sorters can handle cell sorting at rates of at least 10,000 cells per second, more preferably at least 50,000 per second, more preferably at least 100,000 per second.

**Gel microdroplet encapsulation**

The gel microdroplet technology has had significance in amplifying the signals available in flow cytometric analysis, and in permitting the screening of microbial strains in strain improvement programs for biotechnology. Wittrup et al., (Biotechnolo. Bioeng. (1993) 42:351-356) developed a microencapsulation selection method which allows the rapid and quantitative screening of >$10^6$ yeast cells for enhanced secretion of Aspergillus awamori glucoamylase. The method provides a 400-fold single-pass enrichment for high-secretion mutants.

Gel microdroplet or other related technologies can be used in the present invention to localize as well as amplify signals in the high throughput screening of cells. Preferably the screening methods of the present invention are laid out to ensure survival of the producer cell, so that these can be used for further rounds of evolution. However, it is also possible to isolate the expression cassettes and possibly even the artificial chromosomes from the cells and re-insert these into other host cells should this be necessary if the cells are killed by the screen.

Different types of encapsulation strategies and compounds or polymers can be used with the present invention. An encapsulation of particular relevance is the encapsulation in calcium alginate due to its broad applicability. Furthermore, calcium alginate beads can be made at room temperature and be dissolved by gentle procedures leaving the encapsulated cells alive.

A further feature of particular interest is the possibility of coating the beads (or gel microdroplets) with a lipid layer in order to make them impermeable to small molecules. This ensures that small molecules do not leak to the surroundings and that the connection between producer cell and small molecule is not lost during screening and sorting of gel microdroplets.

Encapsulation techniques may be employed to localize signal, even in cases where cells are no longer viable. Gel microdrops (GMDs) are small (25 to 200 μm in diameter) particles made with a biocompatible matrix. In cases of viable cells, these microdrops serve as miniaturized petri dishes because cell progeny are retained next to each other, allowing isolation of cells based on clonal growth. The basic method has a significant degree of automation and high throughput. Cells are encapsulated together with substrates and particles containing a positive clones are sorted. Fluorescent substrate labeled glass beads can also be loaded inside the GMDs. In cases of non-viable cells, GMDs can be employed to ensure localization of signal.

Encapsulation can be in beads, high temperature agaroses, gel microdroplets made from agarose, polysacchoride, carbohydrate, alginate, carrageenan, chitosan, cellulose, pectin, dextran, or polyacrylamide, cells, such as ghost red blood cells or macrophages, liposomes, or any other means of encapsulating and localizing molecules.

Gel encapsulated cells may further be enclosed in a layer essentially non-penetrable to the compounds being-screened. Thereby it is ensured that the compounds remain within the vicinity of the cell and that the physical connection between cell and compound is not lost. Furthermore leakage from gel droplet to gel droplet is prevented. The non-penetrable material may a lipid material.

Advantageously, cells are encapsulated into gel droplets comprising reporter system(s) prior to sorting by FACS so that the advantages of FACS are combined with the advantages of gel droplet screening.

The cells and the reporter system(s) may be encapsulated into one layer of gel droplets. The cell may also be encapsulated in one layer of the gel droplet and at least one reporter system is encapsulated in another layer of the same gel droplet. Furthermore, the cell may be encapsulated in one layer of the gel droplet and a first reporter system is encapsulated in another layer of the same gel droplet and at least a second reporter system is encapsulated into yet another layer of the same gel droplet.

For example, methods of preparing liposomes have been described (i.e., U.S. Pat. Nos. 5,653,996, 5,393,530 and 5,651,981), as well as the use of liposomes to encapsulate a variety of molecules U.S. Pat. Nos. 5,595,756, 5,605,703, 5,627,159, 5,652,225, 5,567,433, 4,235;871, 5,227,170). Entrapment of proteins, viruses, bacteria and DNA in erythrocytes during endocytosis has been described, as well (Journal of Applied Biochemistry 4, 418-435 (1982)). Erythrocytes employed as carriers in vitro or in vivo for substances entrapped during hypo-osmotic lysis or dielectric breakdown of the membrane have also been described (reviewed in Ihler, G. M. (1983) J. Pharm. Ther). These techniques are useful in the present invention to encapsulate samples for screening.

An environment suitable for facilitating molecular interactions include, for example, liposomes. Liposomes can be prepared from a variety of lipids including phospholipids, glycolipids, steroids, long-chain alkyl esters; e.g., alkyl phosphates, fatty acid esters; e.g., lecithin, fatty amines and the like. A mixture of fatty material may be employed such a combination of neutral steroid, a charge amphiphile and a phospholipid. Illustrative examples of phospholipids include lecithin, sphingomyelin and dipalmitoylphos-phatidylcholine. Representative steroids include cholesterol, cholestanol and lanosterol. Representative charged amphiphilic compounds generally contain from 12-30 carbon atoms. Mono- or dialkyl phosphate esters, or alkyl amines; e.g., dicetyl phosphate, stearyl amine, hexadecyl amine, dilauryl phosphate, and the like.

**Other screening systems**

As an alternative to gel droplet screening the selection of positive cells meeting the at least one screening criterion may be performed by means of an overlay assay, said overlay assay comprising reporter system(s), and manual or automatic picking of positive cells.

Other systems for screening include the selection of positive cells meeting the at least one screening criterion is performed by means of placing a single clonal cell line in one well of a microtiterplate, said well comprising reporter system(s), and manual or automatic picking of positive cells. This system takes advantage of the many systems developed for automatic handling and analysis of microtiterplates.

Cells may also simply be plated on medium and positive cells can be picked either automatically of manually.

Cells may also be engineered so that only positive cells are able to survive. These cells may be grown in liquid media or be plated.

**Evolution towards mutliple parameters**

Evolution at its most general is a process, whereby a set of replicating and varying patterns are subjected to a selection process that favours the replication of certain of the variant patterns. The selection process acts on an emergent property (phenotype) that is encoded by the pattern and that varies as a consequence of the underlying variation in the pattern. Over the course of a series of replication events those patterns whose replication is most favoured come to dominate the population.

Variation in the patterns occurs as the result of changes in individual patterns or as the result of mixing of individual patterns. Which patterns come to dominate the population is partly a consequence of the selection criteria used and partly a function of the starting population.

In living organisms and cells the predominant replicating pattern consists of nucleotide sequences (DNA or - in some vira - RNA) and the criteria on which selection acts it typically mediated through other molecules such as (but not limited to) proteins, metabolites, and structural macromolecules that are encoded by the nucleotide sequence either directly or indirectly.

In genetic algorithms the replicating pattern consists of software defined magnetic states and the variation on which selection acts is typically (but not limited to) the solution of a mathematical algorithm encoded by the magnetic states either directly or indirectly.

The ability of a pattern to replicate in a given set of environmental parameters is often referred to as the "fitness" of the pattern. Fitness can be regarded as a mathematical property that replicating patterns "attempt to" optimise. The higher the fitness of any given pattern, the greater the chance it will produce one or more copies of itself, the higher the number of copies it will on average produce, and the lower the chance it will be destroyed prior to replication. As with any mathematical function the property that is optimised may itself be a complex function of otherwise independent properties. Thus evolution can optimise across more than one criteria. For instance the mating calls of many male insects are optimised to attract females of the same species whilst not attracting predators. The oxygen binding proteins in whale blood are optimised to bind oxygen under one set of conditions and release it under another set of conditions.

Cells containing genetic material are thus in principle able to evolve by virtue of the variations in the genetic sequence that occur within each cell and the consquences of this variation upon the fitness of the cell in a given set of environmental parameters and the ability of the cell to pass these genetic sequences on to descendant cells

For the purposes of this invention the term "Fitness Function" shall be taken to mean a mathemetical or algebraic equation that calculates a score and where the variable elements in the equation are output variables that vary between different cells within a *cell population.*

For the purposes of this invention the term "Fitness Score" shall be the score generated by the *fitness function* equation.

It shall be understood that any selection process conducted on cells may therefore be conducted according to the following general procedures:

- The fitness function (F') is defined so that it encapsulates the desired phenotype of the cell and mathematically relates this to measurable parameters
- Each cell or group of cells is measured on one or more parameters
- F' for the cell is calculated according to the measured parameters
- Those cells with the highest F' scores are removed from the screening locality and allowed to grow. Cells with lower F' scores are discarded. By the highest F' score is meant a predetermined percentage of the cells with the highest score, such as the best 1%, 5%, 10 % or 50%, or for very high selection pressures the best 1‰, the best 0.1 ‰, the best 0.01 ‰, the best 0.001 ‰, or the best 0.0001‰.

It is an important teaching of evolution that the criteria on which certain patterns are selected over other patterns is essentially arbitrary - in principle any criterion can be used. That arbitrary, human imposed criteria can be used to generate an evolutionary process in a whole organism is exemplified by the evolution of melanism in moths as a result of industrialisation, the evolution of pedigree dogs with various properties and the evolution of plants with e.g. enhanced levels of commercially valuable oils or more even fruiting times or more attractive scents and colours. The term "breeding" is often used to describe human imposed evolution. Such organisms have increased their fitness according to a given set of human imposed criteria. It shall be obvious from the these examples that it is not necessary for the fitness function equation to be explicitly described for the evolution to take place.

It is a further teaching that fitness functions and consequent selection pressures can lead to the organism expressing phenotypes that impose high costs on (and even in some cases kill) the organism. All that is required for this to be the case is that they confer a countervailing benefit that allows the underlying pattern that produces the phenotype to spread. One example is the evolution of the peacock's tail, which whilst making it highly visible and vulnerable to competitors and predators, improves its ability to attract mates and hence replicate. In organisms with, diploid or higher ploidy and with sexual reproduction it is even possible for patterns that have a net cost to be maintained in the population at reasonable levels. One example of this is the maintenance of the sickle cell anaemia mutation in west african human populations. The heterozygote form of the mutation confers a benefit (by making the carrier more resistant to malaria) whilst the homozygote is costly (causing severe anaemia). The positive benefit of the heterozygote results in the underlying pattern being maintained in the population at a relatively high frequency.

It is a further teaching that multiple selection pressures, acting on a population at different locations and times help develop and maintain the variability of replicating patterns in the population.

It is a further teaching that if two identical selection pressures are applied to two independent but apparently identical populations then although such populations will each evolve similar phenotypes the genetic patterns that come to dominate the population (and that confer the evolved phenotype) may differ between the populations. An example of different genetic patterns conferring the same phenotype is streptomycin resistance in bacteria.

From the above it should be clear that organisms are capable of complex evolutionary responses to a wide range of environmental pressures.

The evolution according to the present invention is based on a series or cycle of steps of subjecting a composition of cells to screening and selecting cells exhibiting a predetermined functionality, as shown in Fig. 22. The cycles are repeated until the desired functionality, for example a target specificity and activity is obtained. Another example of general screening strategy is illustrated in Figure 21.

In other words, the method of evolution according to the present invention is based on the provision of

1. a suitable set of diverse genetic patterns <u>and also</u>
2. a way of selecting for those genetic patterns within this set that encode for phenotypes that are consistent with these properties <u>and also</u>
3. a way of generating novel genetic patterns from those patterns that were selected in step 2.

These steps may then be combined sequentially or in parallel or in some other essentially iterative basis. The present invention sets out how to achieve these requirements.

In another aspect of the invention, the methods may be applied to the generation of a pathway derived from sources from multiple natural kingdoms, phyla or orders in the host cell. An example of this would be the generation of a pathway to produce retinoids or other molecules by means of introduction of genes encoding for the production of careotenoid pathways (obtained from fungi, algae and/or plants) as well as genes encoding for the synthesis of Vitamin A (obtained from mammals) or genes encoding for the production of visual pigments (obtained from insects). By such targeted selection and combination of elements of biochemical pathways across kingdoms or phyla the likelihood of obtaining novel metabolites may be further increased.

As previously described a fitness function (F') can be defined that encapsulates the desired phenotype of the cell and mathematically relates this to one or more measured outputs. For example the fitness function may be defined as the multiple of a cell's absorption at two different wavelengths or alternatively it may be defined as the level of inhibition of one enzyme, divided by the inhibition of another enzyme, or it may be defined as the level of cytotoxic poison that a cell can survive, multiplied by the rate or reproduction of the cell in the absence of the cytotoxic or it may be defined in numerous other ways

In each screening round cells are selected that have outputs that correspond to one or more elements of the fitness function. In a preferred embodiment early screening rounds only measure one output whilst later screening rounds measure multiple outputs.

Those cells with the highest *fitness scores* in the population are removed from the screening environment for later use and/or analysis. Cells with lower F' scores may be discarded. By the highest F' score can be meant a predetermined percentage with the highest score, such as the best 1%, 5%, 10 % or 50%, or for very intense selection or very large cell populations the best 1‰, the best 0.1 ‰, the best 0.01. ‰, the best 0.001 ‰, or the best 0.0001‰ or even the best 0.00001‰. Alternatively an absolute fitness score can be defined and only those cells that exceed this score are selected. By this approach the percentage of cells that are selected may vary.

In a preferred embodiment of this invention the screening and selection processes should be conducted on a repetitive or iterative basis, with each iteration being conducted on a daughter population.

For each iteration of the screening step, the fitness score that the cells are categorised upon is defined and the cell population subjected to screening. Over a series of iterations the fitness score is elaborated such that it progressively approaches the desired target value. The fitness score may be elaborated either by being increased or by having additional factors added into the equation that derives the fitness score

The screening criteria are hence progressively optimised towards the desired functionality through the necessary rounds or cycles of screening and selection. The steps are repeated until at least one cell having the desired functionality has been evolved, such as repeated at least twice, such as at least three times, such as at least four times, such as at least five times, such as at least ten times, such as at least twenty times, such as least fifty times, such as at least one hundred times, such as at least two hundred times

In another embodiment the steps are repeated until at least two cell lines, or at least five cell lines, or at least 10 cell lines, having the desired functionality have been evolved. In a preferred embodiment at least a part of the cell lines evolved have different genetic patterns or genotypes, in a more preferred embodiment all the cell lines evolved have different genetic patterns or genotypes. By the term cell lines is meant cells originating from cells having met the screening criteria related to the determined screening functionality.

The screening criteria (or threshold) for one or more outputs may be increased for each repeat. Increasing criteria may for example be increasing concentration of a chemical, such as a toxin, in growth media for each repeat, or decreasing concentration of one or more nutrition components in the growth media or decreasing sensitivity or proximity of a reporter construct. Other examples of increasing criteria may be repetitive changes of temperature, either increasing or decreasing depending on the cell type chosen.

The screening criteria may also change character per repeat, such as starting with a concentration of a chemical substance in the growth media, and adding a physical parameter, such as light, in the next repeat, or starting with measuring the activity against one enzyme and adding activity against another enzyme in the next repeat.

It is also within the scope of the present invention that screening criteria may be a mixture of the criteria discussed above, ie. increased concentration of a chemical combined with changes of physical parameters, and/or increased concentration of one chemical combined with changed concentration of another.

Through this approach and in accordance with the general principles of evolution, over a series of screening and selection cycles host lines that most demonstrate the required characteristics are selected for and come to dominate the population. Over a series of screens the required fitness score is raised or elaborated, favouring those combinations that have led to an improvement in the expression of the desired characteristics.

In one embodiment the host cell lines that are a priori believed to be interesting for a given target are selected and the selected lines evolved through a series of screens as set out in Figure 22.

In another embodiment the approach is one of an escalator of selection pressure using screens that move from the general / low activity to the specific / high activity with the generation of new genetic patterns between each step.

In another embodiment the fitness score is deliberately raised only marginally between selection cycles, such as by no more than 50% or by no more than 25% or by no more than 10% or by no more than 5% or by no more than 1%. Such gradualist selection pressures allow low level responses to be built upon over a series of selection cycles. By selecting marginal improvements in the fitness score such an approach maximises the genetic diversity at each stage in the selection process.

**Specific strategies for pathway generation**

In another embodiment the approach is to walk down a specific multi-step metabolite pathway in a manner analogous to playing a slot machine. Once the first step of the pathway is obtained the genetic material for that step is put on "hold" by increasing its relative abundance such that most cells in the cell population contain said genetic material and the other genetic materials are then varied (spun or permed) until the second step is achieved, which is then also put on "hold". This process is repeated until the entire pathway is obtained.

In another embodiment the approach is to reverse up a specific multi-step metabolite pathway. Once the last step of the pathway is obtained the genetic material for that step is put on "hold" by increasing its relative abundance such that most cells in the cell population contain said genetic material and the other genetic materials are then varied (spun or permed) until the next but last step is achieved, which is then also put on "hold". This process is repeated until the entire pathway is obtained.

Also, a combination of both embodiments may be conducted, so that the pathway is built up from "both ends".

In one embodiment of the invention the cells are subjected to the selection criteria under conditions that maximise the number of genes expressed by the cells, including the genes being heterologous to the cells. Alternatively the cells are subjected to the selection criteria under conditions that ensure a certain percentage or set of the genes being heterologous to the cells are expressed

It should be understood that the above approaches are general in concept and lend themselves to the construction of many variants, depending on the desired goal.

Furthermore, it should be understood that by using a cell-based system an advantage is that the compounds may be selected also on parameters not being included in the fitness function, in that the system inherently promotes evolution of compounds exhibiting properties such as not being toxic to the cell, as well as compounds that diffuse rapidly within the cell.

Examples of the approaches to build known or structural class focused pathways are as follows:

For small to medium sized pathways, i.e. pathways of up to 6-7 steps from metabolites of the host cell, the screening strategy relies on enriching the founder population with relevant genes and on the reasonably high probability of assembling over a series of selection rounds pathways that produce a low level of the desired property.

For large pathways (i.e. more than 6-7 steps) the screening strategy mostly involves dividing the pathway into subsets and a) defining screening parameters for each subset in order to build a pathway forwards or b) identifying intermediate metabolites that are feed to the cell population in order to assemble the pathway backwards.

For example in the case of retinoid like compounds it is well known that carotenoids are metabolised by specific tissues in specific classes of organisms to produce retinoids. It is thus possible to first evolve a population of cells that produce carotenoids and then mix the genes of this population(s) with those of a population(s) enriched for retinoid genes and in this manner evolve a population that produce retinoid like compounds.

Another example is the case of Taxol like compounds, for which the exact biosynthetic pathway is not known but is predicted to be somewhere between 12 and 20 enzymatic steps from yeast metabolites and several of the intermediate compounds have been isolated. It is thus possible to start by feeding a metabolite that is a few steps from Taxol in order to identify a population of cells able to produce Taxol like compounds from this precursor. Once this is achieved, the genes responsible for that small pathway are locked, e.g., integrated in the host's genome, or incorporated in artificial chromosomes at such high levels that statistically they occur in most cells and a second evolution process is started. This time the precursor being fed to the cell population is an earlier metabolite from the Taxol biosynthesis. By repeating this partial evolutions a number of times, it is possible to evolve a population of cells that produce Taxol like compounds starting with host metabolites.

Finally it should be said it is also possible to produce a class of compounds using a combination of both approaches described, i.e., by starting simultaneous evolution' processes that cover the pathway backwards and forwards:

**Diverse Genetic Patterns**

Given that evolution is a statistical process it is necessary to provide sufficient genetic variation on which selection processes can act. In the present invention, this comprises two elements

- Providing a sufficiently large and diverse population
- Controlling the genetic basis of the diversity and how it expresses

Selection requires genetic diversity on which to operate. Thus the first requirement of the current invention is to provide a population of cells that embodies a genetic diversity. The term "*genetic diversity*" means that substantially all cells are different, in that they comprise different genes, and/or identical genes under control of different control system, such as different promoters, such that almost each cell initially represents a genotype not represented in any of the other cells. Of course due to cell division a few cells may be substantially identical.

The term "Cell Population" shall be taken to mean a population of cells where at least $10^4$ cells, such as at least $10^5$ cells, such as at least $10^6$ cells, such as at least $10^7$ cells, such as at least $10^8$ cells, such as at least $10^9$ cells, such as at least $10^{10}$ cells, such as at least $10^{11}$ cells, such as at least $10^{12}$ cells in the population represent a genotype not represented in any of the other cells.

Thus, the principle of the evolution method according to the invention is to obtain a population of cells having a very high genetic diversity.

One particular embodiment of this principle is to produce cells with combinations of concatemers comprising cassettes with expressible nucleotide sequences from a number of different expression states, which may be from any number of unrelated or distantly or closely related species, or from species from different kingdoms or phylae, novel and random combinations of gene products are produced in one single cell.

By inserting novel genes into the host cell, and especially by inserting a high number of novel genes from different expression states, such as from a wide variety of species into a host cell, the gene products from this array of novel genes will interact with the pool of metabolites of the host cell and with each other and modify known metabolites and/or intermediates in novel ways to create novel compounds. Due to the high number of substantially different cells that can be generated using the methods according to the present invention, for example at least $10^4$ cells, such as at least $10^5$ cells, such as at least $10^6$ cells, such as at least $10^7$ cells, such as at least $10^8$, such as at least $10^9$, for example at least $10^{10}$, such as at least $10^{12}$, it is more or less inevitable or at least likely that such large populations will lead to a sub-population having such an interaction. The sub-population having such interaction may comprise at most $10^{10}$ cells, such as at most $10^9$ cells, such as at most $10^8$, such as at most $10^7$ cells, such as at most $10^6$ cells, such as at most $10^5$ cells, such as at most $10^4$ cells, such as at most $10^3$ cells, such as at most $10^2$ cells or just 10 cells.

**HOST CELLS**

The host cells selected for this purpose are preferably cultivable under standard laboratory conditions using standard culture conditions, such as standard media and protocols. Preferably the host cells comprise a substantially stable cell line, in which the concatemers can be maintained for generations of cell division in a suitable manner. It is also of great advantage that standard techniques for transformation of the host cells are available, especially that methods are known for insertion of artificial chromosomes into the host cells.

It is also of advantage if the host cells are capable of undergoing meiosis to perform sexual recombination. It is also advantageous that meiosis is controllable through external manipulations of the cell culture. One especially advantageous host cell type is one where the cells can be manipulated through external manipulations into different mating types.

The host cell should preferably be conditionally deficient in the abilities to undergo homologous recombination. The host cell should preferably have a codon usage similar to that of the donor organisms. Furthermore, in the case of heterologous genomic DNA, if eukaryotic donor organisms are used, it is preferable that the host cell has the ability to process the donor messenger RNA properly, e.g., splice out introns.

The cells can be bacterial, archaebacteria, or eukaryotic and can constitute a homogeneous cell line or mixed culture. Suitable cells include the bacterial and eukaryotic cell lines commonly used in genetic engineering and protein expression. Suitable mammalian cells include those from, e.g., mouse, rat, hamster, primate, and human, both cell lines and primary cultures.

Preferred prokaryotic host organisms may include but are not limited to Escherichia coli, Bacillus subtilis, B licehniformis, B. cereus, Streptomyces lividans, Streptomyces coelicolor, Pseudomonas aeruginosa, Myxococcus xanthus. Rhodococcus, Streptomycetes, Actinomycetes, Corynebacteria, Bacillus, Pseudomonas, Salmonella, and Erwinia. The complete genome sequences of E. coli and Bacillus subtilis are described by Blattner et al., Science 277, 1454-1462 (1997); Kunst et al., Nature 390, 249-256 (1997)).

Preferred eukaryotic host organisms are mammals, fish, insects, plants, algae and fungi.

Examples of mammalian cells include those from, e.g., monkey, mouse, rat, hamster, primate, and human, both cell lines and primary cultures. Preferred mammalian host cells include but are not limited to those derived from humans, monkeys and rodents, such as chinese hamster ovary (CHO) cells, NIH/3T3, COS, 293, VERO, HeLa etc (see Kriegler M. in "Gene Transfer and Expression: A Laboratory Manual", New York, Freeman & Co. 1990), and stem cells, including embryonic stem cells and hemopoietic stem cells, zygotes, fibroblasts, lymphocytes, kidney, liver, muscle, and skin cells.

Examples of insect cells include baculo lepidoptera.

Examples of plant cells include maize, rice, wheat, cotton, soybean, and sugarcane. Plant cells such as those derived from Nicotiana and Arabidopsis are preferred

Examples of fungi include penicillium, aspergillus, such as Aspergillus nidulans, podospora, neurospora, such as Neurospora crassa, Saccharomyces, such as Saccharomyces cerevisiae (budding yeast), Schizosaccharomyces, such as Schizosaccharomyces pombe (fission yeast), Pichia spp, such as Pichia pastoris, and Hansenula polymorpha (methylotropic yeasts).

The choice of host will depend on a number of factors, depending on the intended use of the engineered host, including pathogenicity, substrate range, environmental hardiness, presence of key intermediates, ease of genetic manipulation, and likelihood of promiscuous transfer of genetic information to other organisms. Particularly advantageous hosts are E. coli, lactobacilli, Streptomycetes, Actinomycetes and filamentous fungi.

A preferred host cell is yeast due to the following characteristics: it is fast growing, eukaryotic, allows scalable culture capabilities, genetic tools are available, it is metabolically flexible, can have a relatively permeable cell membrane/wall and folds more heterologous eukaryotic proteins correctly than prokaryotic cells.

Thus, an illustrative and not limiting list of suitable yeast host cells comprise: baker's yeast, Kluyveromyces marxianus, K. lactis, Candida utilis, Phaffia rhodozyma, Saccharomyces boulardii, Pichia pastoris, Hansenula polymorpha, Yarrowia lipolytica, Candida paraffinica, Schwanniomyces castellii, Pichia stipitis, Candida shehatae, Rhodotorula glutinis, Lipomyces lipofer, Cryptococcos curvatus, Candida spp. (e.g. C. palmioleophila), Yarrowia lipolytica, Candida guilliermondii, Candida, Rhodotorula spp., Saccharomycopsis spp., Aureobasidium pullulans, Candida brumptii, Candida hydrocarbofumarica, Torulopsis, Candida tropicalis, Saccharomyces cerevisiae, Rhodotorula rubra, Candida flaveri, Eremothecium ashbyii, Pichia spp., Pichia pastoris, Schizosaccharomyces pompe (fission yeast), Kluyveromyces, Hansenula, Kloeckera, Pichia, Pachysolen spp., or Torulopsis bombicola.

In any one host cell it is possible to make all sorts of combinations of expressible nucleotide sequences from all possible sources. Furthermore, it is possible to make combinations of promoters and/or spacers and/or introns and/or terminators in combination with one and the same expressible nucleotide sequence.

In a preferred embodiment the cells to be evolved are produced by inserting concatemers comprising the multitude of cassettes into a host cell, in which the concatemers can be maintained and the expressible nucleotide sequences can be expressed in a co-ordinated way. The cassettes comprised in the concatemers may be cut out from the host cell and re-assembled due to their uniform structure with - preferably - compatible restriction sites between the cassettes.

The cells as defined in the present invention are preferably collected into populations for use in the present invention. The composition of cells subjected to evolution is then produced by selecting cells from a population or from several sub-populations. A population of individual cells is a population of expression constructs prepared from randomly assembled or even concatenated expressible nucleotide sequences derived from a plurality of species of donor organisms, in which expressible nucleotide sequences are operably associated with regulatory regions that drives expression of the expressible nucleotide sequences in an appropriate host cell. The host cells used are capable of producing functional gene products of the donor organisms. Upon expression in the host cell, gene products of the donor organism (s) may interact to form novel biochemical pathways.

The population according to this embodiment of the invention may in any one cell comprise a unique and preferably random combination of a high number of expression cassettes being heterologous to the host cells. Through this random combination of expression cassettes novel and unique combinations of gene products are obtained in each cell. Such populations are especially adapted in the discovery of novel metabolic pathways created through the non-native combinations of gene products.

In a preferred embodiment a population may be defined as a population comprising a collection of individual cells, the cells being denoted

$cell_1$, $cell_2$, ...., $cell_i$, wherein $i \geq 2$,

each cell comprising at least one concatemer of individual oligonucleotide cassettes, each concatemer comprising a nucleotide sequence of the following formula:

$$[rs_2\text{-SP-PR-X-TR-SP-}rs_1]_n$$

wherein $rs_1$ and $rs_2$ together denote a restriction site, SP denotes a spacer of at least two bases, X denotes an expressible nucleotide sequence, PR denotes a promoter, capable of regulating the expression of X in the cell, TR denotes a terminator, and $n \geq 2$, and
wherein at least one concatemer of $cell_1$ is different from a concatemer of $ceii_2$.

In the present context the nucleotide sequence of the formula $[rs_2\text{-SP-PR-X-TR-SP-}rs_1]_n$ is also referred to as an expression cassette of the formula $[rs_2\text{-SP-PR-X-TR-SP-}rs_1]_n$.

Sub-populations may comprise cells as defined above for populations, but mostly the cells of a sub-population will have at least one trait in common, such as common promoter combinations, genetic material from a common species, a common phenotype or the like.

The function of the populations and sub-populations is to act as a source of diversity when obtaining the composition of cells to be evolved. Thus, in one embodiment the composition is a collection of subcompositions, wherein a subcomposition is a collection of individual cells having at least one phenotype in common. In a preferred embodiment the composition comprises at least 2 individual subcompositions, said subcompositions being different, such as at least 5 individual sub-compositions, such as at least 10 individual sub-compositions, wherein each sub-composition comprises at least 10 individual cells, such as at least 50 individual cells, such as at least 100 individual cells, such as at least $10^3$ individual cells, such as at least $10^4$ individual cells, such as at least $10^5$ individual cells, such as at least $10^6$ individual cells, such as at least $10^7$ individual cells, such as at least $10^8$ individual cells, such as at least $10^9$ individual cells.

The composition of cells preferably comprises at least 20 individual cells, such as at least 50 individual cells, such as at least 100 individual cells, such as at least 150 individual cells, such as at least 200 individual cells, such as at least 250 individual cells, such as at least 500 individual cells, such as at least 750 individual cells, such as at least 1000 individual cells, such as at least $10^4$ individual cells, such as at least $10^5$ individual cells, such as at least $10^6$ individual cells, such as at least $10^7$ individual cells, such as at least $10^8$ individual cells, such as at least $10^9$ individual cells.

In a preferred embodiment at least a majority of the individual cells have a genetic patterns or genotypes, thereby representing a great diversity.

The term "founding population" or a "founder populations" shall mean a Cell Population that has not itself been subjected to a selection round, in the present context also referred to as composition of cells. Optionally the expression constructs within the cell population are constructed such that .genetic material from species that are known from prior art to produce compounds of a desired structure class, or compounds that have a desired functional effect, or are associated with a desired functional effect independent of knowledge of the compounds, predominate.

The term "daughter population" is a cell population having been subjected to at least one selection round. In the present context the daughter population is also referred to as a further modified composition.

**Controlling The Genetic Basis of the Diversity**

**Sources of Genes**

The natural world contains a significant amount of genetic diversity. Various authorities estimate that there are at least $10^7$ different species, and that each of these species contains on average at least $10^4$ genes. Even allowing for the fact that many of these genes are relatively conserved between species this represents a high level of genetic diversity.

One approach that can be envisaged for the purposes of the current invention is to source genetic material so as to maximise the taxonomic diversity of the genes obtained.

A second is to preferentially source genetic material from organisms that are known or reputed to produce molecules of the structural class or with the functional effects desired or are known or reputed to have a desired functional effect without the molecule being known, or are taxonomically related to any such organism.

A third approach is selection of genes of particular interest.

A fourth approach is to select genes that generally extend the host metabolic pathways.

Optionally these approaches can be combined in any suitable manner.

In one embodiment the heterologous gene is an intact gene comprising all exons and introns, in another embodiment the heterologous gene comprises all exons, but no introns or only some introns of the native gene.

Genes can be sourced through the collection and processing of genetic material of carious forms. The expressible nucleotide sequences that can be inserted into the vectors, concatemers, and cells according to this invention encompass any type of nucleotide such as RNA, DNA. Such a nucleotide sequence could be obtained e.g. from cDNA, which by its nature is expressible. But it is also possible to use sequences of genomic DNA, coding for specific genes. Preferably, the expressible nucleotide sequences correspond to full length genes such as substantially full length cDNA, but nucleotide sequences coding for shorter peptides than the original full length clones may also be used. Shorter peptides may still retain the catalytic activity of the native proteins. Thus, a preferred embodiment of this invention is to source and collect messenger transcripts (mRNA) for obtaining cDNA.

Another way to obtain expressible nucleotide sequences is through chemical synthesis of nucleotide sequences coding for known peptide or protein sequences. Thus the expressible DNA sequences does not have to be a naturally occurring sequence, although it may be preferable for practical purposes to primarily use naturally occurring nucleotide sequences. Whether the DNA is single or double stranded will depend on the vector system used.

By the term "Expression state" is meant a state of gene expression (i.e the mRNA transcript popuilation) in a specific cell, tissue, combination of tissues or organism or organisms of a given species as sampled at at any one time. Different expression states are found in different individuals, or in the same individual at different point in time, or in the same individual at different points its life-cycle or in the same individual under differing external conditions. The expression states of given cells or tissues of a given individual will also vary with respect to other cells or tissues of the same individual. Different expression states may also be obtained in the same organ or tissue in any one species or individual by exposing the tissues or organs to different environmental conditions comprising but not limited to changes in developmental stage, age, disease, infection, drought, humidity, salinity, exposure to xenobiotics, physiological effectors, temperature, pressure, pH, light, gaseous environment, chemicals such as toxins.

In the following the invention is described in the order in which the steps of obtaining a transformed host cell containing an evolvable artificial chromosome may be performed, starting with the entry vector.

In most cases the orientation with respect to the promoter of an expressible nucleotide sequence will be such that the coding strand is transcribed into a proper mRNA. It is however conceivable that the sequence may be reversed generating an antisense transcript in order to block expression of a specific gene.

Each cell of the *cell population* is initially produced by combining genes selected from at least one expression state. It is of course also possible from the onset to combine genes from two, three, four or more expression states in one host cell or to combine genes from different organisms in one cell. In some embodiments of the invention it is preferred to combine genes from a large variety of organisms into a single host in a manner so that each cell comprises at least two expressible nucleotide sequences, said sequences being heterologous to the cell, i.e. the sequences are not found in the native cell type.

A wide variety of combinations of expressible nucleotide sequences from all possible sources may occur in the cells. Furthermore, it is possible to make combinations of promoters and/or spacers and/or introns and/or terminators in combination with one and the same expressible nucleotide sequence.

Thus in any one cell there may preferably be expressible nucleotide sequences from two different expression states. Furthermore, these two different expression states may be from one species or advantageously from two different species. Any one host cell may also comprise expressible nucleotide sequences from at least three species, such as from at least four, five, six, seven, eight, nine or ten species, or from more than 15 species such as from more than 20 species, for example from more than 30, 40 or 50 species, such as from more than 100 different species, for example from more than 300 different species, such as from more than 500 different species, for example from more than 750 different species, thereby obtaining combinations of large numbers of expressible nucleotide sequences from a large number of species. In this way potentially unlimited numbers of combinations of expressible nucleotide sequences can be combined across different expression states. These different expression states may represent at least two different tissues, such as at least two organs, such as at least two species, such as at least two genera. The different species may be from at least two different phylae, such as from at least two different classes, such as from at least two different divisions, more preferably from at least two different sub-kingdoms, such as from at least two different kingdoms. Thus expressible nucleotide sequences may be combined from a eukaryote and a prokaryote into one and the same cell.

According to another embodiment of the invention, the expressible nucleotide sequences may be from one and the same expression state. The products of these sequences may interact with the products of the genes in the host cell and with each other and form new enzyme combinations leading to novel biochemical pathways.

**Sources of genetic diversity**

Examples of groups of species and individual species known to produce compounds witch structural or functional utility include without limitation

| | |
|---|---|
| Bacteria | Streptomyces, Micromonospora, Norcadia, Actinomadura, Actinoplanes, Streptosporangium, Microbispora, Kitasatosporiam, Azobacterium, Rhizobium, Achromobacterium, Enterobacterium, Brucella, Micrococcus, Lactobacillus, Bacillus (B.t. toxins), Clostridium (toxins), Brevibacterium, Pseudomonas, Aerobacter, Vibrio, Halobacterium, Mycoplasma, Cytophaga, Myxococcus |
| Fungi | Amanita muscaria (fly agaric, ibotenic acid, muscimol), Psilocybe (psilocybin) Physarium, Fuligo, Mucor, Phytophtora, Rhizopus, Aspergillus, Penicillium (penicillin), Coprinus, Phanerochaete, Acremonium (Cephalosporin), Trochoderma, Helminthosporium, Fusarium, Alternaria, Myrothecium, Saccharomyces |

| | |
|---|---|
| Algae | Digenea simplex (kainic acid, antihelminthic), Laminaria anqustata (laminine, hypotensive) |
| Lichens | Usnea fasciata (vulpinicacid, antimicrobial; usnic acid, antitumor) |
| Higher Plants | Artemisia (artemisinin), Coleus (forskolin), Desmodium (K channel agonist), Catharanthus (Vinca alkaloids), Digitalis (cardiac glycosides), Podophyllum (podophyllotoxin), Taxus (taxol), Cephalotaxus (homoharringtonine), Camptotheca (Camptothecin), Camellia sinensis (Tea), Cannabis indica, Cannabis sativa (Hemp), Erythroxylum coca (Coca), Lophophora williamsii (PeyoteMyristica fragrans (Nutmeg), Nicotiana, Papaver somniferum (Opium Poppy), Phalaris arundinacea (Reed canary grass) |
| Protozoa | Ptychodiscus brevis; Dinoflagellates (brevitoxin, cardiovascular) |
| Sponges | Microciona prolifera (ectyonin, antimicrobial) Cryptotethya cryta (D-arabino furanosides) |
| Coelenterata | Portuguese Man o War & other jellyfish and medusoid toxins. |
| Corals | Pseudoterogonia species (Pseudoteracins, anti-inflammatory), Erythropodium (erythrolides, anti-inflammatory) |
| Aschelminths | Nematode secretory compounds |
| Molluscs | Conus toxins, sea slug toxins, cephalapod neurotransmitters, squid inks |
| Annelida | Lumbriconereis heteropa (nereistoxin, insecticidal) |
| Arachnids | Dolomedes ("fishing spider" venoms) |
| Crustacea | Xenobalanus (skin adhesives) |
| Insects | Epilachna (mexican bean beetle alkaloids) |
| Spinunculida | Bonellia viridis (bonellin, neuroactive) |
| Bryozoans | Bugula neritina (bryostatins,anti cancer) |
| Echinoderms | Crinoid chemistry |
| Tunicates | Trididemnum solidum (didemnin, anti-tumor and anti-viral; Ecteinascidia turbinata ecteinascidins, antitumor) |
| Vertebrates | Eptatretus stoutii (eptatretin, cardioactive), Trachinus draco (proteinaceous toxins, reduce blood pressure, respiration and reduce heart rate). Dendrobatid frogs (batrachotoxins, pumiliotoxins, histrionicotoxins, and other polyamines); Snake venom toxins; Orinthorhynohus anatinus (duck-billed platypus venom), modified carotenoids, retinoids and steroids; Avians: histrionicotoxins, modified carotenoids, retinoids and steroids |

**Controlling Gene Expression - Expression Cassettes**

Genes primarily give rise to selectable phenotypes through transcription of the gene to RNA and translation of the RNA to protein. Furthermore phenotypes are often the result of interactions between multiple genes and their gene products

Thus it is an element of the current invention that the heterologous genes are provided in a format whereby their individual and collective expression (transcription to RNA) can be controlled.

It is likely that through the combination of a high number of non-native genes in a host cell combinations of genes or single genes are inserted that are lethal or sub-lethal to the host cell. Through the co-ordinated expression of the genes in the host cell it is possible not only to initiate the expression of any subset of genes but also to repress such expression, e.g. of lethal or sub-lethal genes.

Through external regulation of the promoters controlling the expressible nucleotides sequences novel and non-naturally occurring combinations of expressed genes can be obtained. Since these novel and non-natural combinations of gene products are found in one and the same cell, the heterologous gene products may affect the metabolism of the host cell in novel ways and thus cause it to produce novel primary or secondary metabolites and/or known metabolites in novel amounts and/or known metabolites in novel compartments of the cell or outside the cells. The novel metabolic pathways and/or novel or modified metabolites may be obtained without substantially recombining the introduced genes with a segment in the host genome or an episome of the host cells by as well as without intra- or extra concatemeric recombination.

By having expressible nucleotide sequences under the control of a number of independently inducible or repressible promoters, a large number of different expression states can be created inside one single cell by selectively turning on and off groups of the inserted expressible nucleotide sequences. The number of independently inducible and/or repressible promoters in one cell may vary from 1 to 10, such as 2, 3, 4, 5, 6, 7, 8, or 9, or even up to 15, 20, 25 or above 50 promoters.

In the evolution steps the functionality of the controllable promoters of the cells is used, since due to the controllable promoters it is possible during the screening and selection step to switch promoters on and off, thereby creating a greater diversity of expressed genes.

The term promoter is used with its normal meaning, i.e. a DNA sequence to which RNA polymerase binds and initiates transcription. The promoter determines the polarity of the transcript by specifying which strand will be transcribed.

- Bacterial promoters normally consist of -35 and -10 (relative to the transcriptional start) consensus sequences which are bound by a specific sigma factor and RNA polymerase.
- Eukaryotic promoters are more complex. Most promoters utilized in expression vectors are transcribed by RNA polymerase II. General transcription factors (GTFs) first bind specific sequences near the transcriptional start and then recruit the binding of RNA polymerase II. In addition to these minimal promoter elements, small sequence elements are recognized specifically by modular DNA-binding / trans-activating proteins (e.g. AP-1, SP-1) which regulate the activity of a given promoter.
- Viral promoters may serve the same function as bacterial and eukaryotic promoters. Upon viral infection of their host, viral promoters direct • transcription either by using host transcriptional machinery or by supplying virally encoded enzymes to substitute part of the host machinery. Viral promoters are recognised by the transcriptional machinery of a large number of host organisms and are therefore often used in cloning and expression vectors.

Promoters may furthermore comprise regulatory elements, which are DNA sequence elements which act in conjunction with promoters and bind either repressors (e.g., lacO/ LAC Iq repressor system in E. coli) or inducers (e.g., gal1. /GAL4 inducer system in yeast). In either case, transcription is virtually "shut off" until the promoter is derepressed or induced, at which point transcription is "turned-on". The choice of promoter in the cassette is primarily dependent on the host organism into which the cassette is intended to be inserted. An important requirement to this end is that the promoter should preferably be capable of functioning in the host cell, in which the expressible nucleotide sequence is to be expressed.

Preferably the promoter is an externally controllable promoter, such as an inducible promoter and/or a repressible promoter. The promoter may be either controllable (repressible/inducible) by chemicals such as the absence/presence of chemical inducers, e.g. metabolites, substrates, metals, hormones, sugars. The promoter may likewise be controllable by certain physical parameters such as temperature, pH, redox status, growth stage, developmental stage, or the promoter may be inducible/repressible by a synthetic inducer/repressor such as the gal inducer.

In order to avoid unintentional interference with the gene regulation systems of the host cell, and in order to improve controllability of the co-ordinated gene expression the promoter is preferably a synthetic promoter. Suitable promoters are described in US 5,798,227, US 5,667,986. Principles for designing suitable synthetic eukaryotic promoters are disclosed in US 5,559,027, US 5,877,018 or US 6,072,050.

Synthetic inducible eukaryotic promoters for the regulation of transcription of a gene may achieve improved levels of protein expression and lower basal levels of gene expression. Such promoters preferably contain at least two different classes of regulatory elements, usually by modification of a native promoter containing one of the inducible elements by inserting the other of the inducible elements. For example, additional metal responsive elements IR:Es) and/or glucocorticoid responsive elements (GREs) may be provided to native promoters. Additionally, one or more constitutive elements may be functionally disabled to provide the lower basal levels of gene expression.

Preferred examples of promoters include but is not limited to those promoters being induced and/or repressed by any factor selected from the group comprising carbohydrates, e.g. galactose; low inorganic phosphase levels; temperature, e.g. low or high temperature shift; metals or metal ions, e.g. copper ions; hormones, e.g. dihydrotestosterone; deoxycorticosterone; heat shock (e.g. 39°C); methanol; redox-status; growth stage, e.g. developmental stage; synthetic inducers, e.g. gal inducer. Examples of such promoters include ADH 1, PGK 1, GAP 491, TPI, PYK, NO, PMA 1, PHO5 GAL 1, GAL 2, GAL 10, MET25, ADH2, MEL 1, CUP 1, HSE, AOX, MOX, SV40, CaMV, Opaque-2, GRE, ARE, PGK/ARE hybrid, CYC/GRE hybrid, TPI/α2 operator, AOX 1, MOX A.

More preferably, however the promoter is selected from hybrid promoters such as PGK/ARE hybrid, CYC/GRE hybrid or from synthetic promoters. Such promoters can be controlled without interfering too much with the regulation of native genes' in the expression host.

In the following, examples of known yeast promoters that may be used in conjunction with the present invention are shown. The examples are by no way limiting and only serve to indicate to the skilled practitioner how to select or design promoters that are useful according to the present invention.

Although numerous transcriptional promoters which are functional in yeasts have been described in the literature, only some of them have proved effective for the production of polypeptides by the recombinant route. There may be mentioned in particular the promoters of the PGK genes (3-phosphoglycerate kinase, TDH genes encoding GAPDH (Glyceraldehyde phosphate dehydrogenase), TEF1 genes (Elongation factor 1), MFα1 (α sex pheromone precursor) which are considered as strong constitutive promoters or alternatively the regulatable promoter CYCl which is repressed in the presence of glucose or PHO5 which can be regulated by thiamine. However, for reasons which are often unexplained, they do not always allow the effective expression of the genes which they control. In this context, it is always advantageous to be able to have new promoters in order to generate new effective host/vector systems. Furthermore, having a choice of effective promoters in a given cell also makes it possible to envisage the production of multiple proteins in this same' cell (for example several enzymes of the same metabolic chain) while avoiding the problems of recombination between homologous sequences.

In general, a promoter region is situated in the 5' region of the genes and comprises all the elements allowing the transcription of a DNA fragment placed under their control, in particular:

(1) a so-called minimal promoter region comprising the TATA box and the site of initiation of transcription, which determines the position of the site of initiation as well as the basal level of transcription. In Saccharomyces cerevisiae, the length of the minimal promoter region is relatively variable. Indeed, the exact location of the TATA box varies from one gene to another and may be situated from -40 to - 120 nucleotides upstream of the site of the initiation (Chen and Struhl, 1985, EMBO J., 4, 3273-3280)

(2) sequences situated upstream of the TATA box (immediately upstream up to several hundreds of nucleotides) which make it possible to ensure an effective level of transcription either constitutively (relatively constant level of transcription all along the cell cycle, regardless of the conditions of culture) or in a regulatable manner (activation of transcription in the presence of an activator and/or repression in the presence of a repressor). These sequences, may be of several types: activator, inhibitor, enhancer, inducer, repressor and may respond to cellular factors or varied culture conditions.

Examples of such promoters are the ZZA1 and ZZA2 promoters disclosed in US 5,641,66.1, the EF1-α protein promoter and the ribosomal protein S7 gene promoter disclosed in WO 97/44470" the COX 4 promoter and two unknown promoters-(SEQ ID No: 1 and 2 in the document) disclosed in US 5,952,195. Other useful promoters include the HSP150 promoter disclosed in WO 98/54339 and the SV40 and RSV promoters disclosed in US 4,870,013 as well as the PyK and GAPDH promoters disclosed in EP 0 329 203 A1.

More preferably the invention employs the use of synthetic promoters. Synthetic promoters are often constructed by combining the minimal promoter region of one gene with the upstream regulating sequences of another gene. Enhanced promoter control may be obtained by modifying specific sequences in the upstream regulating sequences, e.g. through substitution or deletion or through inserting multiple copies of specific regulating sequences. One advantage of using synthetic promoters is that they can be controlled without interfering too much with the native promoters of the host cell.

One such synthetic yeast promoter comprises promoters or promoter elements of two different yeast-derived genes, yeast killer toxin leader peptide, and amino terminus of IL-1 β (WO 98/54339).

Another example of a yeast synthetic promoter is disclosed in US 5,436,136 (Hinnen et al), which concerns a yeast hybrid promoter including a 5' upstream promoter element comprising upstream activation site(s) of the yeast PHO5 gene and a 3' downstream promoter element of the yeast GAPDH gene starting at nucleotide -300 to -180 and ending at nucleotide -1 of the GAPDH gene.

Another example of a yeast synthetic promoter is disclosed in US 5,089,398 (Rosenberg et al). This disclosure describes a promoter with the general formula

-(P.R.(2)-P.R.(1))-

wherein:

P.R.(1) is the promoter region proximal to the coding sequence and having the transcription initiation site, the RNA polymerase binding site, and including the TATA box, the CAAT sequence, as well as translational regulatory signals, e.g., capping sequence, as appropriate;

P.R.(2) is the promoter region joined to the 5'-end of P.R.(1) associated with enhancing the efficiency of transcription of the RNA polymerase binding region;

In US 4,945,046 (Horii et al) discloses a further example of how to design a synthetic yeast promoter. This specific promoter comprises promoter elements derived both from yeast and from a mammal. The hybrid promoter consists essentially of Saccharomyces cerevisiae PHO5 or GAP-DH promoter from which the upstream activation site (UAS) has been deleted and replaced by the early enhancer region derived from SV40 virus.

Co-ordinated expression of gene subsets can also be utilised to identify which heterologous genes are responsible for the production of a given phenotype.

In the following the sequence of steps to be taken when starting with the isolation of mRNA until insertion to an entry vector for providing the cells according to the invention is described. In short the sequence may include the following steps

i) isolating mRNA from an expression state,

ii) obtaining substantially full length cDNA clones corresponding to the mRNA sequences,

iii) inserting the substantially full length cDNA clones into a cloning site in a cassette in a primary vector, said cassette being of the general formula in 5'→3' direction:

[RS1-RS2-SP-PR-CS-TR-SP-RS2'-RS1']

wherein CS denotes a cloning site.

## Expression cassettes

The expression cassettes according to the present invention are preferably arranged as a cassette of nucleotides in a highly ordered sequence, the cassette having the general formula in 5'→3' direction:

[RS1-RS2-SP-PR-CS-TR-SP-RS2'-RS1']

wherein RS1 and RS1' denote restriction sites, RS2 and RS2' denote restriction sites different from RS1 and RS1', SP denotes a spacer sequence of at least two nucleotide, PR denotes a promoter, CS denotes a cloning site, and TR denotes a terminator, all of them being as discussed elsewhere in this specification.

It is an advantage to have two different restriction sites flanking both sides of the expression construct. By treating the primary vectors with restriction enzymes cleaving both restriction sites, the expression construct and the primary vector will be left with two non-compatible ends. This facilitates a concatenation process, since the empty vectors do not participate in the concatenation of expression constructs.

In principle, any restriction site, for which a restriction enzyme is known can be used. These include the restriction enzymes generally known and used in the field of molecular biology such as those described in Sambrook, Fritsch, Maniatis, "A laboratory Manual", 2nd edition. Cold Spring Habor Laboratory Press, 1989.

The restriction site recognition sequences preferably are of a substantial length, so that the likelihood of occurrence of an identical restriction site within the cassette is minimised. Thus the first restriction site may comprise at least 6 bases, but more preferably the recognition sequence comprises at least 7 or 8 bases. Restriction sites having 7 or more non N bases in the recognition sequence are generally known as "rare restriction sites" (see example 13). However, the recognition sequence may also be at least 10 bases, such as at least 15 bases, for example at least 16 bases, such as at least 17 bases, for example at least 18 bases, such as at least 18 bases, for example at least 19 bases, for example at least 20 bases, such as at least 21 bases, for example at least 22 bases, such as at least 23 bases, for example at least 25 bases, such as at least 30 bases, for example at least 35 bases, such as at least 40 bases, for example at least 45 bases, such as at least 50 bases.

Preferably the first restriction site RS1 and RS1' is recognised by a restriction enzyme generating blunt ends of the double stranded nucleotide sequences. By generating blunt ends at this site, the risk that the vector participates in a subsequent concatenation is greatly reduced. The first restriction site may also give rise to sticky ends, but these are then preferably non-compatible to the sticky ends resulting from the second restriction site, RS2 and RS2'.

According to a preferred embodiment of the invention, the second restriction site, RS2 and RS2' comprises a rare restriction site. Thus, the longer the recognition sequence of the rare restriction site the more rare it is and the less likely is it that the restriction enzyme recognising it will cleave the nucleotide sequence at other - undesired - positions.

The rare restriction site may furthermore serve as a PCR priming site. Thereby it is possible to copy the cassettes via PCR techniques and thus indirectly "excise" the cassettes from a vector.

Single-stranded compatible ends may be created by digestion with restriction enzymes. For concatenation a preferred enzyme for excising the cassettes would be a rare cutter, i.e. an enzyme that recognises a sequence of 7 or more nucleotides. Examples of enzymes that cut very rarely are the meganucleases, many of which are intron encoded, like e.g. I-Ceu I, I-Sce I, I-Ppo I, and PI-Psp I(see example 13d for more). Other preferred enzymes recognize a sequence of 8 nucleotides like e.g. Asc I, AsiS I, CciN I, CspB I, Fse I, MchA I, Not I, Pac I, Sbf I, Sda I, Sgf I, SgrA I, Sse232 I, add Sse8387 I, all of which create single stranded, palindromic compatible ends.

Other preferred rare cutters, which may also be used to control orientation of individual cassettes in the concatemer are enzymes that recognize non-palindromic sequences like e.g. Aar I, Sap I, Sfi I, Sdi I, and Vpa (see example 13c for more).

Alternatively, cassettes can be prepared by the addition of restriction sites to the ends, e.g. by PCR or ligation to linkers (short synthetic dsDNA molecules). Restriction enzymes are continuously being isolated and characterised and it is anticipated that many of such novel enzymes can be used to generate single-stranded compatible ends according to the present invention.

It is conceivable that single stranded compatible ends can be made by cleaving the vector with synthetic cutters. Thus, a reactive chemical group that will normally be able to cleave DNA unspecifically can cut at specific positions when coupled to another molecule that recognises and binds to specific sequences. Examples of molecules that recognise specific dsDNA sequences are DNA, PNA, LNA, phosphothioates, peptides, and amides. See e.g. Armitage, B.(1998) Chem. Rev. 98: 1171-1200, who describes photocleavage using e.g. anthraquinone and UV light; Dervan P.B. & Bürli R.W. (1999) Curr. Opin. Chem. Biol. 3: 688-93 describes the specific binding of polyamides to DNA; Nielsen, P.E. (2001) Curr. Opin. Biotechnol. 12: 16-20 describes the specific binding of PNA to DNA, and Chemical Reviews special thematic issue: RNA/DNA Cleavage (1998) vol. 98 (3) Bashkin J.K. (ed.) ACS publications, describes several examples of chemical DNA cleavers.

Single-stranded compatible ends may also be created e.g. by using PCR primers including dUTP and then treating the PCR product with Uracil-DNA glycosylase (Ref: US 5,035,996) to degrade part of the primer. Alternatively, compatible ends can be created by tailing both the vector and insert with complimentary nucleotides using Terminal Transferase (Chang, LMS, Bollum TJ (1971) J Biol Chem 246:909).

The spacer sequence located between the RS2 and the PR sequence is preferably a non-transcribed spacer sequence. The purpose of the spacer sequence(s) is to minimise recombination between different concatemers present in the same cell or between cassettes present in the same concatemer, but it may also serve the purpose of making the nucleotide sequences in the cassettes more "host" like. A further purpose of the spacer sequence is to reduce the occurrence of hairpin formation between adjacent palindromic sequences, which may occur when cassettes are assembled head to head or tail to tail. Spacer sequences may also be convenient for introducing short conserved nucleotide sequences that may serve e.g. as PCR primer sites or as target for hybridization to e.g. nucleic acid or PNA or LNA probes allowing affinity purification of cassettes.

The cassette may also optionally comprise another spacer sequence of at least two nucleotides between TR and RS2. When cassettes are cut out from a vector and concatenated into concatemers of cassettes, the spacer sequences together ensure that there is a certain distance between two successive identical promoter or terminator sequences. This distance may comprise at least 50 bases, such as at least 60 bases, for example at least 75 bases, such as at least 100 bases, for example at least 150 bases, such as at least 200 bases, for example at least 250 bases, such as at least 300 bases, for example at least 400 bases, for example at least 500 bases, such as at least 750 bases, for example at least 1000 bases, such as at least 1100 bases, for example at least 1200 bases, such as at least 1300 bases, for example at least 1400 bases, such as at least 1500 bases, for example at least 1600 bases, such as at least 1700 bases, for example at least 1800 bases, such as at least 1900 bases, for example at least 2000 bases, such as at least 2100 bases, for example at least 2200 bases, such as at least 2300 bases, for example at least 2400 bases, such as at least 2500 bases, for example at least 2600 bases, such as at least 2700 bases, for example at least 2800 bases, such as at least 2900 bases, for example at least 3000 bases, such as at least 3200 bases, for example at least 3500 bases, such as at least 3800 bases, for example at least 4000 bases, such as at least 4500 bases, for example at least 5000 bases, such as at least 6000 bases.

The number of the nucleotides between the spacer located 5' to the PR sequence and the one located 3' to the TR sequence may be any However, it may be advantageous to ensure that at least one of the spacer sequences comprises between 100 and 2500 bases, preferably between 200 and 2300 bases, more preferably between 300 and 2100 bases, such as between 400 and 1900 bases, more preferably between 500 and 1700 bases, such as between 600 and 1500 bases, more preferably between 700 and 1400 bases.

If the intended host cell is yeast, the spacers present in a concatemer should perferably comprise a combination of a few ARSes with varying lambda phage DNA fragments.

Preferred examples of spacer sequences include but are not limited to: Lamda phage DNA, prokaryotic genomic DNA such as E.-coli genomic DNA, ARSes.

The cloning site in the cassette in the primary vector should be designed so that any nucleotide sequence can be cloned into it.

The cloning site in the cassette preferably allows directional cloning. Hereby is ensured that transcription in a host cell is performed from the coding strand in the intended direction and that the translated peptide is identical to the peptide for which the original nucleotide sequence codes.

However according to some embodiments it may be advantageous to insert the sequence in opposite direction. According to these embodiments, so-called antisense constructs may be inserted which prevent functional expression of specific genes involved in specific pathways. Thereby it may become possible to divert metabolic intermediates from a prevalent pathway to another less dominant pathway.

The cloning site in the cassette may comprise multiple cloning sites, generally known as MCS or polylinker sites, which is a synthetic DNA sequence encoding a series of restriction endonuclease recognition sites. These sites are engineered for convenient cloning of DNA into a vector at a specific position and for directional cloning of the insert.

Cloning of cDNA does not have to involve the use of restriction enzymes. Other alternative systems include but are not limited to:

- Creator™ Cre-IoxP system from Clontech, which uses recombination and IoxP sites
- use of Lambda attachment sites (att-λ), such as the Gateway™ system from Life Technologies.

Both of these systems are directional.

The role of the terminator sequence is to limit transcription to the length of the coding sequence. An optimal terminator sequence is thus one, which is capable of performing this act in the host cell.

In prokaryotes, sequences known as transcriptional terminators signal the RNA polymerase to release the DNA template and stop transcription of the nascent RNA.

In eukaryotes, RNA molecules are transcribed well beyond the end of the mature mRNA molecule. New transcripts are enzymatically cleaved and modified by the addition of a long sequence of adenylic acid residues known as the poly-A tail. A polyadenylation consensus sequence is located about 10 to 30 bases upstream from the actual cleavage site.

Preferred examples of yeast derived terminator sequences include, but are not limited to: ADN1, CYC1, GPD, ADH1 alcohol dehydrogenase.

Depending on the nature of the host cell, it may be advantageous that at least one cassette comprises an intron between the promoter and the expressible nucleotide sequence, more preferable that substantially all cassettes comprise an intron between the promoter and the expressible nucleotide sequence. The choice of intron sequence depends on requirements of the host cell.

Thus, optionally the cassette in the vector comprises an intron sequence, which may be located 5' or 3' to the expressible nucleotide sequence. The design and layout of introns is well known in the art. The choice of intron design largely depends on the intended host cell, in which the expressible nucleotide sequence is eventually to be expressed. The effects of having intron sequence in the expression cassettes are those generally associated with intron sequences.

Examples of yeast introns can be found in the literature and in specific databases such as Ares Lab Yeast Intron Database (Version 2.1) as updated on 15 April 2000. Earlier versions of the database as well as extracts of the database have been published in: "Genome-wide bioinformatic and molecular analysis of introns in Saccharomyces cerevisiae." by Spingola M, Grate L, Haussler D, Ares M Jr. (RNA 1999 Feb;5(2):221-34) and "Test of intron predictions reveals novel splice sites, alternatively spliced mRNAs and new introns in meiotically regulated genes of yeast." by Davis CA, Grate L, Spingola M, Ares M Jr, (Nucleic Acids Res 2000 Apr 15;28(8):1700-6).

**Primary vectors (entry vectors)**

By the term entry vector is meant a vector for storing and amplifying cDNA or other expressible nucleotide sequences using the cassettes according to the present invention. The entry vectors or primary vectors are preferably able to propagate in E. coli or any other suitable standard host cell. It should preferably be amplifiable and amenable to standard normalisation and enrichment procedures.

The entry vector may be of any type of DNA that has the basic requirements of a) being able to replicate itself in at least one suitable host organism and b) allows insertion of foreign DNA which is then replicated together with the vector and c) preferably allows selection of vector molecules that contain insertions of said foreign DNA. In a preferred embodiment the vector is able to replicate in standard hosts like yeasts, bacteria and it should preferably have a high copy number per host cell. It is also preferred that the vector in addition to a host specific origin of replication, contains an origin of replication for a single stranded virus, such as e.g. the f1 origin for filamentous phages. This will allow the production of single stranded nucleic acid which may be useful for normalisation and enrichment procedures of cloned sequences. A vast number of cloning vectors have been described which are commonly used and references may be given to e.g. Sambrook,J; Fritsch, E.F; and Maniatis T. (1989) Molecular Cloning: A laboratory manual. Cold Spring Harbour Laboratory Press, USA, Netherlands Culture Collection of Bacteria (www.cbs.knaw.nl/NCCB/collection.htm) or Department of Microbial Genetics, National Institute of Genetics, Yata 1111 Mishima Shizuoka 411-8540, Japan (www.shigen.nig.ac.ip/cvector/cvector.html). A few type-examples that are the parents of many popular derivatives are M13mp10, pUC18, Lambda gt 10, and pYAC4. Examples of primary vectors include but are not limited to M13K07, pBR322, pUC18, pUC19, pUC118, pUC119, pSP64, pSP65, pGEM-3, pGEM-3Z, pGEM-3Zf(-), pGEM-4, pGEM-4Z, πAN13, pBluescript II, CHARON 4A, $\lambda^+$, CHARON 21 A, CHARON 32, CHARON 33, CHARON 34, CHARON 35, CHARON 40, EMBL3A, λ2001, λDASH, λFIX, λgt10, λgt11, λgt18, λgt20, λgt22, λORF8, λZAP/R, pJB8, c2RB, pcos1 EMBL

methods for cloning of cDNA or genomic DNA into a vector are well known in the art. Reference may be given to J. Sambrook, E.F. Fritsch, T. Maniatis: Molecular Cloning, A Laboratory Manual (2nd edition, Cold Spring Harbor Laboratory Press, 1989).

One example of a circular model entry vector is described in Figure 11, The vector, EVE contains the expression cassette, R1-R2-Spacer-Promoter-Multi Cloning Site-Terminator-Spacer-R2-R1. The vector furthermore contains a gene for ampicillin resistance, AmpR, and an origin of replication for E.coli, ColE1.

The entry vectors EVE4, EVE5, and EVE8 shown in Figures 12, 13 and 14. These all contain SrfI as R1 and AscI as R2. Both of these sites are palindromic and are regarded as rare restriction sites having 8 bases in the recognition sequence. The vectors furthermore contain the AmpR ampicillin resistance gene, and the ColE1 origin or replication for E.coli as well as f1, which is an origin of replication for filamentous phages, such as M13. EVE4 (Fig. 12) contains the MET25 promoter and the ADH1 terminator. Spacer 1 and spacer 2 are short sequences deriving from the multiple cloning site, MCS. EVE5 (Fig. 13) contains the CUP1 promoter and the ADH1 terminator. EVE8 (Fig. 14) contains the CUP1 promoter and the ADH1 terminator. The spacers of EVE8 are a 550 bp lambda phage DNA (spacer 3) and an ARS sequence from yeast (spacer 4).

## Nucleotide library (entry library)

A schematic illustration of the steps leading from expression steps to nucleotide library are illustrated in figure 9

Methods as well as suitable vectors and host cells for constructing and maintaining a library of nucleotides sequences in a cell are well known in the art. The primary requirement for the library is that is should be possible to store and amplify in it a number of primary vectors (constructs) according to this invention, the vectors (constructs) comprising expressible nucleotide sequences from at least one expression state and wherein at least two vectors (constructs) are different.

One specific example of such a library is the well known and widely employed cDNA libraries. The advantage of the cDNA library is mainly that it contains only DNA sequences corresponding to transcribed messenger RNA in a cell. Suitable methods are also present to purify the isolated mRNA or the synthesised cDNA so that only substantially full-length cDNA is cloned into the library.

Methods for optimisation of the process to yield substantially full length cDNA may comprise size selection, e.g. electrophoresis, chromatography, precipitation or may comprise ways of increasing the likelihood of getting full length cDNAs, e.g. the SMART™ method (Clonetech) or the CapTrap™ method (Stratagene).

Preferably the method for making the nucleotide library comprises obtaining a substantially full length cDNA population comprising a normalised representation of cDNA species. More preferably a substantially full length cDNA population comprises a normalised representation of cDNA species characteristic of a given expression state.

Normalisation reduces the redundancy of clones representing abundant mRNA species and increases the relative representation of clones from rare mRNA species.

Methods for normalisation of cDNA libraries are well known in the art. Reference may be given to suitable protocols for normalisation such as those described in US 5,763,239 (DIVERSA) and WO 95/08647 and WO 95/11986. and Bonaldo, Lennon, Scares, Genome Research 1996; 6:791-806; Ali, Holloway, Taylor, Plant Mol Biol Reporter, 2000, 18:123-132.

Enrichment methods are used to isolate clones representing mRNA which are characteristic of a particular expression state. A number of variations of the method broadly termed as subtractive hybrisation are known in the art. Reference may be given to Sive, John, Nucleic Acid Res, 1988, 16:10937; Diatchenko, Lau, Campbell et al, PNAS, 1996, 93:6025-6030; Carninci, Shibata, Hayatsu, Genome Res, 2000, 10:1617-30, Bonaldo, Lennon, Soares, Genome Research 1996, 6:791-806; Ali, Holloway, Taylor, Plant Mol Biol Reporter, 2000, 18:123-132. For example, enrichment may be achieved by doing additional rounds of hybridization similar to normalization procedures, using e.g. cDNA from a library of abundant clones or simply a library representing the uninduced state as a driver against a tester library from the induced state. Alternatively mRNA or PCR amplified cDNA derived from the expression state of choice can be used to subtract common sequences from a tester library. The choice of driver and tester population will depend on the nature of target expressible nucleotide sequences in each particular experiment.

Finally, enrichment may be achieved by subtractive hybridisation followed by colony picking.

In the library an expressible nucleotide sequence coding for one peptide is preferably found in different but similar vectors under the control of different promoters. Preferably the library comprises at least three primary vectors with an expressible nucleotide sequence coding for the same peptide under the control of three different promoters. More preferably the library comprises at least four primary vectors with an expressible nucleotide sequence coding for the same peptide under the control of four different promoters. More preferably the library comprises at least five primary vectors with an expressible nucleotide sequence coding for the same peptide under the control of five different promoters, such as comprises at lest six primary vectors with an expressible nucleotide sequence coding for the same peptide under the control of six different promoters, for example comprises at least seven primary vectors with an expressible nucleotide sequence coding for the same peptide under the control of seven different promoters, for example comprises at least eight primary vectors with an expressible nucleotide sequence coding for the same peptide under the control of eight different promoters, such as comprises at least nine primary vectors with an expressible nucleotide sequence coding for the same peptide under the control of nine different promoters, for example comprises at least ten primary vectors with an expressible nucleotide sequence coding for the same peptide under the control of ten different promoters.

The expressible nucleotide sequence coding for the same peptide preferably comprises essentially the same nucleotide sequence, more preferably the same nucleotide sequence.

By having a library with what may be termed one gene under the control of a number of different promoters in different vectors, it is possible to construct from the nucleotide library an array of combinations of genes and promoters. Preferably, one library comprises a complete or substantially complete combination such as a two dimensional array of genes and promoters, wherein substantially all genes are found under the control of substantially all of a selected number of promoters.

According to another embodiment of the invention the nucleotide library comprises combinations of expressible nucleotide sequences combined in different vectors with different spacer sequences and/or different intron sequences. Thus any one expressible nucleotide sequence may be combined in a two, three, four or five dimensional array with different promoters and/or different spacers and/or different introns and/or different terminators. The two, three, four or five dimensional array may be complete or incomplete, since not all combinations will have to be present.

The library may suitably be maintained in a host cell comprising prokaryotic cells or eukaryotic cells. Preferred prokaryotic host organisms may include but are not limited to Escherichia coli, Bacillus subtilis, Streptomyces lividans, Streptomyces coelicolor Pseudomonas aeruginosa, Myxococcus xanthus.

Yeast species such as Saccharomyces cerevisiae (budding yeast), Schizosaccharomyces pombe (fission yeast), Pichia pastoris, and Hansenula polymorpha (methylotropic yeasts) may also be used. Filamentous ascomycetes, such as Neurospora crassa and Aspergillus nidulans may also be used. Plant cells such as those derived from Nicotiana and, Arabidopsis are preferred. Preferred mammalian host cells include but are not limited to those derived from humans, monkeys and rodents, such as chinese hamster ovary (CHO) cells, NIH/3T3, COS, 293, VERO, HeLa etc (see Kriegler M. in "Gene Transfer and Expression: A Laboratory Manual", New York, Freeman & Co. 1990).

**Concatemers**

For the purposes of providing a method for assembling multiple expression cassettes ("cassettes") into a single host cell, and allowing their facile remixing between cells, the expression cassettes are assembled into concatemers.

A concatemer is a series of linked units. The concatemers according to the invention may comprise a selection of expressible nucleotide sequences from just one expression state and can thus be assembled from one library representing this expression state or it may comprise cassettes from a number of different expression states. The concatemers according to the invention are especially suitable for ligating into an artificial chromosome, which may be inserted into a host cell for coordinated expression. For this purpose, the variation among and between cassettes may be such as to minimise the chance of cross over as the host cell undergoes cell division such as through minimising the level of repeat sequences occurring in any one concatemer, since it is not an object of this embodiment of the invention to obtain recombination of concatemers with a segment in the host genome or an epitope of the host cells nor is it an object to obtain intra- or extra concatemeric recombination.

According to a preferred embodiment of the invention the concatemer comprises at least a first cassette and a second cassette, said first cassette being different from said second cassette. More preferably, the concatemer comprises cassettes, wherein substantially all cassettes are different. The difference between the cassettes may arise from differences between promoters, and/or expressible nucleotide sequences, and/or spacers, and/or introns and/or terminators.

The number of cassettes in a single concatemer is largely determined by the host species into which the concatemer is eventually to be inserted and the vector through which the insertion is carried out. The concatemer thus may comprise at least 10 cassettes, such as at least 15, for example at least 20, such as at least 25, for example at least 30, such as from 30 to 60 or more than 60, such as at least 75, for example at least 100, such as at least 200, for example at least 500, such as at least 750, for example at least 1000, such as at least 1500, for example at least 2000 cassettes.

Each of the cassettes may be laid out as described above.

Thus, in a preferred embodiment a concatemer is used to denote a number of serially linked nucleotide cassettes, wherein at least two of the serially linked nucleotide units comprises a cassette having the basic structure

$$[rs_2\text{-SP-PR-X-TR-SP-}rs_1]$$

wherein

$rs_1$ and $rs_2$ together denote a restriction site,
SP denotes a spacer of at least two nucleotide bases,
PR denotes a promoter, capable of functioning in a cell,
X denotes an expressible nucleotide sequence,
TR denotes a terminator, and
SP denotes a spacer of at least two nucleotide bases.

wherein the variables of the cassette have the meaning as defined elsewhere in this specification. Optionally the cassettes comprise an intron sequence between the promoter and the expressible nucleotide sequence and/or between the terminator and the expressible nucleotide sequence as discussed above.

According to one aspect of the invention, a concatemer comprises cassettes with expressible nucleotide from different expression states, so that non-naturally occurring combinations or non-native combinations of expressible nucleotide sequences are obtained.

According to a preferred embodiment of the invention the concatemer comprises at least a first cassette and a second cassette, said first cassette being different from said second cassette. More preferably, the concatemer comprises cassettes, wherein substantially all cassettes are different. The difference between the cassettes may arise from differences between promoters, and/or expressible nucleotide sequences, and/or spacers, and/or terminators, and/or introns.

The concatenation may be carried out in different ways.

Cassettes to be concatenated are normally excised from a vector or they are synthesised through PCR. After excision the cassettes may be separated from the vector through size fractionation such as gel filtration or through tagging of known sequences in the cassettes. The isolated cassettes may then be ligated together either through interaction between sticky ends or through ligation of blunt ends.

More preferably the cassettes may be concatenated without an intervening purification step through excision from a vector with two restriction enzymes, one leaving sticky ends on the cassettes and the other one leaving blunt ends in the vectors.

An alternative way of producing concatemers free of vector sequences would be to PCR amplify the cassettes from a single stranded primary vector. The PCR product must include the restriction sites RS2 and RS2' which are subsequently cleaved by its cognate enzyme(s). Concatenation can then be performed using the digested PCR product, essentially without interference from the single stranded primary vector template or the small double stranded fragments, which have been cut form the ends.

When the vectors comprising the cassettes are single stranded, the cassettes may be excised and be made double stranded through PCR techniques, which only prime the cassette sequence and not the vector sequence. Sticky ends can be made by cleaving with a restriction enzyme leaving sticky ends and the cassettes can be assembled without interaction from the single stranded vector fragments.

The concatemer may be assembled or concatenated by concatenation of at least two cassettes of nucleotide sequences each cassette comprising a first sticky end, a spacer sequence, a promoter, an expressible nucleotide sequence, a terminator, and a second sticky end.

After concatenation has been completed, concatemers of the desired size may be selected through size selection, such as selection for concatemers having at least 10 cassettes, such as at least 15, for example at least 20, such as at least 25, for example at least 30, such as from 30 to 60 or more than 60, such as at least 75, for example at least 100, such as at least 200, for example at least 500, such as at least 750, for example at least 1000, such as at least 1500, for example at least 2000 cassettes. The number of cassettes in each concatemer may be controlled by size fractionation after concatenation, since the size of the concatemers is approximately proportional to the number of cassettes.

Preferably at least one inserted concatemer in each cell comprises a selectable marker. Selectable markers generally provide a means to select, for growth, only those cells which contain a vector. Such markers are of two types: drug resistance and auxotrophic. A drug resistance marker enables cells to detoxify an exogenously added drug that would otherwise kill the cell. Auxotrophic markers allow cells grow in media lacking an essential component by enabling cells to synthesise the essential component (usually an amino acid).

Illustrative and non-limiting examples of common selectable markers with a brief description of their mode of action follow:

**Prokaryotic**

- Ampicillin: interferes with a terminal reaction in bacterial cell wall synthesis. The resistance gene (bla) encodes beta-lactamase which cleaves the beta-lactam ring of the antibiotic thus detoxifying it.
- Tetracycline: prevents bacterial protein synthesis by binding to the 30S ribosomal subunit. The resistance gene (tet) specifies a protein that modifies the bacterial membrane and prevents transport of the antibiotic into the cell.
- Kanamycin: binds to the 70S ribosomes and causes misreading of messenger RNA. The resistant gene (nptH) modifies the antibiotic and prevents interaction with the ribosome.
- Streptomycin: binds to the 30S ribosomal subunit, causing misreading of messenger RNA. The resistance gene (Sm) modifies the antibiotic and prevents interaction with the ribosome.
- Zeocin: this new bleomycin-family antibiotic intercalates into the DNA and cleaves it. The Zeocin resistance gene encodes a 13,665 dalton protein. This protein confers resistance to Zeocin by binding to the antibiotic and preventing it from binding DNA. Zeocin is effective on most aerobic cells and can be used for selection in mammalian cell lines, yeast, and bacteria.
- Auxotrophic markers.

**Eukaryotic**

- Hygromycin: a aminocyclitol that inhibits protein synthesis by disrupting ribosome translocation and promoting mistranslation. The resistance gene (hph) detoxifies hygromycin -B- phosphorylation.
- Histidinol: cytotoxic to mammalian cells by inhibiting histidyl-tRNA synthesis in histidine free media. The resistance gene (hisD) product inactivates histidinol toxicity by converting it to the essential amino acid, histidine.
- Neomycin (G418): blocks protein synthesis by interfering with ribosomal functions. The resistance gene ADH encodes amino glycoside phosphotransferase which detoxifies G418.
- Uracil: Laboratory yeast strains carrying a mutated gene which encodes μorotidine -5'- phosphate decarboxylase, an enzyme essential for uracil biosynthesis, are unable to grow in the absence of exogenous uracil. A copy of the wild-type gene (ura4+, S. pombe or URA3 S. cerevisiae) carried on the vector will complement this defect in transformed cells.
- Adenosine: Laboratory strains carrying a deficiency in adenosine synthesis maybe complemented by a vector carrying the wild type gene, ADE 2.
- Amino acids: Vectors carrying the wild-type genes for LEU2, TRP 1, HIS 3 or LYS 2 may be used to complement strains of yeast deficient in these genes.
- Zeocin: this new bleomycin-family antibiotic intercalates into the DNA and cleaves it. The Zeocin resistance gene encodes a 13,665 dalton protein. This protein confers resistance to Zeocin by binding to the antibiotic and preventing it from binding DNA. Zeocin is effective on most aerobic cells and can be used for selection in mammalian cell lines, yeast, and bacterial.

The number of concatemers in one single cell may be at least one concatemer per cell, preferably at least 2 concatemers per cell, more preferably 3 per cell, such as 4 per cell, more preferably 5 per cell, such as at least 5 per cell, for example at least 6 per cell, such as 7, 8, 9 or 10 per cell, for example more than 10 per cell. As described above, each concatemer may preferably comprise up to 1000 cassettes, and it is envisages that one concatemer may comprise up to 2000 cassettes. By inserting up to 10 concatemers into one single cell, this cell may thus be enriched with up to 20,000 new expressible genes, which under suitable conditions may be turned on and off by regulation of the regulatable promoters. However it may be more preferable to provide cells having anywhere between 10 and 1000 novel genes, such as 20-900 novel genes, for example 30 to 800 novel genes, such as 40 to 700 novel genes, for example 50 to 600 novel genes, such as from 60 to 300 novel genes. The genes may advantageously be located on 1 to 10 such as from 2 to 5 different concatemers in the cells. Each concatemer may advantageously comprise from 10 to 1000 genes, such as from 10 to 750 genes, such as from 10 to 500 genes, such as from 10 to 200 genes, such as from 20 to 100 genes, for example from 30 to 60 genes.

The concatemers may be inserted into the host cells according to any known transformation technique, preferably according to such transformation techniques that ensure stable and not transient transformation of the host cell. The concatemers may thus be inserted as an artificial chromosome which is replicated by the cells as they divide or they may be inserted into the chromosomes of the host cell. The concatemer may also be inserted in the form of a plasmid such as a plasmid vector, a phage vector, a viral vector, a cosmid vector, that is replicated by the cells as they divide. Any combination of the three insertion methods is also possible. One or more concatemers may thus be integrated into the chromosome(s) of the host cell and one or more concatemers may be inserted as plasmids or artificial chromosomes. One or more concatemers may be inserted as artificial chromosomes and one or more may be inserted into the same cell via a plasmid.

The basic requirements for a functional artificial chromosome have been described in US 4,464,472, the contents of which is hereby incorporated by reference. An artificial chromosome or a functional minichromosome, as it may also be termed must comprise a DNA sequence capable of replication and stable mitotic maintenance in a host cell comprising a DNA segment coding for centromere-like activity during mitosis of said host and a DNA sequence coding for a replication site recognized by said host.

Suitable artificial chromosomes include a Yeast Artificial Chromosome (YAC) (see e.g. Murray et al, Nature 305:189-193; or US 4,464,472), a mega Yeast Artificial Chromosome (mega YAC), a Bacterial Artificial Chromosome (BAC), a mouse artificial chromosome, a Mammalian Artificial Chromosome (MAC) (see e.g. US 6,133,503 or US 6,077,697), an Insect Artificial Chromosome (BUGAC), an Avian Artificial Chromosome (AVAC), a Bacteriophage Artificial Chromosome, a Baculovirus Artificial Chromosome, a plant artificial chromosome (US 5,270,201), a BIBAC vector (US 5,977,439) or a Human Artificial Chromosome (HAC).

The artificial chromosome is preferably so large that the host cell perceives it as a "real" chromosome and maintains it and transmits it as a chromosome. For yeast and other suitable host species, this will often correspond approximately to the size of the smallest native chromosome in the species. For Saccharomyces, the smallest chromosome has a size of 225 Kb.

MACs may be used to construct artificial chromosomes from other species, such as insect and fish species. The artificial chromosomes preferably are fully functional stable chromosomes. Two types of artificial chromosomes may be used. One type, referred to as SATACs [satellite artificial chromosomes] are stable heterochromatic chromosomes, and the other type are minichromosomes based on amplification of euchromatin.

Mammalian artificial chromosomes provide extra-genomic specific integration sites for introduction of genes encoding proteins of interest and permit megabase size DNA integration, such as integration of concatemers according to the invention.

According to another embodiment of the invention, the concatemer may be integrated into the host chromosomes or cloned into other types of vectors, such as a plasmid vector, a phage vector, a viral vector or a cosmid vector.

A preferable artificial chromosome vector is one that is capable of being conditionally amplified in the host cell, e.g. in yeast. The amplification preferably is at least a 10 fold amplification. Furthermore, it is advantageous that the cloning site of the artificial chromosome vector can be modified to comprise the same restriction site as the one bordering the cassettes described above, i.e. RS2 and/or RS2'.

It is also conceivable that recombination can be used to generate concatemers, e.g. through the modification of techniques like the Creator system (Clontech) which uses the Cre-loxP mechanism (ref: Sauer B 1993 Methods Enzymol 225:890-900) to directionally join DNA molecules by recombination or like the Gateway system (Life Technologies, US 5,888,732) using lambda att attachment sites for directional recombination (Landy A 1989, Ann Rev Biochem 58:913). It is envisaged that also lambda cos site dependent systems can be developed to allow concatenation.

The concatemer may be assembled or concatenated by concatenation of at least two cassettes of nucleotide sequences each cassette comprising a first sticky end, a spacer sequence, a promoter, an expressible nucleotide sequence, a terminator, and a second sticky end. A flow chart of the procedure is shown in figure 10a.

Preferably concatenation further comprises

starting from a primary vector

[RS1-RS2-SP-PR-X-TR-SP-RS2'-RS1'],

wherein X denotes an expressible nucleotide sequence,
RS1 and RS1' denote restriction sites,
RS2 and RS2' denote restriction sites different from RS1 and RS1',
SP denotes a spacer sequence of at least two nucleotides,
PR denotes a promoter,
TR denotes a terminator,

i) cutting the primary vector with the aid of at least one restriction enzyme specific for RS2 and RS2' obtaining cassettes having the general formula [$rs_2$-SP-PR-X-TR-SP-$rs_1$] wherein $rs_1$ and $rs_2$ together denote a functional restriction site RS2 or RS2',

ii) assembling the cut out cassettes through interaction between $rs_1$ and $rs_2$.

According to an especially preferred embodiment, vector arms each having a RS2 or RS2' in one end and a non-complementary overhang or a blunt end in the other end are added to the concatenation mixture together with the cassettes described above to further simplify the procedure (see Fig. 10b). One example of a suitable vector for providing vector arms is disclosed in Fig. 16 TRP1, URA3, and HIS3 are auxotrophic marker genes, and AmpR is an antibiotic marker gene. CEN4 is a centromer and TEL are telomeres. ARS1 and PMB1 allow replication in yeast and E. coli respectively. BamH I and Asc I are restriction enzyme recognition sites. The nucleotide sequence of the vector is set forth in SEQ ID NO 4. The vector is digested with BamHI and AscI to liberate the vector arms, which are used for ligation to the concatemer.

The ratio of vector arms to cassettes determines the maximum number of cassettes in the concatemer as illustrated in figure 18a and b. The vector arms preferably are artificial chromosome vector arms such as those described in Fig. 16. Figure 17 illustrates the synthesis of concatamers from entry vector libraries.

It is of course also possible to add stopper fragments to the concatenation solution, the stopper fragments each having a RS2 or RS2' in one end and a non-complementary overhang or a blunt end in the other end. The ratio of stopper fragments to cassettes can likewise control the maximum size of the concatemer.

As an alternative to providing vector arms for the concatenation procedure is possible to ligate the concatemer into an artificial chromosome selected.from the group comprising yeast artificial chromosome, mega yeast artificial chromosome, bacterial artificial chromosome, mouse artificial chromosome, human artificial chromosome.

The number of concatemers in one single cell may be at least one concatemer per cell, preferably at least 2 concatemers per cell, more preferably 3 per cell, such as 4 per cell, more preferably 5 per cell, such as at least 5 per cell, for example at least 6 per cell; such as 7, 8, 9 or 10 per cell, for example more than 10 per cell. As described above, each concatemer may preferably comprise up to 1000 cassettes, and it is envisages that one concatemer may comprise up to 2000 cassettes. By inserting up to 10 concatemers into one single cell, this cell may thus be enriched with up to 20,000 heterologous expressible genes, which under suitable conditions may be turned on and off by regulation of the regulatable promoters.

Often it is more preferable to provide cells having anywhere between 10 and 1000 heterologous genes, such as 20-900 heterologous genes, for example 30 to 800 heterologous genes, such as 40 to 700 heterologous genes, for example 50 to 600 heterologous genes, such as from 60 to 300 heterologous genes or from 100 to 400 heterologous genes which are inserted as 2 to 4 artificial chromosomes each containing one concatemer of genes. The genes may advantageously be located on 1 to 10 such as from 2 to 5 different concatemers in the cells. Each concatemer may advantageously comprise from 10 to 200 genes, such as from 20 to 100 genes, for example from 30 to 60 genes, or from 50 to 100 genes.

## EXAMPLES

**Example 1**: Rescue of expression cassettes from EVAC clones and creation of new EVAC libraries

Cells contain EVAC libraries with the following characteristics:

- EVAC markers: Uracil and Tryptophan
- cDNA libraries used for EVAC construction, , 20 % carrot (root), 20% phaffia (whole organism), 20% carotenoid gene library, 20% Actinidia deliciosa (whole organism), 20% Cantharellus cibarius (whole organism)
- Mixture of entry vectors used to clone the cDNAs, e.g., pEVE4, pEVE5, pEVE13

1) Isolate total DNA (as intact as possible in order to make cassette isolation easier) from an overnight culture using Easy-DNA™ Kit (Invitrogen).

2) Digest over night 10-15 μg chromosomal DNA with 5 units of *Asc*I per μg of DNA. The *Asc*I digests the EVAC into single expression cassettes and the rest of the yeast genome into on average 500 kb fragments.

3) The digested DNA is purified on a "PCR" purification column High Pure™ PCR Product Purification Kit (Roche) (or a filter which holds back fragments bigger then 10 kb) where the chromosomal fragments will be retained and primarily the expression cassettes will be recovered.

4) Digest pEVE1 with AscI and dephosphorylate. (pEVE1 is a modified entry vector, with no linker region in between the *Asc*I sites).

5) Clone expression cassettes into pEVE1 by mixing 0.5 μg of purified expression cassettes, 200ng of entry vector (*Asc*I cut and dephosphorylated) and 0.5 U of T4-DNA ligase (1u/µl) (Roche) in 1 x ligation buffer (Roche). Ligate over night at 16°C.

6) The ligation mixture is then used to transform E.coli by electroporation using 1 µl of 1:10 diluted ligation mixture per transformation (current protocols in molecular biology, section 1.8.4.

7) The synthesis of new EVACs can then be done as described earlier (See examples below).

New EVACs can be made just from these cassettes or by mixing these with cassettes from libraries not previously used. The use of cassettes just from this library would give a larger representation of all possible combinations of the selected genes. This most likely produces more variations of certain classes of molecules and is thus used later on in the evolution process when yeasts with characteristics close to the ideal have been identified.

**Example 2**: Physical re-isolation and re-transformation of EVACs.

1. The EVAC containing population is grown in 5 ml of YPD to an $OD_{600}$ > 1.0
2. Two 100 µl plugs of total DNA are produced as described in BioRad's "CHEF genomic DNA plug kits" manual, Procure n.2
3. EVACs are purified and isolated:

   a. Plugs are cut and loaded into 3 slots of a pulsed field gel
   b. Run PFGE

      i. For EVACs < 1000 kb : Chef III, 1% Agarose, 1/2 strength TBE, 6V/cm, 14°C, 120° angle, 50 - 90 sec. Switch time, 22 h runtime.
      ii. For EVACs > 1000 kb. Chef III, 1% Agarose, 1/2 strength TBE, 6V/cm, 14°C, 120° angle, 60-120 sec. Switch time, 24 h runtime

   c. stain one lane to identify position of EVACs
   d. cut corresponding part of the two non-stained lanes and digest the agarose by agarase treatment following standard procedures e.g. Pulsed Field Gel Electrophoresis. A practical approach.(Ed. A.P. Monaco) Oxford University Press 1995.
   e. Concentrate agarased preparation to 100 µL by ultrafiltration.(e.g. Microcon YM-30, Millipore)
   f. add 400 TE to retentate and repeat concentration step. Repeat and concentrate to 25 µL

4. EVACs are transformed into yeast as before.

**Example 3**: Amplification of EVACs up to 20% of total host DNA prior to EVAC isolation and re-transformation

A YAC vector containing a conditional centromere (the GAL1 promoter in front of the centromere) and a heterologous thymidine kinase (TK) marker can be amplified to constitute up to 20 % of the total DNA content in yeast cells. The centromere is inactivated by inducing transcription from a strong promoter (GAL1) towards conserved sequences of the centromere. The cells are propagated in media containing thymidine, sulfanilamide and methotrexate which selects for cells containing multiple copies of the YAC. (Smith, D. R. et al., 1990, Proc. Natl. Acad. USA, Vol 87, pp. 8242-8246).

**Example 4**: Sexual crosses of yeast cell populations. (Using selective media for diploid selection)

**Cell populations:**

Cell populations with 2 EVACs/cell are obtained by transforming cells that already contain an EVAC with a second EVAC.

Cell population 1 contains EVACs of type 1 and 2 and the host cells are of mating type a.

EVACs of type 1:

- Markers: URA 3, TRP 1, $NPT^{II}$

- cDNA libraries used to make EVACs, e.g., carrot (root), Aloe humilis (flower), Narcissus pseudonarcissus (flower), Licopersicon esculentum (fruit), Olea europaea (leaves)
- Mixture of entry vectors each library is cloned in, e.g., pEVE4, pEVE5, pEVE13 & pEVE14

EVACs of type 2:

- Markers: LEU 2, TRP 1, $NPT^{II}$
- cDNA libraries e.g, phaffia (whole organism), Anubias Barteri (leaves), Acremonium diospyri (whole organism), Phycomyces blakesleeanus (whole organism), Mucor azygosporus (whole organism)
- Mixture of entry vectors each library is cloned in, e.g., pEVE4, pEVE5, pEVE13 & pEVE14

Cell population 2 contains EVACs of type 3 and 4 and the host cells are of mating type α.

EVACs of type 3:

- Markers: URA 3, HIS 3, $NPT^{II}$
- cDNA libraries used, e.g., mouse (skin and placenta), sea urchin (whole organism), carassius auratus, (whole organism), paracheirodon axelrodi (whole organism), cucumaria japonica (whole organism)
- Mixture of entry vectors each library is cloned in, e.g., pEVE4, pEVE5, pEVE13 & pEVE14

EVACs of type 4:

- Markers: LEU 2, HIS 3, $NPT^{II}$
- cDNA libraries used, e.g., Hierodula gransid (head), mouse (eyes), Dyscophus insularis (skin), Gnathonemus petersii (head), Diapherodes jamaicensis (head)
- Mixture of entry vectors each library is cloned in, e.g., pEVE4, pEVE5, pEVE13 & pEVE14

Cell population 3 contains EVACs of type 1 and 2 and the host cells are a 50/50 mixture of mating types a and α.

- cDNA libraries used to produce EVACs, e.g., Skimmia jap. Rubella (leaves), Neurospora crassa (whole organism), Mytilus coruscus (whole organism), Pinus pinaster (leaves), Carica papaya (fruit)

Cell population 4 contains EVACs of type 3 and 4 and the host cells are a 50/50 mixture of mating types a and α.

- cDNA libraries used to produce EVACs, e.g., Cantharellus cibarius (whole organism), Rhizophora mangle (leaves), Fucus vesiculus (leaves), Halichondria okadai (whole organism)

**Remixing round 1:**

1) Construction of Diploid population. Mix cells from freshly grown overnight cultures of cell populations 1 and 2. Distribute cells in agar plates (the plates should allow growth of both haploid strains). Allow mating to proceed for at least 4 hours at 30°C, then wash cells of from plates and incubate the mating mixture in a liquid selective medium that will select for the diploid genotype which contains the 4 different types of EVACs (-URA3, -TRP1, -LEU2,-HIS3).
2) Biological Screen.

a. Induce heterologous genes
b. The diploid cell population is screened for the relevant pharmaceutical property(ies) and a subset of this population is selected
c. Obtain a 10 times representation of selected population by letting it grow on selective medium.
d. Divide selected population in 3 portions, (i) store, (ii) sporulate, (iii) keep for re-screening at a higher selection hurdle

3) Sporulation in liquid media of selected subpopulation.

a. Grow portion (ii) of selected diploid population to an $OD_{600}$ of 2.5 to 3.0 (~8 x $10^7$ cells/ml) in selective medium.
b. Transfer 1 ml culture to a sterile, disposable 15 ml polypropylene tube and centrifuge 5 min at 1200 x g
c. Pour off the supernatant and re-suspend cells in 5 ml sterile water. Vortex to re-suspend cells and spin as in step 2.
d. Pour off supernatant and re-suspend cells in 1 ml of sporulation medium
e. Shake for 2 to 3 days at ≥350 rpm, 30°C, and examine the culture microscopically for spore formation

*Sporulation medium, per liter:*

10g potassium acetate (1%)
1 g yeast extract
0.5 g dextrose

4) Formation of random spores

a. Pellet 1 ml of sporulation culture
b. Re-suspend all the cells from a 1 ml sporulation culture in 5 ml water
c. Add 0.5 ml of a 10000 U Zymolyase-20T solution (ICN Immunobiologicals) and 10 μl of 2-mercaptoethanol. (The Zymolyase will kill any diploid cells that did not sporulate as well as haploid cells that have not mated).
d. Incubate overnight at 30°C with gentle shaking.
e. Add 5 ml of 1.5% Nonidet P-40 (NP-40). Transfer the suspension to a 15 ml disposable tube and set 15 min. on ice.
f. Vortex in the presence of glass beads 30 sec. at 50% to 75% full power, then set on ice 2 min. Repeat twice.
g. Centrifuge spores 10 min. at 1200 x g. Aspirate or pour off supernatant and re-suspend in 5 ml of 1.5% NP-40. Vortex vigorously. Repeat twice.
h. Vortex in the presence of glass beads as in step f (with repeats).
i. Examine the spores by phase contrast microscopy to ensure that no more spores remain stuck together.
j. (If spores remain stuck to each other, add 2 ml glass beads (Type I, Sigma) and shake 30 min. at 300 rpm in an Erlenmeyer flask at 30°C. Let the beads settle and remove the supernatant containing the spores.)
k. Centrifuge spores 10 min. at 1200 x g. Aspirate or pour off supernatant and re-suspend in 5 ml of water. Vortex vigorously. Repeat.
l. Count a 10-fold dilution of the treated spores using a hemocytometer.

5) Selection of haploid cells with at least 1 EVAC:

a. Dilute the spores to get $10^3$ spores/ml in a media that contains G418. (The antibiotic will kill the newly formed haploid cells that do not contain at least 1 EVACS).
b. Allow spores to germinate without allowing cultures $OD_{600}$ to raise above 0.5 (in order to avoid new sexual crosses to occur).
c. Centrifuge 10 min. at 1200 x g and aspirate or pour off supernatant.

The newly formed haploid cell population consists of a 50/50 mixture of a and α cells which have any where from 1 to 4-5 EVACs each with most of the cells containing 2 EVACs.

**Remixing round 2:**

The newly formed haploid population is mated with population 3.

6) Construction of Diploid population.

a. A mixture of the 2 populations is grown overnight.
b. Cells are centrifuged and the supernatant is poured off.
c. Cells are re-suspbnded in as small an amount of rich medium as possible.
d. Distribute cells on agar plates and allow mating to proceed for at least 4 hours at 30°C and then wash cells of from plates.
e. Incubate the mating mixture in a liquid selective medium that will select for the 4 different markers (-URA3, -TRP1, -LEU2, -LYS2). This media selects for at least 70% of all possible diploid combinations. Some haploid cells which contain the 4 markers will also be able to survive at this point but the next time Zymolyase is used, the haploid cells will be killed.

7) Biological Screen.

a. Combine the population obtained in point 6e) with portion (iii) of the population obtained in point 2d).
b. Induce heterologous genes
c. The cell population is screened for the relevant pharmaceutical property(ies) and a subset of these populations are selected
d. Obtain a 10 times representation of selected populations by letting them grow on selective medium.
e. Divide selected population in 3 portions, (i) store, (ii) sporulate, (iii) keep for re-screening at a higher selection hurdle

8) Repeat points 3, 4 and 5

**Remixing round 3:**

The newly formed haploid population is mated with population 4.

9) Construction of Diploid population.

a. A mixture of the 2 populations is grown overnight.
b. Cells are centrifuged and the supernatant is poured off.
c. Cells are re-suspended in as small an amount of rich medium as possible.
d. Distribute cells on agar plates and allow mating to proceed for at least 4 hours at 30°C and then wash cells of from plates.
e. Incubate the mating mixture in a liquid selective medium that will select for the 4 different markers (-URA3, -TRP1, -LEU2, -LYS2). This media selects for at least 90% of all possible diploid combinations. Some haploid cells which contain the 4 markers will also be able to survive at this point but the next time Zymolyase is used, the haploid cells will be killed.

10) Biological Screen.

a. Combine the population obtained in point 9e) with portion (iii) of the population obtained in point 7e).
b. Induce heterologous genes
c. The cell population is screened for the relevant pharmaceutical property(ies) and a subset of these populations are selected
d. Obtain a 10 times representation of selected populations by letting them grow on selective medium.
e. Divide selected population in 3 portions, (i) store, (ii) sporulate, (iii) keep for re-screening at a higher selection hurdle

11) Repeat points 3, 4 and 5

Repeat till desired pharmaceutical properties have been obtained.

**Example 5**: Sexual crosses of yeast cell populations. (Using a fluorescence activated cell sorter (FACS) for diploid selection)

This procedure is very similar to the one described in example 2 but instead of using selective media to select for diploid cells, this selection is done simultaneously with the biological screening.

**Remixing round 1:**

1) Construction of Diploid population. Mix cells from freshly grown overnight cultures of each cell population. Distribute cells in agar plates (the plates should allow growth of both haploid strains). Allow mating to proceed for at least 4 hours at 30°C, then wash cells of from plates and incubate the mating mixture in a rich medium.
2) Biological Screen.

a. Induce heterologous genes
b. The cell population is screened for the relevant pharmaceutical property(ies) and for ploidy. A subset of this population that has the required biological property and is diploid is taken forward.
c. Obtain a 10 times representation of selected copulation.
d. Divide selected population in 3 portions, (i) store, (ii) sporulate, (iii) keep for re-screening at a higher selection hurdle

3) Repeat remaining protocol has described in example 2.

Thus when using FACS for the biological screening it is better to use this method since it shortens each screening round by 24-36 hours.

**Example 6**: Sorting of mating types after formation of haploid cells.

Keeping haploid cells from mating with each other is very difficult and requires very precise control. Thus an alternative is to sort haploid cells every time after haploid formation. For this purpose it is possible to use antibodies (against a and α receptor) or a and α mating factors (conjugated with suitable flourofors/chromofors) to label the cells of different mating types. The cells are then sorted in a FACS. (In principle as in chapter 5 of Flow cytometry : a practical approach / edited by Michael G. Ormerod. 3rd ed., 2000, Oxford university press).

**Example 7**: Remixing of spores.

Another possibility is to mix the spores of 2 different populations and then do random spore separation and mating.

**Example 8**: Combination of sexual remixing with physical rescue of expression cassettes

Every so often in the evolution programme it is advisable to use physical rescue of expression cassettes and transformation of new EVACs into new host cells in order to avoid selection of phenotypes that are due to the creation of resistant mechanisms and mutations in the host cell.

**Example 9**: Biological screening of haploid cells

It is also possible to screen for cells in their haploid state. In this procedure it is essential to have optimised for 90% plus mating efficiency in order not to loose the genetic content of the haploid cells in the remixing step.

**Example 10**: Preparation of EVACs (Evolvable Artificial Chromosomes)

1. Essentially full length cDNA libraries are made.
2. cDNA libraries are made using a pool of 4 entry vectors: pEVE4, pEVE5, pEVE8 and pEVE9 in a proportion of 30:30:1:30. See Figures 12, 13, 14, and 15.
3. Each cDNA library is normalised essentially as method 4 described in Bonaldo, MF et al. (1996) Genome Res. 6: 791-806.
4. Coding sequences from a non-normalised yeast (*Saccharomyces cerevisiae*). cDNA library are amplified by PCR and are used as driver for subtractive hybridization against single stranded circular DNA prepared from the normalized library (Bonaldo, MF et al. (1996) Genome Res. 6: 791-806), in order to remove household genes. Remaining single stranded circles are purified, converted to double stranded DNA and used to transform E.coli DH5α.
5. EVAC (Evolvable Artificial Chromosome) containing cell populations are made using 10 different normalised and enriched cDNA libraries in each.

**Preparation of expression cassettes**

1. Inoculate 5 ml of LB-medium (Sigma) containing 100 μg/L ampicillin with library inoculum corresponding to a 10+ fold representation of library. Grow overnight.
2. make plasmid miniprep from 1.5 ml of culture (E.g. Qiaprep spin miniprep kit)
3. digest plasmid w. Srf 1
4. dephosphorylate fragments and heat inactivate phosphatase (20 min, 80°C)
5. digest w. Asc1
6. run 1/10 of reaction in 1% agarose gel to estimate amount of fragment

**Preparation of pYAC4-Asc arms**

1. inoculate 150 ml of LB medium (Sigma) with a single colony of DH5α containing pYAC4-AscI
2. grow to $OD_{600}$ ~ 1, harvest cells and make plasmid preparation
3. digest 100μg pYAC4-AscI w. BamH1 and Asc1
4. dephosphorylate fragments and heat inactivate phosphatase (20 min, 80°C)
5. purify fragments(e.g. Qiaquick Gel Extraction Kit)
6. run 1 % agarose gel to estimate amount of fragment

***EVAC Synthesis***

1. mix expression cassette fragments with YAC-arms so that cassette/arm ration is ~1000/1
2. if needed concentrate mixture (use e.g. Microcon YM30) so fragment concentration > 75 ng/μL of reaction
3. add 1 U T4 DNA ligase, incubate 16C, 1-3 h . Stop reaction by adding 1 μL of 500 mM EDTA
4. run pulsed field gel (CHEF III, 1% LMP agarose, ½ strength TBE, angle 120, temperature 12 C, voltage 5.6V/cm, switch time ramping 5 - 25 s, run time 30 h) Load sample in 2 lanes.
5. Stain part of the gel that contains molecular weight markers
6. cut sample lanes corresponding to MW. 100 - 500 kb
7. agarose gel in high NaCL agarase buffer. 1 u agarase /100 mg gel
8. concentrate preparation to < 20 μL

**Example 11**: EVAC transformation using electroporation

100 ml of YPD is inoculated with one yeast colony and grown to $OD_{600} \cong 1.3$ to 1.5. The culture is harvested by centrifuging at 4000 × g and 4°C. The cells are re-suspended in 16 ml sterile $H_2O$. Add 2 ml 10 × TE buffer, pH 7.5 and swirl to mix. Add 2 ml 10 × lithium acetate solution (1 M, pH 7.5) and swirl to mix. Shake gently 45 min at 30°C. Add 1.0 ml 0.5 M DTE while swirling. Shake gently 15 min at 30°C. The yeast suspension is diluted to 100 ml with sterile water. The cells are washed and concentrated by centrifuging at 4000 × g, resuspending the pellet in 50 ml ice-cold sterile water, centrifuging at 4000 × g, resuspending the pellet in 5 ml ice-cold sterile water, centrifuging at 4000 × g and resuspending the pellet in 0.1 ml ice-cold sterile 1 M sorbitol. The electroporation was done using a *Bio-Rad Gene Pulser*. In a sterile 1.5-ml microcentrifuge tube 40 μl concentrated yeast cells were mixed with 5 μl 1:10 diluted EVAC preparation. The yeast-DNA mix is transferred to an ice-cold 0.2-cm-gap disposable electroporation cuvette and pulsed at 1.5 kV, 25 μF, 200 Ω. 1 ml ice-cold 1 M sorbitol is added to the cuvette to recover the yeast. Aliquots are spread on selective plates containing 1 M sorbitol. Incubate at 30°C until colonies appear.

**Example 12:** Transformation of EVACs (Evolvable Artificial Chromosomes) into hosts that already contain EVACs

1. Grow the EVAC cell population to mid log, 2 x $10^6$ to 2 x $10^7$ cells/ml in liquid medium, at 30°C and with aeration, under selective conditions for the EVACs.
2. Spin to pellet cells at 400 x g for 5 minutes; discard supernatant.

3. Resuspend cells in a total of 9 ml TE, pH 7.5. Spin to pellet cells and discard supernatant.
4. Gently resuspend cells in 5 ml 0.1 M Lithium/Cesium Acetate solution, pH 7.5.
5. Incubate at 30°C for 1 hour with gentle shaking.
6. Spin at 400 x g for 5 minutes to pellet cells and discard supernatant.
7. Gently resuspend in 1 ml TE, pH 7.5. Cells are now ready for transformation.
8. In a 1.5 ml tube combine:

- 100 µl yeast cells
- 5 µl carrier DNA (10 mg/ml)
- 5 µl Histamine Solution
- 5/100 of an EVAC preparation in a 10 µl volume (max). (One EVAC preparation is made of 100 µg of entry vector library plasmid mixture)

9. Gently mix and incubate at room temperature for 30 minutes.
10. In a separate tube, combine 0.8 ml 50% (w/v) PEG 4000 and 0.1 ml TE and 0.1 ml of 1 M LiAc for each transformation reaction. Add 1 ml of this PEG/TE/LiAc mix to each transformation reaction. Mix cells into solution with gentle pipetting.
11. Incubate at 30°C for 1 hour.
12. Heat shock at 42°C for 15 minutes; cool to 30°C.
13. Pellet cells in a microcentrifuge at high speed for 5 seconds and remove supernatant.
14. Resuspend in 200 µl of rich media and plate in appropriate selective media
15. Incubate at 30°C for 48-120 hours until transformed colonies appear.

**Example 13: Rare restriction enzymes with recognition sequence and cleavage points**

In this example, rare restriction enzymes are listed together with their recognition sequence and cleavage points. W = A or T; N = A, C, G, or T

| 13 a) | Unique, palindromic overhang |
|---|---|
| AscI | GG^CGCG-CC |
| AsiSI | GCG_AT^CGC |
| CciNI | GC^GGCC_GC |
| CspBI | GC^GGCC_GC |
| FseI | GG_CCGG^CC |
| MchAI | GC^GGCC_GC |
| NotI | GC^GGCC_GC |
| PacI | TTA_AT^TAA |
| SbfI | CC_TGCA^GG |
| SdaI | CC_TGCA^SG |
| SgfI | GCG_AT^CGC |
| SgrAI | CR^CCGG_YG |
| Sse232I | CG^CCGG_CG |
| Sse83871 | CC_TGCA^GG |

| 13b) | No overhang |
|---|---|
| BstRZ246I | ATTT^AAAT |
| BstSWI | ATTT^AAAT |
| MspSWI | ATTT^AAAT |
| MssI | GTTT^AAAC |

(continued)

| 13b) | No overhang |
|---|---|
| PmeI | GTTT^AAAC |
| SmiI | ATTT^AAAT |
| SrfI | GCCC^GGGC |
| SwaI | ATTT^AAAT |

| 13c) | Non-palindromic and/or variable overhang |
|---|---|
| AarI | CACCTGCNNNN^NNNN_ |
| AbeI | CC^TCA_GC |
| AloI | ANNNNN_NNNNNNNGAACNNNNNNTCCNNNNNNN_NNNNN^ |
| BaeI | ^NNNNN_NNNNNNNNNNACNNNNGTAYCNNNNNNN_NNNNN^ |
| BbvCI | CC^TCA_GC |
| CpoI | CG^GWC_CG |
| CspI | CG^GWC_CG |
| Pfl27I | RG^GWC_CY |
| PpiI | ^NNNNN_NNNNNNNGAACNNNNNCTCNNNNNNNN_NNNNN^ |
| PpuMI | RG^GWC_CY |
| PpuXI | RG^GWC_CY |
| Psp5II | RG^GWC_CY |
| PspPPI | RG^GWC_CY |
| RsrII | CG^GWC_CG |
| Rsr2I | CG^GWC_CG |
| SanDI | GG^GWC_CC |
| SapI | GCTCTTCN^NNN_ |
| SdiI | GGGCN_NNN^NGGCC |
| SexAI | A^CCWGG_T |
| SfiI | GGCCN_NNN^NGGCC |
| Sse1825I | GG^GWC_CC |
| Sse8647I | AG^GWC_CT |
| VpaK32I | GCTCTTCN^NNN_ |

| 12d) | Meganucleases |
|---|---|
| I-Sce I | TAGGGATAA_CAGG^GTAAT |
| I-Ceu I | ACGGTC_CTAA^GGTAG |
| I-Cre I | AAACGTC_GTGA^GACAGTTT |
| I-Sce II | GGTC_ACCC^TGAAGTA |
| I-Sce III | GTTTTGG_TAAC^TATTTAT |
| Endo. Sce I | GATGCTGC_AGGC^ATAGGCTTGTTTA |
| PI-Sce I | GG_GTGC^GGAGAA |
| PI-Psp I | TGGCAAACAGCTA_TTAT^GGGTATTATGGGT |
| I-Ppo I | CTCTC_TTAA^GGTAG |
| HO | TTTCCGC_AACA^GT |
| I-Tev I | NN_NN^NNTCAGTAGATGTTTTTCTTGGTCTACCGTTT |

More meganucleases have been identified, but their precise sequence of recognition has not been determined, see e.g. www.meganuclease.com

**Example 14: Concatemer size limitation experiments (use of stoppers)**

Materials used:

pYAC4 (Sigma. Burke et al. 1987, science, vol 236, p 806) was digested with EcoR1 and BamH1 and dephosphorylated.
pSE420 (invitrogen) was linearised using EcoR1 and used as the model fragment for concatenation.
T4 DNA ligase (Amersham-pharmacia biotech) was used for ligation according to manufacturers instructions.

Method: Fragments and arms were mixed in the ratios(concentrations are arbitrary units) indicated on figures. Ligation was allowed to proceed for 1 h at 16C. Reaction was stopped by the addition of 1 μL 500 mM EDTA. Products were analysed by standard agarose GE (1 % agarose, ½ strength TBE) or by PFGE(CHEF III, 1% LMP agarose, ½ strength TBE, angle 120, temperature 12 C, voltage 5.6V/cm, switch time ramping 5 - 25 s, run time 30 h)
The results are shown in Figure 18a and 18b.

**Example 15: Expression of different patterns "phenotypes" obtained using the same yeast clones under different expression conditions**

Yeast cells comprising a library of artificial chromosomes comprising genes derived from Daucus carota and Xanthophyllomyces dendrorhous as well as specific carotenoid genes under control of a Cup promoter or a Met promoter.
Colonies selected for carotenoid production were picked with a sterile toothpick and streaked sequentially onto selective plates corresponding to the four repressed and/or induced conditions (-Ura/-Trp (both promoters on), -Ura/-Trp/-Met (Cup promoter off, Met promoter on), -Ura/-Trp/+200 μM $Cu_2SO_4$ (Cup promotor on, Met Promotor off), -Ura/-Trp/-Met/+200 μM $Cu_2SO_4$ (both promoters on)). Results are shown in Figure 27. Replicas on media with one promotor induced and the other expressed showed some color development while the replicas on double inducive medium shoved the brightest colors in a high proportion of replicas.

**Example 16**

**Preparation of cells expressing carotenoids and other antioxidants**

2 EVAC libraries were prepared as described in Example 10, pages 120-122.
EVAC library 1 was made using 5 plant libraries: *Aloe humillis* (flowers), red chilli pepper (fruit), *Gerbera hummingbird* (flower), *Viola cornuta* (flowers) and *Spireae japonica* (leaves) as well as specific carotenoid genes. The plants were used in equal proportions and represented a total of 20% of the cassettes used in the EVAC preparation. The specific genes were also included in equal amounts and represented the remaining 80% of cassettes used in the EVAC preparation. This EVAC library was transformed into a haploid yeast strain of mating type α, using the procedure described in example 11.
The specific carotenoid genes were:

ggps, psy, pds, zds, Icy-b, Icy-e, bhy, zep *(Gentiana sp.),* idi, crtC, crtF *(Rhodobacter capsulatus),* crtE, crtB, crtI, crtY, crtZ *(Erwinia uredovora)*, zds *(Nostoc anabaena),* pds *(Synechococcus PCC7942),* crtE, crtB, crtI, crtY, crtZ *(Erwinia herbicola),* crtM, crtN *(Staphylococcus aureus),* crtl, crtYb *(Xanthophyllomyces dendrorhous)*, ccs, crtL *(Capsicum annuum),* crtL, bchy *(Nicotiana tabacum)*, Icy-b, Icy-e *(Prochlorococcus sp.)*, idi *(Saccharomyces cerevisiae)*, crtI, citYe, crtYf, crtEb *(Corynebacterium sp.)*, psy-1 *(Lycopersicon esculentum)*, al1 *(Neurospora crassa)*

EVAC library 2 was made using 10 plant cDNA libraries, 1 moss library *(Pellia endiviaefolia),* 1 red algae cDNA library *(Polysiphonia fibrillosa)* and one mushroom *(Cantharellus cibarius).* The 10 plants used were *Viburnum fragrans* (flower), carrot (root), *Aloe humillis* (leaves), *Lithops salicola* (leaves), cherry tomato (fruit), red chilli (fruit), coriander (leaves), *Gerbera hummingbird* (flower), *Forsythia intermedia* (leaves & shoots) and papaya fruit. All cDNA libraries were used in equal amounts. This EVAC library was transformed into a yeast strain of mating type a using the procedure described in example 11.
The yeast library containing EVAC preparation 1 was screened for oxidative stress resistance using the following anti-oxidant screen:

a. The screening population was grown and the heterologous genes were induced/de-repressed by re-suspending the cells in selective medium lacking methionine and with 200 μM $Cu_2SO_4$. The cells were grown under induction conditions for 24 hours prior to screening.

b. The screening population was divided in 10 portions.

c. Each sub population was exposed to 1 out of a range of 10 concentrations of Methylene blue. (0,1mM MB & 2 h. irradiation with 200W white fluorescence light kills 100% of the host). Immediately after exposure to Methylene blue, the cells were irradiated with a 200 W lamp for 2 hours. During the procedure it was ensured that the temperature remained constant at 37°C. The yeast host subjected to methylene blue and light, the cell population exposed just to light and the cell population exposed to methylene blue but not light were used as controls throughout the procedure.

d. After exposure to methylene blue and light, the cell populations were washed to remove all traces of poison, re-suspended in selective media and stored at 0°C to avoid cell growth.

e. Plating a dilution series and counting the number of colonies formed after 48 hours determined survival rates.

f. The surviving cell population from the highest concentration of Methylene blue where cells statistically representing 1% of the original cell lines survived was taken forward.

The cell culture subjected to the highest oxidative stress, where 1% of the cells survived (0.4 mM concentration of methylene blue) were mated with the yeast library containing EVAC library 2. Mating of the libraries was done using the following procedure:

a. The selected library and the library containing EVAC library 2 were amplified so that enough copies of each clone exist to be able to mate with all cells of opposite mating type

b. Cells from freshly grown overnight cultures of both populations were mixed and distributed in agar plates (the plates should allow growth of both haploid strains).

c. Mating was allowed to proceed for at least 4 hours at 30°C and then the cells were washed of from plates.

d. The mating mixture was incubated in a liquid selective medium that selects for the diploid genotype which contains all different types of EVACs

The newly formed diploid population was screened for oxidative stress resistance as described herein above. Again the cell culture subjected to the highest oxidative stress, where 1% of the cells survived (1.2 mM concentration of methylene blue) were selected. This selected cell population was sporulated and the newly formed daughter population was mated again to form a new diploid population. Sporulation was done using the following procedure:

f. A portion of the selected diploid population was grown to an $OD_{600}$ of 2.5 to 3.0 (~8 x $10^7$ cells/ml) in selective medium.

g. 1 ml of culture was transferred to a sterile, disposable 15 ml polypropylene tube and centrifuge 5 min at 1200 $\times$ g.

h. The supernatant was poured off and the cells were washed in 5 ml sterile water.

i. The cells were re-suspended in 1 ml of sporulation medium. (Sporulation medium, per litre: 10g potassium acetate (1%), 1g yeast extract, 0.5 g dextrose).

j. Cells were shaken for 2 to 3 days at ≥350 rpm, 30°C, and the culture was examined microscopically for spore formation.

The disruption of spores was done the following way:

a. The sporulation culture was pelleted and re-suspended in 5 ml water.

b. Zymolyase-20T solution (ICN Immunobiologicals) and 2-mercaptoethanol were added and the spores were incubated overnight at 30°C with gentle shaking. (The Zymolyase kills any diploid cells that did not sporulate as well as haploid cells that have not mated).

c. The supernatant is aspirated or poured off and the spores are re-suspended in 5 ml of water. Glass beads were added (Type I, Sigma) and the mixture was vortexed vigorously. The process is repeated until 90% of spores are disrupted.

d. The spore populations are plated onto selection agar plates with $CaCl_2$ (25 mM $CaCl_2$, 0.67% yeast nitrogen base w/o amino acids, 1$\times$CSM (-TRP) (Q-biogen), 2% glucose and 2% or 2.5% agar) using top alginate. A suitable amount of selective top alginate was added so that 100000 cells are spread on 100-mm plates or 1000000 cells on 150-mm plates of selective top alginate: 5 ml (for 100-mm plates) or 12 ml (for 150-mm plates). Top alginate is the same as the selection agar except that agar is substituted with Na-Alginate and $CaCl_2$ is omitted. The use of selective media prevents the growth of cells lacking at least one EVAC.

e. The plates were quickly tilted or rotated from side to side as to distribute the agar solution evenly across the surface of the agar plate. The plates were left standing face up until top alginate has solidified.

f. The plates were incubated for 2-6 days at 30°C until colonies appear.

g. The alginate from the plates was collected and transferred to a 50 ml tube (from two 100-mm plates or one 150-mm plate in one tube). 12 ml (per 100-mm plate) or 33 ml (150-mm plate) of 0.25 M Na-citrate (pH 6.2) were added and mixed thoroughly for 2.5 min.

h. The cells were collected by centrifugation for 5 min at 2500 rpm. The supernatant was poured of and the cells were re-suspended in 50 ml of $H_2O$.

The newly formed haploid population is a mixture of both mating types that will mate as soon as they are transferred from the water into rich media as described herein above.

The new diploid population was subjected to a third round of screening for oxidative stress resistance as described above. Again the cell culture subjected to the highest oxidative stress, where 1% of the cells survived (1.9 mM concentration of methylene blue) were selected.

After 3 rounds of screening for oxidative stress resistance, cells able to survive 20 times more oxidative stress than the untransformed host were obtained (see figure 28).

Also, after the first around of screen only yellow and orange colours were obtained (similar to Example 15, page 125) while after the second and third rounds yeasts producing pink and green colours were also obtained. See figure 29.

Yeast cells producing a number of different carotenoids were identified. Figure 30 shows the carotenoid content of 10 selected clones.

## Claims

1. A method of mixing heterologous genes in expression cassettes located on artificial chromosomes said method comprising the steps of

   (a) providing two initial populations of cells that can mate with each other,
   said initial populations comprising at least 2 cells in each population, and at least two cells in one population having different combinations of heterologous genes and/or different combinations of expression cassettes,
   each cell comprising at least a first type of artificial chromosome, the at least first type of artificial chromosome comprising both at least two expression cassettes comprising heterologous genes and at least one selectable marker,
   the selectable markers being allocated to artificial chromosomes so that each type of artificial chromosome from each population can be individually selected for,
   the expression cassettes are located on a nucleotide the concatemer comprising in the 5'→3' direction a cassette of nucleotide sequence of the general formula

   $$[rs_2\text{-SP-PR-X-TR-SP-}rs_1]_n$$

   wherein

   $rs_1$ and $rs_2$ together denote a functional restriction site,
   SP individually denotes a spacer of at least two nucleotide bases,
   PR denotes a promoter, capable of functioning in a cell,
   X denotes an expressible nucleotide sequence,
   TR denotes a terminator, and

SP individually denotes a spacer of at least two nucleotide bases, and
n ≥ 2, and
wherein at least a first cassette is different from a second cassette.

(b) mating the cells with each other, and
selecting mated cells that carry at least a subset of the selectable markers present on the artificial chromosomes in the two initial populations.

**2.** The method according to claim 1, wherein at least two cells in each population has different combinations of heterologous genes and/or different combinations of expression cassettes.

**3.** The method of claim 1, further comprising causing the selected mated cells to undergo meiosis.

**4.** The method according to claim 1, wherein the subset of markers selected for comprises at least one marker from and artificial chromosome in each of the initial populations to ensure selection of mated cells.

**5.** The method according to any of the preceding claims, wherein the selection for a subset of the selectable markers includes selecting at least 70 % of all diploid types present in the mated population.

**6.** The method according to any of the preceding claims, further comprising screening mated cells for one or more parameters related to a desired functionality(ies) and selecting cells having a predefined selection criterion(a) to undergo meiosis and mating.

**7.** The method according to any of the preceding claims, further comprising screening cells that have undergone meiosis for at least one parameter related to a desired functionality(ies) and selecting cells having a predefined selection criterion(a) to undergo mating and meiosis.

**8.** The method according to claims 6 or 7, wherein the selection threshold(s) associated with the desired functionalty (ies) is increased for each round of mating and meiosis.

**9.** The method according to any of the preceding claims, further comprising repeating the steps of claims 1 and 2 at least twice.

**10.** The method according to any of the preceding claims, further comprising separating cells of the two mating types from each other after meiosis.

**11.** The method according to any of the preceding claims, further comprising mixing spores from different populations prior to mating.

**12.** The method according to any of the preceding claims 3 to 11, further comprising adding a further population of cells with types of artificial chromosomes comprising at least two expression cassettes with heterologous genes, the cells being capable of mating with the cells that have undergone mating and meiosis, the further population comprising at least 2 cells with combinations of expression cassettes different from the combinations in the cells of the initial population, the artificial chromosomes of said further population carrying at least one selectable marker.

**13.** The method according to any of the preceding claims, wherein at least one of the two initial populations of cells that can mate with each other further carry at least a second type of artificial chromosome with expression cassettes comprising heterologous genes, the first and second types of artificial chromosome carrying at least one selectable marker so that said first and second type of artificial chromosome can be individually selected for.

**14.** The method according to any of the preceding claims, wherein the two initial populations of cells that can mate with each other carry from 1 to 10 types of artificial chromosomes, each type of artificial chromosome of each population carrying at least one selectable marker so that each of the types of artificial chromosomes from each of the two populations can be individually selected for.

**15.** The method according to any of the preceding claims, wherein each cell carries 2 artificial chromosomes per cell that can mate.

**16.** The method according to any of the preceding claims, wherein each artificial chromosome carries at least two selectable markers, the selectable markers being allocated to artificial chromosomes so that each type of artificial chromosome from each population can be individually selected for.

**17.** The method according to any of the preceding claims, wherein the two initial populations are of different mating types.

**18.** The method according to any of the preceding claims, wherein type of artificial chromosomes with the same marker or combination of markers differ with respect to combinations of expression cassettes comprising heterologous genes.

**19.** The method according to any of the preceding claims, wherein the species of cells is fungal cells selected from a spore forming species.

**20.** The method according to any of the preceding claims, wherein the species of cells is yeast cells.

**21.** The method according to any of the preceding claims, wherein the mated cells are diploid or tetraploid or hexaploid.

**22.** The method according to any of the preceding claims, comprising nucleotide sequences from at least two expression states.

**23.** The method according to any of the preceding claims, wherein the $rs_1$-$rs_2$ restriction site of essentially all cassettes are recognised by the same restriction enzyme.

**24.** The method according to any of the preceding claims, wherein substantially all expression cassettes on one artificial chromosome are different.

**25.** The method according to any of the preceding claims, wherein at least one expression cassette comprises an intron between the promoter and the expressible nucleotide sequence.

**26.** The method according to any of the preceding claims, wherein the different combinations of expression cassettes comprises different promoters, and/or different expressible nucleotide sequences, and/or different spacers and/or different terminators and/or different introns.

**27.** The method according to claim any of the preceding claims, wherein n is at least 10.

**28.** The method according to any of the preceding claims, wherein the different expression states represent at least two different tissues, such as at least two organs, such as at least two species, such as at least two genera.

**29.** A method of mixing heterologous genes in expression cassettes located on artificial chromosomes said method comprising the steps of
providing two initial populations of protoplasts or cells that can be fused,
said initial populations comprising at least 2 cells in each population, and at least two cells in each population having different combinations of heterologous genes and/or different combinations of expression cassettes,
the expression cassettes are located on a nucleotide the concatemer comprising in the 5'→3' direction a cassette of nucleotide sequence of the general formula

$$[rs_2\text{-}SP\text{-}PR\text{-}X\text{-}TR\text{-}SP\text{-}rS_1]_n$$

wherein

$rs_1$ and $rs_2$ together denote a functional restriction site,
SP individually denotes a spacer of at least two nucleotide bases,
PR denotes a promoter, capable of functioning in a cell,
X denotes an expressible nucleotide sequence,
TR denotes a terminator, and
SP individually denotes a spacer of at least two nucleotide bases, and
$n \geq 2$, and
wherein at least a first cassette is different from a second cassette.

each cell comprising at least a first type of artificial chromosome, said at least first type of artificial chromosome comprising both at least two expression cassettes comprising heterologous genes and at least one selectable marker, the selectable markers being allocated to artificial chromosomes so that each type of artificial chromosome from each population can be individually selected for, performing protoplast fusion and regeneration of cell walls or performing fusion of cells, and
selecting fused cells that carry at least a subset of the selectable markers present on the artificial chromosomes in the two initial populations.

**30.** The method according to claim 29, further comprising repeating the steps of claim 29.

**31.** The method according to claim 29, wherein the species of cells are selected from fungi, algae, plants, prokaryots, animal cells or human cells.

**32.** The method according to any of the preceding claims 29 to 31, further comprising screening cells that result from protoplast fusion for a desired functionalty(ies) and selecting cells having the desired functionalty(ies) above a defined threshold, isolating protoplasts from these cells and performing protoplast fusion and cell regeneration on the selected cells.

**33.** The method according to any of the preceding claims 29 to 31, wherein at least one of the two initial populations of protoplasts that can fuse with each other further carries at least a second type of artificial chromosome with expression cassettes comprising heterologous genes, the first and second type of artificial chromosome from each population carrying at least one selectable marker so that said first and second type of artificial chromosome can be individually selected for.

**34.** The method according to any of the preceding claims 29 to 33, wherein selection of a subset of the selectable markers includes selection for at least 70 % of all fused cell types present in the fused population.

**35.** The method according to any of the preceding claims, further comprising subjecting the populations of cells to physical isolation of artificial chromosomes from the populations for every 2-3 rounds of meiosis and selection, and transferring the isolated artificial chromosomes into new host cells.

**36.** The method according to any of the preceding claims 16-35, wherein the two initial populations of cells carry from 1 to 10 types of artificial chromosomes, each type of artificial chromosome of each population carrying at least one selectable marker so that each of the types of artificial chromosomes from each of the two populations can be individually selected for.

**37.** The method according to any of the preceding claims 16 to 36, further comprising adding a further population of cells with artificial chromosomes comprising at least two expression cassettes with heterologous genes, the cells being capable of fusing with the cells that have undergone fusion, the further population comprising at least 2 cells with combinations of expression cassettes different from the combinations in the cells of the initial population, the artificial chromosomes of said further population carrying at least one selectable marker.

**38.** The method according to claim 37, wherein the further population of cells with artificial chromosomes capable of fusing with the cells that have undergone mating and meiosis carry from 1 to 10 types of artificial chromosomes, each type of artificial chromosome of said further population carrying at least one selectable marker so that each of the types of artificial chromosomes can be individually selected for.

**39.** The method according to any of the preceding claims 29-38, comprising the features of any of the claims 16-18 and 23-29.

**40.** A method of mixing heterologous genes in expression cassettes located on artificial chromosomes, said method comprising the steps of

a) obtaining at least one population of cells, the cells of said at least one population comprising
a concatemer of expression cassettes of the following formula:

$$[rS_2\text{-}SP\text{-}PR\text{-}X\text{-}TR\text{-}SP\text{-}rs_1]_n$$

wherein

$rs_1$ and $rs_2$ together denote a functional restriction site,
SP individually denotes a spacer of at least two nucleotide bases,
PR denotes a promoter, capable of functioning in the cells,
X denotes an expressible nucleotide sequence,
TR denotes a terminator, and
$n \geq 2$,
the cells differing from each other with respect to combinations of expressible nucleotide sequences and/or promoters,

b) isolating at least some of the cassettes of the selected cells by cutting the concatemers with a restriction enzyme cleaving $rs_1rs_2$,
c) amplifying at least some of the isolated cassettes,
d) assembling the expression cassettes of step c) into artificial chromosomes, and
e) optionally transferring the artificial chromosomes into host cells.

**41.** The method according to claim 40, wherein amplification of isolated cassettes comprises PCR with primers for tagging $rs_1$ and $rs_2$.

**42.** The method according to claim 40, wherein amplification of isolated cassettes comprises inserting isolated cassettes into a vector having a cloning site compatible with $rs_1rs_2$ and multiplying this vector in a suitable host.

**43.** The method according to claim 40, further comprising adding further cassettes for the assembly step.

**44.** The method according to any of the preceding claims 40 to 43, further comprising screening cells with assembled artificial chromosomes for a desired functionalty(ies) and selecting cells having the desired functionalty(ies).

**45.** The method according to claim 45, further comprising subjecting the selected cells to further isolation and amplification of cassettes and assembly of artificial chromosomes.

**46.** A method for mixing heterologous genes in expression cassettes located on artificial chromosomes, said method comprising the steps of
providing two initial populations of cells,
said initial populations comprising at least 2 cells in each population, and at least two cells in each population having different combinations of heterologous genes and/or different combinations of expression cassettes,
the expression cassettes are located on a nucleotide the concatemer comprising in the 5'→3' direction a cassette of nucleotide sequence of the general formula

$$[rs_2\text{-SP-PR-X-TR-SP-}rs_1]_n$$

wherein

$rs_1$ and $rs_2$ together denote a functional restriction site,
SP individually denotes a spacer of at least two nucleotide bases,
PR denotes a promoter, capable of functioning in a cell,
X denotes an expressible nucleotide sequence,
TR denotes a terminator, and
SP individually denotes a spacer of at least two nucleotide bases, and
$n \geq 2$, and
wherein at least a first cassette is different from a second cassette,

each cell comprising at least a first type of artificial chromosome, the at least first type of artificial chromosome comprising both at least two expression cassettes comprising heterologous genes and at least one selectable marker, the selectable markers being allocated to artificial chromosomes so that each type of artificial chromosome from each population can be individually selected for, mating the cells with each other,
amplifying the artificial chromosomes in the host cells,
isolating the artificial chromosomes,

mixing the isolated artificial chromosomes,
transferring subsets of said isolated and mixed artificial chromosomes into host cells, and
selecting cells that carry at least a subset of the selectable markers present on the artificial chromosomes in the two initial populations.

**47.** The method according to claim 46, further comprising repeating the mixing process at least once.

**48.** The method according to claim 46, wherein the host cells into which the subsets of mixed type of artificial chromosomes are transferred already contain artificial chromosomes with expression cassettes with heterologous genes.

**49.** A method of mixing heterologous genes in expression cassettes located on plasmids said method comprising the steps of
providing two initial populations of cells that can mate with each other,
said initial populations comprising at least 2 cells in each population, and at least two cells in each population having different combinations of heterologous genes and/or different combinations of expression cassettes,
wherein the expression cassettes are located on a nucleotide concatemer comprising in the 5'→3' direction a cassette of nucleotide sequence of the general formula

$$[rs_2\text{-SP-PR-X-TR-SP-}rs_1]_n$$

wherein

$rs_1$ and $rs_2$ together denote a functional restriction site,
SP individually denotes a spacer of at least two nucleotide bases,
PR denotes a promoter, capable of functioning in a cell,
X denotes an expressible nucleotide sequence,
TR denotes a terminator, and
SP individually denotes a spacer of at least two nucleotide bases, and
$n \geq 2$, and
wherein at least a first cassette is different from a second cassette.

each cell comprising at least a first plasmid, the at least first plasmid comprising both at least two expression cassettes comprising heterologous genes and at least one selectable marker,
the selectable markers being allocated to plasmids so that each type of plasmid from each population can be individually selected for,
mating the cells with each other, and
selecting mated cells that carry at least a subset of the selectable markers present on the plasmids in the two initial populations.

## Patentansprüche

**1.** Verfahren zum Mischen von heterologen Genen in Expressionskassetten, die sich auf künstlichen Chromosomen befinden, wobei das Verfahren die Schritte umfasst:

(a) Bereitstellen von zwei Ausgangspopulationen von Zellen, die sich miteinander verbinden/paaren können, wobei die Ausgangspopulationen mindestens zwei Zellen in jeder Population umfassen, und wobei mindestens zwei Zellen in einer Population unterschiedliche Kombinationen von heterologen Genen und/oder unterschiedliche Kombinationen von Expressionskassetten aufweisen,
wobei jede Zelle mindestens einen ersten Typ eines künstlichen Chromosoms umfasst, wobei der mindestens erste Typ eines künstlichen Chromosoms mindestens zwei Expressionskassetten umfassend heterologe Gene und mindestens einen auswählbaren Marker umfasst,
wobei die auswählbare Marker den künstlichen Chromosomen zugeteilt sind, so dass jeder Typ eines künstlichen Chromosoms von jeder Population individuell ausgewählt werden kann,
wobei sich die Expressionskassetten auf einem Nukleotid-Concatemer befinden, der in 5'→3' Richtung eine Kassette mit Nukleotidsequenz aufweist mit der generellen Formel

$$[rs_2\text{-SP-PR-X-TR-SP-}rs_1]_n$$

wobei

$rs_1$ und $rs_2$ zusammen eine funktionale Restriktionsstelle bezeichnen,
SP einzeln einen Spacer aus mindestens zwei Nukleotidbasen bezeichnet,
PR einen Promotor bezeichnet, der fähig ist, in einer Zelle zu funktionieren,
X eine exprimierbare Nukleotidsequenz bezeichnet,
TR einen Terminator bezeichnet, und
SP einzeln einen Spacer aus mindestens zwei Nukleotidbasen bezeichnet, und
$n \geq 2$, und
wobei sich mindestens eine erste Kassette von einer zweiten Kassette unterscheidet,

(b) Verbinden der Zellen miteinander, und
Auswählen der verbundenen/gepaarten Zellen, die mindestens eine Teilmenge der in den künstlichen Chromosomen in den zwei Ausgangspopulationen vorhandenen auswählbaren Marker tragen.

**2.** Verfahren nach Anspruch 1, worin mindestens zwei Zellen in jeder Population unterschiedliche Kombinationen von heterologen Genen und/oder andere Kombinationen von Expressionskassetten aufweisen.

**3.** Verfahren nach Anspruch 1, weiter umfassend Herbeiführen eines Durchlaufens der Meiose der ausgewählten verbundenen/gepaarten Zellen.

**4.** Verfahren nach Anspruch 1, worin die Teilmenge ausgewählter Marker mindestens einen Marker von und künstliches Chromosom in jeder der Ausgangspopulationen umfasst, um die Auswahl verbundener/gepaarter Zellen zu gewährleisten.

**5.** Verfahren nach einem der vorstehenden Ansprüche, worin die Auswahl für eine Teilmenge der auswählbaren Marker die Auswahl von mindestens 70 % aller in der verbundenen Population vorhandenen diploider Typen umfasst.

**6.** Verfahren nach einem der vorstehenden Ansprüche, weiter umfassend Durchmustern verbundener Zellen auf einen oder mehrere Parameter, die in Bezug zu (einer) erwünschten Funktionalität(en) steht bzw. stehen, und Auswählen von Zellen, die das vorbestimmte Auswahlkriterium eines Durchlaufens der Meiose und der Verbindung/Paarung aufweisen.

**7.** Verfahren nach einem der vorstehenden Ansprüche, weiter umfassend Durchmustern von Zellen, die die Meiose durchlaufen haben, auf mindestens ein Parameter, der in Bezug zu (einer) erwünschten Funktionalität(en) steht bzw. stehen, und Auswählen von Zellen, die das vorbestimmte Auswahlkriterium eines Durchlaufens der Verbindung/ Paarung und der Meiose ausweisen.

**8.** Verfahren nach Ansprüchen 6 oder 7, worin die mit der/den erwünschten Funktionalität(en) verbundenen Auswahlschwelle(n) für jede Runde Meiose und Verbindung/Paarung erhöht ist bzw. sind.

**9.** Verfahren nach einem der vorstehenden Ansprüche, weiter umfassend mindestens zweimaliges Wiederholen der Schritte 1 und 2.

**10.** Verfahren nach einem der vorstehenden Ansprüche, weiter umfassend Trennen von Zellen der zwei Verbindungs-/ Paarungstypen voneinander nach der Meiose.

**11.** Verfahren nach einem der vorstehenden Ansprüche, weiter umfassend Mischen von Sporen aus unterschiedlichen Populationen vor der Verbindung/Paarung.

**12.** Verfahren nach einem der vorstehenden Ansprüche 3 bis 11, weiter umfassend Zugeben einer weiteren Population von Zellen mit Typen künstlicher Chromosomen, die mindestens zwei Expressionskassetten mit heterologen Genen umfassen, wobei die Zellen fähig sind sich mit den Zellen, die eine Verbindung/Paarung eingegangen sind und die Meiose durchlaufen haben, zu verbinden/paaren, wobei die weitere Population mindestens zwei Zellen mit Kombinationen von Expressionskassetten umfasst, die von den Kombinationen in den Zellen der Ausgangspopulation verschieden sind, wobei die künstlichen Chromosomen der weiteren Population mindestens einen auswählbaren Marker tragen.

**13.** Verfahren nach einem der vorstehenden Ansprüche, wobei wenigstens eine der zwei Ausgangspopulationen von Zellen, die sich miteinander verbinden/paaren können, weiterhin mindestens einen zweiten Typ eines künstlichen Chromosoms mit Expressionskassetten trägt, die heterologe Gene umfassen, wobei die ersten und zweiten Typen des künstlichen Chromosoms mindestens einen auswählbaren Marker tragen, so dass der erste und zweite Typ des künstlichen Chromosoms individuell ausgewählt werden kann.

**14.** Verfahren nach einem der vorstehenden Ansprüche, wobei die zwei Ausgangspopulationen von Zellen, die sich miteinander verbinden/paaren können, einen bis zehn Typen von künstlichen Chromosomen tragen, wobei jeder Typ eines künstlichen Chromosoms jeder Population mindestens einen auswählbaren Marker trägt, so dass jeder der Typen der künstlichen Chromosomen von jeder der zwei Populationen individuell ausgewählt werden kann.

**15.** Verfahren nach einem der vorstehenden Ansprüche, wobei jede Zelle zwei künstliche Chromosomen pro Zelle trägt, die sich verbinden/paaren kann.

**16.** Verfahren nach einem der vorstehenden Ansprüche, wobei jedes künstlichen Chromosom mindestens zwei auswählbare Marker trägt, wobei die auswählbaren Marker künstlichen Chromosomen zugeordnet sind, so dass jeder Typ eines künstlichen Chromosoms von jeder Population individuell ausgewählt werden kann.

**17.** Verfahren nach einem der vorstehenden Ansprüche, wobei die zwei Ausgangspopulationen unterschiedliche Verbindungs/Paarungstypen sind.

**18.** Verfahren nach einem der vorstehenden Ansprüche, wobei die Typen künstlicher Chromosomen mit dem gleichen Marker oder Kombinationen von Markern sich in Bezug auf die Kombination von Expressionskassetten unterscheiden, die heterologe Gene umfassen.

**19.** Verfahren nach einem der vorstehenden Ansprüche, wobei die Arten von Zellen Pilzzellen sind, ausgewählt unter Sporen bildenden Arten.

**20.** Verfahren nach einem der vorstehenden Ansprüche, wobei die Arten von Zellen Hefezellen sind.

**21.** Verfahren nach einem der vorstehenden Ansprüche, wobei die verbundenen/gepaarten Zellen diploid, tetraploid oder hexaploid sind.

**22.** Verfahren nach einem der vorstehenden Ansprüche, umfassend Nukleotidsequenzen aus mindestens zwei Expressionsstadien.

**23.** Verfahren nach einem der vorstehenden Ansprüche, wobei die $rs_1$-$rs_2$ Restriktionsstelle von im wesentlichen allen Kassetten durch das selbe Restriktionsenzym erkannt wird.

**24.** Verfahren nach einem der vorstehenden Ansprüche, wobei im Wesentlichen alle Expressionskassetten auf einem künstlichen Chromosom unterschiedlich sind.

**25.** Verfahren nach einem der vorstehenden Ansprüche, wobei mindestens eine Expressionskassette ein Intron zwischen dem Promotor und der exprimierbaren Nukleotidsequenz umfasst.

**26.** Verfahren nach einem der vorstehenden Ansprüche, wobei die unterschiedlichen Kombinationen von Expressionskassetten unterschiedliche Promotoren und/oder unterschiedliche exprimierbare Nukleotidsequenzen und/oder unterschiedliche Spacer und/oder unterschiedliche Terminatoren und/oder unterschiedliche Introns umfassen.

**27.** Verfahren nach einem der vorstehenden Ansprüche, wobei n mindestens 10 ist.

**28.** Verfahren nach einem der vorstehenden Ansprüche, wobei die unterschiedlichen Expressionsstadien wenigstens zwei unterschiedliche Gewebe vertreten, bspw. wie mindestens zwei Organe, bspw. wie mindestens zwei Arten, bspw. wie mindestens zwei Genera.

**29.** Verfahren zum Mischen von heterologen Genen in Expressionskassetten, die sich auf künstlichen Chromosomen befinden, wobei das Verfahren die Schritte umfasst:

Bereitstellen von zwei Ausgangspopulationen von Protoplasten oder Zellen, die fusioniert werden können, wobei die Ausgangspopulationen mindestens zwei Zellen in jeder Population umfassen, und wobei mindestens zwei Zellen in jeder Population unterschiedliche Kombinationen von heterologen Genen und/oder unterschiedliche Kombinationen von Expressionskassetten aufweisen,
wobei sich die Expressionskassetten auf einem Nukleotid-Concatemer befinden, der in 5'→3' Richtung eine Kassette mit Nukleotidsequenz aufweist mit der generellen Formel

$$[rs_2\text{-SP-PR-X-TR-SP-}rs_1]_n$$

wobei

$rs_1$ und $rs_2$ zusammen eine funktionale Restriktionsstelle bezeichnen,
SP einzeln einen Spacer aus mindestens zwei Nukleotidbasen bezeichnet,
PR einen Promotor bezeichnet, der fähig ist, in einer Zelle zu funktionieren,
X eine exprimierbare Nukleotidsequenz bezeichnet,
TR einen Terminator bezeichnet, und
SP einzeln einen Spacer aus mindestens zwei Nukleotidbasen bezeichnet, und
$n \geq 2$, und
wobei sich mindestens eine erste Kassette von einer zweiten Kassette unterscheidet,

wobei jede Zelle mindestens einen ersten Typ eines künstlichen Chromosoms umfasst, wobei der erste Typ eines künstlichen Chromosoms mindestens zwei Expressionskassetten umfassend heterologe Gene und mindestens einen auswählbaren Marker umfasst,
wobei die auswählbaren Marker künstlichen Chromosomen zugeteilt sind, so dass jeder Typ eines künstlichen Chromosoms von jeder Population individuell ausgewählt werden kann, Durchführen einer Protoplastenfusion und Regenerieren von Zellwänden oder Durchführen einer Fusion von Zellen, und
Auswählen fusionierter Zellen, die mindestens eine Teilmenge der in den künstlichen Chromosomen in den zwei Ausgangspopulationen vorhandenen auswählbaren Marker tragen,

**30.** Verfahren nach Anspruch 29, weiter umfassend Wiederholen der Schritte von Anspruch 29.

**31.** Verfahren nach Anspruch 29, wobei die Arten von Zellen ausgewählt sind unter Pilz-, Algen-, Pflanzen-, Prokaryoten-, Tierzellen oder humanen Zellen.

**32.** Verfahren nach einem der vorstehenden Ansprüche 29 bis 31, weiter umfassend Durchmustern von Zellen, die aus der Protoplastenfusion herrühren, auf (eine) erwünschte Funktionalität(en) und Auswählen von Zellen, die die erwünschte(n) Funktionalität(en) über einer definierten Schwelle aufweist bzw. aufweisen, Isolieren von Protoplasten von diesen Zellen und Durchführen einer Protoplastenfusion und Zellregeneration an den ausgewählten Zellen.

**33.** Verfahren nach einem der vorstehenden Ansprüche 29 bis 31, wobei wenigstens eine der zwei Ausgangspopulationen von Protoplasten, die miteinander fusionieren können, weiterhin mindestens einen zweiten Typ eines künstlichen Chromosoms mit Expressionskassette trägt, die heterologe Gene umfasst, wobei die ersten und zweiten Typen künstlicher Chromosomen jeder Population mindestens einen auswählbaren Marker tragen, so dass die ersten und zweiten Typen künstlicher Chromosomen individuell ausgewählt werden können.

**34.** Verfahren nach einem der vorstehenden Ansprüche 29 bis 33, wobei die Auswahl einer Teilmenge der auswählbaren Marker eine Auswahl von mindestens 70% aller fusionierten Zelltypen beinhaltet, die in der fusionierten Population vorkommen.

**35.** Verfahren nach einem der vorstehenden Ansprüche, weiter umfassend Aussetzen der Populationen von Zellen einer physischen Isolierung von künstlichen Chromosomen der Populationen für jede zwei bis drei Runden der Meiose und Auswahl, und Überführen der isolierten künstlichen Chromosomen in neue Wirtszellen.

**36.** Verfahren nach einem der vorstehenden Ansprüche 16-35, wobei die zwei Ausgangspopulationen von Zellen ein bis zehn Typen künstlicher Chromosomen tragen, wobei jeder Typ eines künstlichen Chromosoms jeder Zellpopulation mindestens einen auswählbaren Marker trägt, so dass jeder Typ der künstlichen Chromosomen von jeder der zwei Zellpopulationen individuell ausgewählt werden kann.

**37.** Verfahren nach einem der vorstehenden Ansprüche 16-36, weiter umfassend Zugeben einer weiteren Population von Zellen mit künstlichen Chromosomen, die mindestens zwei Expressionskassetten mit heterologen Genen umfassen, wobei die Zellen fähig sind mit den Zellen zu fusionieren, die eine Fusion eingegangen sind, wobei die weitere Population mindestens zwei Zellen mit Kombinationen von Expressionskassetten umfasst, die von den Kombinationen in den Zellen der Ausgangspopulation unterschiedlich sind, wobei die künstlichen Chromosomen der weiteren Population mindestens einen auswählbaren Marker tragen.

**38.** Verfahren nach Anspruch 37, wobei die weitere Population von Zellen mit künstlichen Chromosomen, die fähig sind, mit den Zellen zu fusionieren, die eine Verbindung/Paarung und Meiose eingegangen sind, von einem bis zehn Typen von künstlichen Chromosomen trägt, wobei jeder Typ eines unterschiedlichen Chromosoms der weiteren Population mindestens einen auswählbaren Marker trägt, so dass jeder der Typen eines künstlichen Chromosoms individuell ausgewählt werden kann.

**39.** Verfahren nach einem der vorstehenden Ansprüche 29-38, umfassend die Merkmale eines der Ansprüche 16-18 und 23-29.

**40.** Verfahren zum Mischen von heterologen Genen in Expressionskassetten, die sich auf künstlichen Chromosomen befinden, wobei das Verfahren die Schritte umfasst:

a) Erhalten von mindestens einer Population von Zellen, wobei die Zellen der mindestens einen Population umfassen
einen Concatemer von Expressionskassetten mit der folgenden Formel:

$$[rs_2\text{-SP-PR-X-TR-SP-}rs_1]_n$$

wobei

$rs_1$ und $rs_2$ zusammen eine funktionale Restriktionsstelle bezeichnen,
SP einzeln einen Spacer aus mindestens zwei Nukleotidbasen bezeichnet,
PR einen Promotor bezeichnet, der fähig ist, in einer Zelle zu funktionieren,
X eine exprimierbare Nukleotidsequenz bezeichnet,
TR einen Terminator bezeichnet, und
$n \geq 2$,
wobei die Zellen sich voneinander in Bezug auf die Kombinationen von exprimierbaren Nukleotidsequenzen und/oder Promotoren unterscheiden,

b) Isolieren wenigstens einiger der Kassetten der ausgewählten Zellen mittels Schneiden des Concatemers mit einem Restriktionsenzym, das $rs_1rs_2$ schneidet,
c) Amplifizieren wenigstens einiger der isolierten Kassetten,
d) Zusammenfügen der Expressionskassetten aus Schritt c) in künstliche Chromosomen, und
e) wahlweise Übertragen der künstlichen Chromosomen in Wirtszellen.

**41.** Verfahren nach Anspruch 40, wobei Amplifizieren von isolierten Kassetten eine PCR mit Primern für das Identifizieren (Tagging) von $rs_1$ und $rs_2$ umfasst.

**42.** Verfahren nach Anspruch 40, wobei Amplifizieren von isolierten Kassetten Einfügen von isolierten Kassetten in einen Vektor umfasst, der eine mit $rs_1rs_2$ kompatible Klonierungsstelle aufweist und Vervielfältigen dieses Vektors in einem geeigneten Wirt.

**43.** Verfahren nach Anspruch 40, weiter umfassend Zugeben weiterer Kassetten für den Schritt des Zusammenfügens.

**44.** Verfahren nach einem der vorstehenden Ansprüche 40 bis 43, weiter umfassend Durchmustern der Zellen mit zusammengefügten künstlichen Chromosomen auf (eine) gewünschte Funktionalität(en) und Auswählen der Zellen mit der bzw. den gewünschte(n) Funktionalität(en).

**45.** Verfahren nach Anspruch 44, weiter umfassend Unterwerfen der ausgewählten Zellen einer weiteren Isolierung und Amplifizierung der Kassetten und Zusammenfügen von künstlichen Chromosomen.

**46.** Verfahren zum Mischen von heterologen Genen in Expressionskassetten, die sich auf künstlichen Chromosomen befinden, wobei das Verfahren die Schritte umfasst:

Bereitstellen von zwei Ausgangspopulationen von Zellen,
wobei die Ausgangspopulationen mindestens zwei Zellen in jeder Population umfassen, und wobei mindestens zwei Zellen in jeder Population unterschiedliche Kombinationen von heterologen Genen und/oder unterschiedliche Kombinationen von Expressionskassetten aufweisen,
wobei sich die Expressionskassetten auf einem Nukleotid-Coneatemer befinden, der in 5'→3' Richtung eine Kassette mit Nukleotidsequenz aufweist mit der generellen Formel

$$[rs_2\text{-SP-PR-X-TR-SP-}rs_1]_n$$

wobei

$rs_1$ und $rs_2$ zusammen eine funktionale Restriktionsstelle bezeichnen,
SP einzeln einen Spacer aus mindestens zwei Nukleotidbasen bezeichnet,
PR einen Promotor bezeichnet, der fähig ist, in einer Zelle zu funktionieren,
X eine exprimierbare Nukleotidsequenz bezeichnet,
TR einen Terminator bezeichnet, und
SP einzeln einen Spacer aus mindestens zwei Nukleotidbasen bezeichnet, und
$n \geq 2$, und
wobei sich mindestens eine erste Kassette von einer zweiten Kassette unterscheidet,

wobei jede Zelle mindestens einen ersten Typ eines künstlichen Chromosoms umfasst, wobei der mindestens erste Typ eines künstlichen Chromosoms mindestens zwei Expressionskassetten umfassend heterologe Gene und mindestens einen auswählbaren Marker umfasst, wobei die auswählbaren Marker den künstlichen Chromosomen zugeteilt sind, so dass jeder Typ eines künstlichen Chromosoms von jeder Population individuell ausgewählt werden kann,
Verbinden/Paaren der Zellen miteinander,
Amplifizieren der künstlichen Chromosomen in den Wirtszellen,
Isolieren der künstlichen Chromosomen,
Mischen der isolierten künstlichen Chromosomen,
Übertragen von Teilmengen der isolierten und gemischten künstlichen Chromosomen in Wirtszellen, und
Auswählen von Zellen, die mindestens eine Teilmenge der auswählbaren Marker tragen, die auf den künstlichen Chromosomen der zwei Ausgangspopulationen vorhanden sind.

**47.** Verfahren nach Anspruch 46, weiter umfassend mindestens einmaliges Wiederholen des Mischvorgangs.

**48.** Verfahren nach Anspruch 46, wobei die Wirtszellen, in die die Teilmengen der gemischten Typen unterschiedlicher Chromosomen übertragen werden, bereits künstliche Chromosomen mit Expressionskassetten mit heterologen Genen umfassen.

**49.** Verfahren zum Mischen von heterologen Genen in Expressionskassetten, die sich auf Plasmiden befinden, wobei das Verfahren die Schritte umfasst:

Bereitstellen von zwei Ausgangspopulationen von Zellen, die sich miteinander verbinden/paaren können,
wobei die Ausgangspopulationen mindestens zwei Zellen in jeder Population umfassen, und wobei mindestens zwei Zellen in jeder Population unterschiedliche Kombinationen von heterologen Genen und/oder unterschiedliche Kombinationen von Expressionskassetten aufweisen,
wobei sich die Expressionskassetten auf einem Nukleotid-Concatemer befinden, der in 5'→3' Richtung eine Kassette mit Nukleotidsequenz aufweist mit der generellen Formel

$$[rs_2\text{-SP-PR-X-TR-SP-}rs_1]_n$$

wobei

$rs_1$ und $rs_2$ zusammen eine funktionale Restriktionsstelle bezeichnen,
SP einzeln einen Spacer aus mindestens zwei Nukleotidbasen bezeichnet,

PR einen Promotor bezeichnet, der fähig ist, in einer Zelle zu funktionieren,
X eine exprimierbare Nukleotidsequenz bezeichnet,
TR einen Terminator bezeichnet, und
SP einzeln einen Spacer aus mindestens zwei Nukleotidbasen bezeichnet, und
$n \geq 2$, und
wobei sich mindestens eine erste Kassette von einer zweiten Kassette unterscheidet,

wobei jede Zelle mindestens ein erstes Plasmid umfasst, wobei das mindestens erste Plasmid mindestens zwei Expressionskassetten umfassend heterologe Gene und mindestens einen auswählbaren Marker umfasst, wobei die auswählbaren Marker Plasmiden zugeteilt sind, so dass jeder Typ eines Plasmids von jeder Population individuell ausgewählt werden kann,
Verbinden/Paaren der Zellen miteinander,
Auswählen von verbundenen/gepaarten Zellen, die mindestens eine Teilmenge der auswählbaren Marker tragen, die auf den Plasmiden der zwei Ausgangspopulationen vorhanden sind.

## Revendications

**1.** Procédé de mélange de gènes hétérologues dans des casettes d'expression se trouvant sur des chromosomes artificiels, ledit procédé comprenant les étapes consistant à

(a) fournir deux populations initiales de cellules qui peuvent s'apparier les unes aux autres,
lesdites populations initiales comprenant au moins 2 cellules dans chaque population, et au moins deux cellules dans une population ayant des combinaisons différentes de gènes hétérologues et/ou des combinaisons différentes de cassettes d'expression,
chaque cellule comprenant au moins un premier type de chromosome artificiel, le ou les premiers types de chromosome artificiel comprenant à la fois au moins deux cassettes d'expression comprenant des gènes hétérologues et au moins un marqueur de sélection,
les marqueurs de sélection étant répartis entre les chromosomes artificiels de manière à ce que chaque type de chromosome artificiel de chaque population puisse être individuellement choisi,
les cassettes d'expression étant situées sur un concatémère de nucléotides comprenant dans la direction 5'→3' une cassette de séquence nucléotidique de formule générale

$$[rs_2\text{-SP- PR- X- TR- SP- }rs_1]_n$$

dans laquelle

$rs_1$ et $rs_2$ représentent ensemble un site de restriction fonctionnel,
SP représente individuellement un espaceur d'au moins deux bases nucléotidiques,
PR représente un promoteur, capable de fonctionner dans une cellule,
X représente une séquence nucléotidique pouvant être exprimée,
TR représente un terminateur, et
SP représente individuellement un espaceur d'au moins deux bases nucléotidiques, et
$n \geq 2$, et

dans lequel au moins une première cassette est différente d'une seconde cassette,
(b) apparier les cellules les unes avec les autres, et
choisir des cellules appariées qui portent au moins un sous-ensemble des marqueurs de sélection présents sur les chromosomes artificiels dans les deux populations initiales.

**2.** Procédé selon la revendication 1, dans lequel au moins deux cellules dans chaque population ont des combinaisons différentes de gènes hétérologues et/ou des combinaisons différentes de cassettes d'expression.

**3.** Procédé selon la revendication 1, comprenant en outre le fait de faire subir une méiose aux cellules appariées.

**4.** Procédé selon la revendication 1, dans lequel le sous-ensemble de marqueurs choisi comprend au moins un marqueur d'un chromosome artificiel dans chacune des populations initiales pour assurer la sélection des cellules appariées.

**5.** Procédé selon l'une quelconque des revendications précédentes, dans lequel le choix d'un sous-ensemble des marqueurs de sélection comprend le choix d'au moins 70 % de tous les types diploïdes présents dans la population appariée.

**6.** Procédé selon l'une quelconque des revendications précédentes, comprenant en outre le criblage des cellules appariées à la recherche d'un ou de plusieurs paramètres liés à une (des) fonctionnalité(s) souhaitée(s) et le choix de cellules ayant un (des) critère(s) de sélection prédéfini(s) pour leur faire subir une méiose et les faire s'apparier.

**7.** Procédé selon l'une quelconque des revendications précédentes, comprenant en outre le criblage de cellules qui ont subi une méiose à la recherche d'au moins un paramètre lié à une (des) fonctionnalité(s) souhaitée(s) et le choix de cellules ayant un (des) critère(s) de sélection prédéfini(s) pour les faire s'apparier et leur faire subir une méiose.

**8.** Procédé selon les revendications 6 ou 7, dans lequel le (s) seuil (s) de sélection associé (s) à la (aux) fonctionnalité (s) souhaitée(s) est (sont) augmenté(s) pour chaque cycle d'appariement et de méiose.

**9.** Procédé selon l'une quelconque des revendications précédentes, comprenant en outre la répétition des étapes des revendications 1 et 2 au moins deux fois.

**10.** Procédé selon l'une quelconque des revendications précédentes, comprenant en outre la séparation des cellules les unes des autres des deux types s'appariant après la méiose.

**11.** Procédé selon l'une quelconque des revendications précédentes, comprenant en outre le mélange de spores de différentes populations avant l'appariement.

**12.** Procédé selon l'une quelconque des revendications 3 à 11 précédentes, comprenant en outre l'ajout d'une population de cellules supplémentaire avec des types de chromosomes artificiels comprenant au moins deux cassettes d'expression avec des gènes hétérologues, les cellules étant capables de s'apparier avec les cellules qui ont subi un appariement et une méiose, la population supplémentaire comprenant au moins 2 cellules avec des combinaisons de cassettes d'expression différentes des combinaisons dans les cellules de la population initiale, les chromosomes artificiels de ladite population supplémentaire portant au moins un marqueur de sélection.

**13.** Procédé selon l'une quelconque des revendications précédentes, dans lequel au moins l'une des deux populations initiales de cellules qui peuvent s'apparier les unes aux autres porte en outre au moins un second type de chromosome artificiel avec des cassettes d'expression comprenant des gènes hétérologues, les premier et second types de chromosome artificiel portant au moins un marqueur de sélection de manière à ce que lesdits premier et second types de chromosome artificiel puissent être individuellement choisis.

**14.** Procédé selon l'une quelconque des revendications précédentes, dans lequel les deux populations initiales de cellules qui peuvent s'apparier les unes aux autres portent de 1 à 10 types de chromosomes artificiels, chaque type de chromosome artificiel de chaque population portant au moins un marqueur de sélection de telle manière que chacun des types de chromosomes artificiels de chacune des deux populations puisse être individuellement choisi.

**15.** Procédé selon l'une quelconque des revendications précédentes, dans lequel chaque cellule porte 2 chromosomes artificiels par cellule qui peut s'apparier.

**16.** Procédé selon l'une quelconque des revendications précédentes, dans lequel chaque chromosome artificiel porte au moins deux marqueurs de sélection, les marqueurs de sélection étant répartis entre les chromosomes artificiels de manière à ce que chaque type de chromosome artificiel de chaque population puisse être individuellement choisi.

**17.** Procédé selon l'une quelconque des revendications précédentes, dans lequel deux populations initiales sont de différents types d'appariement.

**18.** Procédé selon l'une quelconque des revendications précédentes, dans lequel un type de chromosomes artificiels ayant le même marqueur ou la même combinaison de marqueur diffère relativement aux combinaisons de cassettes d'expression comprenant les gènes hétérologues.

**19.** Procédé selon l'une quelconque des revendications précédentes, dans lequel l'espèce de cellules est des cellules fongiques choisies parmi une espèce sporulée.

**20.** Procédé selon l'une quelconque des revendications précédentes, dans lequel l'espèce de cellules est des cellules de levure.

**21.** Procédé selon l'une quelconque des revendications précédentes, dans lequel les cellules appariées sont diploïdes ou tétraploïdes ou hexaploïdes.

**22.** Procédé selon l'une quelconque des revendications précédentes, qui comprend les séquences nucléotidiques d'au moins deux états d'expression.

**23.** Procédé selon l'une quelconque des revendications précédentes, dans lequel le site de restriction $rs_1$-$rs_2$ de sensiblement toutes les cassettes est reconnu par la même enzyme de restriction.

**24.** Procédé selon l'une quelconque des revendications précédentes, dans lequel sensiblement toutes les cassettes d'expression sur un chromosome artificiel sont différentes.

**25.** Procédé selon l'une quelconque des revendications précédentes, dans lequel au moins une cassette d'expression comprend un intron entre le promoteur et la séquence nucléotidique pouvant être exprimée.

**26.** Procédé selon l'une quelconque des revendications précédentes, dans lequel les différentes combinaisons de cassettes d'expression comprennent différents promoteurs, et/ou différentes séquences nucléotidiques pouvant être exprimées, et/ou différents espaceurs et/ou différents terminateurs et/ou différents introns.

**27.** Procédé selon l'une quelconque des revendications précédentes, dans lequel n vaut au moins 10.

**28.** Procédé selon l'une quelconque des revendications précédentes, dans lequel les différents états d'expression représentent au moins deux tissus différents, par exemple au moins deux organes, par exemple au moins deux espèces, par exemple au moins deux genres.

**29.** Procédé de mélange de gènes hétérologues dans des casettes d'expression se trouvant sur des chromosomes artificiels, ledit procédé comprenant les étapes consistant à
fournir deux populations initiales de protoplastes ou de cellules qui peuvent être fusionné(e)s,
lesdites populations initiales comprenant au moins 2 cellules dans chaque population, et au moins deux cellules dans chaque population ayant des combinaisons différentes de gènes hétérologues et/ou des combinaisons différentes de cassettes d'expression,
les cassettes d'expression étant situées sur un concatémère de nucléotides comprenant dans la direction 5'→3' une cassette de séquence nucléotidique de formule générale

$$[\mathbf{rs_2\text{-}SP\text{-}\ PR\text{-}\ X\text{-}\ TR\text{-}\ SP\text{-}\ rs_1}]_n$$

dans laquelle

$rs_1$ et $rs_2$ représentent ensemble un site de restriction fonctionnel,
SP représente individuellement un espaceur d'au moins deux bases nucléotidiques,
PR représente un promoteur, capable de fonctionner dans une cellule,
X représente une séquence nucléotidique pouvant être exprimée,
TR représente un terminateur, et
SP représente individuellement un espaceur d'au moins deux bases nucléotidiques, et
$n \geq 2$, et

dans lequel au moins une première cassette est différente d'une seconde cassette,
chaque cellule comprenant au moins un premier type de chromosome artificiel, ledit premier type de chromosome artificiel ou plus comprenant à la fois au moins deux cassettes d'expression comprenant des gènes hétérologues et au moins un marqueur de sélection,
les marqueurs de sélection étant répartis entre les chromosomes artificiels de manière à ce que chaque type de chromosome artificiel de chaque population puisse être individuellement choisi,
réaliser une fusion de protoplaste et une régénération des parois cellulaires ou réaliser une fusion de cellules, et
choisir des cellules fusionnées qui portent au moins un sous-ensemble des marqueurs de sélection présents sur les chromosomes artificiels dans les deux populations initiales.

**30.** Procédé selon la revendication 29, comprenant en outre la répétition des étapes selon la revendication 29.

**31.** Procédé selon la revendication 29, dans lequel les espèces de cellules sont choisies parmi les champignons, les algues, les plantes, les procaryotes, les cellules animales ou les cellules humaines.

**32.** Procédé selon l'une quelconque des revendications 29 à 31 précédentes, comprenant en outre le criblage de cellules qui résultent d'une fusion de protoplastes à la recherche d'une (de) fonctionnalité(s) souhaitée (s) et le choix de cellules ayant la (les) fonctionnalité(s) souhaitées au-dessus d'un seuil défini, l'isolement de protoplastes de ces cellules et la réalisation d'une fusion de protoplastes et d'une régénération cellulaire sur les cellules choisies.

**33.** Procédé selon l'une quelconque des revendications 29 à 31 précédentes, dans lequel au moins l'une des deux populations initiales de protoplastes qui peuvent fusionner les uns aux autres porte en outre au moins un second type de chromosome artificiel avec des cassettes d'expression comprenant des gènes hétérologues, les premier et second types de chromosome artificiel de chaque population portant au moins un marqueur de sélection de manière à ce que lesdits premier et second types de chromosome artificiel puissent être individuellement choisis.

**34.** Procédé selon l'une quelconque des revendications 29 à 33 précédentes, dans lequel le choix d'un sous-ensemble des marqueurs de sélection comprend le choix d'au moins 70 % de tous les types de cellules fusionnées présents dans la population fusionnée.

**35.** Procédé selon l'une quelconque des revendications précédentes, comprenant en outre la soumission des populations de cellules à un isolement physique de chromosomes artificiels des populations tous les 2 à 3 cycles de méiose et de sélection, et le transfert des chromosomes artificiels isolés dans de nouvelles cellules hôtes.

**36.** Procédé selon l'une quelconque des revendications 16 à 35 précédentes, dans lequel les deux populations initiales de cellules portent de 1 à 10 types de chromosomes artificiels, chaque type de chromosome artificiel de chaque population portant au moins un marqueur de sélection de telle manière que chacun des types de chromosomes artificiels de chacune des deux populations puisse être individuellement choisi.

**37.** Procédé selon l'une quelconque des revendications 16 à 36 précédentes, comprenant en outre l'ajout d'une population de cellules supplémentaire avec des chromosomes artificiels comprenant au moins deux cassettes d'expression avec des gènes hétérologues, les cellules étant capables de fusionner avec les cellules qui ont subi une fusion, la population supplémentaire comprenant au moins 2 cellules avec des combinaisons de cassettes d'expression différentes des combinaisons dans les cellules de la population initiale, les chromosomes artificiels de ladite population supplémentaire portant au moins un marqueur de sélection.

**38.** Procédé selon la revendication 37, dans lequel la population supplémentaire de cellules avec des chromosomes artificiels capables de fusionner avec les cellules qui ont subi un appariement et une méiose porte de 1 à 10 type de chromosomes artificiels, chaque type de chromosome artificiel de ladite population supplémentaire portant au moins un marqueur de sélection de manière à ce que chacun des types de chromosomes artificiels puisse être individuellement choisi.

**39.** Procédé selon l'une quelconque des revendications 29 à 38 précédentes, comprenant en outre les caractéristiques selon l'une quelconque des revendications 16 à 18 et 23 à 29.

**40.** Procédé de mélange de gènes hétérologues dans des cassettes d'expression se trouvant sur des chromosomes artificiels, ledit procédé comprenant les étapes consistant à

a) obtenir au moins une population de cellules, les cellules de ladite ou desdites populations comprenant un concatémère de cassettes d'expression de la formule suivante :

$$\mathbf{[rs_2\text{-}SP\text{-} PR\text{-} R\text{-} X\text{-} TR\text{-} SP\text{-} rs_1]_n}$$

dans laquelle

$rs_1$ et $rs_2$ représentent ensemble un site de restriction fonctionnel,
SP représente individuellement un espaceur d'au moins deux bases nucléotidiques,
PR représente un promoteur, capable de fonctionner dans les cellules,

X représente une séquence nucléotidique pouvant être exprimée,
TR représente un terminateur, et
$n \geq 2$,

les cellules différant les unes des autres relativement aux combinaisons de séquences nucléotidiques pouvant être exprimées et/ou de promoteurs,
b) isoler au moins une partie des cassettes des cellules choisies en coupant les concatémères avec une enzyme de restriction clivant $rs_1rs_2$,
c) amplifier au moins une partie des cassettes isolées,
d) assembler les cassettes d'expression de l'étape c) dans les chromosomes artificiels, et
e) facultativement transférer les chromosomes artificiels dans les cellules hôtes.

**41.** Procédé selon la revendication 40, dans lequel l'amplification de la cassette isolée comprend une PCR avec des amorces pour marquer $rs_1$ et $rs_2$.

**42.** Procédé selon la revendication 40, dans lequel l'amplification de cassettes isolées comprend l'insertion des cassettes isolées dans un vecteur ayant un site de clonage compatible avec $rs_1rs_2$ et la multiplication de ce vecteur dans un hôte adapté.

**43.** Procédé selon la revendication 40, comprenant en outre des cassettes d'expression pour l'étape d'assemblage.

**44.** Procédé selon l'une quelconque des revendications 40 à 43 précédentes, comprenant en outre le criblage de cellules avec des chromosomes artificiels assemblés à la recherche d'une (de) fonctionnalité(s) souhaitée (s) et le choix de cellules ayant la (les) fonctionnalité(s) souhaitée(s).

**45.** Procédé selon la revendication 44, comprenant en outre la soumission des cellules choisies à un isolement et à une amplification supplémentaires des cassettes et à l'assemblage des chromosomes artificiels.

**46.** Procédé de mélange de gènes hétérologues dans des cassettes d'expression se trouvant sur des chromosomes artificiels, ledit procédé comprenant les étapes consistant à
fournir deux populations de cellules initiales,
lesdites populations initiales comprenant au moins 2 cellules dans chaque population, et au moins deux cellules dans chaque population ayant des combinaisons différentes de gènes hétérologues et/ou des combinaisons différentes de cassettes d'expression,
les cassettes d'expression étant situées sur un concatémère de nucléotides comprenant dans la direction 5'→3' une cassette de séquence nucléotidique de formule générale

$$[rs_2\text{-}SP\text{-}PR\text{-}R\text{-}X\text{-}TR\text{-}SP\text{-}rs_1]_n$$

dans laquelle

$rs_1$ et $rs_2$ représentent ensemble un site de restriction fonctionnel,
SP représente individuellement un espaceur d'au moins deux bases nucléotidiques,
PR représente un promoteur, capable de fonctionner dans une cellule,
X représente une séquence nucléotidique pouvant être exprimée,
TR représente un terminateur, et
SP représente individuellement un espaceur d'au moins deux bases nucléotidiques, et
$n \geq 2$, et

dans lequel au moins une première cassette est différente d'une seconde cassette,
chaque cellule comprenant au moins un premier type de chromosome artificiel, le ou les premiers types de chromosome artificiel comprenant à la fois au moins deux cassettes d'expression comprenant des gènes hétérologues et au moins un marqueur de sélection,
les marqueurs de sélection étant répartis entre les chromosomes artificiels de manière à ce que chaque type de chromosome artificiel de chaque population puisse être individuellement choisi,
apparier les cellules les unes avec les autres, amplifier les chromosomes artificiels dans les cellules hôtes,
isoler les chromosomes artificiels,
mélanger les chromosomes artificiels isolés,

transférer des sous-ensembles desdits chromosomes artificiels isolés et mélangés dans des cellules hôtes, et choisir des cellules qui portent au moins un sous-ensemble de marqueurs de sélection présents sur les chromosomes artificiels dans les deux populations initiales.

**47.** Procédé selon la revendication 46, comprenant en outre la répétition au moins une fois du procédé de mélange.

**48.** Procédé selon la revendication 46, dans lequel les cellules hôtes dans lesquelles les sous-ensembles de type mélangé de chromosomes artificiels sont transférés contiennent déjà des chromosomes artificiels avec des cassettes d'expression avec des gènes hétérologues.

**49.** Procédé de mélange de gènes hétérologues dans des casettes d'expression se trouvant sur des plasmides, ledit procédé comprenant les étapes consistant à
fournir deux populations initiales de cellules qui s'apparient les unes aux autres,
lesdites populations initiales comprenant au moins 2 cellules dans chaque population, et au moins deux cellules dans chaque population ayant des combinaisons différentes de gènes hétérologues et/ou des combinaisons différentes de cassettes d'expression,
dans lequel les cassettes d'expression se trouvent sur un concatémère de nucléotides comprenant dans la direction 5'→3' une cassette de séquence nucléotidique de formule générale

$$[rs_2\text{-SP- PR- X- TR- SP- }rs_1]_n$$

dans laquelle

$rs_1$ et $rs_2$ représentent ensemble un site de restriction fonctionnel,
SP représente individuellement un espaceur d'au moins deux bases nucléotidiques,
PR représente un promoteur, capable de fonctionner dans une cellule,
X représente une séquence nucléotidique pouvant être exprimée,
TR représente un terminateur, et
SP représente individuellement un espaceur d'au moins deux bases nucléotidiques, et
$n \geq 2$, et

dans lequel au moins une première cassette est différente d'une seconde cassette,
chaque cellule comprenant au moins un premier plasmide, le ou les premiers plasmides comprenant à la fois au moins deux cassettes d'expression comprenant des gènes hétérologues et au moins un marqueur de sélection,
les marqueurs de sélection étant répartis entre les plasmides de manière à ce que chaque type de plasmide de chaque population puisse être individuellement choisi,
apparier les cellules les unes avec les autres, et choisir des cellules appariées qui portent au moins un sous-ensemble des marqueurs de sélection présents sur les plasmides dans les deux populations initiales.

Fig. 1

Remixing Strategy

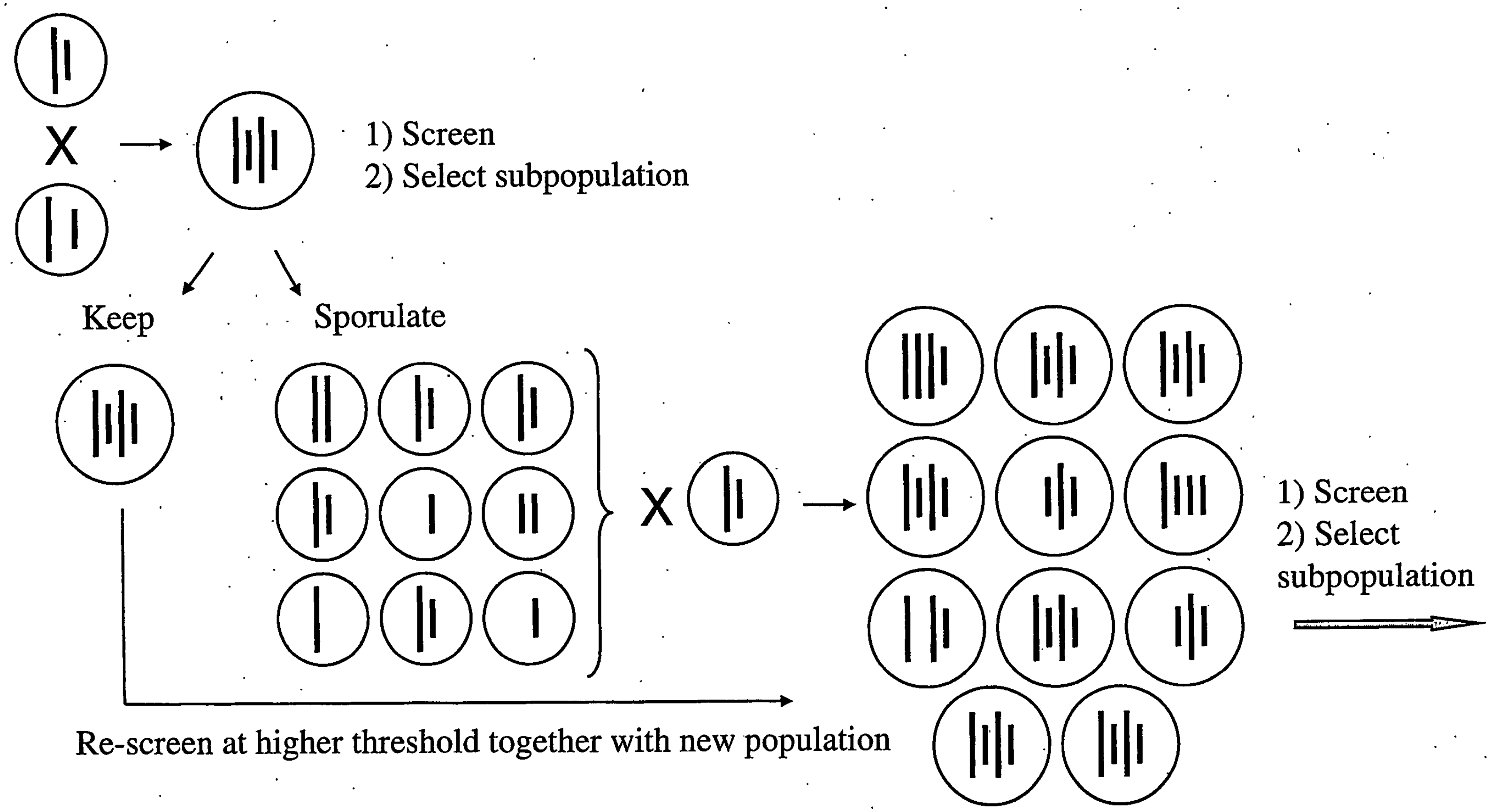

Fig. 1 cont'd

Remixing Strategy

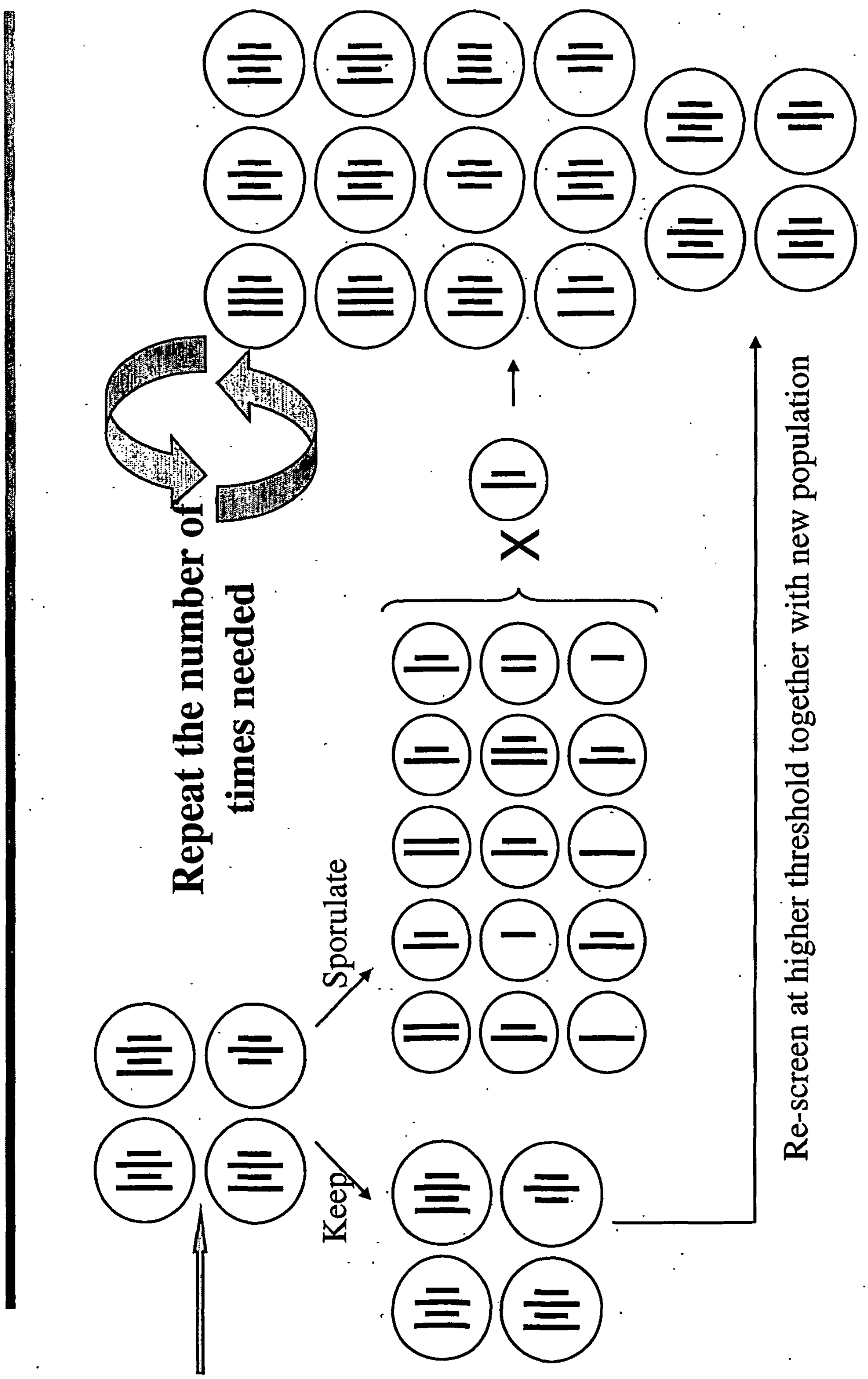

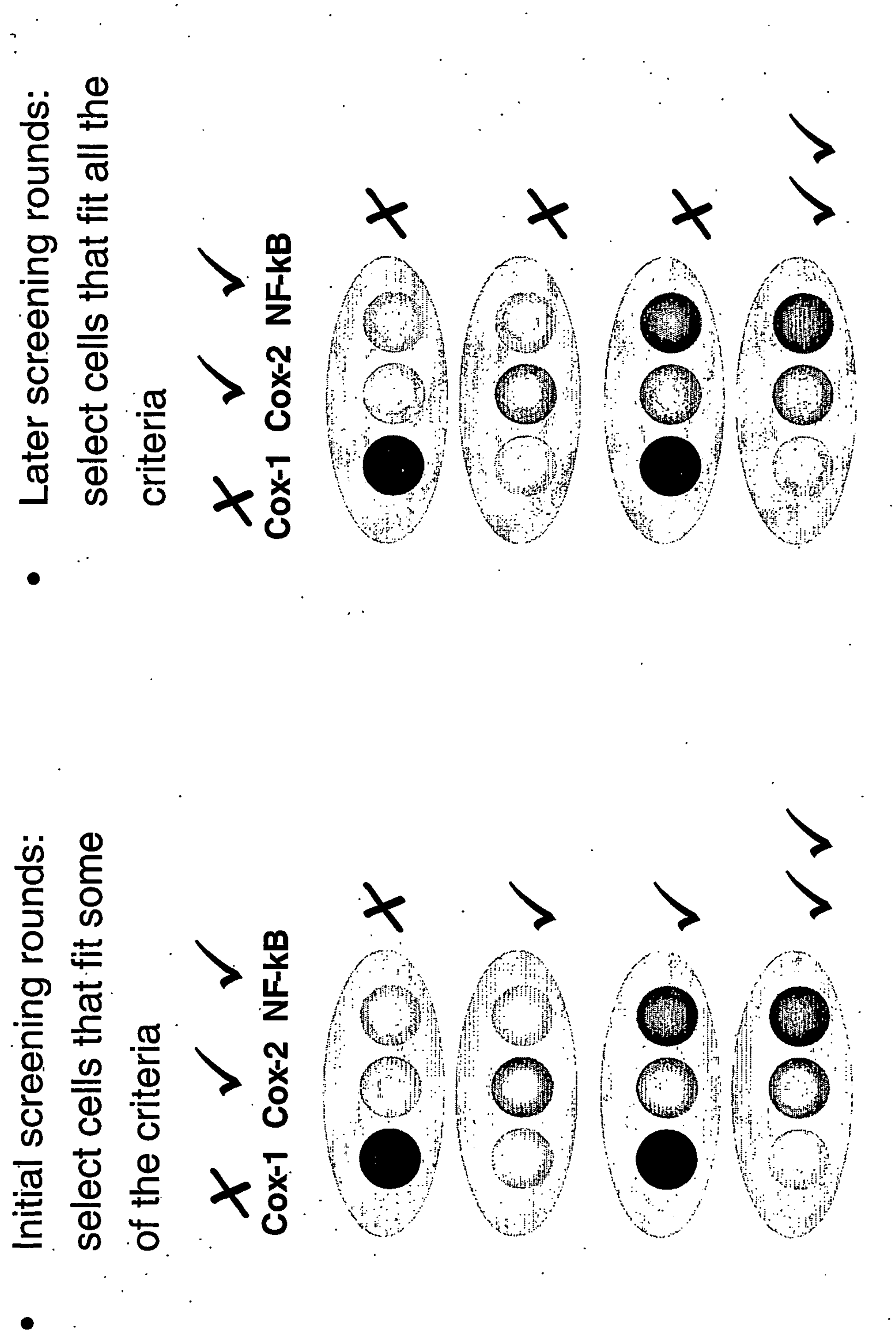
Initial screening rounds: select cells that fit some of the criteria
Cox-1 Cox-2 NF-kB
Later screening rounds: select cells that fit all the criteria
Cox-1 Cox-2 NF-kB

Fig. 2

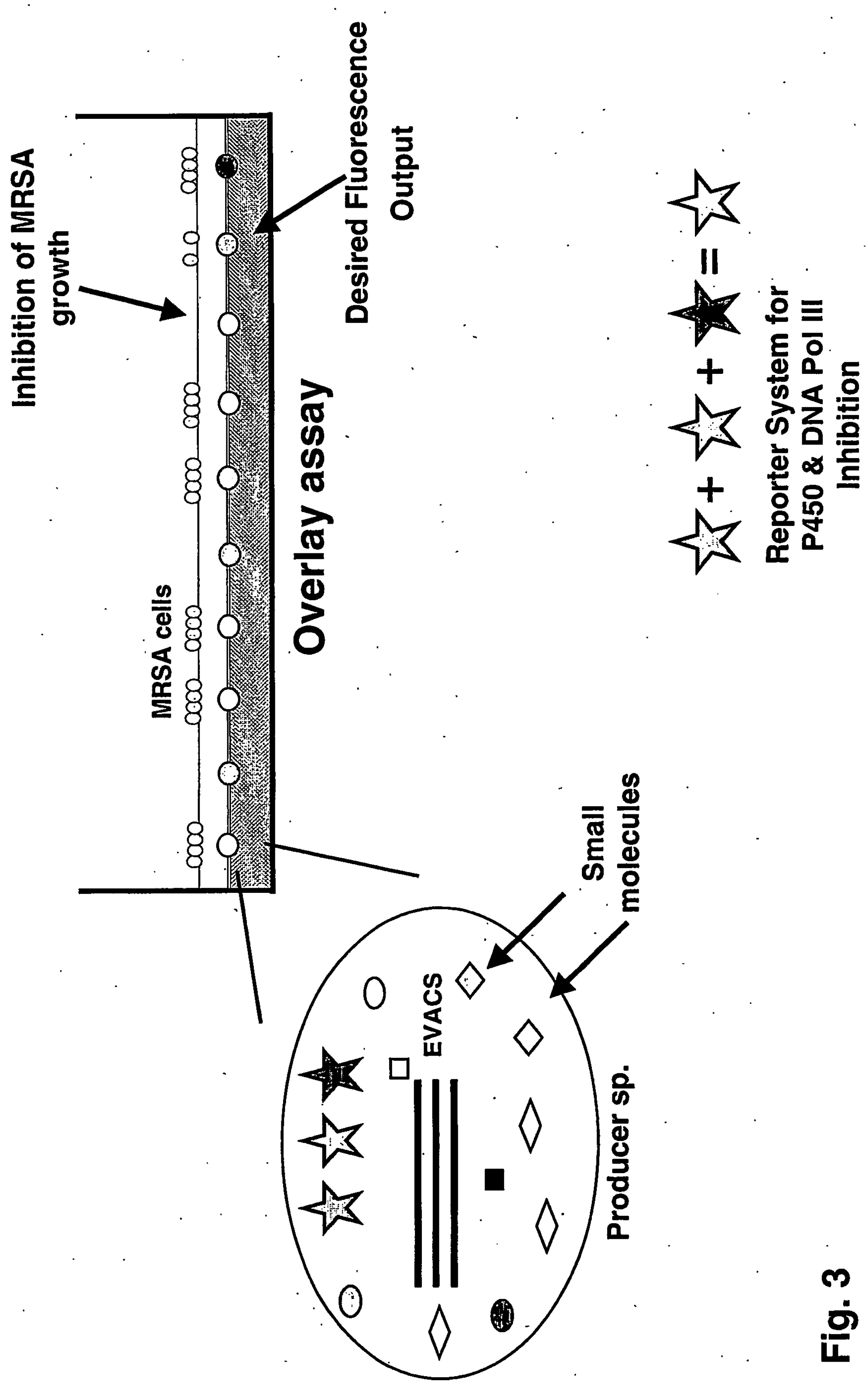
Inhibition of MRSA growth
Desired Fluorescence Output
MRSA cells
Overlay assay
Small molecules
EVACS
Producer sp.
Reporter System for P450 & DNA Pol III Inhibition

Fig. 3

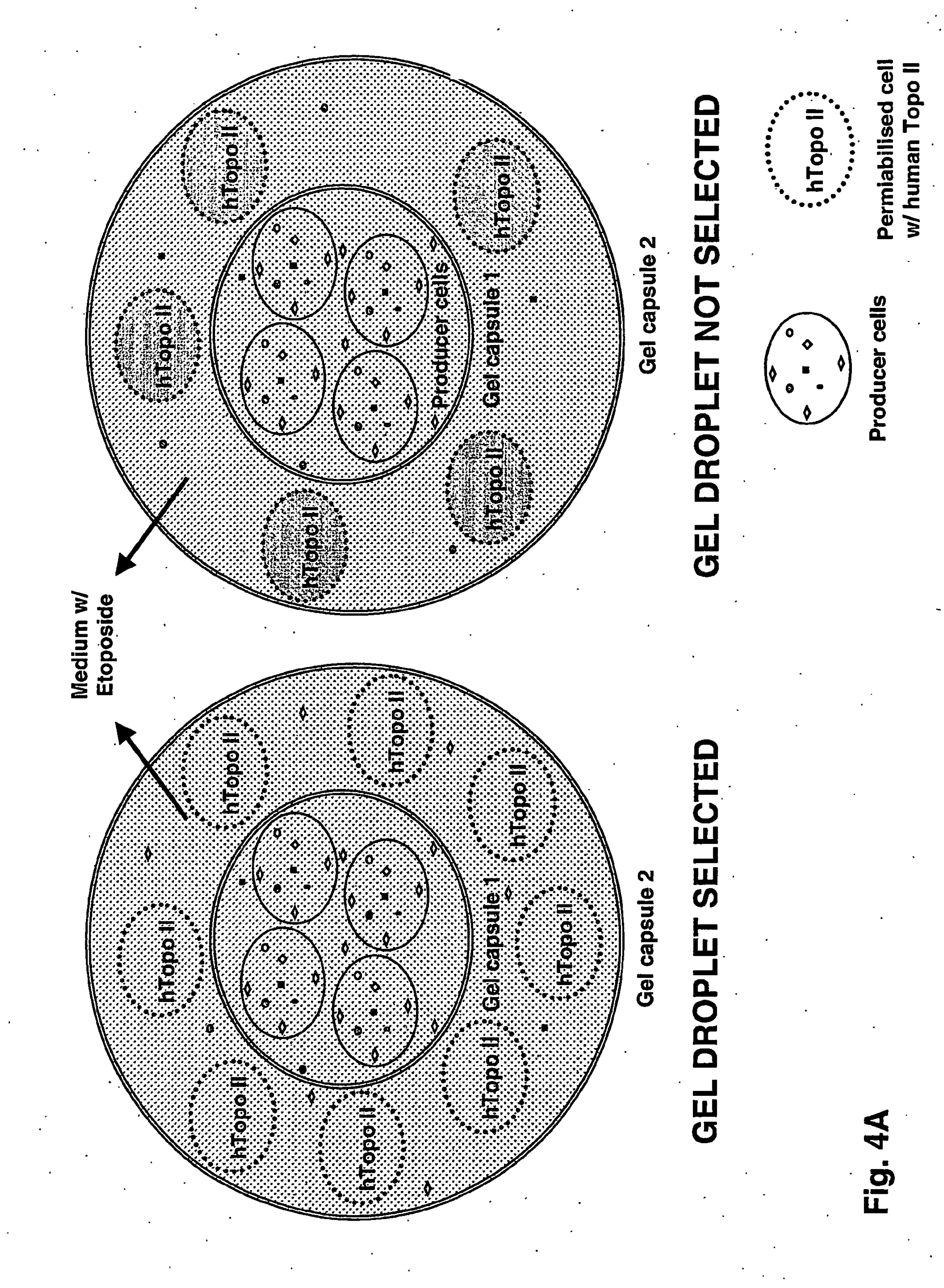
Medium w/
Etoposide
hTopo II
Producer cells
Gel capsule 1
Gel capsule 2
GEL DROPLET NOT SELECTED
GEL DROPLET SELECTED
Producer cells
Permiabilised cell
w/ human Topo II

Fig. 4A

Fig. 4B

# Evolving Topo II Compounds

- **Gel encapsulate single producer yeast**
  - Allow growth to 100 cell clonal colony
  - Induce
- **Gel encapsulate reporter yeast**
  - Procedure avoids excessive reporter yeast proliferation
- **Add precursor**
- **Screen & sort: (JN394) (JN394top2-5)**

**Gel droplet selected**

**Compound is a topo II inhibitor**

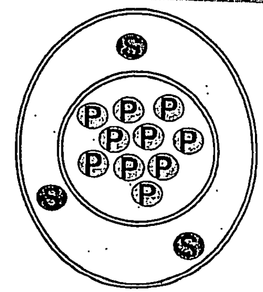

**Gel droplet <u>not</u> selected**

**Compounds do not Inhibit topo II**

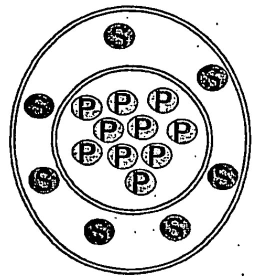

**Gel droplet <u>not</u> selected**

**Compound probably very toxic**

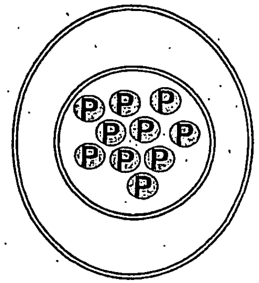

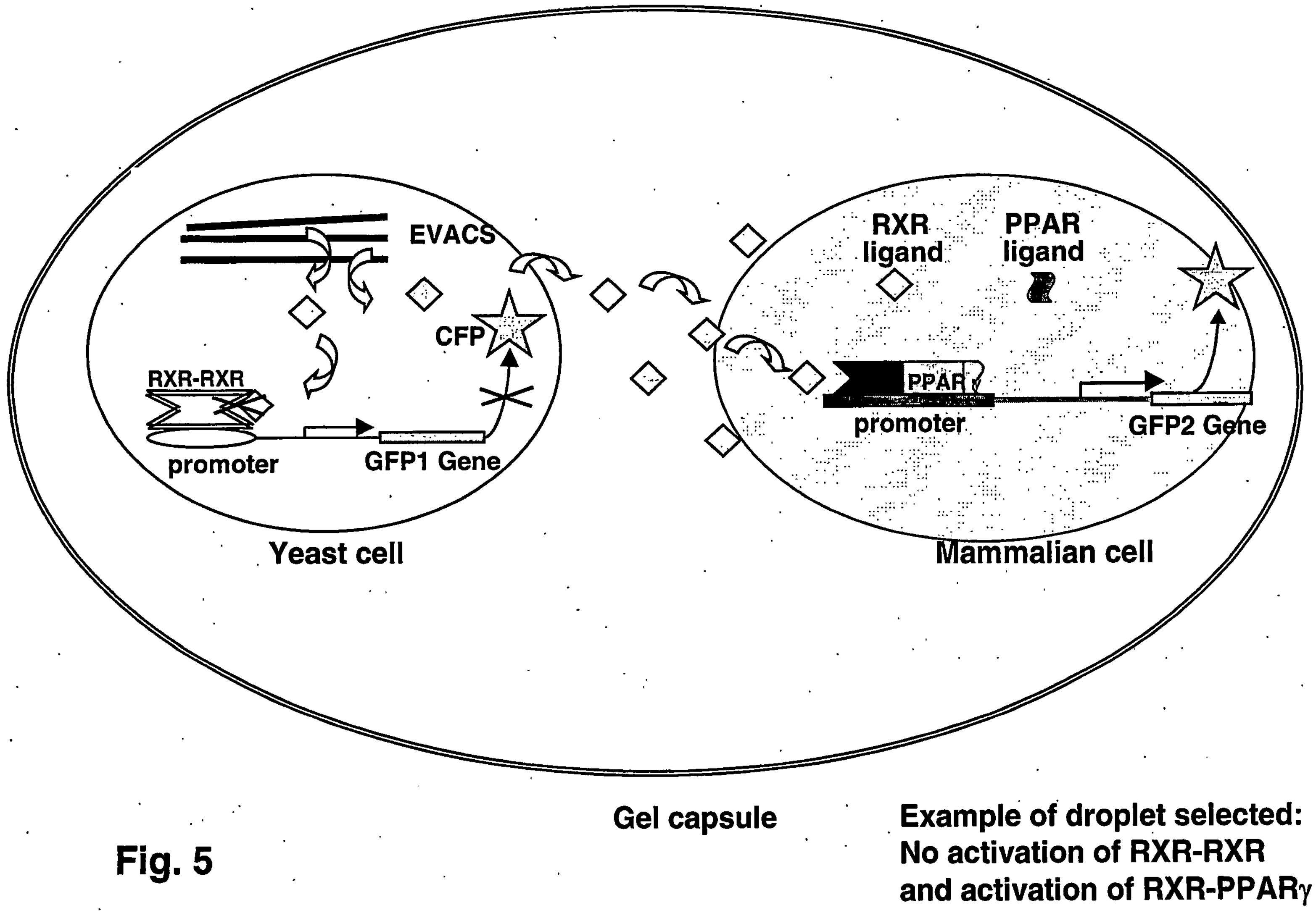


Example of droplet selected:
No activation of RXR-RXR
and activation of RXR-PPARγ

Fig. 5

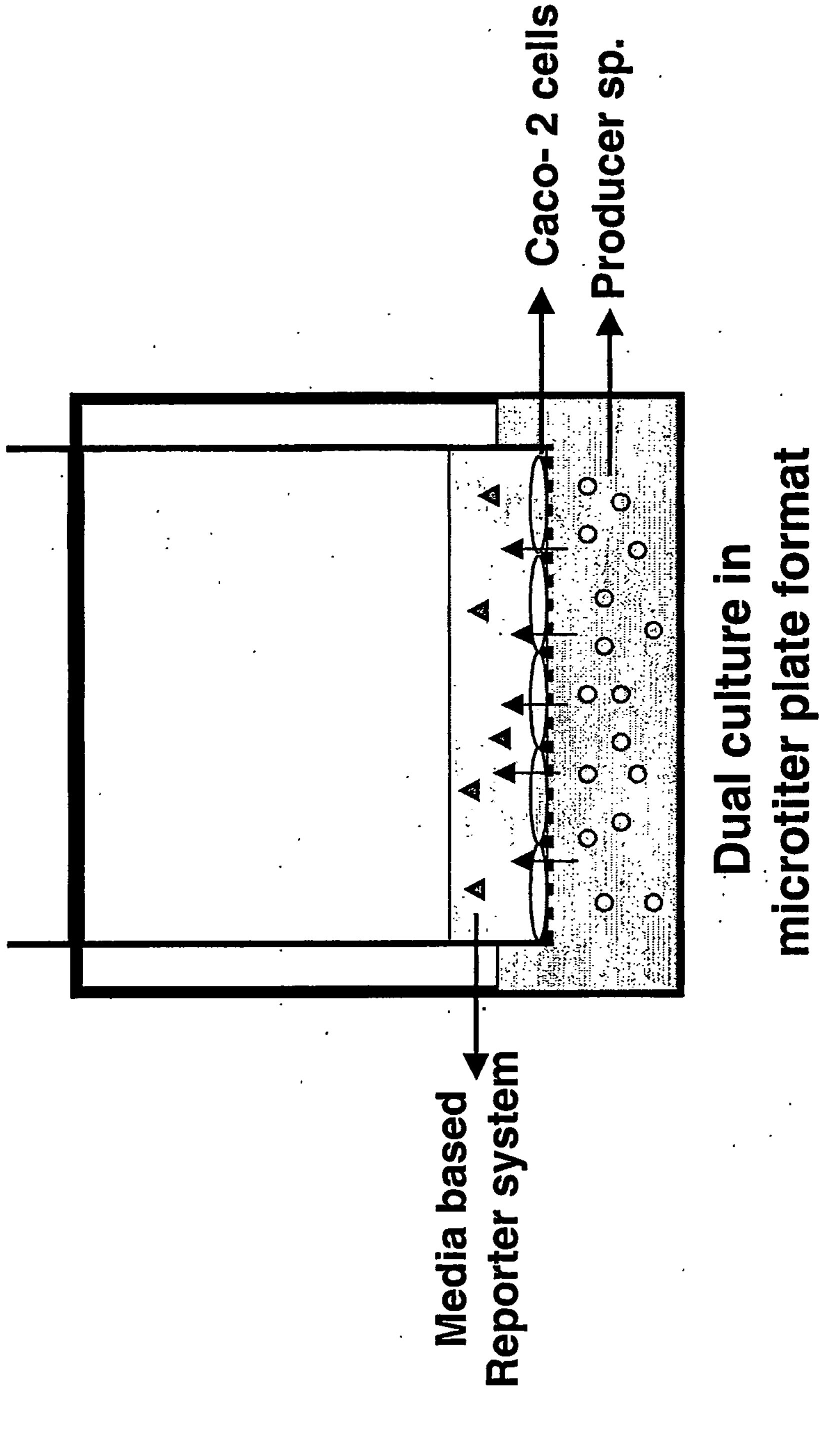
Caco- 2 cells
Producer sp.
Media based
Reporter system
Dual culture in
microtiter plate format

Fig. 6

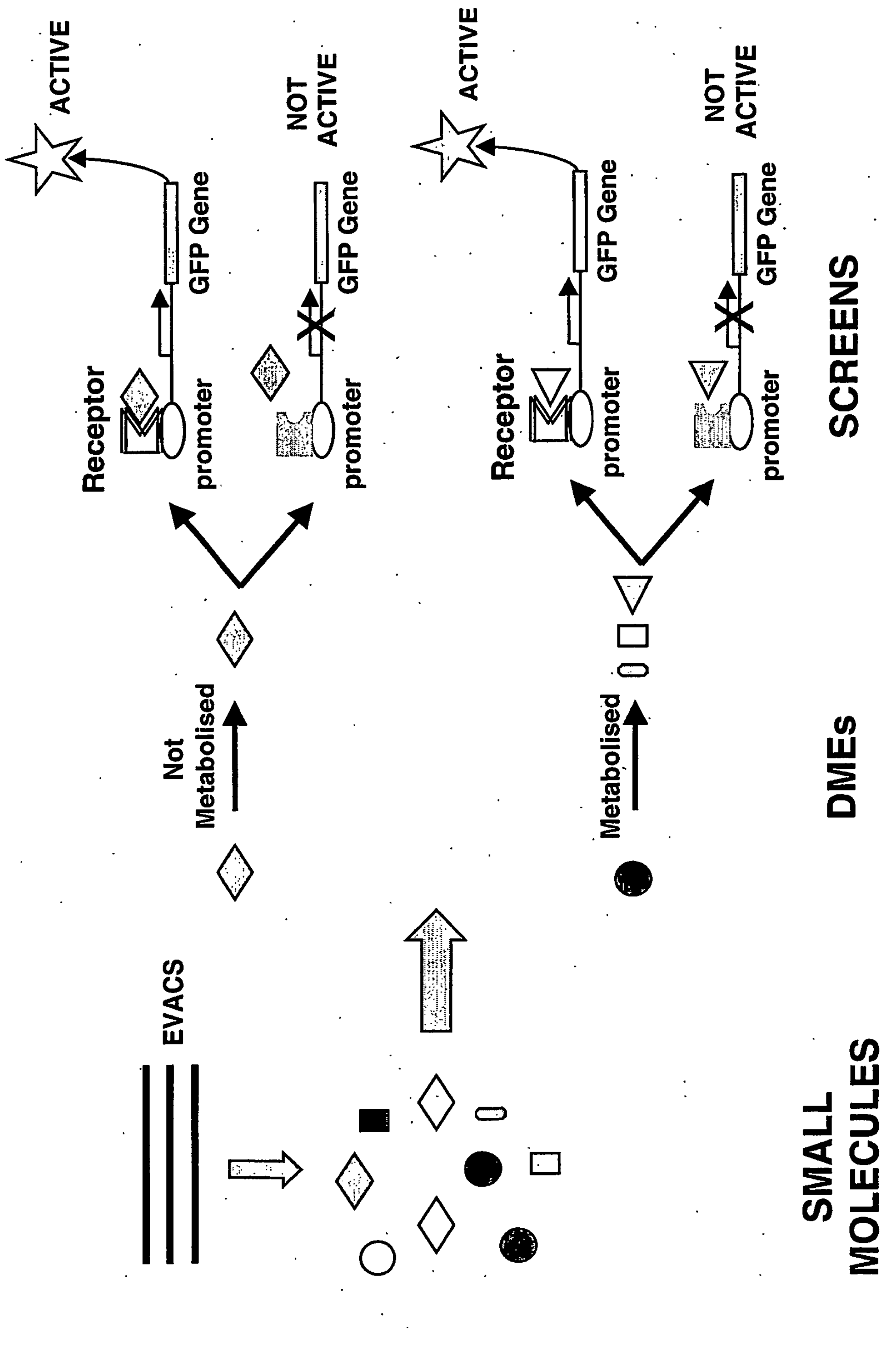
EVACS
SMALL MOLECULES
Not Metabolised
Metabolised
DMEs
Receptor
promoter
GFP Gene
ACTIVE
NOT ACTIVE
Receptor
promoter
GFP Gene
ACTIVE
NOT ACTIVE
SCREENS

Fig. 7

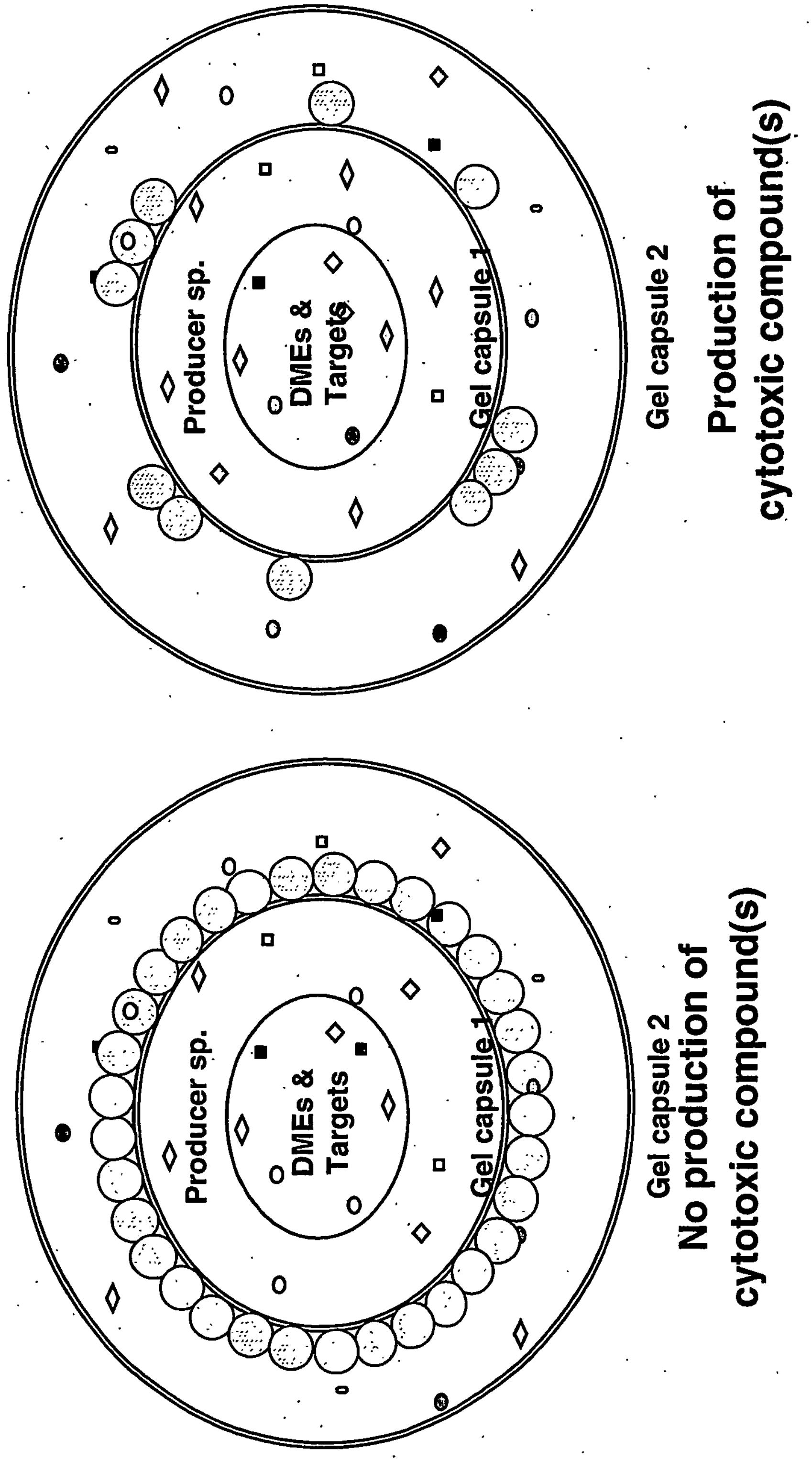

Producer sp.
DMEs &
Targets
Gel capsule 1
Gel capsule 2
No production of
cytotoxic compound(s)
Producer sp.
DMEs &
Targets
Gel capsule 1
Gel capsule 2
Production of
cytotoxic compound(s)


Fig. 8

Fig. 9

**Constructing entry libraries**

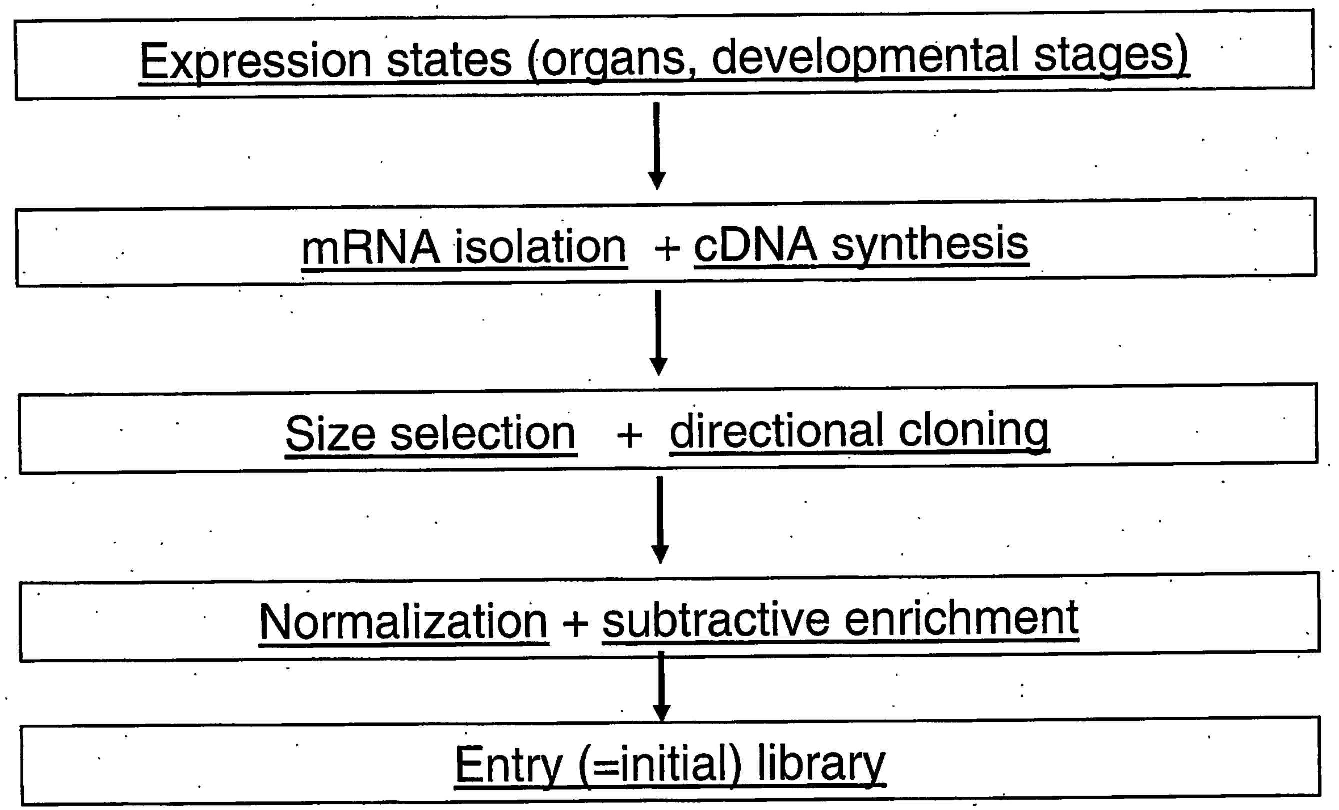

Fig. 10a

**Entry library to evolvable cell**

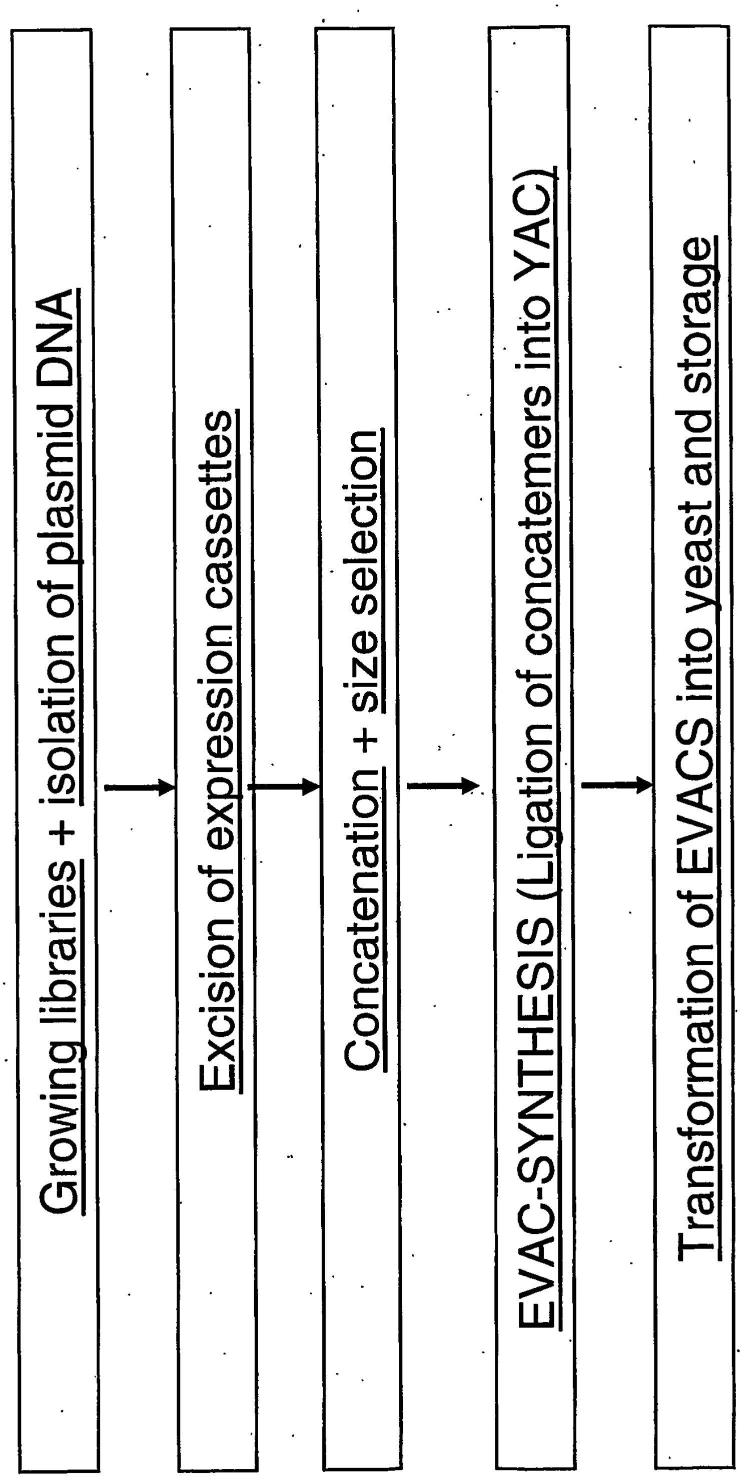

Fig. 10b **Entry library to evolvable cell**

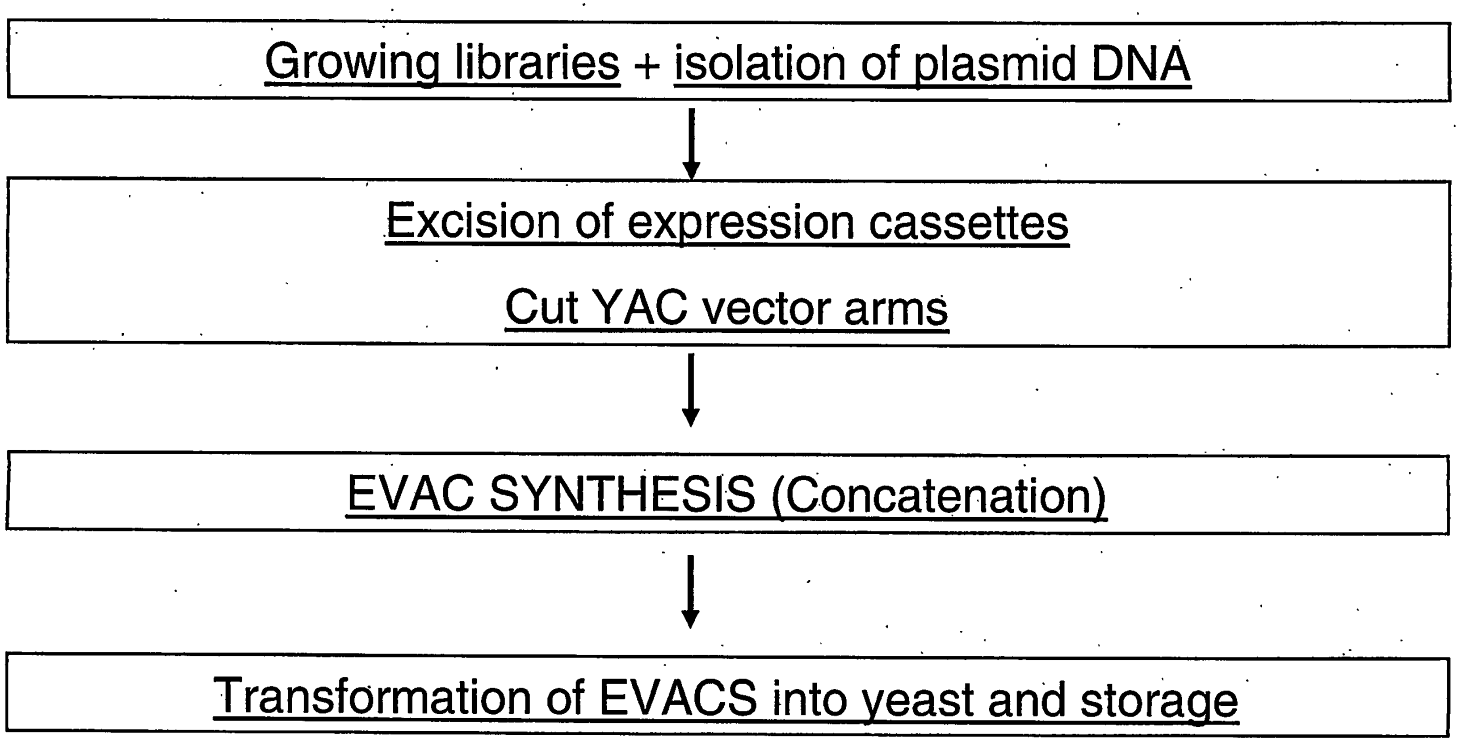

# Model Entry Vector

**Fig. 11**

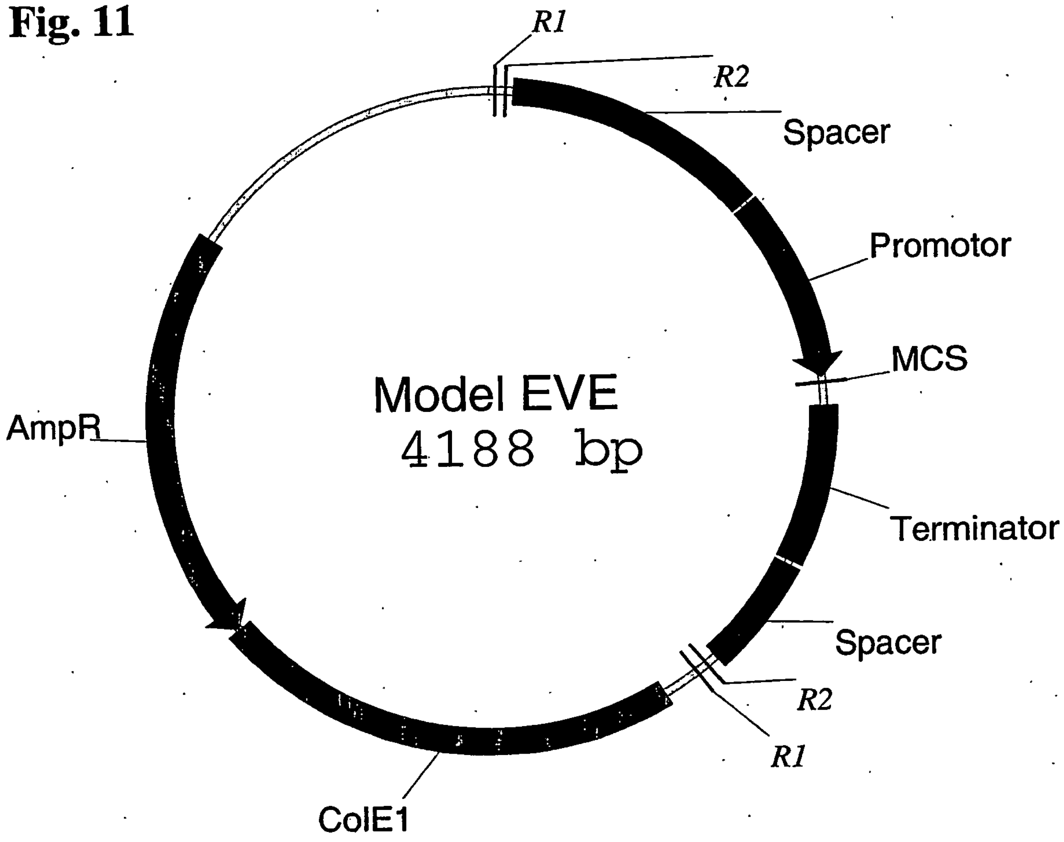

# EVE4 entry vector

**Fig. 12**

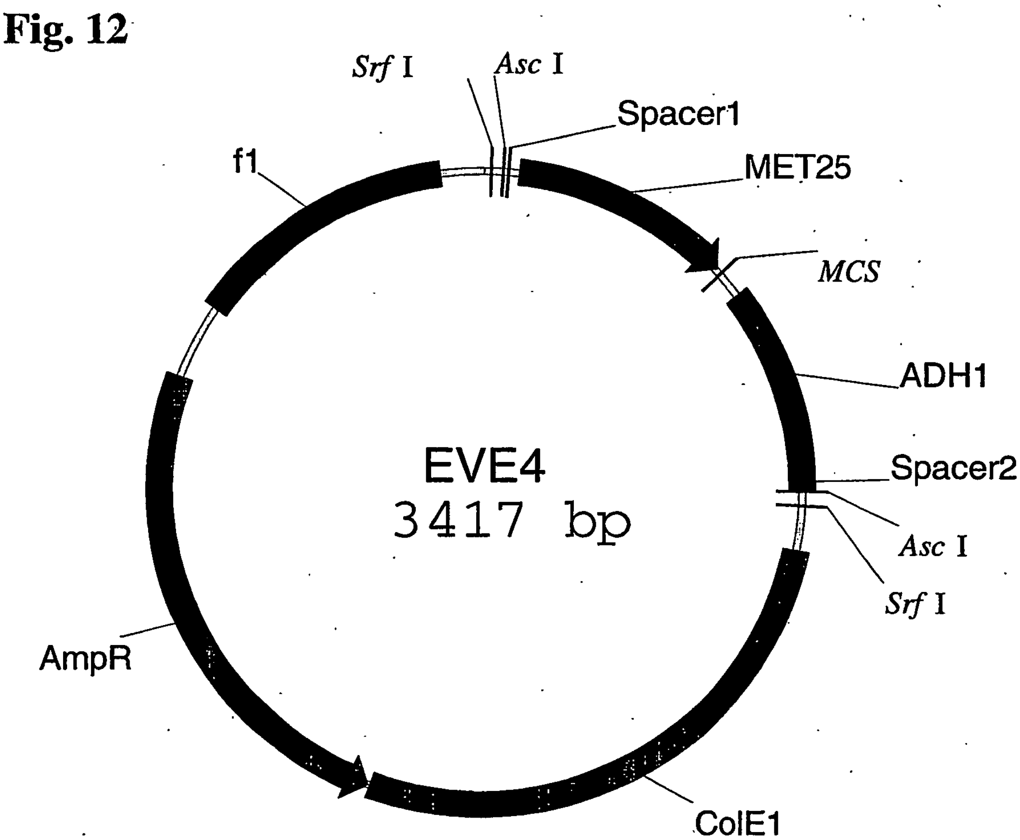

# EVE5 entry vector

**Fig. 13**

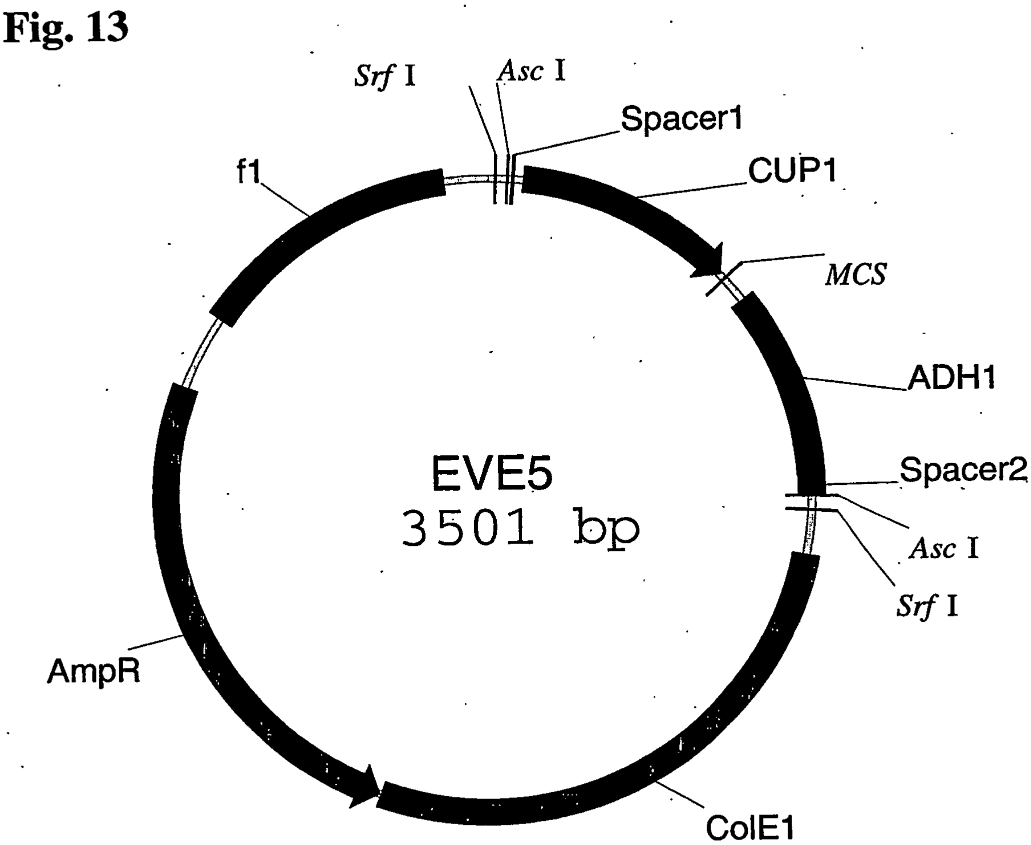

EVE8 entry vector

**Fig. 14**

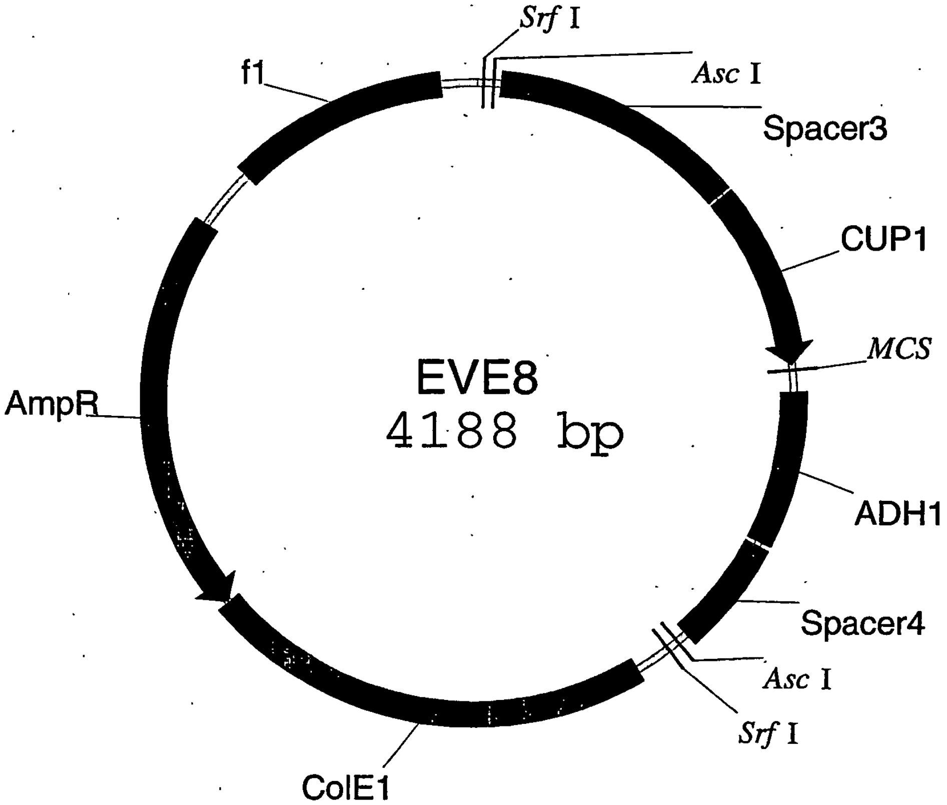

# EVE9 entry vector

**Fig. 15**

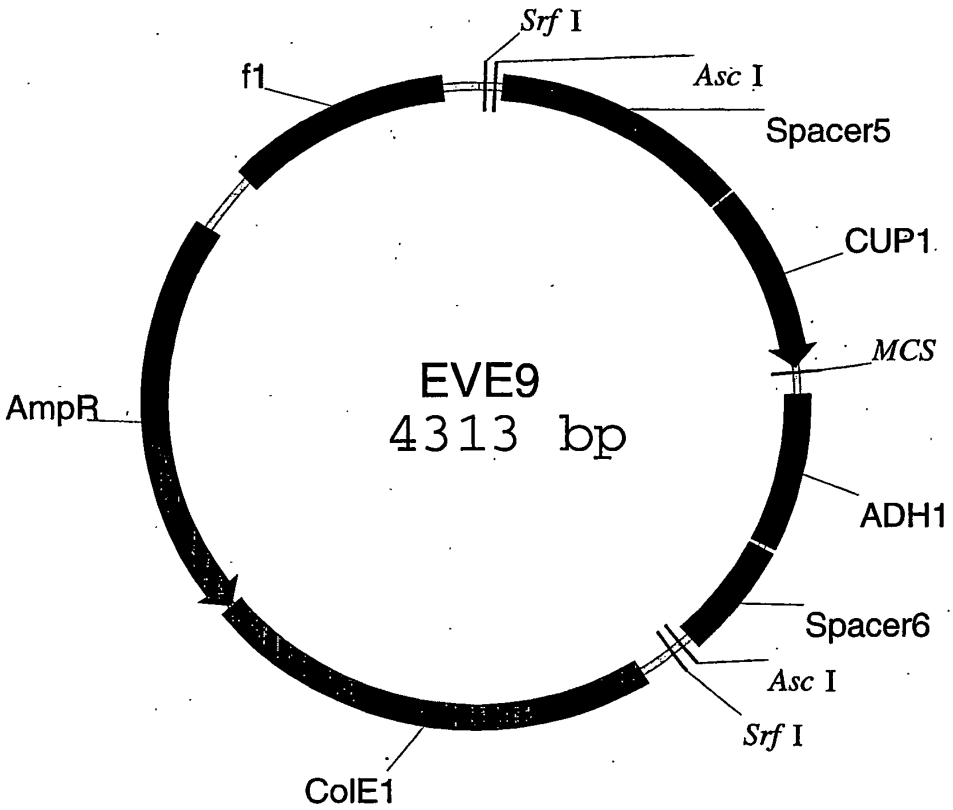

# pYAC4-AscI

Vector for providing EVACS arms

**Fig. 16**

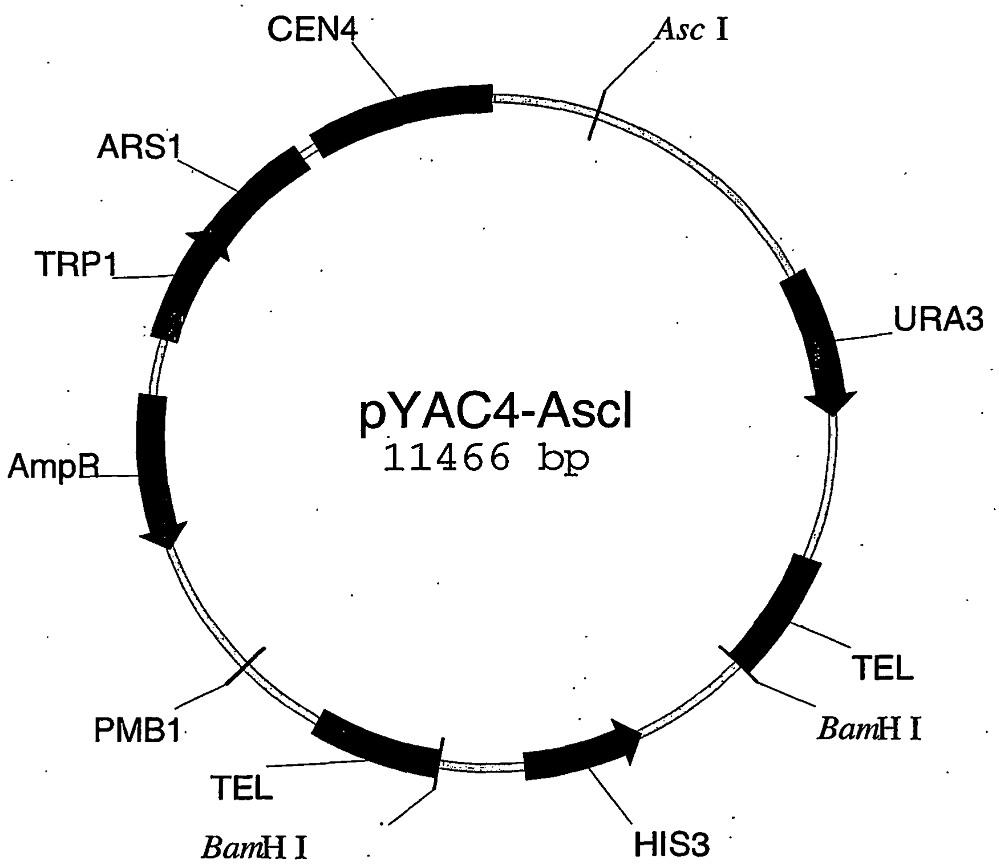

## Synthesis of Concatemers

Fig. 17

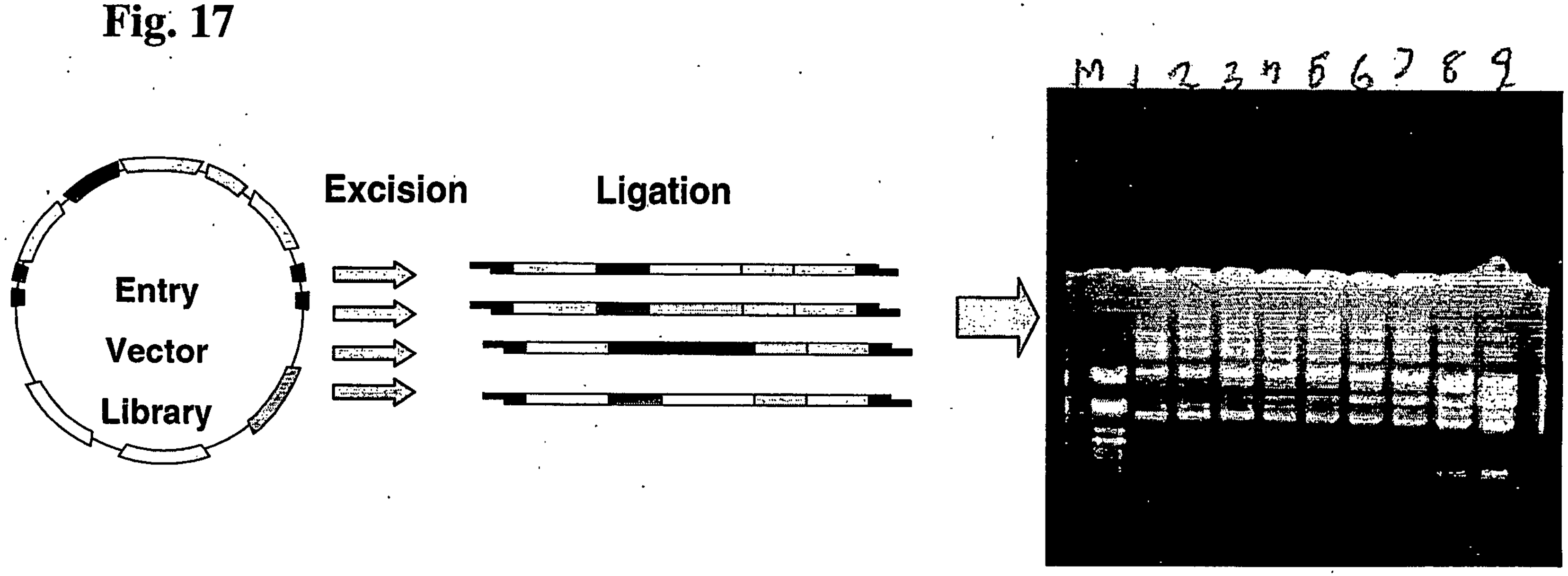

Fig. 18a

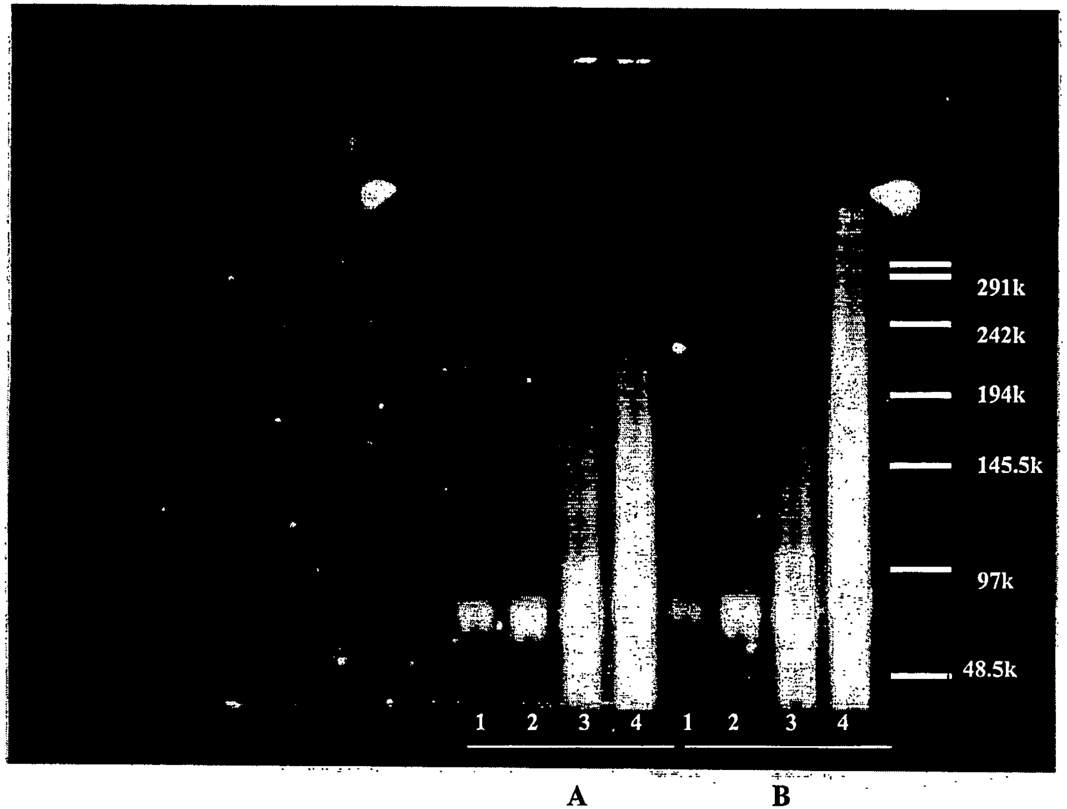


A B

A: F/Y = 100, B: F/Y= 1000

1: fragment conc.= 1
2: fragment conc.= 2
3: fragment conc.= 5
4: fragment conc.= 10

3+4: same amount loaded on gel
But concentration in 4 = 2x concentration in 3

Fig. 18b

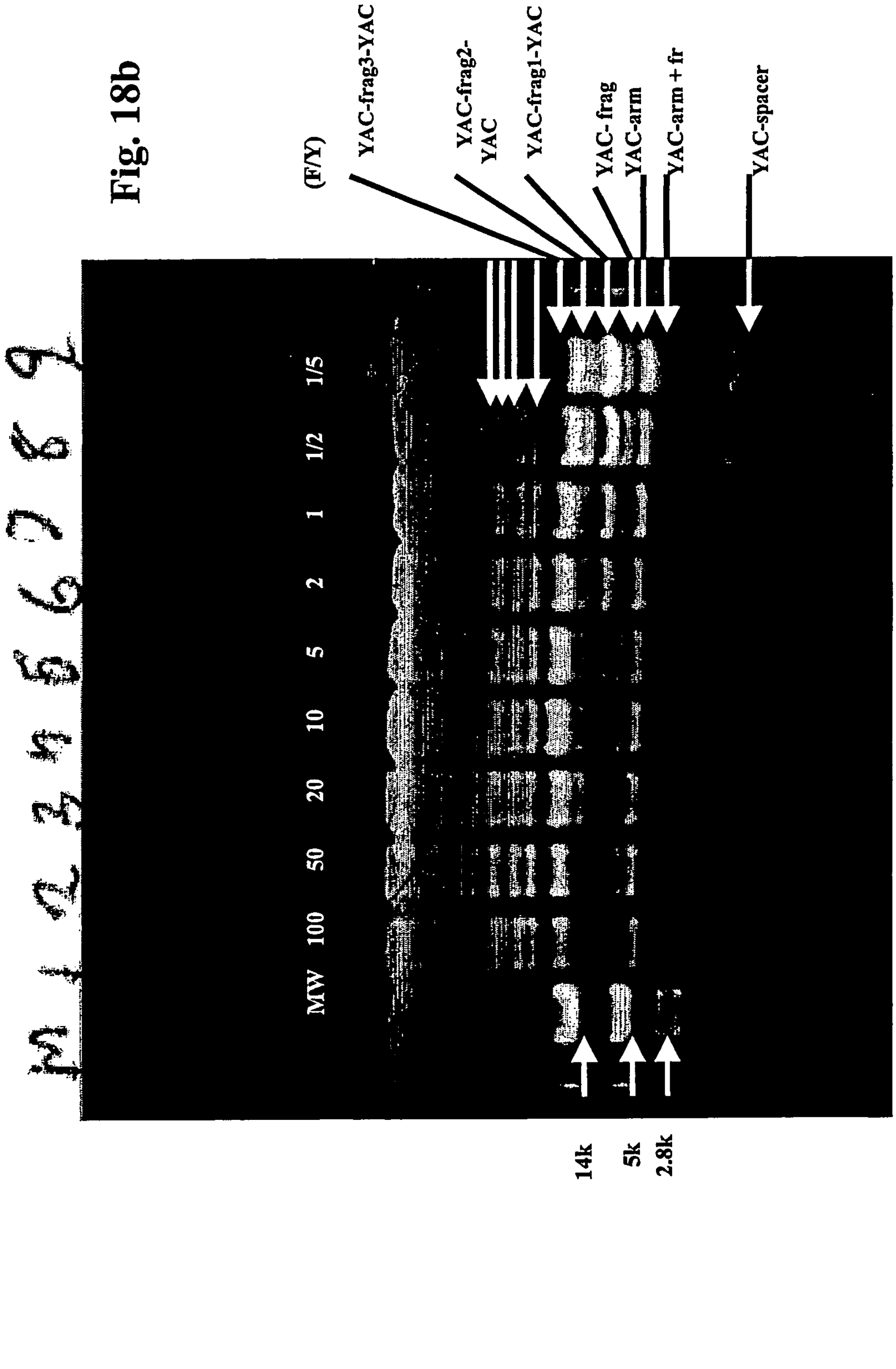
(F/Y)
YAC-frag3-YAC
YAC-frag2-YAC
YAC-frag1-YAC
YAC-frag
YAC-arm
YAC-arm + fr
YAC-spacer
MW 100 50 20 10 5 2 1 1/2 1/5
14k
5k
2.8k

Fig. 19

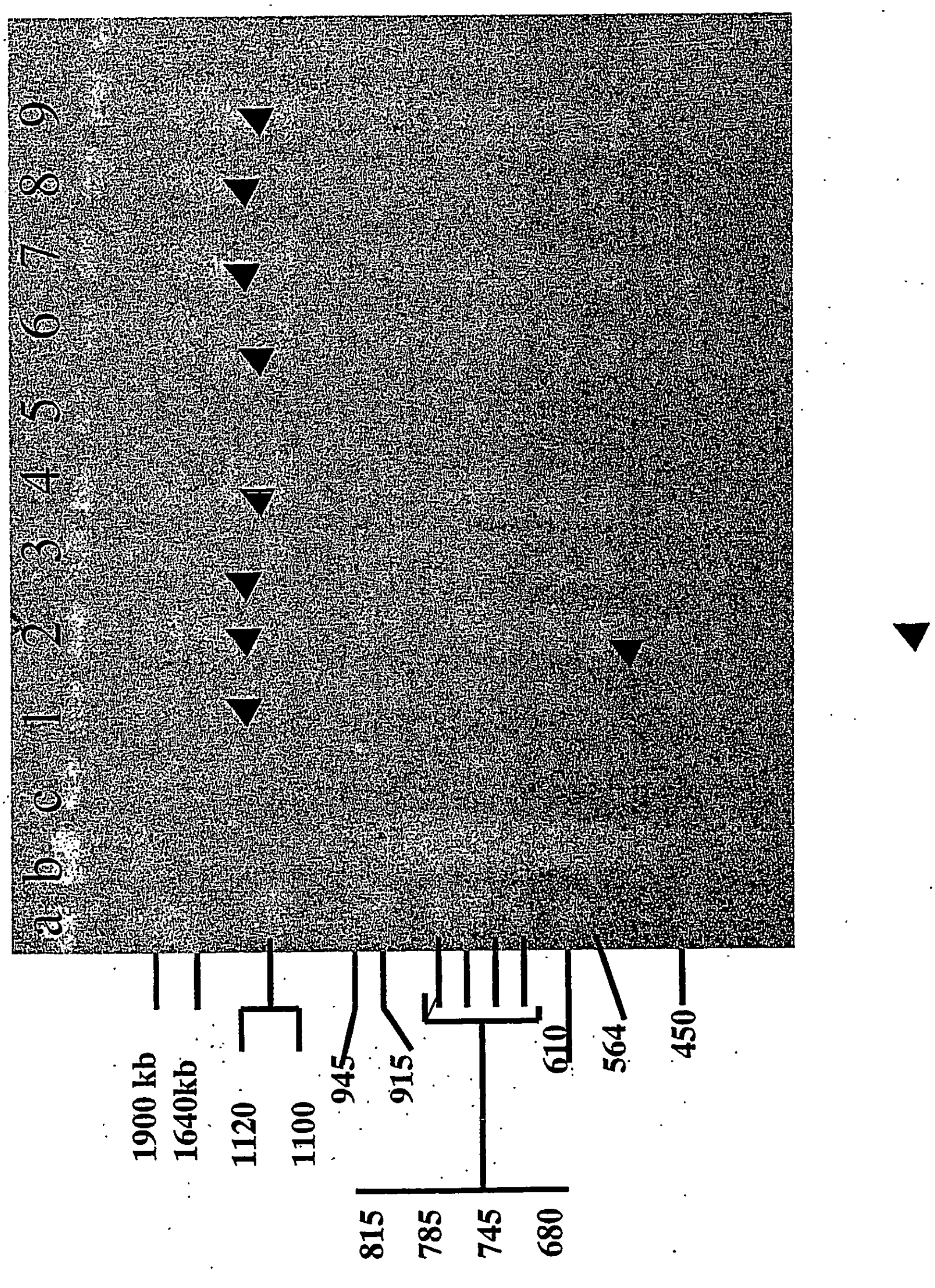
a b c 1 2 3 4 5 6 7 8 9
1900 kb
1640kb
1120
1100
945
915
815
785
745
680
610
564
450

# Generation of EVAC containing cell populations

Fig. 20

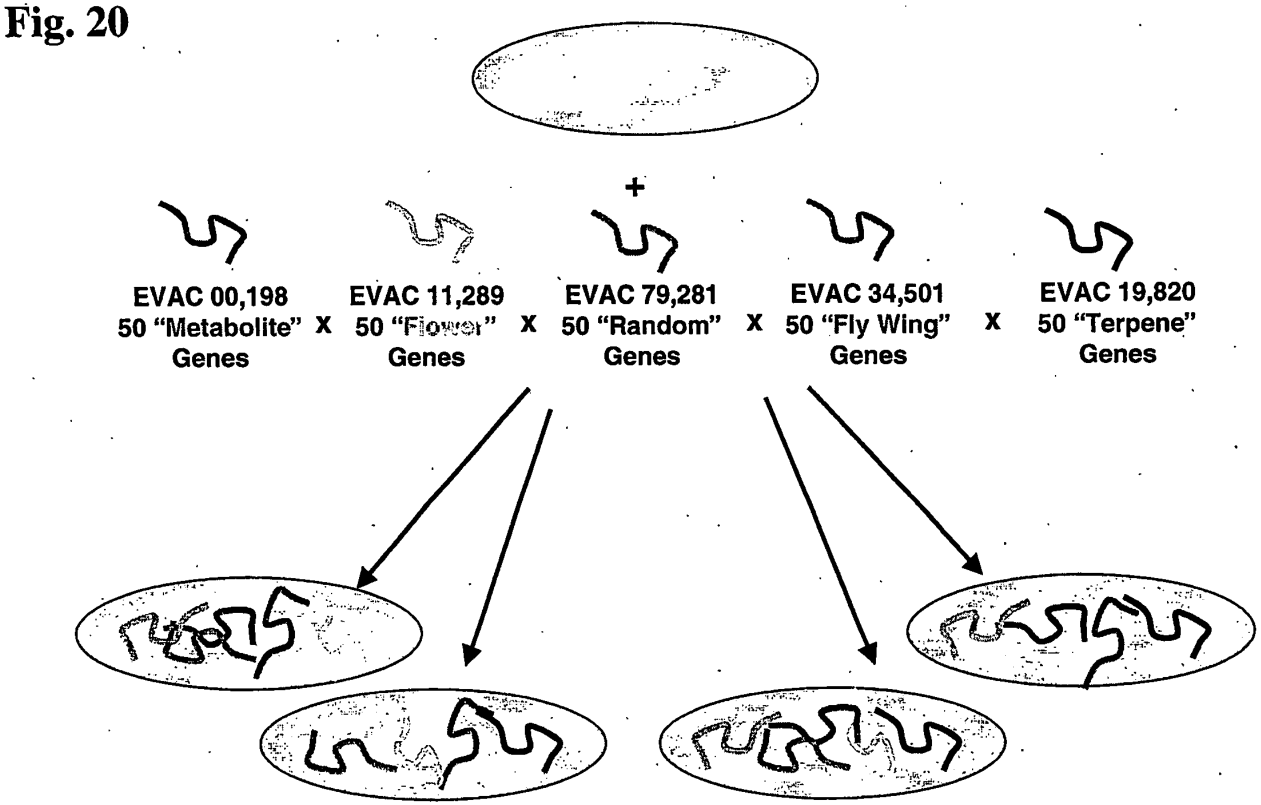

Fig. 21

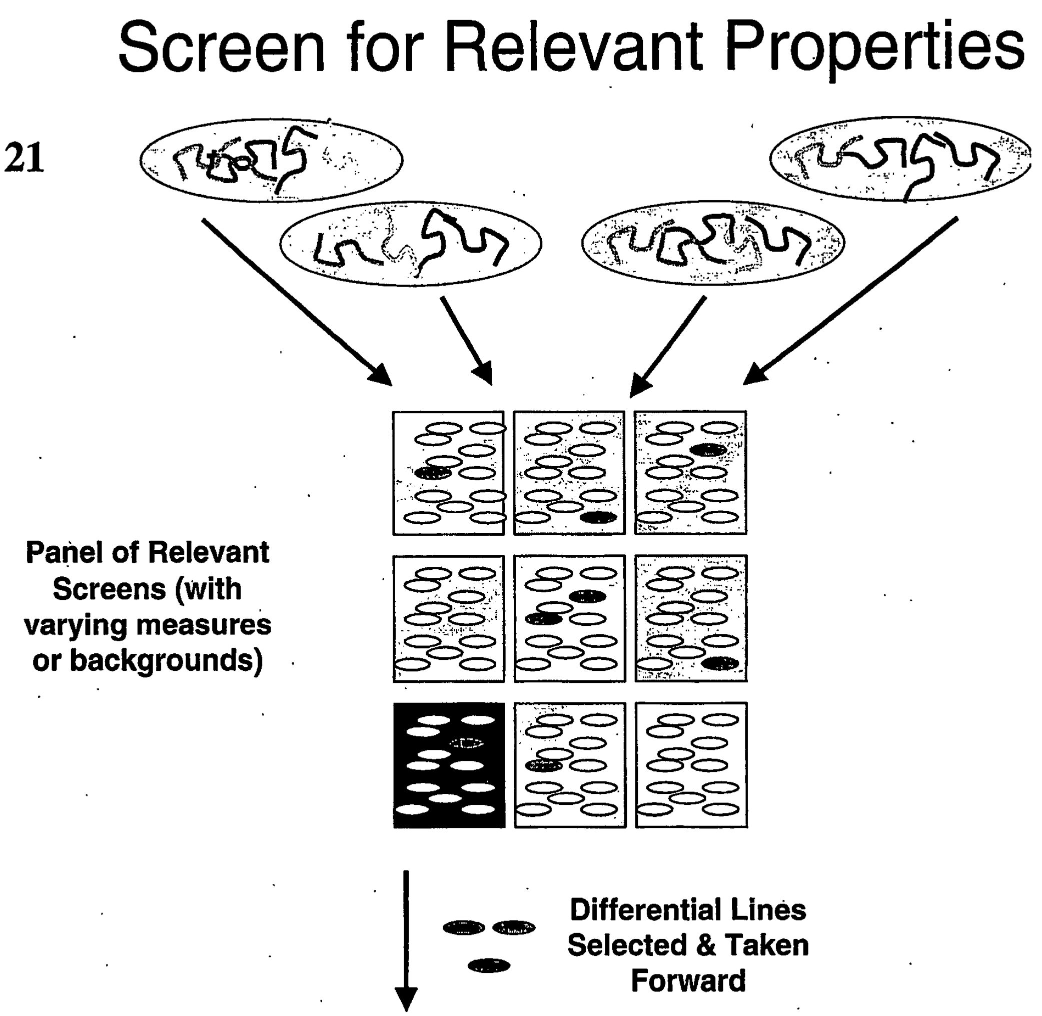
Screen for Relevant Properties
Panel of Relevant Screens (with varying measures or backgrounds)
Differential Lines Selected & Taken Forward

# Evolve Through Tiered Screens

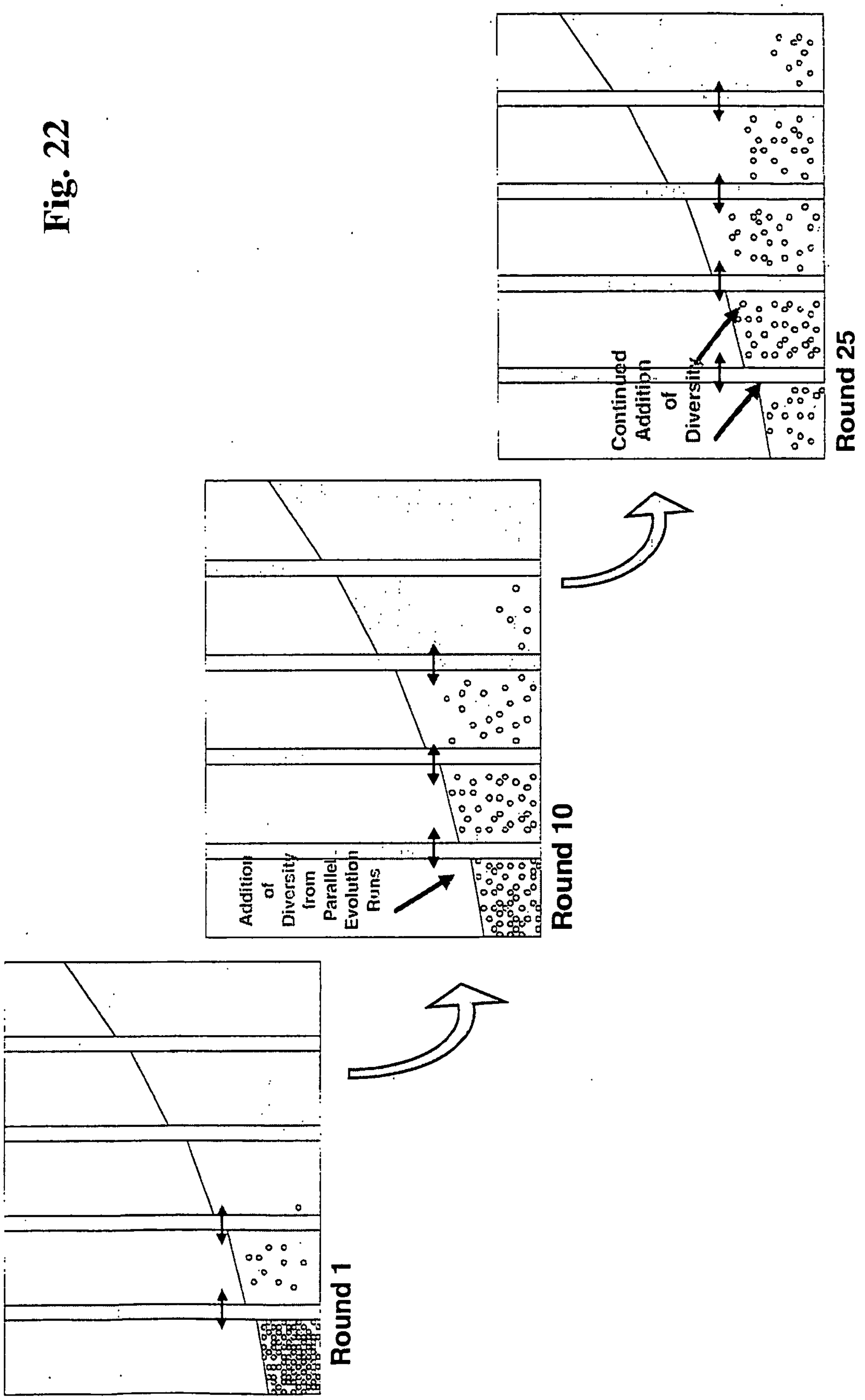


Fig. 22

Fig. 23

General Screening Strategy

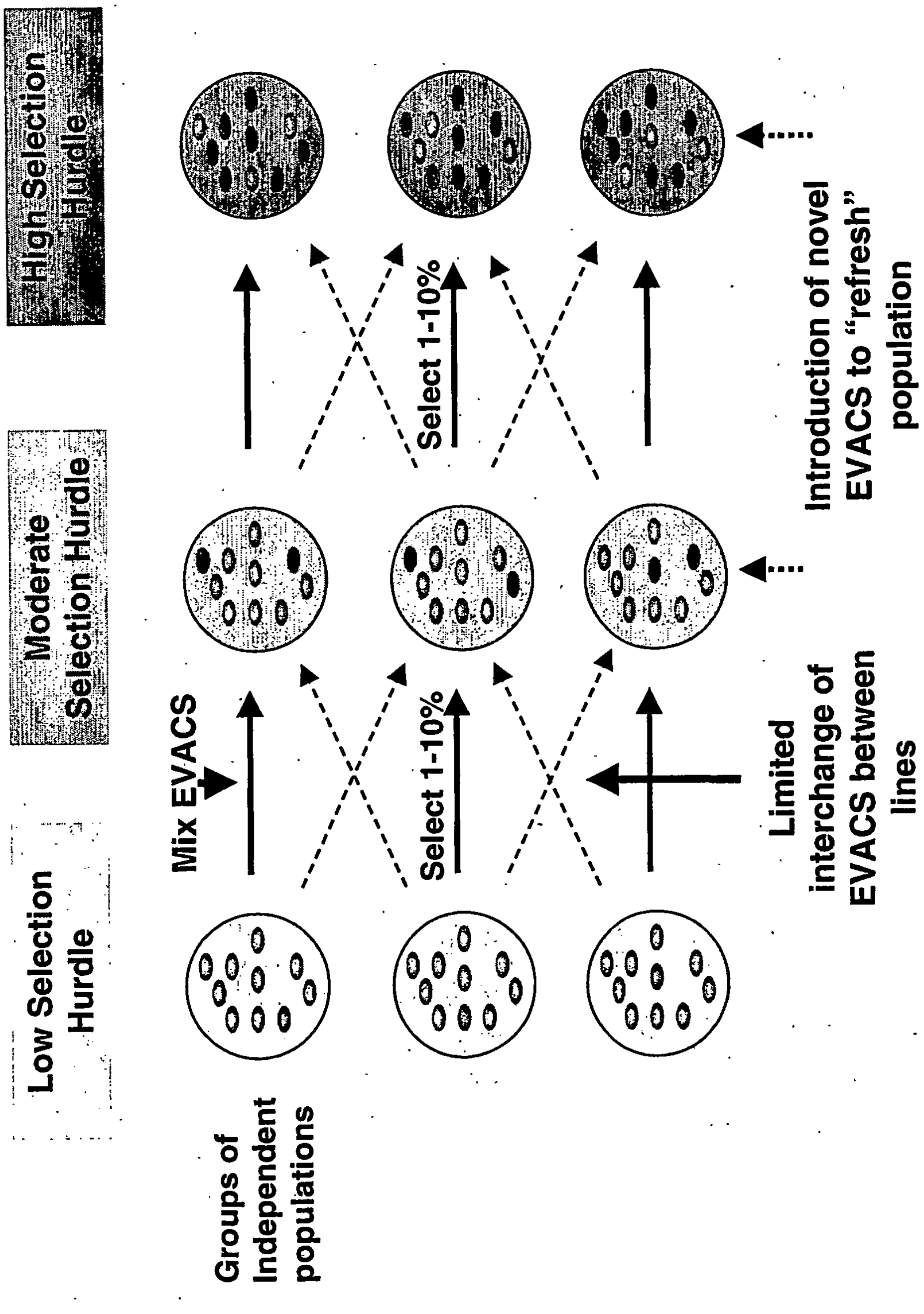

Fig. 24

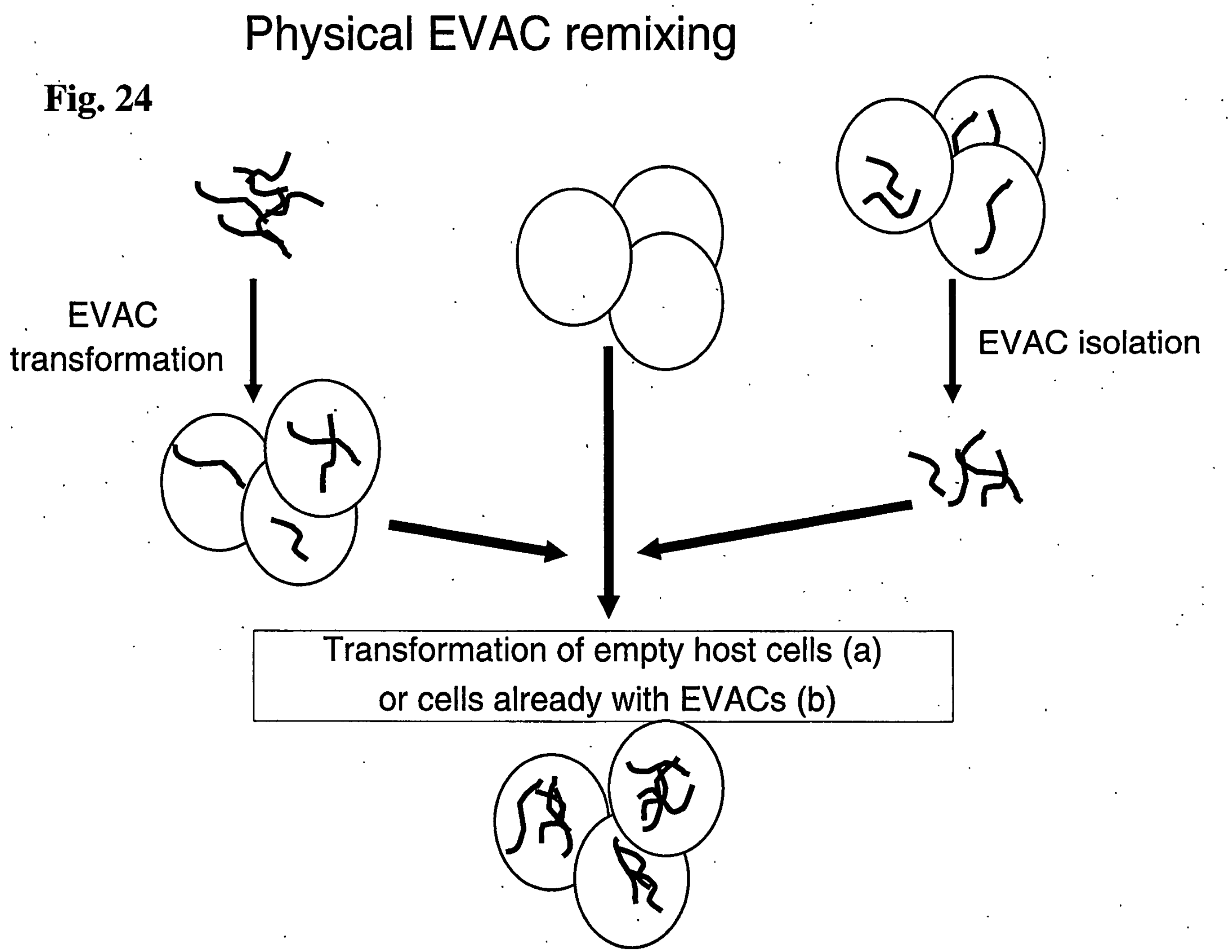
Physical EVAC remixing
EVAC transformation
EVAC isolation
Transformation of empty host cells (a)
or cells already with EVACs (b)

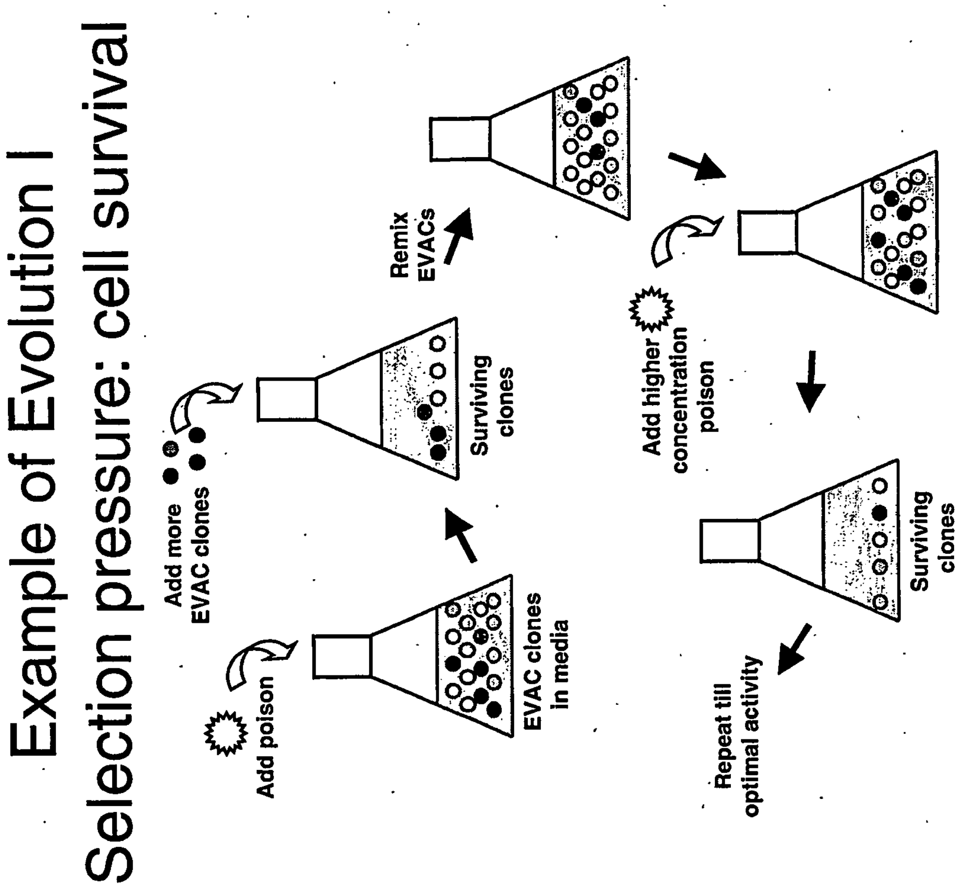
Example of Evolution I
Selection pressure: cell survival
Add poison
EVAC clones in media
Add more EVAC clones
Surviving clones
Remix EVACs
Add higher concentration poison
Surviving clones
Repeat till optimal activity

Fig. 25

# Example of Evolution II
## Selection pressure: activation of a reporter system

Fig. 26

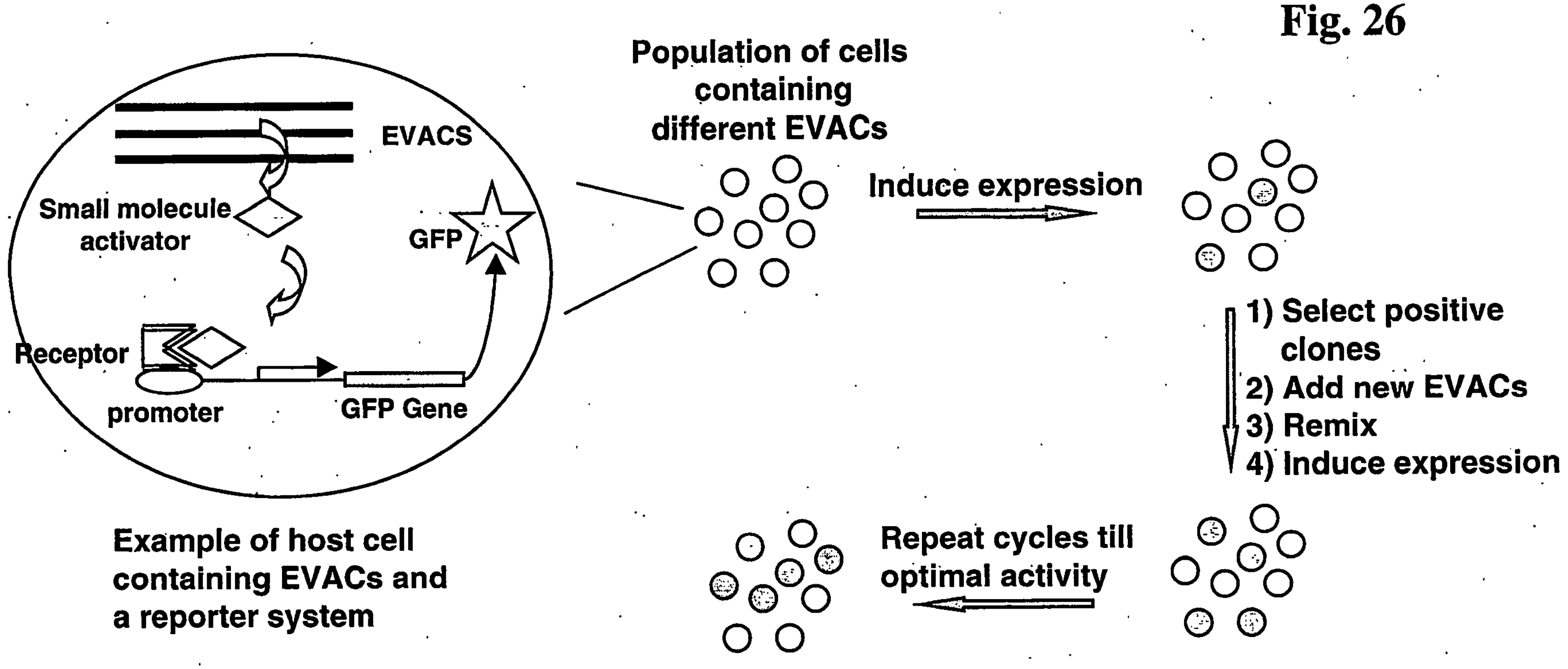

Fig. 27

## Example of controllable gene expression

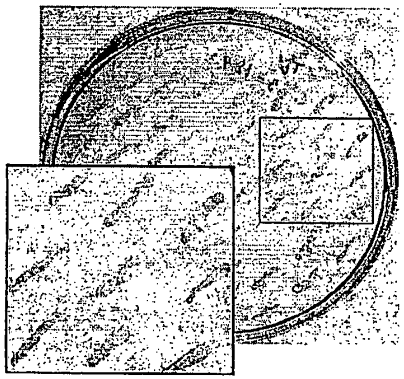

Plate 1: Promoters not induced/repressed

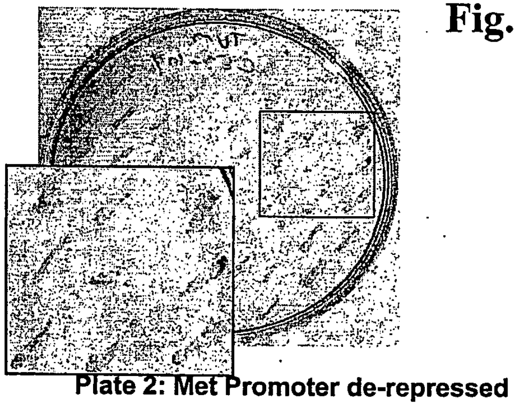

Plate 2: Met Promoter de-repressed

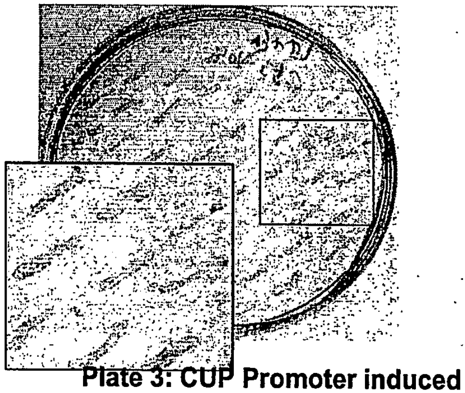

Plate 3: CUP Promoter induced

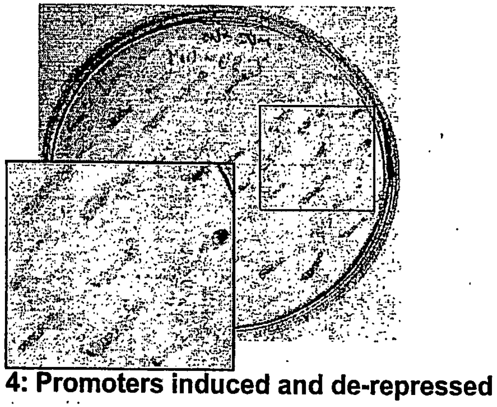

Plate 4: Promoters induced and de-repressed

Fig. 28

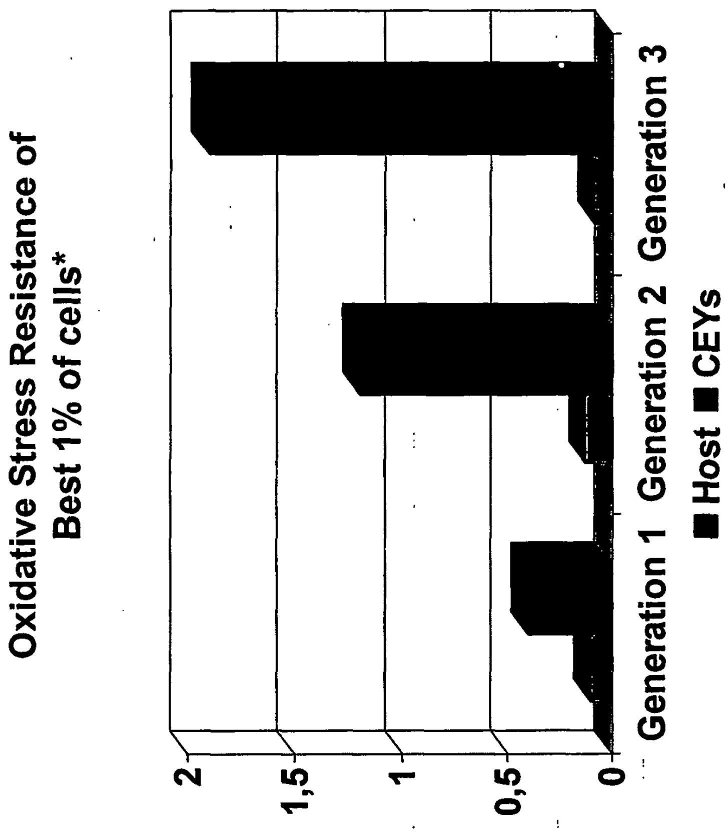


* Methylene Blue Concentration (mM)
where 1% cell population survived
after 2 hours irradiation

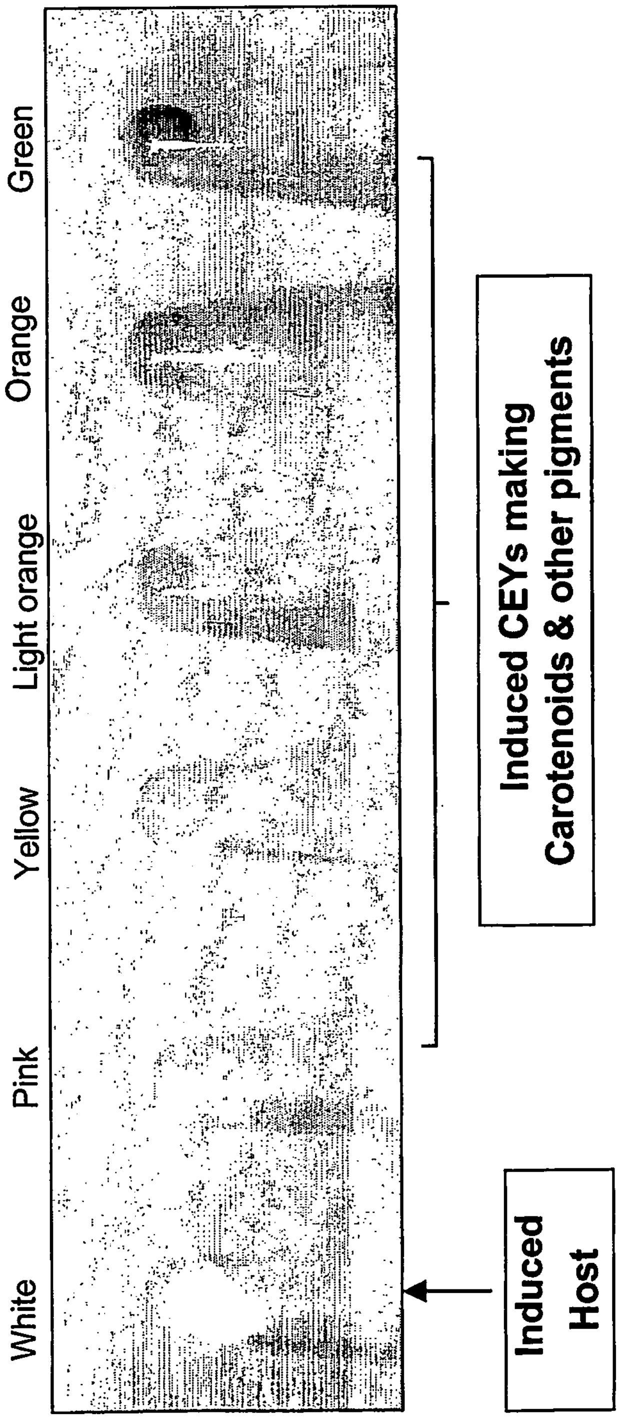
White
Pink
Yellow
Light orange
Orange
Green
Induced Host
Induced CEYs making Carotenoids & other pigments

Fig. 29

Fig. 30

Carotenoid content (µg carotenoid/g dry weigth)

| | Ceya1 | Cey2 | Cey3 | Cey4 | Cey5 | Cey8(b) | Cey9(a) | Cey9(b) | Cey11(a2) | Cey11b |
|---|---|---|---|---|---|---|---|---|---|---|
| absolute | 65,33 | 33,6 | 11 | 91,3 | 11,1 | 50,5 | 32,50 | 84 | 150 | 134,7 |
| astaxanthin | | | | | | | | 11,72 | | 6,85 |
| canthaxanthin | 3,16 | 1,95 | | 7,4 | | | 11,05 | 20,96 | 3,3 | 34,88 |
| echinenone | 18,08 | 6,54 | | 4,55 | 2,45 | | 11,99 | 6,09 | 36,55 | 18,46 |
| lycopene | | | | 9,5 | 1,2 | | | 17,81 | | 11,5 |
| neurosporene | 1,8 | 3,21 | 0,34 | 6,15 | | 15,57 | 3,26 | | 13,11 | |
| betacarotene | 42,29 | 3,69 | 9,8 | 44,17 | 6,35 | | 3,27 | | 84,1 | 14,95 |
| 427nm-carotenoid, not identified | | | | | | | | | | |
| 405nm-keto carotenoid, oxidation product | | | 0,11 | | | | | | | |
| 440nm-ketocarotenoid, untypically | | | 0,41 | | | | | | | |
| didehydrolycopene | | | | 6,41 | | | | | | |
| beta-cryptoxanthin | | | | | | | | 5,34 | | |
| 460nm-carotenoid | | | | 12,76 | | | | 22,05 | | 14,84 |
| beta zeaxanthin | | | | | | | | | 10,5 | |
| zeaxanthin | | | | | | 17,69 | | | | |
| keto carotenoid, not identified | | | | | | 17,26 | | | | |
| 465nm keto carotenoid, not identified | | | | | | | | | | 17,38 |
| 460nm keto carotenoid, oxidation product | | | | | | | | | | 5,34 |
| 445nm keto carotenoid, not identified | | | | | | | | | | 10,62 |
| phytoene | | | 4,1 | | 1,3 | | 4 | | | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9831837 A
- WO 9735966 A
- WO 0004190 A
- WO 0052180 A
- WO 9817811 A
- WO 017300 A
- WO 9634112 A
- US 6368558 B
- US 5653996 A
- US 5393530 A
- US 5651981 A
- US 5595756 A
- US 5605703 A
- US 5627159 A
- US 5652225 A
- US 5567433 A
- US 4235871 A
- US 5227170 A
- US 5798227 A
- US 5667986 A
- US 5559027 A
- US 5877018 A
- US 6072050 A
- US 564166 A
- WO 9744470 A
- US 5952195 A
- WO 9854339 A
- US 4870013 A
- EP 0329203 A1
- US 5436136 A, Hinnen
- US 5089398 A, Rosenberg
- US 4945046 A, Horii
- US 5035996 A
- US 5763239 A
- WO 9508647 A
- WO 9511986 A
- US 4464472 A
- US 6133503 A
- US 6077697 A
- US 5270201 A
- US 5977439 A
- US 5888732 A


### Non-patent literature cited in the description


- **Smith et al.** *PNAS,* 1990, vol. 87, 8242-48
- **Hugerat Y ; Spencer F ; Zenwirth D ; Simchen G.** *Genomics,* 1994, vol. 22 (1), 108-117
- **Curran BP ; Bugeja VC.** *Methods Mol. Biol.,* 1996, vol. 53, 45-49
- *Cancer J.,* 1998, vol. 4, S92
- *Int. J. Biochem. Cell. Biol.,* 1997, vol. 29 (6), 867
- *Faseb,* 1998, vol. 12, 1063
- *J. Nat. Cancer Inst.,* 1998, vol. 90 (11), 802
- *Inflamm. Res.,* 1997, vol. 46, 282
- **Malonne, H. ; Atassi, G.** *Anti-Cancer Drugs,* 1997, vol. 8, 811-822
- **Sehested et al.** *Cancer Research,* 1998, vol. 58, 1460-1468
- **Inui K-I ; Yamamoto M ; Saito H. T.** *J Pharmacol Exp Ther,* 1992, vol. 261, 195-201
- **Lu S ; Guttendorf RJ ; Stewart BH.** *Pharm Res,* 1994, vol. 11, S-258
- **Bjorge S ; Halelehle KL ; Homan R ; Rose SE ; Turluck DA ; Wright DS.** *Pharm Res,* 1991, vol. 8, 1441-1443
- **Ohgiya S ; Komori M ; Fujitani T ; Miura T ; Shinriki N ; Kamataki T.** *Biochem Int,* 1989, vol. 18, 429-438
- **Winters DK ; Cederbaum AI.** *Biochim Biophys Acta,* 1992, vol. 1156, 43-49
- **Crespi CL ; Gonzalez FJ ; Steimel DT ; Turner TR ; Gelboin HV ; Penman BW ; Langenbach R.** *Chem Res Toxicol,* 1991, vol. 4, 566-572
- **Nelson, D.R.** *Arch. Biochem. Biophys.,* 1999, vol. 369, 1-10
- **Guengerich, F.P.** Cytochrome P450: Structure, Mechanism, and Biochemistry. Plenum Press, 1995, 450
- **Shimada, T. et al.** *J. Pharmacol. Exp. Ther.,* 1994, vol. 270, 414-23
- **Ortiz de Montellano, P.R.** Cytochrome P450: Structure, Mechanism, and Biochemistry. Plenum Press, 1995, 450
- **Yamazaki, H. et al.** *J. Biol. Chem.,* 1996, vol. 271, 27438-44
- **Rettie, A.E. ; Fisher, M.B.** Handbook of Drug Metabolism. Marcel Dekker, Inc, 1999, 131-147
- **Ziegler, D.M.** *Annu. Rev. Pharmacol. Toxicol.,* 1993, vol. 33, 179

- **Coffman, B. et al.** *Drug Metab. Dispos.,* 1996, vol. 25, 1-4
- **Coffman, B. et al.** *Drug Metab. Dispos.,* 1997, vol. 25, 1032-8
- **Radominska-Pandya, A. et al.** *Drug Metab. Rev.,* 1999, vol. 31, 817-99.11
- **Radominska- Pandya, A. et al.** *Drug Metab. Rev.,* 1999, vol. 31, 817-99
- **Weber, W.** Pharmacogenetics. Oxford University Press, 1997
- **McCall, J. et al.** *J. Med. Chem.,* 1983, vol. 26, 1791-3
- **Miller, J.A.** *Chem. Bio. Interact.,* 1994, vol. 92, 329-41
- **Weinshilboum, R.M. et al.** *FASEB J.,* 1997, vol. 11, 3-14
- **Raftogianis, R.B. et al.** *BBRC,* 1997, vol. 239, 298-304
- **Wittrup et al.** *Biotechnolo. Bioeng.,* 1993, vol. 42, 351-356
- *Journal of Applied Biochemistry,* 1982, vol. 4, 418-435
- **Ihler, G. M.** *J. Pharm. Ther,* 1983
- **Blattner et al.** *Science,* 1997, vol. 277, 1454-1462
- **Kunst et al.** *Nature,* 1997, vol. 390, 249-256
- **Kriegler M.** Gene Transfer and Expression: A Laboratory Manual. Freeman & Co, 1990
- **Chen ; Struhl.** *EMBO J.,* 1985, vol. 4, 3273-3280
- **Sambrook ; Fritsch ; Maniatis.** A laboratory Manual. Cold Spring Habor Laboratory Press, 1989
- **Armitage, B.** *Chem. Rev.,* 1998, vol. 98, 1171-1200
- **Dervan P.B. ; Bürli R.W.** *Curr. Opin. Chem. Biol.,* 1999, vol. 3, 688-93
- **Nielsen, P.E.** *Curr. Opin. Biotechnol.,* 2001, vol. 12, 16-20
- Chemical Reviews special thematic issue: RNA/DNA Cleavage. ACS publications, 1998, vol. 98
- **Chang ; LMS ; Bollum TJ.** *J Biol Chem,* 1971, vol. 246, 909
- **Spingola M ; Grate L ; Haussler D ; Ares M Jr.** Genome-wide bioinformatic and molecular analysis of introns in Saccharomyces cerevisiae. *RNA,* February 1999, vol. 5 (2), 221-34
- **Davis CA ; Grate L ; Spingola M ; Ares M Jr.** Test of intron predictions reveals novel splice sites, alternatively spliced mRNAs and new introns in meiotically regulated genes of yeast. *Nucleic Acids Res,* 15 April 2000, vol. 28 (8), 1700-6
- **Maniatis T.** Molecular Cloning: A laboratory manual. Cold Spring Harbour Laboratory Press, 1989
- **J. Sambrook ; E.F. Fritsch ; T. Maniatis.** Molecular Cloning, A Laboratory Manual. Cold Spring Harbor Laboratory Press, 1989
- **Bonaldo ; Lennon ; Scares.** *Genome Research,* 1996, vol. 6, 791-806
- **Ali ; Holloway ; Taylor.** *Plant Mol Biol Reporter,* 2000, vol. 18, 123-132
- **Sive ; John.** *Nucleic Acid Res,* 1988, vol. 16, 10937
- **Diatchenko ; Lau ; Campbell et al.** *PNAS,* 1996, vol. 93, 6025-6030
- **Carninci ; Shibata ; Hayatsu.** *Genome Res,* 2000, vol. 10, 1617-30
- **Bonaldo ; Lennon ; Soares.** *Genome Research,* 1996, vol. 6, 791-806
- **Murray et al.** *Nature,* vol. 305, 189-193
- **Sauer B.** *Methods Enzymol,* 1993, vol. 225, 890-900
- **Landy A.** *Ann Rev Biochem,* 1989, vol. 58, 913
- Pulsed Field Gel Electrophoresis. A practical approach. Oxford University Press, 1995
- **Smith, D. R. et al.** *Proc. Natl. Acad. USA,* 1990, vol. 87, 8242-8246
- chapter 5 of Flow cytometry : a practical approach. Oxford university press, 2000
- **Bonaldo, MF et al.** *Genome Res.,* 1996, vol. 6, 791-806
- **Burke et al.** *science,* 1987, vol. 236, 806